# EUROPÄISCHE PATENTSCHRIFT

(11) **EP 0 876 332 B1**
(45) Veröffentlichungstag und Bekanntmachung des Hinweises auf die Patenterteilung: **27.06.2001**
(21) Anmeldenummer: 96934659.2
(22) Anmeldetag: 11.10.1996
(51) Int. Cl.: C07C 251/60, A01N 37/36

(54) **PHENYLESSIGSÄUREDERIVATE, VERFAHREN UND ZWISCHENPRODUKTE ZU IHRER HERSTELLUNG UND SIE ENTHALTENDE MITTEL**
PHENYLACETIC ACID DERIVATIVES, PROCESS AND INTERMEDIATE PRODUCTS FOR USE IN PRODUCING THEM AND AGENTS CONTAINING THEM
DERIVES D'ACIDE PHENYLACETIQUE, PROCEDE ET INTERMEDIAIRES UTILISES POUR LEUR PRODUCTION ET AGENTS LES CONTENANT

(30) Priorität: 23.10.1995 DE 19539324
(43) Veröffentlichungstag der Anmeldung: 11.11.1998
(73) Patentinhaber: BASF AKTIENGESELLSCHAFT, 67056 Ludwigshafen (DE)
(72) Erfinder: GROTE, Thomas, D-67105 Schifferstadt (DE); BAYER, Herbert, D-68159 Mannheim (US); MÜLLER, Ruth, D-67159 Friedelsheim (DE); SAUTER, Hubert, D-68167 Mannheim (DE); KIRSTGEN, Reinhard, D-67434 Neustadt (DE); HARRIES, Volker, D-67227 Frankenthal (DE); LORENZ, Gisela, D-67434 Hambach (DE); AMMERMANN, Eberhard, D-64646 Heppenheim (DE); STRATHMANN, Siegfried, D-67117 Limburgerhof (DE)
(86) Internationale Anmeldenummer: EP9604446
(87) Internationale Veröffentlichungsnummer: WO9715552

(56) Entgegenhaltungen:
- WO-A-90/07493
- WO-A-95/18789
- WO-A-95/21153
- WO-A-95/21154
- J.Chem.Soc., Perkin Trans. II, 1987, 523-29
- Chem. Abstracts vol.71, 1969, 38176r

## Beschreibung

Die vorliegende Erfindung betrifft Phenylessigsäurederivate der Formel I 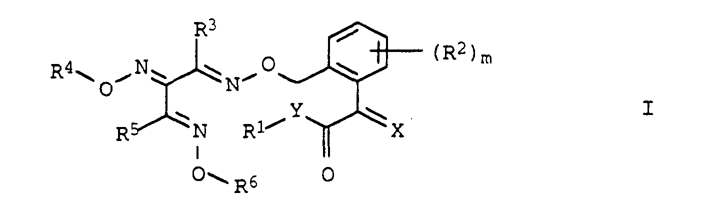 in der die Substituenten und der Index die folgende Bedeutung haben:
- X: NOCH₃, CHOCH₃, CHCH₃;
- Y: O, NR
- R¹,R: unabhängig voneinander Wasserstoff und C₁-C₄-Alkyl;
- R²: Cyano, Nitro, Trifluormethyl, Halogen, C₁-C₄-Alkyl und C₁-C₄-Alkoxy;
- m: 0, 1 oder 2, wobei die Reste R² verschieden sein können, wenn m für 2 steht;
- R³: Wasserstoff, Cyano, C₁-C₄-Alkyl, C₁-C₄-Halogenalkyl, C₃-C₆-Cycloalkyl;
- R⁴,R⁶: unabhängig voneinander Wasserstoff,
C₁-C₁₀-Alkyl, C₃-C₆-Cycloalkyl, C₂-C₁₀-Alkenyl, C₂-C₁₀-Alkinyl, C₁-C₁₀-Alkylcarbonyl, C₂-C₁₀-Alkenylcarbonyl, C₃-C₁₀-Alkinylcarbonyl oder C₁-C₁₀-Alkylsulfonyl, wobei diese Reste partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, C₃-C₆-Cycloalkyl, C₃-C₆-Cycloalkyloxy, Heterocyclyl, Heterocyclyloxy, Benzyl, Benzyloxy, Aryl, Aryloxy, Arylthio, Hetaryl, Hetaryloxy und Hetarylthio, wobei die cyclischen Gruppen ihrerseits partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkyloxycarbonyl, C₁-C₆-Alkylthio, C₁-C₆-Alkylamino, Di-C₁-C₆-Alkylamino, C₁-C₆-Alkylaminocarbonyl, Di-C₁-C₆-Alkylaminocarbonyl, C₁-C₆-Alkylaminothiocarbonyl, Di-C₁-C₆-Alkylaminothiocarbonyl, C₂-C₆-Alkenyl, C₂-C₆-Alkenyloxy, Benzyl, Benzyloxy, Aryl, Aryloxy, Arylthio, Hetaryl, Hetaryloxy, Hetarylthio oder C(=NOR⁷)-Aₙ-R⁸;
Aryl, Arylcarbonyl, Arylsulfonyl, Hetaryl, Hetarylcarbonyl oder Hetarylsulfonyl, wobei diese Reste partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkyloxycarbonyl, C₁-C₆-Alkylthio, C₁-C₆-Alkylamino, Di-C₁-C₆-Alkylamino, C₁-C₆-Alkylaminocarbonyl, Di-C₁-C₆-Alkylaminocarbonyl, C₁-C₆-Alkylaminothiocarbonyl, Di-C₁-C₆-Alkylaminothiocarbonyl, C₂-C₆-Alkenyl, C₂-C₆-Alkenyloxy, Benzyl, Benzyloxy, Aryl, Aryloxy, Hetaryl, Hetaryloxy oder C(=NOR⁷)-Aₙ-R⁸;
- R⁵: Wasserstoff,
C₁-C₆-Alkyl, C₂-C₆-Alkenyl, C₂-C₆-Alkinyl, wobei die Kohlenwasserstoffreste dieser Gruppen partiell oder vollständig halogeniert sein können oder einen bis drei der folgenden Reste tragen können: Cyano, Nitro, Hydroxy, Mercapto, Amino, Carboxyl, Aminocarbonyl, Aminothiocarbonyl, Halogen, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyloxy, C₃-C₆-Cycloalkyl, C₃-C₆-Cycloalkyloxy, Heterocyclyl, Heterocyclyloxy, Aryl, Aryloxy, Aryl-C₁-C₄-alkoxy, Arylthio, Aryl-C₁-C₄-alkylthio, Hetaryl, Hetaryloxy, Hetaryl-C₁-C₄-alkoxy, Hetarylthio, Hetaryl-C₁-C₄-alkylthio, wobei die cyclischen Reste ihrerseits partiell oder vollständig halogeniert sein können und/oder ein bis drei der folgenden Gruppen tragen können: Cyano, Nitro, Hydroxy, Mercapto, Amino, Carboxyl, Aminocarbonyl, Aminothiocarbonyl, C₁-C₆-Alkyl, C₁-C₆-Halogenalkyl, C₁-C₆-Alkylsulfonyl, C₁-C₆-Alkylsulfoxyl, C₃C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Arylthio, Hetaryl, Hetaryloxy, Hetarylthio und C (=NOR⁷)-Aₙ-R⁸;
C₃-C₆-Cycloalkyl, C₃-C₆-Cycloalkenyl, Heterocyclyl, Aryl, Hetaryl, wobei die cyclischen Reste partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Hetaryl und Hetaryloxy;
wobei
- A: für Sauerstoff, Schwefel oder Stickstoff steht und wobei der Stickstoff Wasserstoff oder C₁-C₆-Alkyl trägt;
- n: 0 oder 1 bedeutet;
- R⁷: Wasserstoff oder C₁-C₆-Alkyl bedeutet und
- R⁸: Wasserstoff oder C₁-C₆-Alkyl bedeutet,
sowie deren Salze.

Außerdem betrifft die Erfindung Verfahren und Zwischenprodukte zur Herstellung dieser Verbindungen sowie sie enthaltende Mittel zur Bekämpfung tierischer Schädlinge und Schadpilze.

Aus der Literatur sind Phenylessigsäurederivate zur Schädlingsbekämpfung bekannt (EP-A 422 597, EP-A 463 488, EP-A 370 629, EP-A 460 575, EP-A 472 300, WO-A 90/07,493, WO-A 92/13,830, WO-A 92/18,487, WO-A 95/18,789, WO-A 95/21,153, WO-A 95/21,154).

Der vorliegenden Erfindung lagen neue Verbindungen mit verbesserter Wirkung als Aufgabe zugrunde.

Demgemäß wurden die eingangs definierten Phenylessigsäurederivate I gefunden. Außerdem wurden Verfahren und Zwischenprodukte zu ihrer Herstellung sowie sie enthaltende Mittel zur Bekämpfung von tierischen Schädlingen und Schadpilzen und ihre Verwendung in diesem Sinne gefunden.

Die Verbindungen I sind auf verschiedenen Wegen nach an sich in der Literatur bekannten Verfahren erhältlich.

Grundsätzlich ist es bei der Synthese der Verbindungen I unerheblich, ob zunächst die Gruppierung -C(X)-COYR¹ oder die Gruppierung 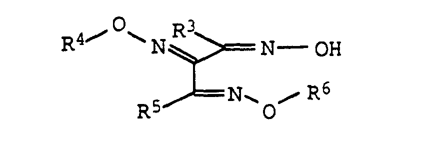 aufgebaut wird.

Der Aufbau der Gruppierung -C(X)-CO₂R¹ ist beispielsweise aus der eingangs zitierten Literatur bekannt.

1. Für die Synthese der Verbindungen der Formel I geht man im allgemeinen so vor, daß man ein Benzylderivat der Formel II mit einem Hydroxyimin der Formel III umsetzt. 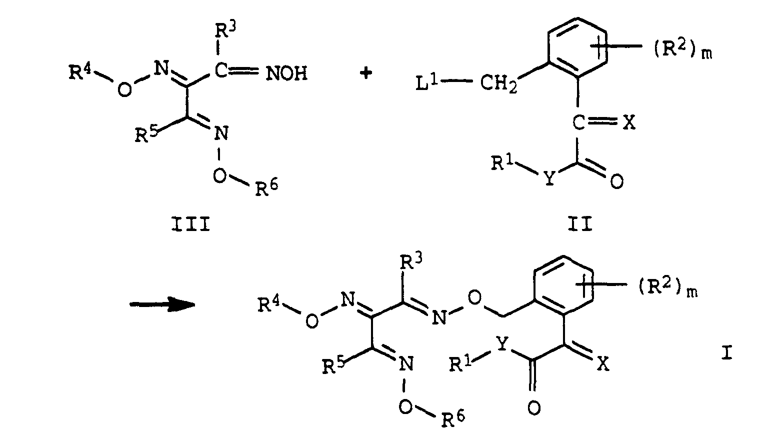 L¹ in der Formel II steht für eine nukleophil austauschbare Abgangsgruppe, z.B. Halogen oder Sulfonatgruppen, vorzugsweise Chlor, Brom, Iod, Mesylat, Tosylat oder Triflat.

Die Umsetzung erfolgt in an sich bekannter Weise in einem inerten organischen Lösungsmittel in Gegenwart einer Base, z.B. Natriumhydrid, Kaliumhydroxid, Kaliumcarbonat und Triethylamin gemäß den in Houben-Weyl, Bd. E 14b, S. 370f und Houben-Weyl, Bd. 10/1, S. 1189f beschriebenen Methoden.

Die benötigten Hydroxyimine III erhält man beispielsweise wie in Schema 1 beschrieben durch eine Folge von Nitrosierungs-, Alkylierungs- und Oximierungsreaktionen aus entsprechenden Carbonylverbindungen XIV.

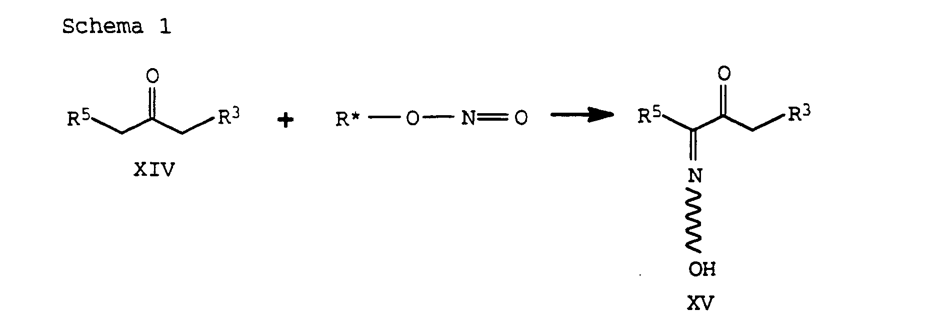

Durch basische oder saure Katalyse kann aus dem Keton XIV mit einem organischen Nitrit gemäß den in Houben-Weyl Bd. 10/4 S. 17f beschriebenen Methoden das a-Ketooxim XV hergestellt werden

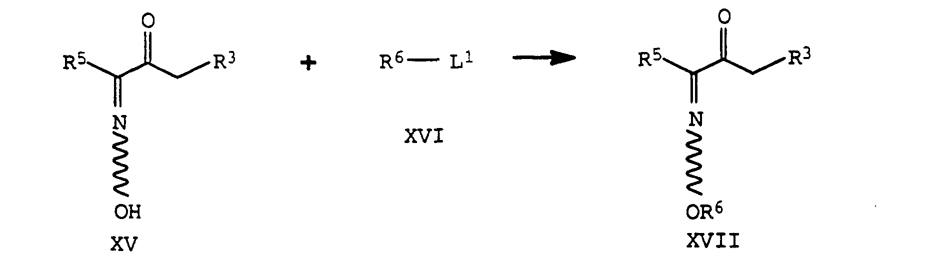

Den benötigten Ketooximether XVII erhält man beispielsweise durch Umsetzung von XV mit einem nukleophil substituierten Reagenz XVI.

L¹ in der Formel XVI steht für eine nukleophil austauschbare Abgangsgruppe, z.B. Halogen oder Sulfonatgruppen, vorzugsweise Chlor, Brom, Iod, Mesylat, Tosylat oder Triflat.

Die Umsetzung erfolgt in an sich bekannter Weise in einem inerten organischen Lösungsmittel in Gegenwart einer Base, z.B. Kaliumcarbonat, Kaliumhydroxid, Natriumhydrid, Triethylamin und Pyridin gemäß den im Houben-Weyl, Bd. 14b S. 307f, S. 370f und S. 385f; Houben-Weyl Bd. 10/4 S. 55f, S. 180f und S. 217f; Houben-Weyl Bd. E5, S. 780f, beschriebenen Methoden.

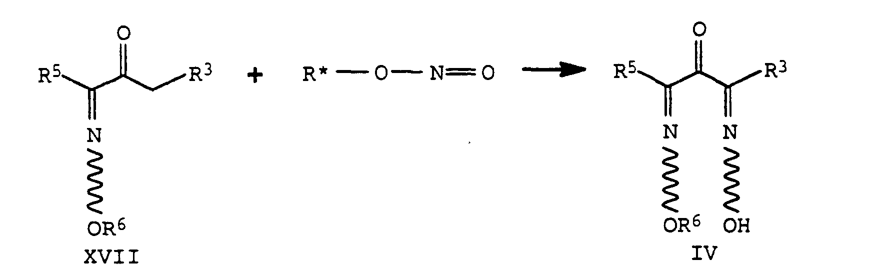

Durch basische oder saure Katalyse kann aus dem Keton XVII mit einem organischen Nitrit gemäß den in Houben-Weyl Bd. 10/4 S. 17f beschriebenen Methoden das α-Ketoxim IV hergestellt werden.

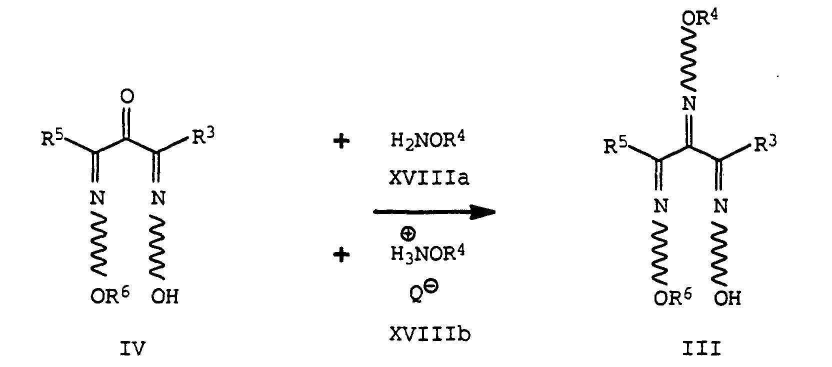

Das benötigte Hydroxyimin III erhält man beispielsweise durch Umsetzung eines entsprechenden α-Ketoxims IV mit einem Oxyamin XVIIIa oder seinem Salz XVIIIb. Q^{⊖} in der Formel XVIIIb steht für das Anion einer Säure, insbesondere einer anorganischen Säure, z.B. Halogenid wie Chlorid.

Die Umsetzung erfolgt in an sich bekannter Weise in einem inerten organischen Lösungsmittel gemäß den in EP-A 513 580; Houben-Weyl Bd. 10/4 S. 73f; Houben-Weyl Bd. E14b S. 369f und S. 385f beschriebenen Methoden.

1.1 Alternativ können die Verbindungen I auch dadurch erhalten werden, daß das Benzylderivat II zunächst mit dem Carbonylhydroxyiminoderivat IV in ein entsprechendes Benzyloxyimin der Formel V umgesetzt wird, wobei anschließend mit dem Hydroxylamin VIa bzw. dessen Salz VIb zu I umgesetzt wird. 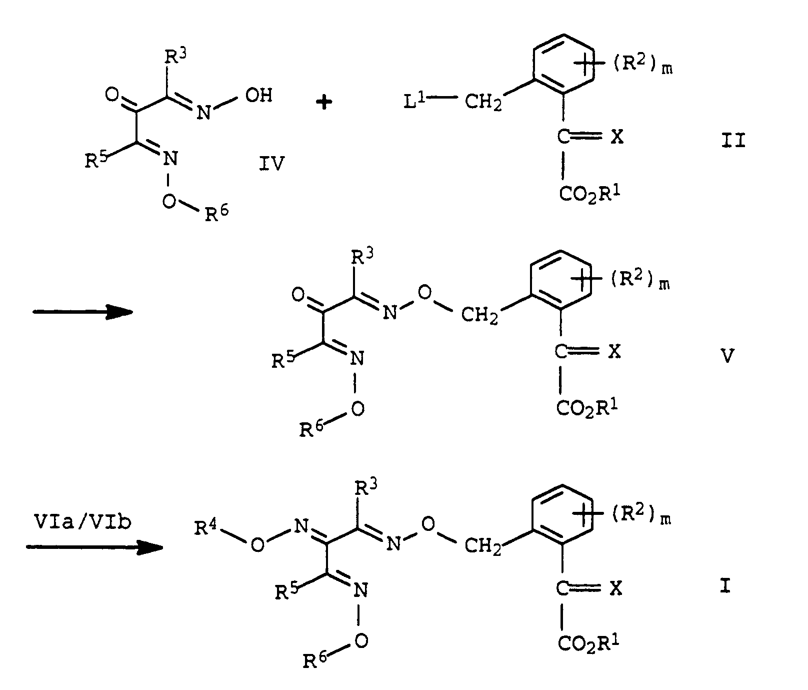 Die Umsetzung erfolgt in an sich bekannter Weise in einem inerten organischen Lösungsmittel gemäß den in Houben-Weyl, Bd. E 14b, S. 369f; Houben-Weyl, Bd. 10/1, S. 1189f und Houben-Weyl, Bd. 10/4, S. 73f oder EP-A 513 580 beschriebenen Methoden.

1.2 Eine weitere Möglichkeit zur Herstellung der Verbindungen I ist die Umsetzung des Benzylderivats II mit N-Hydroxyphthalimid und nachfolgender Hydrazinolyse zum Benzylhydroxylamin IIa und die weitere Umsetzung von IIa mit einer Carbonylverbindung VII.

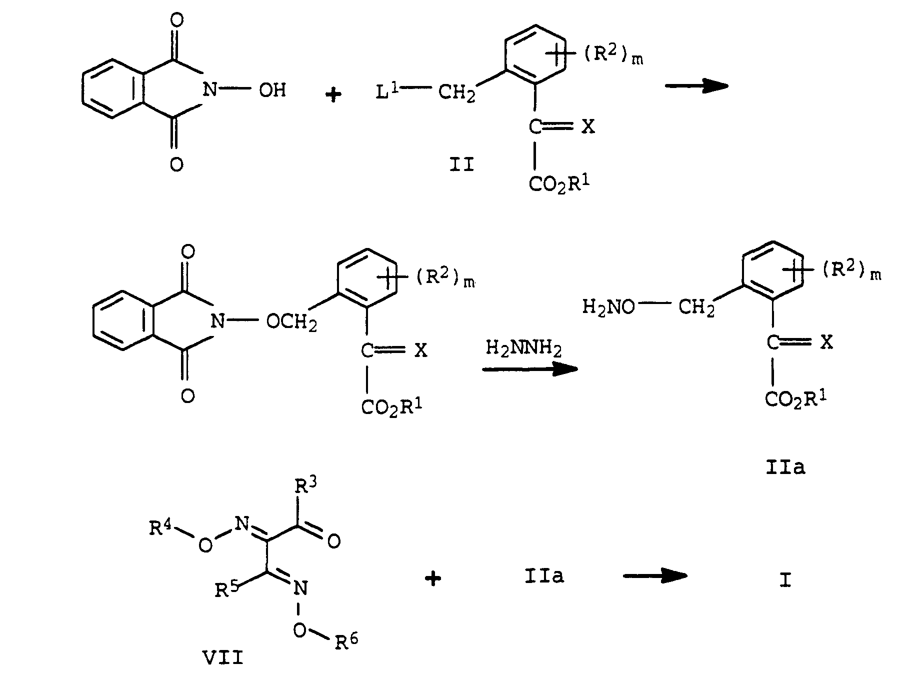

2. Verbindungen I erhält man bevorzugt dadurch, daß man eine Verbindung IV gemäß den in EP-A 493 711 beschriebenen Methoden mit einem Lacton IX zunächst in die entsprechende Benzoesäure X überführt und X über die entsprechenden Halogenide in die Cyanocarbonsäuren XI überführt, welche im Wege der Pinner-Reaktion (Angew. Chem. 94, 1 (1982)) in die a-Ketoester XII überführt und weiter zu den α-Ketoamiden XIII umgesetzt werden (vgl. EP 348 766, DE 37 05 389, EP 178 826, DE 36 23 92; Houben-Weyl Bd. E5 S. 941f).

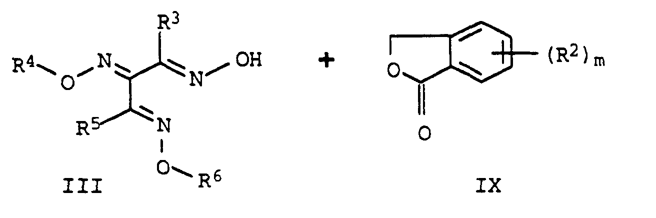

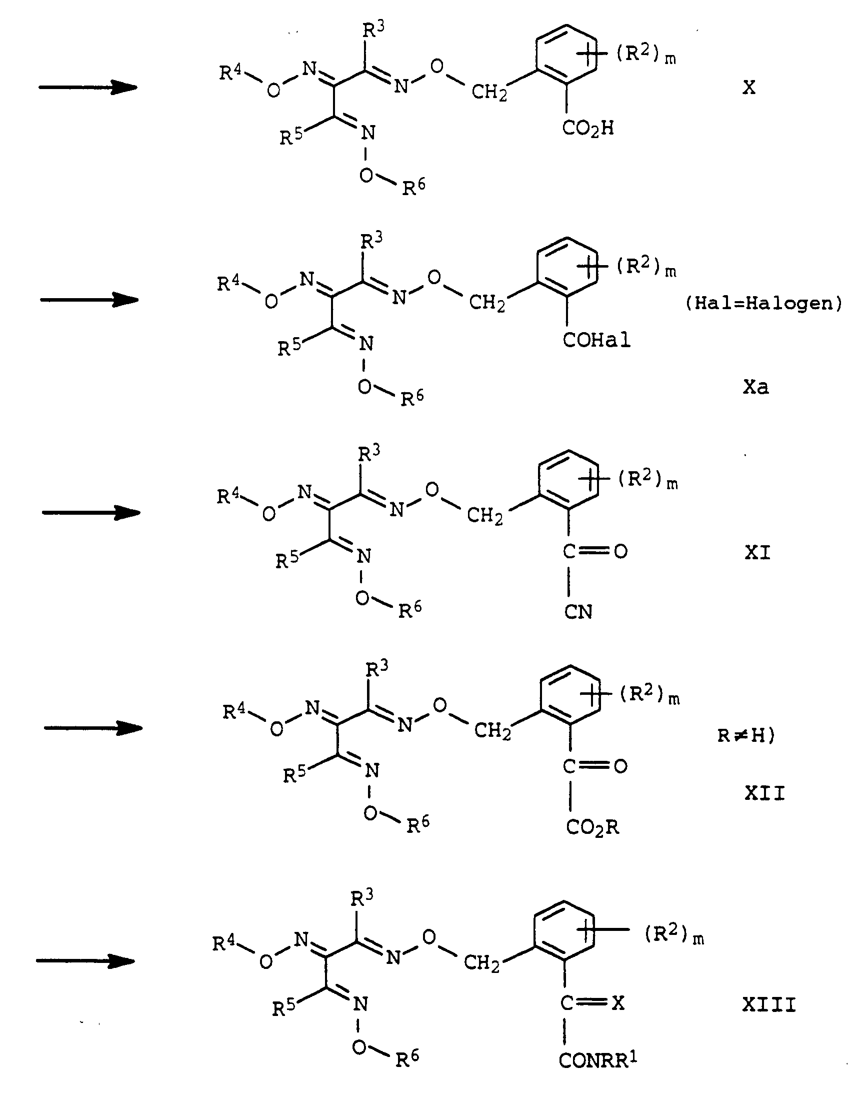

Die α-Ketoester XII und die a-Ketoamide XIII können gemäß üblicher Verfahren in die Verbindungen I überführt werden (vgl. EP-A 178 926, EP-A 513 580, DE-A 36 23 921, EP-A 398 692).

Verbindungen I, in denen R¹ Wasserstoff bedeutet, erhält man nach diesem Verfahren durch Verseifung der Ester XV und anschließende Umsetzung zu I.

Die Verbindungen I, in denen Y für NH steht, können auch aus den entsprechenden Estern (Y = 0) durch Umsetzung mit Aminen der Formeln R¹NH₂ erhalten werden.

Die Verbindungen II sind bekannt (EP-A 513 580, EP-A 477 631, EP-A 463 488, EP-A 251 082, EP 400 417, EP 585 751) oder können nach den dort beschriebenen Methoden hergestellt werden.

Die Verbindungen I können bei der Herstellung aufgrund ihrer C=C und C=N Doppelbindungen als E/Z-Isomerengemische anfallen, die z.B. durch Kristallisation oder Chromatographie in üblicher Weise in die Einzelverbindungen getrennt werden können.

Sofern bei der Synthese Isomerengemische anfallen, ist im allgemeinen jedoch eine Trennung nicht unbedingt erforderlich, da sich die einzelnen Isomere teilweise während der Aufbereitung für die Anwendung oder bei der Anwendung (z.B. unter Licht-, Säure- oder Baseneinwirkung) ineinander umwandeln können. Entsprechende Umwandlungen können auch nach der Anwendung, beispielsweise bei der Behandlung von Pflanzen in der behandelten Pflanze oder im zu bekämpfenden Schadpilz oder tierischen Schädling erfolgen.

In Bezug auf die C=X Doppelbindung werden hinsichtlich ihrer Wirksamkeit die E-Isomere der Verbindungen I bevorzugt (Konfiguration bezogen auf die -OCH₃ bzw. die -CH₃-Gruppe im Verhältnis zur -CO₂R¹ Gruppe).

In Bezug auf die -C(R³)=NOCH₂- Doppelbindung werden hinsichtlich ihrer Wirksamkeit die cis-Isomere der Verbindungen I bevorzugt (Konfiguration bezogen auf den Rest R³ im Verhältnis zur -OCH₂- Gruppe).

Bei der eingangs angegebenen Definitionen der Verbindungen I wurden Sammelbegriffe verwendet, die allgemein repräsentativ für die folgenden Gruppen stehen:

### Halogen: Fluor, Chlor, Brom und Jod;

**Alkyl:** geradkettige oder verzweigte Alkylgruppen mit 1 bis 4, 6 oder 10 Kohlenstoffatomen, z.B. C₁-C₆-Alkyl wie Methyl, Ethyl, Propyl, 1-Methylethyl, Butyl, 1-Methyl-propyl, 2-Methylpropyl, 1,1-Dimethylethyl, Pentyl, 1-Methylbutyl, 2-Methylbutyl, 3-Methylbutyl, 2,2-Di-methylpropyl, 1-Ethylpropyl, Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl und 1- Ethyl-2-methylpropyl;

**Halogenalkyl;** geradkettige oder verzweigte Alkylgruppen mit 1 bis 6 Kohlenstoffatomen, wobei in diesen Gruppen teilweise oder vollständig die Wasserstoffatome durch Halogenatome wie vorstehend genannt ersetzt sein können, z.B. C₁-C₂-Halogenalkyl wie Chlormethyl, Dichlormethyl, Trichlormethyl, Fluormethyl, Difluormethyl, Trifluormethyl, Chlorfluormethyl, Dichlorfluormethyl, Chlordifluormethyl, 1-Fluorethyl, 2-Fluorethyl, 2,2-Difluorethyl, 2,2,2-Trifluorethyl, 2-Chlor-2-fluorethyl, 2-Chlor-2,2-difluorethyl, 2,2-Dichlor-2-fluorethyl, 2,2,2-Trichlorethyl und Pentafluorethyl;

**Cycloalkyl:** monocyclische Alkylgruppen mit 3 bis 6 Kohlenstoffringgliedern, z.B. Cyclopropyl, Cyclobutyl, Cyclopentyl und Cyclohexyl;

**Alkenyl:** geradkettige oder verzweigte Alkenylgruppen mit 2 bis 6 oder 10 Kohlenstoffatomen und einer Doppelbindung in einer beliebigen Position, z.B. C₂-C₆-Alkenyl wie Ethenyl, 1-Propenyl, 2-Propenyl, 1-Methylethenyl, 1-Butenyl, 2-Butenyl, 3-Butenyl, 1-Methyl-1-propenyl, 2-Methyl-1-propenyl, 1-Methyl-2-propenyl, 2-Methyl-2-propenyl, 1-Pentenyl, 2-Pentenyl, 3-Pentenyl, 4-Pentenyl, 1-Methyl-1-butenyl, 2-Methyl-1-butenyl, 3-Methyl-1-butenyl, 1-Methyl-2-butenyl, 2-Methyl-2-butenyl, 3-Methyl-2-butenyl, 1-Methyl-3-butenyl, 2-Methyl-3-butenyl, 3-Methyl-3-butenyl, 1,1-Dimethyl-2-propenyl, 1,2-Dimethyl-l-propenyl, 1,2-Dimethyl-2-propenyl, 1-Ethyl-1-propenyl, 1-Ethyl-2-propenyl, 1-Hexenyl, 2-Hexenyl, 3-Hexenyl, 4-Hexenyl, 5-Hexenyl, 1-Methyl-1-pentenyl, 2-Methyl-1-pentenyl, 3-Methyl-1-pentenyl, 4-Methyl-1-pentenyl, 1-Methyl-2-pentenyl, 2-Methyl-2-pentenyl, 3-Methyl-2-pentenyl, 4-Methyl-2-pentenyl, 1-Methyl-3-pentenyl, 2-Methyl-3-pentenyl, 3-Methyl-3-pentenyl, 4-Methyl-3-pentenyl, 1-Methyl-4-pentenyl, 2-Methyl-4-pentenyl, 3-Methyl-4-pentenyl, 4-Methyl-4-pentenyl, 1,1-Dimethyl-2-butenyl, 1,1-Di-methyl-3-butenyl, 1,2-Dimethyl-1-butenyl, 1,2-Dimethyl-2-butenyl, 1,2-Dimethyl-3-butenyl, 1,3-Dimethyl-1-butenyl, 1,3-Dimethyl-2-butenyl, 1,3-Dimethyl-3-butenyl, 2,2-Dimethyl-3-butenyl, 2,3-Dimethyl-1-butenyl, 2,3-Dimethyl-2-butenyl, 2,3-Dimethyl-3-butenyl, 3,3-Dimethyl-1-butenyl, 3,3-Dimethyl-2-butenyl, 1-Ethyl-1-butenyl, 1-Ethyl-2-butenyl, 1-Ethyl-3-butenyl, 2-Ethyl-1-butenyl, 2-Ethyl-2-butenyl, 2-Ethyl-3-butenyl, 1,1,2-Trimethyl-2-propenyl, 1-Ethyl-1-methyl-2-propenyl, l-Ethyl-2-methyl-1-propenyl und 1-Ethyl-2-methyl-2-propenyl;

**Alkinyl:** geradkettige oder verzweigte Alkinylgruppen mit 2 bis 10 Kohlenstoffatomen und einer Dreifachbindung in einer beliebigen Position, z.B. C₂-C₆-Alkinyl wie Ethinyl, 2-Propinyl, 2-Butinyl, 3-Butinyl, 1-Methyl-2-propinyl, 2-Pentinyl, 3-Pentinyl, 4-Pentinyl, 1-Methyl-2-butinyl, 1-Methyl-3-butinyl, 2-Methyl-3-butinyl, 1,1-Dimethyl-2-propinyl, 1-Ethyl-2-propinyl, 2-Hexinyl, 3-Hexinyl, 4-Hexinyl, 5-Hexinyl, 1-Methyl-2-pentinyl, 1-Methyl-3-pentinyl, 1-Methyl-4-pentinyl, 2-Methyl-3-pentinyl, 2-Methyl-4-pentinyl, 3-Methyl-4-pentinyl, 4-Methyl-2-pentinyl, 1,1-Dimethyl-2-butinyl, 1,1-Dimethyl-3-butinyl, 1,2-Dimethyl-3-butinyl, 2,2-Dimethyl-3-butinyl, 1-Ethyl-2-butinyl, 1-Ethyl-3-butinyl, 2-Ethyl-3-butinyl und 1-Ethyl-1-methyl-2-propinyl;

**Heterocyclyl bzw, Heterocyclyloxy, Heterocyclylthio und Heterocyclylamino:** drei- bis sechsgliedrige, gesättigte oder partiell ungesättigte mono- oder polycyclische Heterocyclen, die ein bis drei Hereroatome ausgewählt aus einer Gruppe bestehend aus Sauerstoff, Stickstoff und Schwefel enthalten, und welche direkt bzw. (Heterocyclyloxy) über ein Sauerstoffatom oder (Heterocyclylthio) über ein Schwefelatom oder (Heterocyclylamino) über ein Stickstoffatom an das Gerüst gebunden sind, wie z.B. 2-Tetrahydrofuranyl, Oxiranyl, 3-Tetrahydrofuranyl, 2-Tetrahydrothienyl, 3-Tetrahydrothienyl, 2-Pyrrolidinyl, 3-Pyrrolidinyl, 3-Isoxazoldinyl, 4-Isoxazolidinyl, 5-Isoxazolidinyl, 3-Isothiazolidinyl, 4-Isothiazolidinyl, 5-Isothiazolidinyl, 3-Pyrazolidinyl, 4-Pyrazolidinyl, 5-Pyrazolidinyl, 2-Oxazolidinyl, 4-Oxazolidinyl, 5-Oxazolidinyl, 2-Thiazolidinyl, 4-Thiazolidinyl, 5-Thiazolidinyl, 2-Imidazolidinyl, 4-Imidazolidinyl, 1,2,4-Oxadiazolidin-3-yl, 1,2,4-Oxadiazolidin-5-yl, 1,2,4-Thiadiazolidin-3-yl, 1,2,4-Thiadiazolidin-5-yl, 1,2,4-Triazolidin-3-yl, 1,3,4-Oxadiazolidin-2-yl, 1,3,4-Thiadiazolidin-2-yl, 1,3,4-Triazolidin-2-yl, 2,3-Dihydrofur-2-yl, 2,3-Dihydrofur-3-yl, 2,3-Dihydro-fur-4-yl, 2,3-Dihydro-fur-5-yl, 2,5-Dihydro-fur-2-yl, 2,5-Dihydro-fur-3-yl, 2,3-Dihydrothien-2-yl, 2,3-Dihydrothien-3-yl, 2,3-Dihydrothien-4-yl, 2,3-Dihydrothien-5-yl, 2,5-Dihydrothien-2-yl, 2,5-Dihydrothien-3-yl, 2,3-Dihydropyrrol-2-yl, 2,3-Dihydropyrrol-3-yl, 2,3-Dihydropyrrol-4-yl, 2,3-Dihydropyrrol-5-yl, 2,5-Dihydropyrrol-2-yl, 2,5-Dihydropyrrol-3-yl, 2,3-Dihydroisoxazol-3-yl, 2,3-Dihydroisoxazol-4-yl, 2,3-Dihydroisoxazol-5-yl, 4,5-Dihydroisoxazol-3-yl, 4,5-Dihydroisoxazol-4-yl, 4,5-Dihydroisoxazol-5-yl, 2,5-Dihydroisothiazol-3-yl, 2,5-Dihydroisothiazol-4-yl, 2,5-Dihydroisothiazol-5-yl, 2,3-Dihydroisopyrazol-3-yl, 2,3-Dihydroisopyrazol-4-yl, 2,3-Dihydroisopyrazol-5-yl, 4,5-Dihydroisopyrazol-3-yl, 4,5-Dihydroisopyrazol-4-yl, 4,5-Dihydroisopyrazol-5-yl, 2,5-Dihydroisopyrazol-3-yl, 2,5-Dihydroisopyrazol-4-yl, 2,5-Dihydroisopyrazol-5-yl, 2,3-Dihydrooxazol-3-yl, 2,3-Dihydrooxazol-4-yl, 2,3-Dihydrooxazol-5-yl, 4,5-Dihydrooxazol-3-yl, 4,5-Dihydrooxazol-4-yl, 4,5-Dihydrooxazol-5-yl, 2,5-Dihydrooxazol-3-yl, 2,5-Dihydrooxazol-4-yl, 2,5-Dihydrooxazol-5-yl, 2,3-Dihydrothiazol-2-yl, 2,3-Dihydrothiazol-4-yl, 2,3-Dihydrothiazol-5-yl, 4,5-Dihydrothiazol-2-yl, 4,5-Dihydrothiazol-4-yl, 4,5-Dihydrothiazol-5-yl, 2,5-Dihydrothiazol-2-yl, 2,5-Dihydrothiazol-4-yl, 2,5-Dihydrothiazol-5-yl, 2,3-Dihydroimidazol-2-yl, 2,3-Dihydroimidazol-4-yl, 2,3-Dihydroimidazol-5-yl, 4,5-Dihydroimidazol-2-yl, 4,5-Dihydroimidazol-4-yl, 4,5-Dihydroimidazol-5-yl, 2,5-Dihydroimidazol-2-yl, 2,5-Dihydroimidazol-4-yl, 2,5-Dihydroimidazol-5-yl, 2-Morpholinyl, 3-Morpholinyl, 2-Piperidinyl, 3-Piperidinyl, 4-Piperidinyl, 3-Tetrahydropyridazinyl, 4-Tetrahydropyridazinyl, 2-Tetrahydropyrimidinyl, 4-Tetrahydropyrimidinyl, 5-Tetrahydropyrimidinyl, 2-Tetrahydropyrazinyl, 1,3,5-Tetrahydrotriazin-2-yl, 1,2,4-Tetrahydrotriazin-3-yl, 1,3-Dihydrooxazin-2-yl, 1,3-Dithian-2-yl, 2-Tetrahydropyranyl, 1,3-Dioxolan-2-yl, 3,4,5,6-Tetrahydropyridin-2-yl, 4H-1,3-Thiazin-2-yl, 4H-3,1-Benzothiazin-2-yl, 1,1-Dioxo-2,3,4,5-tetrahydrothien-2-yl, 2H-1,4-Benzothiazin-3-yl, 2H-1,4-Benzoxazin-3-yl, 1,3-Dihydrooxazin-2-yl, 1,3-Dithian-2-yl,

**Aryl bzw, Aryloxy, Arylthio, Arylcarbonyl und Arylsulfonyl:** aromatische mono- oder polycyclische Kohlenwasserstoffreste welche direkt bzw. (Aryloxy) über ein Sauerstoffatom (-O-) oder (Arylthio) ein Schwefelatom (-S-), (Arylcarbonyl) über eine Carbonylgruppe (-CO-) oder (Arylsulfonyl) über eine Sulfonylgruppe (-SO₂-) an das Gerüst gebunden sind, z.B. Phenyl, Naphthyl und Phenanthrenyl bzw. Phenyloxy, Naphthyloxy und Phenanthrenyloxy und die entsprechenden Carbonyl- und Sulfonylreste;

**Hetaryl bzw. Hetaryloxy, Hetarylthio, Hetarylcarbonyl und Hetarylsulfonyl:** aromatische mono- oder polycyclische Reste welche neben Kohlenstoffringgliedern zusätzlich ein bis vier Stickstoffatome oder ein bis drei Stickstoffatome und ein Sauerstoff- oder ein Schwefelatom oder ein Sauerstoff- oder ein Schwefelatom enthalten können und welche direkt bzw. (Hetaryloxy) über ein Sauerstoffatom (-O-) oder (Hetarylthio) ein Schwefelatom (-S-), (Hetarylcarbonyl) über eine Carbonylgruppe (-CO-) oder (Hetarylsulfonyl) über eine Sulfonylgruppe (-SO₂-) an das Gerüst gebunden sind, z.B.
- 5-gliedriges Heteroaryl enthaltend ein bis drei Stickstoffatome: 5-Ring Heteroarylgruppen, welche neben Kohlenstoffatomen ein bis drei Stickstoffatome als Ringglieder enthalten können, z.B. 2-Pyrrolyl, 3-Pyrrolyl, 3-Pyrazolyl, 4-Pyrazolyl, 5-Pyrazolyl, 2-Imidazolyl, 4-Imidazolyl, 1,2,4-Triazol-3-yl und 1,3,4-Triazol-2-yl;
- 5-gliedriges Heteroaryl, enthaltend ein bis vier Stickstoffatome oder ein bis drei Stickstoffatome und ein Schwefel- oder Sauerstoffatom oder ein Sauerstoff oder ein Schwefelatom: 5-Ring Heteroarylgruppen, welche neben Kohlenstoffatomen ein bis vier Stickstoffatome oder ein bis drei Stickstoffatome und ein Schwefel- oder Sauerstoffatom oder ein Sauerstoff- oder Schwefelatom als Ringglieder enthalten können, z.B. 2-Furyl, 3-Furyl, 2-Thienyl, 3-Thienyl, 2-Pyrrolyl, 3-Pyrrolyl, 3-Isoxazolyl, 4-Isoxazolyl, 5-Isoxazolyl, 3-Isothiazolyl, 4-Isothiazolyl, 5-Isothiazolyl, 3-Pyrazolyl, 4-Pyrazolyl, 5-Pyrazolyl, 2-Oxazolyl, 4-Oxazolyl, 5-Oxazolyl, 2-Thiazolyl, 4-Thiazolyl, 5-Thiazolyl, 2-Imidazolyl, 4-Imidazolyl, 1,2,4-Oxadiazol-3-yl, 1,2,4-Oxadiazol-5-yl, 1,2,4-Thiadiazol-3-yl, 1,2,4-Thiadiazol-5-yl, 1,2,4-Triazol-3-yl, 1,3,4-Oxadiazol-2-yl, 1,3,4-Thiadiazol-2-yl, 1,3,4-Triazol-2-yl;
- benzokondensiertes 5-gliedriges Heteroaryl, enthaltend ein bis drei Stickstoffatome oder ein Stickstoffatom und/oder ein Sauerstoff- oder Schwefelatom: 5-Ring Heteroarylgruppen, welche neben Kohlenstoffatomen ein bis vier Stickstoffatome oder ein bis drei Stickstoffatome und ein Schwefel- oder Sauerstoffatom oder ein Sauerstoff- oder ein Schwefelatom als Ringglieder enthalten können, und in welchen zwei benachbarte Kohlenstoffringglieder oder ein Stickstoff- und ein benachbartes Kohlenstoffringglied durch eine Buta-1,3-dien-1,4-diylgruppe verbrückt sein können;
- über Stickstoff gebundenes 5-gliedriges Heteroaryl, enthaltend ein bis vier Stickstoffatome, oder über Stickstoff gebundenes benzokondensiertes 5-gliedriges Heteroaryl, enthaltend ein bis drei Stickstoffatome; 5-Ring Heteroarylgruppen, welche neben Kohlenstoffatomen ein bis vier Stickstoffatome bzw. ein bis drei Stickstoffatome als Ringglieder enthalten können, und in welchen zwei benachbarte Kohlenstoffringglieder oder ein Stickstoff- und ein benachbartes Kohlenstoffringglied durch eine Buta-1,3-dien- 1,4-diylgruppe verbrückt sein können, wobei diese Ringe über eines der Stickstoffringglieder an das Gerüst gebunden sind;
- 6-gliedriges Heteroaryl, enthaltend ein bis drei bzw, ein bis vier Stickstoffatome: 6-Ring Heteroarylgruppen, welche neben Kohlenstoffatomen ein bis drei bzw. ein bis vier Stickstoffatome als Ringglieder enthalten können, z.B. 2-Pyridinyl, 3-Pyridinyl, 4-Pyridinyl, 3-Pyridazinyl, 4-Pyridazinyl, 2-Pyrimidinyl, 4-Pyrimidinyl, 5-Pyrimidinyl, 2-Pyrazinyl, 1,3,5-Triazin-2-yl, 1,2,4-Triazin-3-yl und 1,2,4,5-Tetrazin-3-yl;
- benzokondensiertes 6-gliedriges Heteroaryl, enthaltend ein bis vier Stickstoffatome: 6-Ring Heteroarylgruppen in welchen zwei benachbarte Kohlenstoffringglieder durch eine Buta-1,3-dien-1,4-diylgruppe verbrückt sein können, z.B. Chinolin, Isochinolin, Chinazolin und Chinoxalin,
bzw. die entsprechenden Oxy-, Thio-, Carbonyl- oder Sulfonylgruppen.

**Hetarylamino:** aromatische mono- oder polycyclische Reste, welche neben Kohlenstoffringgliedern zusätzlich ein bis vier Stickstoffatome oder ein bis drei Stickstoffatome und ein Sauerstoff- oder ein Schwefelatom enthalten können und welche über ein Stickstoffatom an das Gerüst gebunden sind.

Die Angabe "partiell oder vollständig halogeniert" soll zum Ausdruck bringen, daß in den derart charakterisierten Gruppen die Wasserstoffatome zum Teil oder vollständig durch gleiche oder verschiedene Halogenatome wie vorstehend genannt ersetzt sein können.

Im Hinblick auf ihre biologische Wirkung sind Verbindungen der Formel I bevorzugt, in denen m für 0 steht.

Gleichermaßen bevorzugt sind Verbindungen der Formel I, in denen R¹ für Methyl steht.

Daneben werden Verbindungen I bevorzugt, in denen R³ für Wasserstoff, Cyano, Cyclopropyl, Methyl, Ethyl, 1-Methylethyl oder CF₃ steht.

Außerdem werden Verbindungen I bevorzugt, in denen R³ für Methyl steht.

Daneben werden Verbindungen I bevorzugt, in denen R³ für Cyano steht.

Weiterhin werden Verbindungen I bevorzugt, in denen R³ für Cyclopropyl steht.

Des weiteren werden Verbindungen I bevorzugt, in denen R³ für CF₃ steht.

Des weiteren werden Verbindungen I bevorzugt, in denen R⁵ für Wasserstoff, Cyclopropyl, Methyl, Ethyl, iso-Propyl, ggf. subst. Aryl oder Hetaryl steht.

Außerdem werden Verbindungen I bevorzugt, in denen R⁵ für Methyl steht.

Des weiteren werden Verbindungen I bevorzugt, in denen R⁵ für Ethyl steht.

Außerdem werden Verbindungen I bevorzugt, in denen R⁵ für Iso-Propyl steht.

Außerdem werden Verbindungen I bevorzugt, in denen R⁵ für Cyclopropyl steht.

Außerdem werden Verbindungen I bevorzugt, in denen R⁵ für CF₃ steht.

Des weiteren werden Verbindungen I bevorzugt, in denen R⁵ für ggf. subst. Aryl oder Hetaryl steht.

Des weiteren werden Verbindungen I bevorzugt, in denen R⁵ für ggf. subst. Pyridyl, Pyrimidyl, Pyrazinyl, Pyridazinyl oder Triazinyl steht.

Des weiteren werden Verbindungen I bevorzugt, in denen R⁵ für ggf. subst. Furyl, Thienyl oder Pyrrolyl steht.

Des weiteren werden Verbindungen I bevorzugt, in denen R⁵ für ggf. subst. Oxazolyl, Thiazolyl, Isoxazolyl, Isothiazolyl, Pyrazolyl oder Imidazolyl steht.

Des weiteren werden Verbindungen I bevorzugt, in denen R⁵ für ggf. subst. Oxdiazolyl, Thiadiazolyl oder Triazolyl steht.

Außerdem werden Verbindungen I bevorzugt, in denen R⁵ für Phenyl steht, welches unsubstituiert ist oder ein oder zwei der folgenden Gruppen trägt: Nitro, Cyano, Hydroxy, Amino, Aminocarbonyl, Aminothiocarbonyl, Halogen, C₁-C₄-Alkyl, C₁-C₄-Halogenalkyl, C₁-C₄-Alkoxy, C₁-C₄-Halogenalkoxy, C₁-C₄-Alkylamino, Di-C₁-C₄-Alkylamino, C₁-C₄-Alkylsulfonyl, C₁-C₄-Alkoxycarbonyl, C₁-C₄-Alkylaminocarbonyl oder Di-C₁-C₄-Alkylaminocarbonyl.

Außerdem werden Verbindungen I bevorzugt, in denen R⁴ für Wasserstoff, C₁-C₆-Alkyl, C₂-C₆-Alkenyl, C₂-C₆-Alkinyl, Allyl, Arylalkyl, Hetarylalkyl, Aryloxyalkyl, Hetaryloxyalkyl, Aryl oder Hetaryl steht.

Des weiteren werden Verbindungen I bevorzugt, in denen R⁴ für C₁-C₆-Alkyl steht.

Außerdem werden Verbindungen I bevorzugt, in denen R⁴ für Methyl, Ethyl, 2-Propenyl oder 2-Propinyl steht.

Des weiteren werden Verbindungen der Formel I bevorzugt, in denen R⁶ für C₁-C₆-Alkyl, C₂-C₆-Alkenyl, C₂-C₆-Alkinyl, Arylalkyl, Hetarylalkyl, Aryloxyalkyl oder Hetaryloxyalkyl steht.

Außerdem werden Verbindungen I bevorzugt, in denen R⁶ für Methyl, Ethyl oder Propargyl steht.

Des weiteren werden solche Verbindungen I bevorzugt, in denen R⁶ für Arylalkyl oder Hetarylalkyl steht.

Außerdem werden solche Verbindungen I bevorzugt, in denen R⁶ für Aryloxyalkyl oder Hetaryloxyalkyl steht.

Daneben werden Verbindungen der Formel I bevorzugt, in denen X für NOCH₃ steht.

Daneben werden Verbindungen der Formel I bevorzugt, in denen X für CHOCH₃ steht.

Daneben werden Verbindungen der Formel I bevorzugt, in denen X für CHCH₃ steht.

Des weiteren werden Verbindungen der Formel I bevorzugt, in denen Y für O steht.

Des weiteren werden Verbindungen der Formel I bevorzugt, in denen Y für NH steht.

Weiterhin werden Verbindungen der Formel I bevorzugt, in denen die Substituenten aus einer Kombination der oben beschriebenen bevorzugten Substituenten ausgewählt sind.

Insbesondere sind im Hinblick auf ihre Verwendung die in den folgenden Tabellen zusammengestellten Verbindungen I bevorzugt. Insbesondere sind im Hinblick auf ihre Verwendung die in den folgenden Tabellen zusammengestellten Verbindungen I der Formeln I.1 - I.4 bevorzugt:

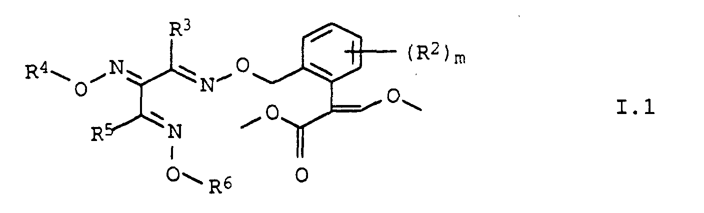

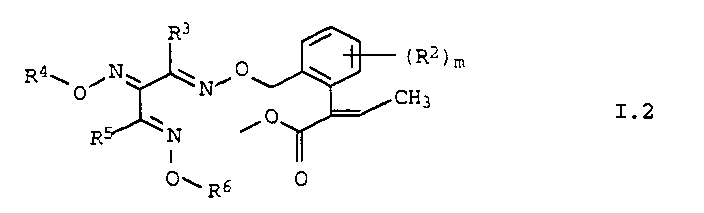

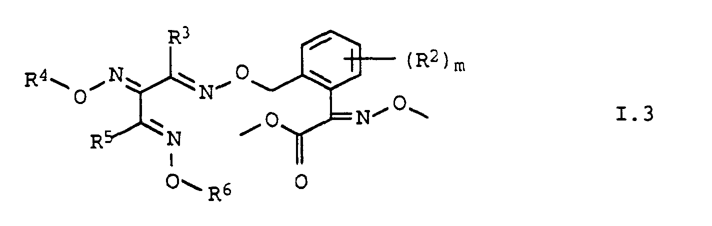

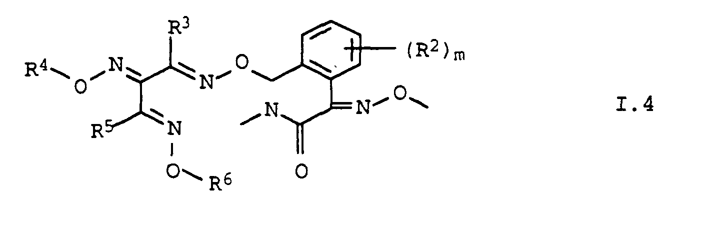

**Tabelle A**

| Nr. | R⁶ |
|---|---|
| 1 | H |
| 2 | CH₃ |
| 3 | CH₂CH₃ |
| 4 | CH₂CH₂CH₃ |
| 5 | CH(CH₃)₂ |
| 6 | cyclopropyl |
| 7 | (CH₂)₃CH₃ |
| 8 | CH(CH₃)CH₂CH₃ |
| 9 | CH₂CH(CH₃)₂ |
| 10 | C(CH₃)₃ |
| 11 | cyclobutyl |
| 12 | (CH₂)₄CH₃ |
| 13 | CH(CH₃)(CH₂)₂CH₃ |
| 14 | CH₂CH(CH₃)CH₂CH₃ |
| 15 | (CH₂)₂CH(CH₃)₂ |
| 16 | CH₂C(CH₃)₃ |
| 17 | CH(CH₂CH₃)₂ |
| 18 | C(CH₃)₂CH₂CH₃ |
| 19 | CH(CH₃)CH(CH₃)₂ |
| 20 | cyclopentyl |
| 21 | (CH₂)₅CH₃ |
| 22 | CH(CH₃)(CH₂)₃CH₃ |
| 23 | CH(CH₂CH₃)(CH₂)₂CH₃ |
| 24 | CH₂CH(CH₃)(CH₂)₂CH₃ |
| 25 | (CH₂)₂CH(CH₃)CH₂CH₃ |
| 26 | (CH₂)₃CH(CH₃)₂ |
| 27 | (CH₂)₂C(CH₃)₃ |
| 28 | CH₂CH(CH₂CH₃)₂ |
| 29 | CH(CH₃)CH(CH₃)CH₂CH₃ |
| 30 | CH(CH₃)CH₂CH(CH₃)₂ |
| 31 | CH₂CH(CH₃)CH(CH₃)₂ |
| 32 | CH(CH₃)C(CH₃)₃ |
| 33 | CH(CH₂CH₃)CH(CH₃)₂ |
| 34 | C(CH₃)₂CH₂CH₂CH₃ |
| 35 | CH₂C(CH₃)₂CH₂CH₃ |
| 36 | C(CH₃)₂CH(CH₃)₂ |
| 37 | cyclohexyl |
| 38 | CH₂CN |
| 39 | (CH₂)₂CN |
| 40 | (CH₂)₃CN |
| 41 | (CH₂)₄CN |
| 42 | CH₂NO₂ |
| 43 | (CH₂)₂NO₂ |
| 44 | (CH₂)₃NO₂ |
| 45 | (CH₂)₄NO₂ |
| 46 | (CH₂)₂OH |
| 47 | (CH₂)₃OH |
| 48 | (CH₂)₄OH |
| 49 | (CH₂)₂NH₂ |
| 50 | (CH₂)₃NH₂ |
| 51 | (CH₂)₄NH₂ |
| 52 | (CH₂)₂NHCH₃ |
| 53 | (CH₂)₃NHCH₃ |
| 54 | (CH₂)₄NHCH₃ |
| 55 | (CH₂)₂N(CH₃)₂ |
| 56 | (CH₂)₃N(CH₃)₂ |
| 57 | (CH₂)₄N(CH₃)₂ |
| 58 | (CH₂)₂N(CH₂CH₃)₂ |
| 59 | (CH₂)₃N(CH₂CH₃)₂ |
| 60 | (CH₂)₄N(CH₂CH₃)₂ |
| 61 | (CH₂)₂OCH₃ |
| 62 | (CH₂)₃OCH₃ |
| 63 | (CH₂)₄OCH₃ |
| 64 | (CH₂)₂OCH₂CH₃ |
| 65 | (CH₂)₃OCH₂CH₃ |
| 66 | (CH₂)₄OCH₂CH₃ |
| 67 | (CH₂)₂O(CH₂)₂CH₃ |
| 68 | (CH₂)₃O(CH₂)₂CH₃ |
| 69 | (CH₂)₄O(CH₂)₂CH₃ |
| 70 | (CH₂)₂OCH(CH₃)₂ |
| 71 | (CH₂)₃OCH(CH₃)₂ |
| 72 | (CH₂)₄OCH(CH₃)₂ |
| 73 | (CH₂)₂OC(CH₃)₃ |
| 74 | (CH₂)₃OC(CH₃)₃ |
| 75 | (CH₂)₄OC(CH₃)₃ |
| 76 | (CH₂)₂OCF₃ |
| 77 | (CH₂)₃OCF₃ |
| 78 | (CH₂)₄OCF₃ |
| 79 | (CH₂)₂SCH₃ |
| 80 | CH₃(CH₂)₃SCH₃ |
| 81 | (CH₂)₄SCH₃ |
| 82 | (CH₂)₂SOCH₃ |
| 83 | (CH₂)₃SOCH₃ |
| 84 | (CH₂)₄SOCH₃ |
| 85 | (CH₂)₂SO₂CH₃ |
| 86 | (CH₂)₃SO₂CH₃ |
| 87 | (CH₂)₄SO₂CH₃ |
| 88 | CH₂-cyclopropyl |
| 89 | (CH₂)₂-cyclopropyl |
| 90 | (CH₂)₃-cyclopropyl |
| 91 | (CH₂)₄-cyclopropyl |
| 92 | CH₂-cyclopentyl |
| 93 | (CH₂)₂-cyclopentyl |
| 94 | (CH₂)₃-cyclopentyl |
| 95 | (CH₂)₄-cyclopentyl |
| 96 | CH₂-cyclohexyl |
| 97 | (CH₂)₂-cyclohexyl |
| 98 | (CH₂)₃-cyclohexyl |
| 99 | (CH₂)₄-cyclohexyl |
| 100 | CHF₂ |
| 101 | CF₃ |
| 102 | CH₂CHF₂ |
| 103 | CH₂CF₃ |
| 104 | CHFCHF₂ |
| 105 | CH₂CH₂F |
| 106 | CHFCH₃ |
| 107 | CHFCF₃ |
| 108 | CF₂CHF₂ |
| 109 | CF₂CHFCF₃ |
| 110 | CH₂CCl₃ |
| 111 | CF₂CHCl₂ |
| 112 | CF₂CHFCl |
| 113 | CF₂CHFBr |
| 114 | CH(CF₃)₂ |
| 115 | CH(CF₃)CH₃ |
| 116 | CH₂CH₂CF₃ |
| 117 | CH₂CHFCH₃ |
| 118 | CH₂CF₂CF₃ |
| 119 | CH₂CH₂CH₂F |
| 120 | CH₂CF₂CF₂CF₃ |
| 121 | CH₂CH₂CHFCH₃ |
| 122 | CH₂CH₂CH₂CH₂F |
| 123 | CH₂CH₂Cl |
| 124 | CH₂CHClCH₃ |
| 125 | CH₂CH₂CH₂Cl |
| 126 | CH₂CH₂CHClCH₃ |
| 127 | CH₂CH₂CH₂CH₂Cl |
| 128 | CH₂CH₂Br |
| 129 | CH₂CHBrCH₃ |
| 130 | CH₂CH₂CH₂Br |
| 131 | CH₂CH₂CHBrCH₃ |
| 132 | CH₂CH₂CH₂CH₂Br |
| 133 | CH₂-C₆H₅ |
| 134 | CH(CH₃)CN |
| 135 | CH(CH₃)CH₂CN |
| 136 | CH₂CH(CH₃)CN |
| 137 | CH(CH₃)CH(CH₃)CN |
| 138 | CH(CH₃)(CH₂)₂CN |
| 139 | CH₂CH(CH₃)CH₂CN |
| 140 | (CH₂)₂CH(CH₃)CN |
| 141 | CH(CH₃)CH(CH₃)CH₂CN |
| 142 | CH(CH₃)CH₂CH(CH₃)CN |
| 143 | CH₂CH(CH₃)CH(CH₃)CN |
| 144 | CH(CH₃)CH(CH₃)CH(CH₃)CN |
| 145 | CH(CH₃)(CH₂)₃CN |
| 146 | CH(CH₃)NO₂ |
| 147 | CH(CH₃)CH₂NO₂ |
| 148 | CH₂CH(CH₃)NO₂ |
| 149 | CH(CH₃)CH(CH₃)NO₂ |
| 150 | CH(CH₃) (CH₂)₂NO₂ |
| 151 | CH₂CH(CH₃)CH₂NO₂ |
| 152 | (CH₂)₂CH(CH₃)NO₂ |
| 153 | CH(CH₃)CH(CH₃)CH₂NO₂ |
| 154 | CH(CH₃)CH₂CH(CH₃)NO₂ |
| 155 | CH₂CH(CH₃)CH(CH₃)NO₂ |
| 156 | CH(CH₃)CH(CH₃)CH(CH₃)NO₂ |
| 157 | CH(CH₃)(CH₂)₃NO₂ |
| 158 | CH(CH₃)CH₂OH |
| 159 | CH₂CH(CH₃)O |
| 160 | CH(CH₃)CH(CH₃)OH |
| 161 | CH(CH₃)(CH₂)₂OH |
| 162 | CH₂CH(CH₃)CH₂OH |
| 163 | (CH₂)₂CH(CH₃)OH |
| 164 | CH(CH₃)CH(CH₃)CH₂OH |
| 165 | CH(CH₃)CH₂CH(CH₃)OH |
| 166 | CH₂CH(CH₃)CH(CH₃)OH |
| 167 | CH(CH₃)CH(CH₃)CH(CH₃)OH |
| 168 | CH(CH₃)(CH₂)₃OH |
| 169 | CH(CH₃)CH₂OCH₃ |
| 170 | CH₂CH(CH₃)OCH₃ |
| 171 | CH(CH₃)CH(CH₃)OCH₃ |
| 172 | CH(CH₃)(CH₂)₂OCH₃ |
| 173 | CH₂CH(CH₃)CH₂OCH₃ |
| 174 | (CH₂)₂CH(CH₃)OCH₃ |
| 175 | CH(CH₃)CH(CH₃)CH₂OCH₃ |
| 176 | CH(CH₃)CH₂CH(CH₃)OCH₃ |
| 177 | CH₂CH(CH₃)CH(CH₃)OCH₃ |
| 178 | CH(CH₃)CH(CH₃)CH(CH₃)OCH₃ |
| 179 | CH(CH₃)(CH₂)₃OCH₃ |
| 180 | CH(CH₃)CH₂OCH₂CH₃ |
| 181 | CH₂CH(CH₃)OCH₂CH₃ |
| 182 | CH(CH₃)CH(CH₃)OCH₂CH₃ |
| 183 | CH(CH₃) (CH₂)₂OCH₂CH₃ |
| 184 | CH₂CH(CH₃)CH₂OCH₂CH₃ |
| 185 | (CH₂)₂CH(CH₃)OCH₂CH₃ |
| 186 | CH(CH₃)CH(CH₃)CH₂OCH₂CH₃ |
| 187 | CH(CH₃)CH₂CH(CH₃)OCH₂CH₃ |
| 188 | CH₂CH(CH₃)CH(CH₃)OCH₂CH₃ |
| 189 | CH(CH₃)CH(CH₃)CH(CH₃)OCH₂CH₃ |
| 190 | CH(CH₃)(CH₂)₃OCH₂CH₃ |
| 191 | CH(CH₃)CH₂O(CH₂)₂CH₃ |
| 192 | CH₂CH(CH₃)O(CH₂)₂CH₃ |
| 193 | CH(CH₃)CH(CH₃)O(CH₂)₂CH₃ |
| 194 | CH(CH₃)(CH₂)₂O(CH₂)₂CH₃ |
| 195 | CH₂CH(CH₃)CH₂O(CH₂)₂CH₃ |
| 196 | (CH₂)₂CH(CH₃)O(CH₂)₂CH₃ |
| 197 | CH(CH₃)CH(CH₃)CH₂O(CH₂)₂CH₃ |
| 198 | CH(CH₃)CH₂CH(CH₃)O(CH₂)₂CH₃ |
| 199 | CH₂CH(CH₃)CH(CH₃)O(CH₂)₂CH₃ |
| 200 | CH(CH₃)CH(CH₃)CH(CH₃)O(CH₂)₂CH₃ |
| 201 | CH(CH₃)(CH₂)₃O(CH₂)₂CH₃ |
| 202 | CH(CH₃)CH₂OCH(CH₃)₂ |
| 203 | CH₂CH(CH₃)OCH(CH₃)₂ |
| 204 | CH(CH₃)CH(CH₃)OCH(CH₃)₂ |
| 205 | CH(CH₃)(CH₂)₂OCH(CH₃)₂ |
| 206 | CH₂CH(CH₃)CH₂OCH(CH₃)₂ |
| 207 | (CH₂)₂CH(CH₃)OCH(CH₃)₂ |
| 208 | CH(CH₃)CH(CH₃)CH₂OCH(CH₃)₂ |
| 209 | CH(CH₃)CH₂CH(CH₃)OCH(CH₃)₂ |
| 210 | CH₂CH(CH₃)CH(CH₃)OCH(CH₃)₂ |
| 211 | CH(CH₃)CH(CH₃)CH(CH₃)OCH(CH₃)₂ |
| 212 | CH(CH₃)(CH₂)₃OCH(CH₃)₂ |
| 213 | CH(CH₃)CH₂OC(CH₃)₃ |
| 214 | CH₂CH(CH₃)OC(CH₃)₃ |
| 215 | CH(CH₃)CH(CH₃)OC(CH₃)₃ |
| 216 | CH(CH₃)(CH₂)₂OC(CH₃)₃ |
| 217 | CH₂CH(CH₃)CH₂OC(CH₃)₃ |
| 218 | (CH₂)₂CHCH₃)OC(CH₃)₃ |
| 219 | CH(CH₃)CH(CH₃)CH₂OC(CH₃)₃ |
| 220 | CH(CH₃)CH₂CH(CH₃)OC(CH₃)₃ |
| 221 | CH₂CH(CH₃)CH(CH₃)OC(CH₃)₃ |
| 222 | CH(CH₃)CH(CH₃)CH(CH₃)OC(CH₃)₃ |
| 223 | CH(CH₃)(CH₂)₃OC(CH₃)₃ |
| 224 | CH(CH₃)CH₂OCF₃ |
| 225 | CH₂CH(CH₃)OCF₃ |
| 226 | CH(CH₃)CH(CH₃)OCF₃ |
| 227 | CH(CH₃)(CH₂)₂OCF₃ |
| 228 | CH₂CH(CH₃)CH₂OCF₃ |
| 229 | (CH₂)₂CH(CH₃)OCF₃ |
| 230 | CH(CH₃)CH(CH₃)CH₂OCF₃ |
| 231 | CH(CH₃)CH₂CH(CH₃)OCF₃ |
| 232 | CH₂CH(CH₃)CH(CH₃)OCF₃ |
| 233 | CH(CH₃)CH(CH₃)CH(CH₃)OCF₃ |
| 234 | CH(CH₃)(CH₂)₃OCF₃ |
| 235 | CH(CH₃)CH₂SCH₃ |
| 236 | CH₂CH(CH₃)SCH₃ |
| 237 | CH(CH₃)CH(CH₃)SCH₃ |
| 238 | CH(CH₃)(CH₂)₂SCH₃ |
| 239 | CH₂CH(CH₃)CH₂SCH₃ |
| 240 | (CH₂)₂CH(CH₃)SCH₃ |
| 241 | CH(CH₃)CH(CH₃)CH₂SCH₃ |
| 242 | CH(CH₃)CH₂CH(CH₃)SCH₃ |
| 243 | CH₂CH(CH₃)CH(CH₃)SCH₃ |
| 244 | CH(CH₃)CH(CH₃)CH(CH₃)SCH₃ |
| 245 | CH(CH₃)(CH₂)₃SCH₃ |
| 246 | CH(CH₃)CH₂SOCH₃ |
| 247 | CH₂CH(CH₃)SOCH₃ |
| 248 | CH(CH₃)CH(CH₃)SOCH₃ |
| 249 | CH(CH₃)(CH₂)₂SOCH₃ |
| 250 | CH₂CH(CH₃)CH₂SOCH₃ |
| 251 | (CH₂)₂CH(CH₃)SOCH₃ |
| 252 | CH(CH₃)CH(CH₃)CH₂SOCH₃ |
| 253 | CH(CH₃)CH₂CH(CH₃)SOCH₃ |
| 254 | CH₂CH(CH₃)CH(CH₃)SOCH₃ |
| 255 | CH(CH₃)CH(CH₃)CH(CH₃)SOCH₃ |
| 256 | CH(CH₃)(CH₂)₃SOCH₃ |
| 257 | CH(CH₃)CH₂SO₂CH₃ |
| 258 | CH₂CH(CH₃)SO₂CH₃ |
| 259 | CH(CH₃)CH(CH₃)SO₂CH₃ |
| 260 | CH(CH₃)(CH₂)₂SO₂CH₃ |
| 261 | CH₂CH(CH₃)CH₂SO₂CH₃ |
| 262 | (CH₂)₂CH(CH₃)SO₂CH₃ |
| 263 | CH(CH₃)CH(CH₃)CH₂SO₂CH₃ |
| 264 | CH(CH₃)CH₂CH(CH₃)SO₂CH₃ |
| 265 | CH₂CH(CH₃)CH(CH₃)SO₂CH₃ |
| 266 | CH(CH₃)CH(CH₃)CH(CH₃)SO₂CH₃ |
| 267 | CH(CH₃)(CH₂)₃SO₂CH₃ |
| 268 | CH(CH₃)-cyclopropyl |
| 269 | CH(CH₃)CH₂-cyclopropyl |
| 270 | CH₂CH(CH₃)-cyclopropyl |
| 271 | CH(CH₃)CH(CH₃)-cyclopropyl |
| 272 | CH(CH₃)(CH₂)₂-cyclopropyl |
| 273 | CH₂CH(CH₃)CH₂-cyclopropyl |
| 274 | (CH₂)₂CH(CH₃)-cyclopropyl |
| 275 | CH(CH₃)CH(CH₃)CH₂-cyclopropyl |
| 276 | CH(CH₃)CH₂CH(CH₃)-cyclopropyl |
| 277 | CH₂CH(CH₃)CH(CH₃)-cyclopropyl |
| 278 | CH(CH₃)CH(CH₃)CH(CH₃)-cyclopropyl |
| 279 | CH(CH₃)(CH₂)₃-cyclopropyl |
| 280 | CH(CH₃)-cyclopentyl |
| 281 | CH(CH₃)CH₂-cyclopentyl |
| 282 | CH₂CH(CH₃)-cyclopentyl |
| 283 | CH(CH₃)CH(CH₃)-cyclopentyl |
| 284 | CH(CH₃)(CH₂)₂-cyclopentyl |
| 285 | CH₂CH(CH₃)CH₂-cyclopentyl |
| 286 | (CH₂)₂CH(CH₃)-cyclopentyl |
| 287 | CH(CH₃)CH(CH₃)CH₂-cyclopentyl |
| 288 | CH(CH₃)CH₂CH(CH₃)-cyclopentyl |
| 289 | CH₂CH(CH₃)CH(CH₃)-cyclopentyl |
| 290 | CH(CH₃)CH(CH₃)CH(CH₃)-cyclopentyl |
| 291 | CH(CH₃)(CH₂)₃-cyclopentyl |
| 292 | CH(CH₃)-cyclohexyl |
| 293 | CH(CR₃)CR₂-cyclohexyl |
| 294 | CH₂CH(CH₃)-cyclohexyl |
| 295 | CH(CH₃)CH(CH₃)-cyclohexyl |
| 296 | CH(CH₃)(CH₂)₂-cyclohexyl |
| 297 | CH₂CH(CH₃)CH₂-cyclohexyl |
| 298 | (CH₂)₂CH(CH₃)-cyclohexyl |
| 299 | CH(CH₃)CH(CR₃)CH₂-cyclohexyl |
| 300 | CH(CH₃)CH₂CH(CH₃)-cyclohexyl |
| 301 | CH₂CH(CH₃)CH(CH₃)-cyclohexyl |
| 302 | CH(CH₃)CH(CH₃)CH(CH₃)-cyclohexyl |
| 303 | CH(CH₃) (CH₂)₃-cyclohexyl |
| 304 | CH(CH₃)CHF₂ |
| 305 | CF(CH₃)CHF₂ |
| 306 | CH(CH₃)CH₂F |
| 307 | CF(CH₃)CH₃ |
| 308 | CF(CH₃)CF₃ |
| 309 | CH(CH₃)CCl₃ |
| 310 | CH(CH₃)CH₂CF₃ |
| 311 | CH₂CH(CH₃)CF₃ |
| 312 | CH(CH₃)CH(CH₃)CF₃ |
| 313 | CH(CH₃)CF₂CF₃ |
| 314 | CH(CH₃)-phenyl |
| 315 | CH(CH₃)CH₂-phenyl |
| 316 | CH₂CH(CH₃)-phenyl |
| 317 | CH(CH₃)CH(CH₃)-phenyl |
| 318 | CH(CH₃)(CH₂)₂-phenyl |
| 319 | CH₂CH(CH₃)CH₂-phenyl |
| 320 | (CH₂)₂CH(CH₃)-phenyl |
| 321 | CH(CH₃)CH(CH₃)CH₂-phenyl |
| 322 | CH(CH₃)CH₂CH(CH₃)-phenyl |
| 323 | CH₂CH(CH₃)CH(CH₃)-phenyl |
| 324 | CH(CH₃)CH(CH₃)CH(CH₃)-phenyl |
| 325 | CH(CH₃)(CH₂)₃-phenyl |
| 326 | 2-F-C₆H₄-CH₂ |
| 327 | 3-F-C₆H₄-CH₂ |
| 328 | 4-F-C₆H₄-CH₂ |
| 329 | 2,3-F₂-C₆H₃-CH₂ |
| 330 | 2,4-F₂-C₆H₃-CH₂ |
| 331 | 2,5-F₂-C₆H₃-CH₂ |
| 332 | 2,6-F₂-C₆H₃-CH₂ |
| 333 | 3,4-F₂-C₆H₃-CH₂ |
| 334 | 3,5-F₂-C₆H₃-CH₂ |
| 335 | 2-Cl-C₆H₄-CH₂ |
| 336 | 3-Cl-C₆H₄-CH₂ |
| 337 | 4-Cl-C₆H₄-CH₂ |
| 338 | 2,3-Cl₂-C₆H₃-CH₂ |
| 339 | 2,4-Cl₂-C₆H₃-CH₂ |
| 340 | 2,5-Cl₂-C₆H₃-CH₂ |
| 341 | 2,6-Cl₂-C₆H₃-CH₂ |
| 342 | 3,4-Cl₂-C₆H₃-CH₂ |
| 343 | 3,5-Cl₂-C₆H₃-CH₂ |
| 344 | 2,3,4-Cl₃-C₆H₂-CH₂ |
| 345 | 2,3,5-Cl₃-C₆H₂-CH₂ |
| 346 | 2,3,6-Cl₃-C₆H₂-CH₂ |
| 347 | 2,4,5-Cl₃-C₆H₂-CH₂ |
| 348 | 2,4,6-Cl₃-C₆H₂-CH₂ |
| 349 | 3,4,5-Cl₃-C₆H₂-CH₂ |
| 350 | 2-Br-C₆H₄-CH₂ |
| 351 | 3-Br-C₆H₄-CH₂ |
| 352 | 4-Br-C₆H₄-CH₂ |
| 353 | 2,3-Br₂-C₆H₃-CH₂ |
| 354 | 2,4-Br₂-C₆H₃-CH₂ |
| 355 | 2,5-Br₂-C₆H₃-CH₂ |
| 356 | 2,6-Br₂-C₆H₃-CH₂ |
| 357 | 3,4-Br₂-C₆H₃-CH₂ |
| 358 | 3,5-Br₂-C₆H₃-CH₂ |
| 359 | 2-F, 3-Cl-C₆H₃-CH₂ |
| 360 | 2-F, 4-Cl-C₆H₃-CH₂ |
| 361 | 2-F, 5-Cl-C₆H₃-CH₂ |
| 362 | 2-F, 3-Br-C₆H₃-CH₂ |
| 363 | 2-F, 4-Br-C₆H₃-CH₂ |
| 364 | 2-F, 5-Br-C₆H₃-CH₂ |
| 365 | 2-Cl, 3-F-C₆H₃-CH₂ |
| 366 | 2-Cl, 4-F-C₆H₃-CH₂ |
| 367 | 2-Cl, 5-F-C₆H₃-CH₂ |
| 368 | 2-Cl, 3-Br-C₆H₃-CH₂ |
| 369 | 2-Cl, 4-Br-C₆H₃-CH₂ |
| 370 | 2-Cl, 5-Br-C₆H₃-CH₂ |
| 371 | 2-Br, 3-F-C₆H₃-CH₂ |
| 372 | 2-Br, 4-F-C₆H₃-CH₂ |
| 373 | 2-Br, 5-F-C₆H₃-CH₂ |
| 374 | 2-Br, 3-Cl-C₆H₃-CH₂ |
| 375 | 2-Br, 4-Cl-C₆H₃-CH₂ |
| 376 | 2-Br, 5-Cl-C₆H₃-CH₂ |
| 377 | 4-Cl, 3,5-Br₂-C₆H₂-CH₂ |
| 378 | 2-CN-C₆H₄-CH₂ |
| 379 | 3-CN-C₆H₄-CH₂ |
| 380 | 4-CN-C₆H₄-CH₂ |
| 381 | 2-NO₂-C₆H₄-CH₂ |
| 382 | 3-NO₂-C₆H₄-CH₂ |
| 383 | 4-NO₂-C₆H₄-CH₂ |
| 384 | 2-CH₃-C₆H₄-CH₂ |
| 385 | 3-CH₃-C₆H₄-CH₂ |
| 386 | 4-CH₃-C₆H₄-CH₂ |
| 387 | 2,3-(CH₃)₂-C₆H₃-CH₂ |
| 388 | 2,4-(CH₃)₂-C₆H₃-CH₂ |
| 389 | 2,5-(CH₃)₂-C₆H₃-CH₂ |
| 390 | 2,6-(CH₃)₂-C₆H₃-CH₂ |
| 391 | 3,4-(CH₃)₂-C₆H₃-CH₂ |
| 392 | 3,5-(CH₃)₂-C₆H₃-CH₂ |
| 393 | 2-CH₂CH₃-C₆H₄-CH₂ |
| 394 | 3-CH₂CH₃-C₆H₄-CH₂ |
| 395 | 4-CH₂CH₃-C₆H₄-CH₂ |
| 396 | 2-CH(CH₃)₂-C₆H₄-CH₂ |
| 397 | 3-CH(CH₃)₂-C₆H₄-CH₂ |
| 398 | 4-CH(CH₃)₂-C₆H₄-CH₂ |
| 399 | 3-C(CH₃)₃-C₆H₄-CH₂ |
| 400 | 4-C(CH₃)₃-C₆R₄-CH₂ |
| 401 | 2-C₆H₅-C₆H₄-CH₂ |
| 402 | 3-C₆H₅-C₆H₄-CH₂ |
| 403 | 4-C₆H₅-C₆H₄-CH₂ |
| 404 | 2-OCH₃-C₆H₄-CH₂ |
| 405 | 3-OCH₃-C₆H₄-CH₂ |
| 406 | 4-OCH₃-C₆H₄-CH₂ |
| 407 | 2,3-(OCH₃)₂-C₆H₃-CH₂ |
| 408 | 2,4-(OCH₃)₂-C₆H₃-CH₂ |
| 409 | 2,5-(OCH₃)₂-C₆H₃-CH₂ |
| 410 | 2,6-(OCH₃)₂-C₆H₃-CH₂ |
| 411 | 3,4-(OCH₃)₂-C₆H₃-CH₂ |
| 412 | 3,5-(OCH₃)₂-C₆H₃-CH₂ |
| 413 | 3,4,5-(OCH₃)₃-C₆H₂-CH₂ |
| 414 | 2-OCH₂CH₃-C₆H₄-CH₂ |
| 415 | 3-OCH₂CH₃-C₆H₄-CH₂ |
| 416 | 4-OCH₂CH₃-C₆H₄-CH₂ |
| 417 | 2-O(CH₂)₂CH₃-C₆H₄-CH₂ |
| 418 | 3-O(CH₂)₂CH₃-C₆H₄-CH₂ |
| 419 | 4-O(CH₂)₂CH₃-C₆H₄-CH₂ |
| 420 | 2-OCH(CH₃)₂-C₆H₄-CH₂ |
| 421 | 3-OCH(CH₃)₂-C₆H₄-CH₂ |
| 422 | 4-OCH(CH₃)₂-C₆H₄-CH₂ |
| 423 | 3-OC(CH₃)₃-C₆H₄-CH₂ |
| 424 | 4-OC(CH₃)₃-C₆H₄-CH₂ |
| 425 | 2-OCH₂CH=CH₂-C₆H₄-CH₂ |
| 426 | 3-OCH₂CH=CH₂-C₆H₄-CH₂ |
| 427 | 4-OCH₂CH=CH₂-C₆H₄-CH₂ |
| 428 | 2-CF₃-C₆H₄-CH₂ |
| 429 | 3-CF₃-C₆H₄-CH₂ |
| 430 | 4-CF₃-C₆H₄-CH₂ |
| 431 | 2-CO₂CH₃-C₆H₄-CH₂ |
| 432 | 3-CO₂CH₃-C₆H₄-CH₂ |
| 433 | 4-CO₂CH₃-C₆H₄-CH₂ |
| 434 | 2-CO₂CH₂CH₃-C₆H₄-CH₂ |
| 435 | 3-CO₂CH₂CH₃-C₆H₄-CH₂ |
| 436 | 4-CO₂CH₂CH₃-C₆H₄-CH₂ |
| 437 | 2-CONH₂-C₆H₄-CH₂ |
| 438 | 3-CONH₂-C₆H₄-CH₂ |
| 439 | 4-CONH₂-C₆H₄-CH₂ |
| 440 | 2-CON(CH₃)₂-C₆H₄-CH₂ |
| 441 | 3-CON(CH₃)₂-C₆H₄-CH₂ |
| 442 | 4-CON(CH₃)₂-C₆H₄-CH₂ |
| 443 | 2-CONHCH₃-C₆H₄-CH₂ |
| 444 | 3-CONHCH₃-C₆H₄-CH₂ |
| 445 | 4-CONHCH₃-C₆H₄-CH₂ |
| 446 | 2-NH₂-C₆H₄-CH₂ |
| 447 | 3-NH₂-C₆H₄-CH₂ |
| 448 | 4-NH₂-C₆H₄-CH₂ |
| 449 | 2-N(CH₃)₂-C₆H₄-CH₂ |
| 450 | 3-N(CH₃)₂-C₆H₄-CH₂ |
| 451 | 4-N(CH₃)₂-C₆H₄-CH₂ |
| 452 | 2-NHCH₃-C₆H₄-CH₂ |
| 453 | 3-NHCH₃-C₆H₄-CH₂ |
| 454 | 4-NHCH₃-C₆H₄-CH₂ |
| 455 | 2-CSNH₂-C₆H₄-CH₂ |
| 456 | 3-CSNH₂-C₆H₄-CH₂ |
| 457 | 4-CSNH₂-C₆H₄-CH₂ |
| 458 | 2-SCH₃-C₆H₄-CH₂ |
| 459 | 3-SCH₃-C₆H₄-CH₂ |
| 460 | 4-SCH₃-C₆H₄-CH₂ |
| 461 | 2-SOCH₃-C₆H₄-CH₂ |
| 462 | 3-SOCH₃-C₆H₄-CH₂ |
| 463 | 4-SOCH₃-C₆H₄-CH₂ |
| 464 | 2-SO₂CH₃-C₆H₄-CH₂ |
| 465 | 3-SO₂CH₃-C₆H₄-CH₂ |
| 466 | 4-SO₂CH₃-C₆H₄-CH₂ |
| 467 | 2-OCF₃-C₆H₄-CH₂ |
| 468 | 3-OCF₃-C₆H₄-CH₂ |
| 469 | 4-OCF₃-C₆H₄-CH₂ |
| 470 | 2-OCHF₂-C₆H₄-CH₂ |
| 471 | 3-OCHF₂-C₆H₄-CH₂ |
| 472 | 4-OCHF₂-C₆H₄-CH₂ |
| 473 | 3-CF₃, 4-OCF₃-C₆H₃-CH₂ |
| 474 | 2-CH₂CH₂F-C₆H₄-CH₂ |
| 475 | 3-CH₂CH₂F-C₆H₄-CH₂ |
| 476 | 4-CH₂CH₂F-C₆H₄-CH₂ |
| 477 | 2-CH₂CF₃-C₆H₄-CH₂ |
| 478 | 3-CH₂CF₃-C₆H₄-CH₂ |
| 479 | 4-CH₂CF₃-C₆H₄-CH₂ |
| 480 | 2-CF₂CHF₂-C₆H₄-CH₂ |
| 481 | 3-CF₂CHF₂-C₆H₄-CH₂ |
| 482 | 4-CF₂CHF₂-C₆H₄-CH₂ |
| 483 | 2-CHF₂-C₆H₄-CH₂ |
| 484 | 3-CHF₂-C₆H₄-CH₂ |
| 485 | 4-CHF₂-C₆H₄-CH₂ |
| 486 | naphthalin-1-yl-CH₂ |
| 487 | naphthalin-2-yl-CH₂ |
| 488 | pyridin-2-yl-CH₂ |
| 489 | pyridin-3-yl-CH₂ |
| 490 | pyridin-4-yl-CH₂ |
| 491 | 5-CH₃-pyridin-2-yl-CH₂ |
| 492 | 6-CH₃-pyridin-2-yl-CH₂ |
| 493 | 5-CH₃-pyridin-3-yl-CH₂ |
| 494 | 6-CH₃-pyridin-3-yl-CH₂ |
| 495 | 5-OCH₃-pyridin-2-yl-CH₂ |
| 496 | 6-OCH₃-pyridin-2-yl-CH₂ |
| 497 | 5-OCH₃-pyridin-3-yl-CH₂ |
| 498 | 6-OCH₃-pyridin-3-yl-CH₂ |
| 499 | 4-Cl-pyridin-2-yl-CH₂ |
| 500 | 5-Cl-pyridin-2-yl-CH₂ |
| 501 | 6-Cl-pyridin-2-yl-CH₂ |
| 502 | 2-Cl-pyridin-3-yl-CH₂ |
| 503 | 5-Cl-pyridin-3-yl-CH₂ |
| 504 | 6-Cl-pyridin-3-yl-CH₂ |
| 505 | 2-Cl-pyridin-4-yl-CH₂ |
| 506 | 3,5-Cl-pyridin-2-yl-CH₂ |
| 507 | pyrimidin-2-yl-CH₂ |
| 508 | 4-Cl-pyrimidin-2-yl-CH₂ |
| 509 | 5-Cl-pyrimidin-2-yl-CH₂ |
| 510 | 4-CH₃-pyrimidin-2-yl-CH₂ |
| 511 | 5-CH₃-pyrimidin-2-yl-CH₂ |
| 512 | 4-OCH₃-pyrimidin-2-yl-CH₂ |
| 513 | 5-OCH₃-pyrimidin-2-yl-CH₂ |
| 514 | 4-OCH₂CH₃-pyrimidin-2-yl-CH₂ |
| 515 | 5-OCH₂CH₃-pyrimidin-2-yl-CH₂ |
| 516 | pyrimidin-4-yl-CH₂ |
| 517 | 2-Cl-pyrimidin-4-yl-CH₂ |
| 518 | 6-Cl-pyrimidin-4-yl-CH₂ |
| 519 | 2,6-Cl₂-pyrimidin-4-yl-CH₂ |
| 520 | 2-CH₃-pyrimidin-4-yl-CH₂ |
| 521 | 6-CH₃-pyrimidin-4-yl-CH₂ |
| 522 | 2-OCH₃-pyrimidin-4-yl-CH₂ |
| 523 | 6-OCH₃-pyrimidin-4-yl-CH₂ |
| 524 | 2-OCH₂CH₃-pyrimidin-4-yl-CH₂ |
| 525 | 6-OCH₂CH₃-pyrimidin-4-yl-CH₂ |
| 526 | pyrimidin-5-yl-CH₂ |
| 527 | 2-Cl-pyrimidin-5-yl-CH₂ |
| 528 | 2-CH₃-pyrimidin-5-yl-CH₂ |
| 529 | 2-OCH₃-pyrimidin-5-yl-CH₂ |
| 530 | 2-OCH₂CH₃-pyrimidin-5-yl-CH₂ |
| 531 | furan-2-yl-CH₂ |
| 532 | 4-Br-furan-2-yl-CH₂ |
| 533 | 4-Cl-furan-2-yl-CH₂ |
| 534 | 4-CN-furan-2-yl-CH₂ |
| 535 | 4-CH₃-furan-2-yl-CH₂ |
| 536 | 5-Br-furan-2-yl-CH₂ |
| 537 | 5-Cl-furan-2-yl-CH₂ |
| 538 | 5-CN-furan-2-yl-CH₂ |
| 539 | 5-CH₃-furan-2-yl-CH₂ |
| 540 | furan-3-yl-CH₂ |
| 541 | 5-Br-furan-3-yl-CH₂ |
| 542 | 5-Cl-furan-3-yl-CH₂ |
| 543 | 5-CN-furan-3-yl-CH₂ |
| 544 | 5-CH₃-furan-3-yl-CH₂ |
| 545 | thien-2-yl-CH₂ |
| 546 | 4-Br-thien-2-yl-CH₂ |
| 547 | 4-Cl-thien-2-yl-CH₂ |
| 548 | 4-CN-thien-2-yl-CH₂ |
| 549 | 4-CH₃-thien-2-yl-CH₂ |
| 550 | 5-Br-thien-2-yl-CH₂ |
| 551 | 5-Cl-thien-2-yl-CH₂ |
| 552 | 5-CN-thien-2-yl-CH₂ |
| 553 | 5-CH₃-thien-2-yl-CH₂ |
| 554 | thien-3-yl-CH₂ |
| 555 | 5-Br-thien-3-yl-CH₂ |
| 556 | 5-Cl-thien-3-yl-CH₂ |
| 557 | 5-CN-thien-3-yl-CH₂ |
| 558 | 5-CH₃-thien-3-yl-CH₂ |
| 559 | oxazol-2-yl-CH₂ |
| 560 | 4-Br-oxazol-2-yl-CH₂ |
| 561 | 4-Cl-oxazol-2-yl-CH₂ |
| 562 | 4-CN-oxazol-2-yl-CH₂ |
| 563 | 4-CH₃-oxazol-2-yl-CH₂ |
| 564 | 5-Br-oxazol-2-yl-CH₂ |
| 565 | 5-Cl-oxazol-2-yl-CH₂ |
| 566 | 5-CN-oxazol-2-yl-CH₂ |
| 567 | 5-CH₃-oxazol-2-yl-CH₂ |
| 568 | oxazol-4-yl-CH₂ |
| 569 | 2-Br-oxazol-4-yl-CH₂ |
| 570 | 2-Cl-oxazol-4-yl-CH₂ |
| 571 | 2-CN-oxazol-4-yl-CH₂ |
| 572 | 2-CH₃-oxazol-4-yl-CH₂ |
| 573 | 2-C₆H₅-oxazol-4-yl-CH₂ |
| 574 | 5-Br-oxazol-4-yl-CH₂ |
| 575 | 5-Cl-oxazol-4-yl-CH₂ |
| 576 | 5-CN-oxazol-4-yl-CH₂ |
| 577 | 5-CH₃-oxazol-4-yl-CH₂ |
| 578 | oxazol-5-yl-CH₂ |
| 579 | 4-Br-oxazol-5-yl-CH₂ |
| 580 | 4-Cl-oxazol-5-yl-CH₂ |
| 581 | 4-CN-oxazol-5-yl-CH₂ |
| 582 | 4-CH₃-oxazol-5-yl-CH₂ |
| 583 | 2-Br-oxazol-5-yl-CH₂ |
| 584 | 2-Cl-oxazol-5-yl-CH₂ |
| 585 | 2-CN-oxazol-5-yl-CH₂ |
| 586 | 2-CH₃-oxazol-5-yl-CH₂ |
| 587 | isoxazol-3-yl-CH₂ |
| 588 | 4-Br-isoxazol-3-yl-CH₂ |
| 589 | 4-Cl-isoxazol-3-yl-CH₂ |
| 590 | 4-CN-isoxazol-3-yl-CH₂ |
| 591 | 4-CH₃-isoxazol-3-yl-CH₂ |
| 592 | 5-Br-isoxazol-3-yl-CH₂ |
| 593 | 5-Cl-isoxazol-3-yl-CH₂ |
| 594 | 5-CN-isoxazol-3-yl-CH₂ |
| 595 | 5-CH₃-isoxazol-3-yl-CH₂ |
| 596 | isoxazol-4-yl-CH₂ |
| 597 | 3-Br-isoxazol-4-yl-CH₂ |
| 598 | 3-Cl-isoxazol-4-yl-CH₂ |
| 599 | 3-CN-isoxazol-4-yl-CH₂ |
| 600 | 3-CH₃-isoxazol-4-yl-CH₂ |
| 601 | 5-Br-isoxazol-4-yl-CH₂ |
| 602 | 5-Cl-isoxazol-4-yl-CH₂ |
| 603 | 5-CN-isoxazol-4-yl-CH₂ |
| 604 | 5-CH₃-isoxazol-4-yl-CH₂ |
| 605 | 3,5-(CH₃)₂-isoxazol-4-yl-CH₂ |
| 606 | isoxazol-5-yl-CH₂ |
| 607 | 3-Br-isoxazol-5-yl-CH₂z |
| 608 | 3-Cl-isoxazol-5-yl-CH₂ |
| 609 | 3-CN-isoxazol-5-yl-CH₂ |
| 610 | 3-CH₃-isoxazol-5-yl-CH₂ |
| 611 | 3-C₆H₅-isoxazol-5-yl-CH₂ |
| 612 | 4-Cl, 3-C₆H₅-isoxazol-5-yl-CH₂ |
| 613 | 4-Br, 3-C₆H₅-isoxazol-5-yl-CH₂ |
| 614 | 4-Br-isoxazol-5-yl-CH₂ |
| 615 | 4-Cl-isoxazol-5-yl-CH₂ |
| 616 | 4-CN-isoxazol-5-yl-CH₂ |
| 617 | 4-CH₃-isoxazol-5-yl-CH₂ |
| 618 | thiazol-2-yl-CH₂ |
| 619 | 4-Br-thiazol-2-yl-CH₂ |
| 620 | 4-Cl-thiazol-2-yl-CH₂ |
| 621 | 4-CN-thiazol-2-yl-CH₂ |
| 622 | 4-CH₃-thiazol-2-yl-CH₂ |
| 623 | 5-Br-thiazol-2-yl-CH₂ |
| 624 | 5-Cl-thiazol-2-yl-CH₂ |
| 625 | 5-CN-thiazol-2-yl-CH₂ |
| 626 | 5-CH₃-thiazol-2-yl-CH₂ |
| 627 | thiazol-4-yl-CH₂ |
| 628 | 2-Br-thiazol-4-yl-CH₂ |
| 629 | 2-Cl-thiazol-4-yl-CH₂ |
| 630 | 2-CN-thiazol-4-yl-CH₂ |
| 631 | 2-CH₃-thiazol-4-yl-CH₂ |
| 632 | 5-Br-thiazol-4-yl-CH₂ |
| 633 | 5-Cl-thiazol-4-yl-CH₂ |
| 634 | 5-CN-thiazol-4-yl-CH₂ |
| 635 | 5-CH₃-thiazol-4-yl-CH₂ |
| 636 | thiazol-5-yl-CH₂ |
| 637 | 4-Br-thiazol-5-yl-CH₂ |
| 638 | 4-Cl-thiazol-5-yl-CH₂ |
| 639 | 4-CN-thiazol-5-yl-CH₂ |
| 640 | 4-CH₃-thiazol-5-yl-CH₂ |
| 641 | 2-Br-thiazol-5-yl-CH₂ |
| 642 | 2-Cl-thiazol-5-yl-CH₂ |
| 643 | 2-CN-thiazol-5-yl-CH₂ |
| 644 | 2-CH₃-thiazol-5-yl-CH₂ |
| 645 | isothiazol-3-yl-CH₂ |
| 646 | 4-Br-isothiazol-3-yl-CH₂ |
| 647 | 4-Cl-isothiazol-3-yl-CH₂ |
| 648 | 4-CN-isothiazol-3-yl-CH₂ |
| 649 | 4-CH₃-isothiazol-3-yl-CH₂ |
| 650 | 5-Br-isothiazol-3-yl-CH₂ |
| 651 | 5-Cl-isothiazol-3-yl-CH₂ |
| 652 | 5-CN-isothiazol-3-yl-CH₂ |
| 653 | 5-CH₃-isothiazol-3-yl-CH₂ |
| 654 | isothiazol-4-yl-CH₂ |
| 655 | 3-Br-isothiazol-4-yl-CH₂ |
| 656 | 3-Cl-isothiazol-4-yl-CH₂ |
| 657 | 3-CN-isothiazol-4-yl-CH₂ |
| 658 | 3-CH₃-isothiazol-4-yl-CH₂ |
| 659 | 5-Br-isothiazol-4-yl-CH₂ |
| 660 | 5-Cl-isothiazol-4-yl-CH₂ |
| 661 | 5-CN-isothiazol-4-yl-CH₂ |
| 662 | 5-CH₃-isothiazol-4-yl-CH₂ |
| 663 | 3,5-(CH₃)₂-isothiazol-4-yl-CH₂ |
| 664 | isothiazol-5-yl-CH₂ |
| 665 | 3-Br-isothiazol-5-yl-CH₂ |
| 666 | 3-Cl-isothiazol-5-yl-CH₂ |
| 667 | 3-CN-isothiazol-5-yl-CH₂ |
| 668 | 3-CH₃-isothiazol-5-yl-CH₂ |
| 669 | 4-Br-isothiazol-5-yl-CH₂ |
| 670 | 4-Cl-isothiazol-5-yl-CH₂ |
| 671 | 4-CN-isothiazol-5-yl-CH₂ |
| 672 | 4-CH₃-isothiazol-5-yl-CH₂ |
| 673 | imidazol-2-yl-CH₂ |
| 674 | 1-Cl-imidazol-2-yl-CH₂ |
| 675 | 1-Br-imidazol-2-yl-CH₂ |
| 676 | 1-CN-imidazol-2-yl-CH₂ |
| 677 | 1-CH₃-imidazol-2-yl-CH₂ |
| 678 | 4-Cl-imidazol-2-yl-CH₂ |
| 679 | 4-Br-imidazol-2-yl-CH₂ |
| 680 | 4-CN-imidazol-2-yl-CH₂ |
| 681 | 4-CH₃-imidazol-2-yl-CH₂ |
| 682 | 1-CH₃, 5-Cl-imidazol-2-yl-CH₂ |
| 683 | 1,4-(CH₃)₂-imidazol-2-yl-CH₂ |
| 684 | 1,5-(CH₃)₂-imidazol-2-yl-CH₂ |
| 685 | imidazol-4-yl-CH₂ |
| 686 | 2-Cl-imidazol-4-yl-CH₂ |
| 687 | 2-Br-imidazol-4-yl-CH₂ |
| 688 | 2-CN-imidazol-4-yl-CH₂ |
| 689 | 1-CH₃-imidazol-4-yl-CH₂ |
| 690 | 2-CH₃-imidazol-4-yl-CH₂ |
| 691 | 5-Cl-imidazol-4-yl-CH₂ |
| 692 | 5-Br-imidazol-4-yl-CH₂ |
| 693 | 5-CN-imidazol-4-yl-CH₂ |
| 694 | 5-CH₃-imidazol-4-yl-CH₂ |
| 695 | 1-CH₃, 5-Cl-imidazol-4-yl-CH₂ |
| 696 | 1,2-(CH₃)₂-imidazol-4-yl-CH₂ |
| 697 | 1,5-(CH₃)₂-imidazol-4-yl-CH₂ |
| 698 | pyrazol-3-yl-CH₂ |
| 699 | 5-Br-pyrazol-3-yl-CH₂ |
| 700 | 5-Cl-pyrazol-3-yl-CH₂ |
| 701 | 5-CN-pyrazol-3-yl-CH₂ |
| 702 | 5-CH₃-pyrazol-3-yl-CH₂ |
| 703 | 1-C₆H₅-pyrazol-3-yl-CH₂ |
| 704 | 4-Br-pyrazol-3-yl-CH₂ |
| 705 | 4-Cl-pyrazol-3-yl-CH₂ |
| 706 | 4-CN-pyrazol-3-yl-CH₂ |
| 707 | 4-CH₃-pyrazol-3-yl-CH₂ |
| 708 | 1-CH₃-pyrazol-3-yl-CH₂ |
| 709 | 1,4-(CH₃)₂-pyrazol-3-yl-CH₂ |
| 710 | 1,5-(CH₃)₂-pyrazol-3-yl-CH₂ |
| 711 | 1-CH₃, 4-Cl-pyrazol-3-yl-CH₂ |
| 712 | 1-CH₃, 5-Cl-pyrazol-3-yl-CH₂ |
| 713 | pyrazol-4-yl-CH₂ |
| 714 | 3-Br-pyrazol-4-yl-CH₂ |
| 715 | 3-Cl-pyrazol-4-yl-CH₂ |
| 716 | 3-CN-pyrazol-4-yl-CH₂ |
| 717 | 3-CH₃-pyrazol-4-yl-CH₂ |
| 718 | 1-CH₃-pyrazol-4-yl-CH₂ |
| 719 | 1,5-(CH₃)₂-pyrazol-4-yl-CH₂ |
| 720 | 1,3-(CH₃)₂-pyrazol-4-yl-CH₂ |
| 721 | 1-CH₃, 3-Cl-pyrazol-4-yl-CH₂ |
| 722 | 1-CH₃, 5-Cl-pyrazol-4-yl-CH₂ |
| 723 | pyrazol-5-yl-CH₂ |
| 724 | 3-Br-pyrazol-5-yl-CH₂ |
| 725 | 3-Cl-pyrazol-5-yl-CH₂ |
| 726 | 3-CN-pyrazol-5-yl-CH₂ |
| 727 | 3-CH₃-pyrazol-5-yl-CH₂ |
| 728 | 1-CH₃-pyrazol-5-yl-CH₂ |
| 729 | 4-Br-pyrazol-5-yl-CH₂ |
| 730 | 4-Cl-pyrazol-5-yl-CH₂ |
| 731 | 4-CN-pyrazol-5-yl-CH₂ |
| 732 | 4-CH₃-pyrazol-5-yl-CH₂ |
| 733 | 1,3-(CH₃)₂-pyrazol-5-yl-CH₂ |
| 734 | 1,4-(CH₃)₂-pyrazol-5-yl-CH₂ |
| 735 | 1-CH₃, 3-Cl-pyrazol-5-yl-CH₂ |
| 736 | 1-CH₃, 4-Cl-pyrazol-5-yl-CH₂ |
| 737 | 1,3,4-oxadiazol-5-yl-CH₂ |
| 738 | 2-CH₃-1,3,4-oxadiazol-5-yl-CH₂ |
| 739 | 2-CF₃-1,3,4-oxadiazol-5-yl-CH₂ |
| 740 | 2-OCH₃-1,3,4-oxadiazol-5-yl-CH₂ |
| 741 | 2-Cl-1,3,4-oxadiazol-5-yl-CH₂ |
| 742 | 2-CH(CH₃)₂-1,3,4-oxadiazol-5-yl-CH₂ |
| 743 | 1,3,4-oxadiazol-2-yl-CH₂ |
| 744 | 5-CH₃-1,3,4-oxadiazol-2-yl-CH₂ |
| 745 | 5-CF₃-1,3,4-oxadiazol-2-yl-CH₂ |
| 746 | 5-OCH₃-1,3,4-oxadiazol-2-yl-CH₂ |
| 747 | 5-Cl-1,3,4-oxadiazol-2-yl-CH₂ |
| 748 | 5-CH(CH₃)₂-1,3,4-oxadiazol-2-yl-CH₂ |
| 749 | 5-C₆H₅-1,3,4-oxadiazol-2-yl-CH₂ |
| 750 | 1,2,4-oxadiazol-3-yl-CH₂ |
| 751 | 5-CH₃-1,2,4-oxadiazol-3-yl-CH₂ |
| 752 | 5-CF₃-1,2,4-oxadiazol-3-yl-CH₂ |
| 753 | 5-OCH₃-1,2,4-oxadiazol-3-yl-CH₂ |
| 754 | 5-Cl-1,2,4-oxadiazol-3-yl-CH₂ |
| 755 | 5-CH(CH₃)₂-1,2,4-oxadiazol-3-yl-CH₂ |
| 756 | 1,2,4-triazol-3-yl-CH₂ |
| 757 | 1-CH₃-1,2,4-triazol-3-yl-CH₂ |
| 758 | 5-CH₃-1,2,4-triazol-3-yl-CH₂ |
| 759 | 5-CF₃-1,2,4-triazol-3-yl-CH₂ |
| 760 | 5-OCH₃-1,2,4-triazol-3-yl-CH₂ |
| 761 | 5-Cl-1,2,4-triazol-3-yl-CH₂ |
| 762 | 5-CH(CH₃)2-1,2,4-triazol-3-yl-CH₂ |
| 763 | 1-C₆H₅-1,2,4-triazol-3-yl-CH₂ |
| 764 | 1,3,4-thiadiazol-5-yl-CH₂ |
| 765 | 2-CH₃-1,3,4-thiadiazol-5-yl-CH₂ |
| 766 | 2-CF₃-1,3,4-thiadiazol-5-yl-CH₂ |
| 767 | 2-OCH₃-1,3,4-thiadiazol-5-yl-CH₂ |
| 768 | 2-CH₂OCH₃-1,3,4-thiadiazol-5-yl-CH₂ |
| 769 | 2-Cl-1,3,4-thiadiazol-5-yl-CH₂ |
| 770 | 2-CH(CH₃)₂-1,3,4-thiadiazol-5-yl-CH₂ |
| 771 | 1,3,4-thiadiazol-2-yl-CH₂ |
| 772 | 5-CH₃-1,3,4-thiadiazol-2-yl-CH₂ |
| 773 | 5-CF₃-1,3,4-thiadiazol-2-yl-CH₂ |
| 774 | 5-OCH₃-1,3,4-thiadiazol-2-yl-CH₂ |
| 775 | 5-Cl-1,3,4-thiadiazol-2-yl-CH₂ |
| 776 | 5-CH(CH₃)₂-1,3,4-thiadiazol-2-yl-CH₂ |
| 777 | 5-C₆H₅-1,3,4-thiadiazol-2-yl-CH₂ |
| 778 | 1,2,4-thiadiazol-3-yl-CH₂ |
| 779 | 5-CH₃-1,2,4-thiadiazol-3-yl-CH₂ |
| 780 | 5-CF₃-1,2,4-thiadiazol-3-yl-CH₂ |
| 781 | 5-OCH₃-1,2,4-thiadiazol-3-yl-CH₂ |
| 782 | 5-Cl-1,2,4-thiadiazol-3-yl-CH₂ |
| 783 | 5-CH(CH₃)₂-1,2,4-thiadiazol-3-yl-CH₂ |
| 784 | pyrrol-2-yl-CH₂ |
| 785 | 4-Cl-pyrrol-2-yl-CH₂ |
| 786 | 4-Br-pyrrol-2-yl-CH₂ |
| 787 | 4-CH₃-pyrrol-2-yl-CH₂ |
| 788 | 4-C₆H₅-pyrrol-2-yl-CH₂ |
| 789 | benzimidazol-2-yl-CH₂ |
| 790 | chinolin-2-yl-CH₂ |
| 791 | oxiranyl-CH₂ |
| 792 | 2-CH₃-oxiran-2-yl-CH₂ |
| 793 | 2-CH₃-oxiran-3-yl-CH₂ |
| 794 | 2,2-(CH₃)₂-oxiran-3-yl-CH₂ |
| 795 | 2,3-(CH₃)₂-oxiran-3-yl-CH₂ |
| 796 | 2,3,3-(CH₃)₃-oxiran-2-yl-CH₂ |
| 797 | oxiranyl-CH(CH₃) |
| 798 | 2-CH₃-oxiran-2-yl-CH(CH₃) |
| 799 | 2-CH₃-oxiran-3-yl-CH(CH₃) |
| 800 | 2,2-(CH₃)₂-oxiran-3-yl-CH(CH₃) |
| 801 | 2,3-(CH₃)₂-oxiran-3-yl-CH(CH₃) |
| 802 | 2,3,3-(CH₃)₃-oxiran-2-yl-CH(CH₃) |
| 803 | 1,1-Cl₂-cyclopropan-2-yl-CH₂ |
| 804 | 2-CH₃, 1,1-Cl₂-cyclopropan-2-yl-CH₂ |
| 805 | 2-CH₃, 1,1-Cl₂-cyclopropan-3-yl-CH₂ |
| 806 | 2,2-(CH₃)₂, 1,1-Cl₂-cyclopropan-3-yl-CH₂ |
| 807 | 2,3-(CH₃)₂, 1,1-Cl₂-cyclopropan-3-yl-CH₂ |
| 808 | 2,3,3-(CH₃)₃, 1'1-Cl₂-cyclopropan-2-yl-CH₂ |
| 809 | 1,1-Cl₂-cyclopropan-2-yl-CH(CH₃) |
| 810 | 2-CH₃, 1,1-Cl₂-cyclopropan-2-yl-CH(CH₃) |
| 811 | 2-CH₃, 1,1-Cl₂-cyclopropan-3-yl-CH(CH₃) |
| 812 | 2,2-(CH₃)₂, 1,1-Cl₂-cyclopropan-3-yl-CH(CH₃) |
| 813 | 2,3-(CH₃)₂, 1,1-Cl₂-cyclopropan-3-yl-CH(CH₃) |
| 814 | 2,3,3-(CH₃)₃, 1,1-Cl₂-cyclopropan-2-yl-CH(CH₃) |
| 815 | 1,1-Br₂-cyclopropan-2-yl-CH₂ |
| 816 | 2-CH₃, 1,1-Br₂-cyclopropan-2-yl-CH₂ |
| 817 | 2-CH₃, 1,1-Br₂-cyclopropan-3-yl-CH₂ |
| 818 | 2,2-(CH₃)₂, 1,1-Br₂-cyclopropan-3-yl-CH₂ |
| 819 | 2,3-(CH₃)₂, 1,1-Br₂-cyclopropan-3-yl-CH₂ |
| 820 | 2,3,3-(CH₃)₃, 1,1-Br₂-cyclopropan-2-yl-CH₂ |
| 821 | 1,1-Br₂-cyclopropan-2-yl-CH(CH₃) |
| 822 | 2-CH₃, 1,1-Br₂-cyclopropan-2-yl-CH(CH₃) |
| 823 | 2-CH₃, 1,1-Br₂-cyclopropan-3-yl-CH(CH₃) |
| 824 | 2,2-(CH₃)₂, 1,1-Br₂-cyclopropan-3-yl-CH(CH₃) |
| 825 | 2,3-(CH₃)₂, 1,1-Br₂-cyclopropan-3-yl-CH(CH₃) |
| 826 | 2,3,3-(CH₃)₃, 1,1-Br₂-cyclopropan-2-yl-CH (CH₃) |
| 827 | CH₂CH=CH₂ |
| 828 | CH₂CCl=CH₂ |
| 829 | CH₂CH=CHCl (E) |
| 830 | CH₂CH=CHCl (Z) |
| 831 | CH₂CCl=CHCl (E) |
| 832 | CH₂CCl=CHCl (Z) |
| 833 | CH₂CH=CCl₂ |
| 834 | CH₂CCl=CCl₂ |
| 835 | CH₂CBr=CH₂ |
| 836 | CH₂CH=CHBr (E) |
| 837 | CH₂CH=CHBr (Z) |
| 838 | CH₂CBr=CHBr (E) |
| 839 | CH₂CBr=CHBr (Z) |
| 840 | CH₂CH=CBr₂ |
| 841 | CH₂CBr=CBr₂ |
| 842 | CH₂C(CH₃)=CH₂ |
| 843 | CH₂CH=CHCH₃ (E) |
| 844 | CH₂CH=CHCH₃ (Z) |
| 845 | CH₂C(CH₃)=CHCH₃ (E) |
| 846 | CH₂C(CH₃)=CHCH₃ (Z) |
| 847 | CH₂CH=C(CH₃)₂ |
| 848 | CH₂CH₂CH=CH₂ |
| 849 | CH₂CCl=CHCH₃ (E) |
| 850 | CH₂CCl=CHCH₃ (Z) |
| 851 | CH₂CH=CClCH₃ (E) |
| 852 | CH₂CH=CClCH₃ (Z) |
| 853 | CH₂C(CH₃)=C(CH₃)₂ |
| 854 | CH₂CBr=CHCH₃ (E) |
| 855 | CH₂CBr=CHCH₃ (Z) |
| 856 | CH₂CH=CBrCH₃ (E) |
| 857 | CH₂CH=CBrCH₃ (Z) |
| 858 | CH₂CH=CHCH₂Cl (E) |
| 859 | CH₂CH=CHCH₂Cl (Z) |
| 860 | CH₂CH=CHCH₂CH₃ (E) |
| 861 | CH₂CH=CHCH₂CH₃ (Z) |
| 862 | CH₂CH=CHCH₂Br (E) |
| 863 | CH₂CH=CHCH₂Br (Z) |
| 864 | CH₂CCl=CClCH₂Cl (E) |
| 865 | CH₂CCl=CClCH₂Cl (Z) |
| 866 | CH₂CF=CH₂ |
| 867 | CH₂CH=CHF (E) |
| 868 | CH₂CH=CHF (Z) |
| 869 | CH₂CH=CF₂ |
| 870 | CH₂CF=CHF (E) |
| 871 | CH₂CF=CHF (Z) |
| 872 | CH(CH₃)CH=CH₂ |
| 873 | CH (CH₃) CCI=CH₂ |
| 874 | CH(CH₃)CH=CHCl (E) |
| 875 | CH(CH₃)CH=CHCl (Z) |
| 876 | CH(CH₃)CCl=CHCl (E) |
| 877 | CH(CH₃)CCl=CHCl (Z) |
| 878 | CH(CH₃)CH=CCl₂ |
| 879 | CH(CH₃)CCl=CCl₂ |
| 880 | CH(CH₃)CBr=CH₂ |
| 881 | CH(CH₃)CH=CHBr (E) |
| 882 | CH(CH₃)CH=CHBr (Z) |
| 883 | CH(CH₃)CBr=CHBr (E) |
| 884 | CH(CH₃)CBr=CHBr (Z) |
| 885 | CH(CH₃)CH=CBr₂ |
| 886 | CH(CH₃)CBr=CBr₂ |
| 887 | CH(CH₃)C(CH₃)=CH₂ |
| 888 | CH(CH₃)CH=CHCH₃ (E) |
| 889 | CH(CH₃)CH=CHCH₃ (Z) |
| 890 | CH(CH₃)C(CH₃)=CHCH₃ (E) |
| 891 | CH(CH₃)C(CH₃)=CHCH₃ (Z) |
| 892 | CH(CH₃)CH=C(CH₃)₂ |
| 893 | CH(CH₃)CCl=CHCH₃ (E) |
| 894 | CH(CH₃)CCl=CHCH₃ (Z) |
| 895 | CH(CH₃)CH=CClCH₃ (E) |
| 896 | CH(CH₃)CH=CClCH₃ (Z) |
| 897 | CH(CH₃)CBr=CHCH₃ (E) |
| 898 | CH(CH₃)CBr=CHCH₃ (Z) |
| 899 | CH(CH₃)CH=CBrCH₃ (E) |
| 900 | CH(CH₃)CH=CBrCH₃ (Z) |
| 901 | CH(CH₃)CH=CHCH₂Cl (E) |
| 902 | CH(CH₃)CH=CHCH₂Cl (Z) |
| 903 | CH(CH₃)CH=CHCH₂CH₃ (E) |
| 904 | CH(CH₃)CH=CHCH₂CH₃ (Z) |
| 905 | CH(CH₃)CH=CHCH₂Br (E) |
| 906 | CH(CH₃)CH=CHCH₂Br (Z) |
| 907 | CH(CH₃)CCl=CClCH₂Cl (E) |
| 908 | CH(CH₃)CCl=CClCH₂Cl (Z) |
| 909 | CH(CH₃)CF=CH₂ |
| 910 | CH(CH₃)CH=CHF (E) |
| 911 | CH(CH₃)CH=CHF (Z) |
| 912 | CH(CH₃)CH=CF₂ |
| 913 | CH(CH₃)CF=CHF (E) |
| 914 | CH(CH₃)CF=CHF (Z) |
| 915 | CH₂CHClCH=CH₂ |
| 916 | CH₂CH₂CH=C(CH₃)₂ |
| 917 | CH₂CH₂C(CH₃)=CHCH₃ (E) |
| 918 | CH₂CH₂C(CH₃)=CHCH₃ (Z) |
| 919 | CH₂C≡CH |
| 920 | CH₂C≡CCl |
| 921 | CH₂C≡CBr |
| 922 | CH₂C≡CI |
| 923 | CH₂C≡CCH₃ |
| 924 | CH₂C≡CCH₂CH₃ |
| 925 | CH₂C≡CCH₂OH |
| 926 | CH₂C≡CCH₂NH₂ |
| 927 | CH₂C≡CCH₂Cl |
| 928 | CH₂C≡CCH₂OCH₃ |
| 929 | CH₂C≡CCH₂OCH₂CH₃ |
| 930 | CH₂C≡CCH₂SCH₃ |
| 931 | CH₂C≡CCH₂N(CH₃)₂ |
| 932 | CH₂C≡CCC₆H₅ |
| 933 | CH₂CH₂C≡CH |
| 934 | CH₂CH₂C≡CCl |
| 935 | CH₂CH₂C≡CBr |
| 936 | CH₂CH₂C≡CI |
| 937 | CH₂CH₂C≡CCH₃ |
| 938 | CH₂CH₂C≡CCH₂CH₃ |
| 939 | CH₂CH₂C≡CCH₂OH |
| 940 | CH₂CH₂C≡CCH₂NH₂ |
| 941 | CH₂CH₂C≡CCH₂Cl |
| 942 | CH₂CH₂C≡CCH₂OCH₃ |
| 943 | CH₂CH₂C≡CCH₂OCH₂CH₃ |
| 944 | CH₂CH₂C≡CCH₂SCH₃ |
| 945 | CH₂CH₂C≡CCH₂N(CH₃)₂ |
| 946 | CH₂CH₂C≡CC₆H₅ |
| 947 | CH₂CH₂CH₂C≡CH |
| 948 | CH₂CH₂CH₂C≡CCl |
| 949 | CH₂CH₂CH₂C≡CBr |
| 950 | CH₂CH₂CH₂C≡CI |
| 951 | CH₂CH₂CH₂C≡CCH₃ |
| 952 | CH₂CH₂CH₂C≡CCH₂CH₃ |
| 953 | CH₂CH₂CH₂C≡CCH₂OH |
| 954 | CH₂CH₂CH₂C≡CCH₂NH₂ |
| 955 | CH₂CH₂CH₂C≡CCH₂Cl |
| 956 | CH₂CH₂CH₂C≡CCH₂OCH₃ |
| 957 | CH₂CH₂CH₂C≡CCH₂OCH₂CH₃ |
| 958 | CH₂CH₂CH₂C≡CCH₂SCH₃ |
| 959 | CH₂CH₂CH₂C≡CCH₂N(CH₃)₂ |
| 960 | CH₂CH₂CH₂C≡CC₆H₅ |
| 961 | CH(CH₃)C≡CH |
| 962 | CH(CH₃)C≡CCl |
| 963 | CH(CH₃)C≡CBr |
| 964 | CH(CH₃)C≡CI |
| 965 | CH(CH₃)C≡CCH₃ |
| 966 | CH(CH₃)C≡CCH₂CH₃ |
| 967 | CH(CH₃)C≡CCH₂OH |
| 968 | CH(CH₃)C≡CCH₂NH₂ |
| 969 | CH(CH₃)C≡CCH₂Cl |
| 970 | CH(CH₃)C≡CCH₂OCH₃ |
| 971 | CH(CH₃)C≡CCH₂OCH₂CH₃ |
| 972 | CH(CH₃)C≡CCH₂SCH₃ |
| 973 | CH(CH₃)C≡CCH₂N(CH₃)₂ |
| 974 | CH(CH₃)C≡CC₆H₅ |

Tabelle 1: (Verbindungen 1.1 - 1.974)
Verbindungen der allgemeinen Formel I.1, in denen
R³ = Methyl
R⁴ = CH₃
R⁵ = H
bedeutet und der Substituent R⁶ für eine Verbindung einer Zeile der Tabelle A entspricht.

Tabelle 2: (Verbindungen 2.1 - 2.974)
Verbindungen der allgemeinen Formel I.2, in denen
R³ = Methyl
R⁴ = CH₃
R⁵ = H
bedeutet und der Substituent R⁶ für eine Verbindung einer Zeile der Tabelle A entspricht.

Tabelle 3: (Verbindungen 3.1 - 3.974)
Verbindungen der allgemeinen Formel I.3, in denen
R³ = Methyl
R⁴ = CH₃
R⁵ = H
bedeutet und der Substituent R⁶ für eine Verbindung einer Zeile der Tabelle A entspricht.

Tabelle 4: (Verbindungen 4.1 - 4.974)
Verbindungen der allgemeinen Formel I.4, in denen
R³ = Methyl
R⁴ = CH₃
R⁵ = H
bedeutet und der Substituent R⁶ für eine Verbindung einer Zeile der Tabelle A entspricht.

Tabelle 5: (Verbindungen 5.1 - 5.974)
Verbindungen der allgemeinen Formel I.1, in denen
R³ = Methyl
R⁴ = CH₃
R⁵ = CH₃
bedeutet und der Substituent R⁶ für eine Verbindung einer Zeile der Tabelle A entspricht.

Tabelle 6: (Verbindungen 6.1 - 6.974)
Verbindungen der allgemeinen Formel I.2, in denen
R³ = Methyl
R⁴ = CH₃
R⁵ = CH₃
bedeutet und der Substituent R⁶ für eine Verbindung einer Zeile der Tabelle A entspricht.

Tabelle 7: (Verbindungen 7.1 - 7.974)
Verbindungen der allgemeinen Formel I.3, in denen
R³ = Methyl
R⁴ = CH₃
R⁵ = CH₃
bedeutet und der Substituent R⁶ für eine Verbindung einer Zeile der Tabelle A entspricht.

Tabelle 8: (Verbindungen 8.1 - 8.974)
Verbindungen der allgemeinen Formel I.4, in denen
R³ = Methyl
R⁴ = CH₃
R⁵ = CH₃
bedeutet und der Substituent R⁶ für eine Verbindung einer Zeile der Tabelle A entspricht.

Tabelle 9: (Verbindungen 9.1 - 9.974)
Verbindungen der allgemeinen Formel I.1, in denen
R³ = Methyl
R⁴ = CH₃
R⁵ = C₂H₅
bedeutet und der Substituent R⁶ für eine Verbindung einer Zeile der Tabelle A entspricht.

Tabelle 10: (Verbindungen 10.1 - 10.974)
Verbindungen der allgemeinen Formel I.2, in denen
R³ = Methyl
R⁴ = CH₃
R⁵ = C₂H₅
bedeutet und der Substituent R⁶ für eine Verbindung einer Zeile der Tabelle A entspricht.

Tabelle 11: (Verbindungen 11.1 - 11.974)
Verbindungen der allgemeinen Formel I.3, in denen
R³ = Methyl
R⁴ = CH₃
R⁵ = C₂H₅
bedeutet und der Substituent R⁶ für eine Verbindung einer Zeile der Tabelle A entspricht.

Tabelle 12: (Verbindungen 12.1 - 12.974)
Verbindungen der allgemeinen Formel I.4, in denen
R³ = Methyl
R⁴ = CH₃
R⁵ = C₂H₅
bedeutet und der Substituent R⁶ für eine Verbindung einer Zeile der Tabelle A entspricht.

Tabelle 13: (Verbindungen 13.1 - 13.974)
Verbindungen der allgemeinen Formel I.1, in denen
R³ = Methyl
R⁴ = CH₃
R⁵ = n-C₃H₇
bedeutet und der Substituent R⁶ für eine Verbindung einer Zeile der Tabelle A entspricht.

Tabelle 14: (Verbindungen 14.1 - 14.974)
Verbindungen der allgemeinen Formel I.2, in denen
R³ = Methyl
R⁴ = CH₃
R⁵ = n-C₃H₇
bedeutet und der Substituent R⁶ für eine Verbindung einer Zeile der Tabelle A entspricht.

Tabelle 15: (Verbindungen 15.1 - 15.974)
Verbindungen der allgemeinen Formel I.3, in denen
R³ = Methyl
R⁴ = CH₃
R⁵ = n-C₃H₇
bedeutet und der Substituent R⁶ für eine Verbindung einer Zeile der Tabelle A entspricht.

Tabelle 16: (Verbindungen 16.1 - 16.974)
Verbindungen der allgemeinen Formel I.4, in denen
R³ = Methyl
R⁴ = CH₃
R⁵ = n-C₃H₇
bedeutet und der Substituent R⁶ für eine Verbindung einer Zeile der Tabelle A entspricht.

Tabelle 17: (Verbindungen 17.1 - 17.974)
Verbindungen der allgemeinen Formel I.1, in denen
R³ = Methyl
R⁴ = CH₃
R⁵ = i-C₃H₇
bedeutet und der Substituent R⁶ für eine Verbindung einer Zeile der Tabelle A entspricht.

Tabelle 18: (Verbindungen 18.1 - 18.974)
Verbindungen der allgemeinen Formel I.2, in denen
R³ = Methyl
R⁴ = CH₃
R⁵ = i-C₃H₇
bedeutet und der Substituent R⁶ für eine Verbindung einer Zeile der Tabelle A entspricht.

Tabelle 19: (Verbindungen 19.1 - 19.974)
Verbindungen der allgemeinen Formel I.3, in denen
R³ = Methyl
R⁴ = CH₃
R⁵ = i-C₃H₇
bedeutet und der Substituent R⁶ für eine Verbindung einer Zeile der Tabelle A entspricht.

Tabelle 20: (Verbindungen 20.1 - 20.974)
Verbindungen der allgemeinen Formel I.4, in denen
R³ = Methyl
R⁴ = CH₃
R⁵ = i-C₃H₇
bedeutet und der Substituent R⁶ für eine Verbindung einer Zeile der Tabelle A entspricht.

Tabelle 21: (Verbindungen 21.1 - 21.974)
Verbindungen der allgemeinen Formel I.1, in denen
R³ = Methyl
R⁴ = CH₃
R⁵ = Cyclopropyl
bedeutet und der Substituent R⁶ für eine Verbindung einer Zeile der Tabelle A entspricht.

Tabelle 22: (Verbindungen 22.1 - 22.974)
Verbindungen der allgemeinen Formel I.2, in denen
R³ = Methyl
R⁴ = CH₃
R⁵ = Cyclopropyl
bedeutet und der Substituent R⁶ für eine Verbindung einer Zeile der Tabelle A entspricht.

Tabelle 23: (Verbindungen 23.1 - 23.974)
Verbindungen der allgemeinen Formel I.3, in denen
R³ = Methyl
R⁴ = CH₃
R⁵ = Cyclopropyl
bedeutet und der Substituent R⁶ für eine Verbindung einer Zeile der Tabelle A entspricht.

Tabelle 24: (Verbindungen 24.1 - 24.974)
Verbindungen der allgemeinen Formel I.4, in denen
R³ = Methyl
R⁴ = CH₃
R⁵ = Cyclopropyl
bedeutet und der Substituent R⁶ für eine Verbindung einer Zeile der Tabelle A entspricht.

Tabelle 25: (Verbindungen 25.1 - 25.974)
Verbindungen der allgemeinen Formel I.1, in denen
R³ = Methyl
R⁴ = CH₃
R⁵ = CN
bedeutet und der Substituent R⁶ für eine Verbindung einer Zeile der Tabelle A entspricht.

Tabelle 26: (Verbindungen 26.1 - 26.974)
Verbindungen der allgemeinen Formel I.2, in denen
R³ = Methyl
R⁴ = CH₃
R⁵ = CN
bedeutet und der Substituent R⁶ für eine Verbindung einer Zeile der Tabelle A entspricht.

Tabelle 27: (Verbindungen 27.1 - 27.974)
Verbindungen der allgemeinen Formel I.3, in denen
R³ = Methyl
R⁴ = CH₃
R⁵ = CN
bedeutet und der Substituent R⁶ für eine Verbindung einer Zeile der Tabelle A entspricht.

Tabelle 28: (Verbindungen 28.1 - 28.974)
Verbindungen der allgemeinen Formel I.4, in denen
R³ = Methyl
R⁴ = CH₃
R⁵ = CN
bedeutet und der Substituent R⁶ für eine Verbindung einer Zeile der Tabelle A entspricht.

Tabelle 29: (Verbindungen 29.1 - 29.974)
Verbindungen der allgemeinen Formel I.1, in denen
R³ = Methyl
R⁴ = CH₃
R⁵ = C₆H₅
bedeutet und der Substituent R⁶ für eine Verbindung einer Zeile der Tabelle A entspricht.

Tabelle 30: (Verbindungen 30.1 - 30.974)
Verbindungen der allgemeinen Formel I.2, in denen
R³ = Methyl
R⁴ = CH₃
R⁵ = C₆H₅
bedeutet und der Substituent R⁶ für eine Verbindung einer Zeile der Tabelle A entspricht.

Tabelle 31: (Verbindungen 31.1 - 31.974)
Verbindungen der allgemeinen Formel I.3, in denen
R³ = Methyl
R⁴ = CH₃
R⁵ = C₆H₅
bedeutet und der Substituent R⁶ für eine Verbindung einer Zeile der Tabelle A entspricht.

Tabelle 32: (Verbindungen 32.1 - 32.974)
Verbindungen der allgemeinen Formel I.4, in denen
R³ = Methyl
R⁴ = CH₃
R⁵ = C₆H₅
bedeutet und der Substituent R⁶ für eine Verbindung einer Zeile der Tabelle A entspricht.

Tabelle 33: (Verbindungen 33.1 - 33.974)
Verbindungen der allgemeinen Formel I.1, in denen
R³ = Methyl
R⁴ = CH₃
R⁵ = 2-F-C₆H₄
bedeutet und der Substituent R⁶ für eine Verbindung einer Zeile der Tabelle A entspricht.

Tabelle 34: (Verbindungen 34.1 - 34.974)
Verbindungen der allgemeinen Formel I.2, in denen
R³ = Methyl
R⁴ = CH₃
R⁵ = 2-F-C₆H₄
bedeutet und der Substituent R⁶ für eine Verbindung einer Zeile der Tabelle A entspricht.

Tabelle 35: (Verbindungen 35.1 - 35.974)
Verbindungen der allgemeinen Formel I.3, in denen
R³ = Methyl
R⁴ = CH₃
R⁵ = 2-F-C₆H₄
bedeutet und der Substituent R⁶ für eine Verbindung einer Zeile der Tabelle A entspricht.

Tabelle 36: (Verbindungen 36.1 - 36.974)
Verbindungen der allgemeinen Formel I.4, in denen
R³ = Methyl
R⁴ = CH₃
R⁵ = 2-F-C₆H₄
bedeutet und der Substituent R⁶ für eine Verbindung einer Zeile der Tabelle A entspricht.

Tabelle 37: (Verbindungen 37.1 - 37.974)
Verbindungen der allgemeinen Formel I.1, in denen
R³ = Methyl
R⁴ = CH₃
R⁵ = 3-F-C₆H₄
bedeutet und der Substituent R⁶ für eine Verbindung einer Zeile der Tabelle A entspricht.

Tabelle 38: (Verbindungen 38.1 - 38.974)
Verbindungen der allgemeinen Formel I.2, in denen
R³ = Methyl
R⁴ = CH₃
R⁵ = 3-F-C₆H₄
bedeutet und der Substituent R⁶ für eine Verbindung einer Zeile der Tabelle A entspricht.

Tabelle 39: (Verbindungen 39.1 - 39.974)
Verbindungen der allgemeinen Formel I.3, in denen
R³ = Methyl
R⁴ = CH₃
R⁵ = 3-F-C₆H₄
bedeutet und der Substituent R⁶ für eine Verbindung einer Zeile der Tabelle A entspricht.

Tabelle 40: (Verbindungen 40.1 - 40.974)
Verbindungen der allgemeinen Formel I.4, in denen
R³ = Methyl
R⁴ = CH₃
R⁵ = 3-F-C₆H₄
bedeutet und der Substituent R⁶ für eine Verbindung einer Zeile der Tabelle A entspricht.

Tabelle 41: (Verbindungen 41.1 - 41.974)
Verbindungen der allgemeinen Formel I.1, in denen
R³ = Methyl
R⁴ = CH₃
R⁵ = 4-F-C₆H₄
bedeutet und der Substituent R⁶ für eine Verbindung einer Zeile der Tabelle A entspricht.

Tabelle 42: (Verbindungen 42.1 - 42.974)
Verbindungen der allgemeinen Formel I.2, in denen
R³ = Methyl
R⁴ = CH₃
R⁵ = 4-F-C₆H₄
bedeutet und der Substituent R⁶ für eine Verbindung einer Zeile der Tabelle A entspricht.

Tabelle 43: (Verbindungen 43.1 - 43.974)
Verbindungen der allgemeinen Formel I.3, in denen
R³ = Methyl
R⁴ = CH₃
R⁵ = 4-F-C₆H₄
bedeutet und der Substituent R⁶ für eine Verbindung einer Zeile der Tabelle A entspricht.

Tabelle 44: (Verbindungen 44.1 - 44.974)
Verbindungen der allgemeinen Formel I.4, in denen
R³ = Methyl
R⁴ = CH₃
R⁵ = 4-F-C₆H₄
bedeutet und der Substituent R⁶ für eine Verbindung einer Zeile der Tabelle A entspricht.

Tabelle 45: (Verbindungen 45.1 - 45.974)
Verbindungen der allgemeinen Formel I.1, in denen
R³ = Methyl
R⁴ = CH₃
R⁵ = 2-Cl-C₆H₄
bedeutet und der Substituent R⁶ für eine Verbindung einer Zeile der Tabelle A entspricht.

Tabelle 46: (Verbindungen 46.1 - 46.974)
Verbindungen der allgemeinen Formel I.2, in denen
R³ = Methyl
R⁴ = CH₃
R⁵ = 2-Cl-C₆H₄
bedeutet und der Substituent R⁶ für eine Verbindung einer Zeile der Tabelle A entspricht.

Tabelle 47: (Verbindungen 47.1 - 47.974)
Verbindungen der allgemeinen Formel I.3, in denen
R³ = Methyl
R⁴ = CH₃
R⁵ = 2-Cl-C₆H₄
bedeutet und der Substituent R⁶ für eine Verbindung einer Zeile der Tabelle A entspricht.

Tabelle 48: (Verbindungen 48.1 - 48.974)
Verbindungen der allgemeinen Formel I.4, in denen
R³ = Methyl
R⁴ = CH₃
R⁵ = 2-Cl-C₆H₄
bedeutet und der Substituent R⁶ für eine Verbindung einer Zeile der Tabelle A entspricht.

Tabelle 49: (Verbindungen 49.1 - 49.974)
Verbindungen der allgemeinen Formel I.1, in denen
R³ = Methyl
R⁴ = CH₃
R⁵ = 3-Cl-C₆H₄
bedeutet und der Substituent R⁶ für eine Verbindung einer Zeile der Tabelle A entspricht.

Tabelle 50: (Verbindungen 50.1 - 50.974)
Verbindungen der allgemeinen Formel I.2, in denen
R³ = Methyl
R⁴ = CH₃
R⁵ = 3-Cl-C₆H₄
bedeutet und der Substituent R⁶ für eine Verbindung einer Zeile der Tabelle A entspricht.

Tabelle 51: (Verbindungen 51.1 - 51.974)
Verbindungen der allgemeinen Formel I.3, in denen
R³ = Methyl
R⁴ = CH₃
R⁵ = 3-Cl-C₆H₄
bedeutet und der Substituent R⁶ für eine Verbindung einer Zeile der Tabelle A entspricht.

Tabelle 52: (Verbindungen 52.1 - 52.974)
Verbindungen der allgemeinen Formel I.4, in denen
R³ = Methyl
R⁴ = CH₃
R⁵ = 3-Cl-C₆H₄
bedeutet und der Substituent R⁶ für eine Verbindung einer Zeile der Tabelle A entspricht.

Tabelle 53: (Verbindungen 53.1 - 53.974)
Verbindungen der allgemeinen Formel I.1, in denen
R³ = Methyl
R⁴ = CH₃
R⁵ = 4-Cl-C₆H₄
bedeutet und der Substituent R⁶ für eine Verbindung einer Zeile der Tabelle A entspricht.

Tabelle 54: (Verbindungen 54.1 - 54.974)
Verbindungen der allgemeinen Formel I.2, in denen
R³ = Methyl
R⁴ = CH₃
R⁵ = 4-Cl-C₆H₄
bedeutet und der Substituent R⁶ für eine Verbindung einer Zeile der Tabelle A entspricht.

Tabelle 55: (Verbindungen 55.1 - 55.974)
Verbindungen der allgemeinen Formel I.3, in denen
R³ = Methyl
R⁴ = CH₃
R⁵ = 4-Cl-C₆H₄
bedeutet und der Substituent R⁶ für eine Verbindung einer Zeile der Tabelle A entspricht.

Tabelle 56: (Verbindungen 56.1 - 56.974)
Verbindungen der allgemeinen Formel I.4, in denen
R³ = Methyl
R⁴ = CH₃
R⁵ = 4-Cl-C₆H₄
bedeutet und der Substituent R⁶ für eine Verbindung einer Zeile der Tabelle A entspricht.

Tabelle 57: (Verbindungen 57.1 - 57.974)
Verbindungen der allgemeinen Formel I.1, in denen
R³ = Methyl
R⁴ = CH₃
R⁵ = 2-Br-C₆H₄
bedeutet und der Substituent R⁶ für eine Verbindung einer Zeile der Tabelle A entspricht.

Tabelle 58: (Verbindungen 58.1 - 58.974)
Verbindungen der allgemeinen Formel I.2, in denen
R³ = Methyl
R⁴ = CH₃
R⁵ = 2-Br-C₆H₄
bedeutet und der Substituent R⁶ für eine Verbindung einer Zeile der Tabelle A entspricht.

Tabelle 59: (Verbindungen 59.1 - 59.974)
Verbindungen der allgemeinen Formel I.3, in denen
R³ = Methyl
R⁴ = CH₃
R⁵ = 2-Br-C₆H₄
bedeutet und der Substituent R⁶ für eine Verbindung einer Zeile der Tabelle A entspricht.

Tabelle 60: (Verbindungen 60.1 - 60.974)
Verbindungen der allgemeinen Formel I.4, in denen
R³ = Methyl
R⁴ = CH₃
R⁵ = 2-Br-C₆H₄
bedeutet und der Substituent R⁶ für eine Verbindung einer Zeile der Tabelle A entspricht.

Tabelle 61: (Verbindungen 61.1 - 61.974)
Verbindungen der allgemeinen Formel I.1, in denen
R³ = Methyl
R⁴ = CH₃
R⁵ = 3-Br-C₆H₄
bedeutet und der Substituent R⁶ für eine Verbindung einer Zeile der Tabelle A entspricht.

Tabelle 62: (Verbindungen 62.1 - 62.974)
Verbindungen der allgemeinen Formel I.2, in denen
R³ = Methyl
R⁴ = CH₃
R⁵ = 3-Br-C₆H₄
bedeutet und der Substituent R⁶ für eine Verbindung einer Zeile der Tabelle A entspricht.

Tabelle 63: (Verbindungen 63.1 - 63.974)
Verbindungen der allgemeinen Formel I.3, in denen
R³ = Methyl
R⁴ = CH₃
R⁵ = 3-Br-C₆H₄
bedeutet und der Substituent R⁶ für eine Verbindung einer Zeile der Tabelle A entspricht.

Tabelle 64: (Verbindungen 64.1 - 64.974)
Verbindungen der allgemeinen Formel I.4, in denen
R³ = Methyl
R⁴ = CH₃
R⁵ = 3-Br-C₆H₄
bedeutet und der Substituent R⁶ für eine Verbindung einer Zeile der Tabelle A entspricht.

Tabelle 65: (Verbindungen 65.1 - 65.974)
Verbindungen der allgemeinen Formel I.1, in denen
R³ = Methyl
R⁴ = CH₃
R⁵ = 4-Br-C₆H₄
bedeutet und der Substituent R⁶ für eine Verbindung einer Zeile der Tabelle A entspricht.

Tabelle 66: (Verbindungen 66.1 - 66.974)
Verbindungen der allgemeinen Formel I.2, in denen
R³ = Methyl
R⁴ = CH₃
R⁵ = 4-Br-C₆H₄
bedeutet und der Substituent R⁶ für eine Verbindung einer Zeile der Tabelle A entspricht.

Tabelle 67: (Verbindungen 67.1 - 67.974)
Verbindungen der allgemeinen Formel I.3, in denen
R³ = Methyl
R⁴ = CH₃
R⁵ = 4-Br-C₆H₄
bedeutet und der Substituent R⁶ für eine Verbindung einer Zeile der Tabelle A entspricht.

Tabelle 68: (Verbindungen 68.1 - 68.974)
Verbindungen der allgemeinen Formel I.4, in denen
R³ = Methyl
R⁴ = CH₃
R⁵ = 4-Br-C₆H₄
bedeutet und der Substituent R⁶ für eine Verbindung einer Zeile der Tabelle A entspricht.

Tabelle 69: (Verbindungen 69.1 - 69.974)
Verbindungen der allgemeinen Formel I.1, in denen
R³ = Methyl
R⁴ = CH₃
R⁵ = 2,3-F₂-C₆H₃
bedeutet und der Substituent R⁶ für eine Verbindung einer Zeile der Tabelle A entspricht.

Tabelle 70: (Verbindungen 70.1 - 70.974)
Verbindungen der allgemeinen Formel I.2, in denen
R³ = Methyl
R⁴ = CH₃
R⁵ = 2,3-F₂-C₆H₃
bedeutet und der Substituent R⁶ für eine Verbindung einer Zeile der Tabelle A entspricht.

Tabelle 71: (Verbindungen 71.1 - 71.974)
Verbindungen der allgemeinen Formel I.3, in denen
R³ = Methyl
R⁴ = CH₃
R⁵ = 2,3-F₂-C₆H₃
bedeutet und der Substituent R⁶ für eine Verbindung einer Zeile der Tabelle A entspricht.

Tabelle 72: (Verbindungen 72.1 - 72.974)
Verbindungen der allgemeinen Formel I.4, in denen
R³ = Methyl
R⁴ = CH₃
R5 = 2,3-F₂-C₆H₃
bedeutet und der Substituent R⁶ für eine Verbindung einer Zeile der Tabelle A entspricht.

Tabelle 73: (Verbindungen 73.1 - 73.974)
Verbindungen der allgemeinen Formel I.1, in denen
R³ = Methyl
R⁴ = CH₃
R⁵ = 2,4-F₂-C₆H₃
bedeutet und der Substituent R⁶ für eine Verbindung einer Zeile der Tabelle A entspricht.

Tabelle 74: (Verbindungen 74.1 - 74.974)
Verbindungen der allgemeinen Formel I.2, in denen
R³ = Methyl
R⁴ = CH₃
R⁵ = 2,4-F₂-C₆H₃
bedeutet und der Substituent R⁶ für eine Verbindung einer Zeile der Tabelle A entspricht.

Tabelle 75: (Verbindungen 75.1 - 75.974)
Verbindungen der allgemeinen Formel I.3, in denen
R³ = Methyl
R⁴ = CH₃
R⁵ = 2,4-F₂-C₆H₃
bedeutet und der Substituent R⁶ für eine Verbindung einer Zeile der Tabelle A entspricht.

Tabelle 76: (Verbindungen 76.1 - 76.974)
Verbindungen der allgemeinen Formel I.4, in denen
R³ = Methyl
R⁴ = CH₃
R⁵ = 2,4-F₂-C₆H₃
bedeutet und der Substituent R⁶ für eine Verbindung einer Zeile der Tabelle A entspricht.

Tabelle 77: (Verbindungen 77.1 - 77.974)
Verbindungen der allgemeinen Formel I.1, in denen
R³ = Methyl
R⁴ = CH₃
R⁵ = 2,5-F₂-C₆H₃
bedeutet und der Substituent R⁶ für eine Verbindung einer Zeile der Tabelle A entspricht.

Tabelle 78: (Verbindungen 78.1 - 78.974)
Verbindungen der allgemeinen Formel I.2, in denen
R³ = Methyl
R⁴ = CH₃
R⁵ = 2,5-F₂-C₆H₃
bedeutet und der Substituent R⁶ für eine Verbindung einer Zeile der Tabelle A entspricht.

Tabelle 79: (Verbindungen 79.1 - 79.974)
Verbindungen der allgemeinen Formel I.3, in denen
R³ = Methyl
R⁴ = CH₃
R⁵ = 2,5-F₂-C₆H₃
bedeutet und der Substituent R⁶ für eine Verbindung einer Zeile der Tabelle A entspricht.

Tabelle 80: (Verbindungen 80.1 - 80.974)
Verbindungen der allgemeinen Formel I.4, in denen
R³ = Methyl
R⁴ = CH₃
R⁵ = 2,5-F₂-C₆H₃
bedeutet und der Substituent R⁶ für eine Verbindung einer Zeile der Tabelle A entspricht.

Tabelle 81: (Verbindungen 81.1 - 81.974)
Verbindungen der allgemeinen Formel I.1, in denen
R³ = Methyl
R⁴ = CH₃
R⁵ = 3,4-F₂-C₆H₃
bedeutet und der Substituent R⁶ für eine Verbindung einer Zeile der Tabelle A entspricht.

Tabelle 82: (Verbindungen 82.1 - 82.974)
Verbindungen der allgemeinen Formel I.2, in denen
R³ = Methyl
R⁴ = CH₃
R⁵ = 3,4-F₂-C₆H₃
bedeutet und der Substituent R⁶ für eine Verbindung einer Zeile der Tabelle A entspricht.

Tabelle 83: (Verbindungen 83.1 - 83.974)
Verbindungen der allgemeinen Formel I.3, in denen
R³ = Methyl
R⁴ = CH₃
R⁵ = 3,4-F₂-C₆H₃
bedeutet und der Substituent R⁶ für eine Verbindung einer Zeile der Tabelle A entspricht.

Tabelle 84: (Verbindungen 84.1 - 84.974)
Verbindungen der allgemeinen Formel I.4, in denen
R³ = Methyl
R⁴ = CH₃
R⁵ = 3,4-F₂-C₆H₃
bedeutet und der Substituent R⁶ für eine Verbindung einer Zeile der Tabelle A entspricht.

Tabelle 85: (Verbindungen 85.1 - 85.974)
Verbindungen der allgemeinen Formel I.1, in denen
R³ = Methyl
R⁴ = CH₃
R⁵ = 3,5-F₂-C₆H₃
bedeutet und der Substituent R⁶ für eine Verbindung einer Zeile der Tabelle A entspricht.

Tabelle 86: (Verbindungen 86.1 - 86.974)
Verbindungen der allgemeinen Formel I.2, in denen
R³ = Methyl
R⁴ = CH₃
R⁵ = 3,5-F₂-C₆H₃
bedeutet und der Substituent R⁶ für eine Verbindung einer Zeile der Tabelle A entspricht.

Tabelle 87: (Verbindungen 87.1 - 87.974)
Verbindungen der allgemeinen Formel I.3, in denen
R³ = Methyl
R⁴ = CH₃
R⁵ = 3,5-F₂-C₆H₃
bedeutet und der Substituent R⁶ für eine Verbindung einer Zeile der Tabelle A entspricht.

Tabelle 88: (Verbindungen 88.1 - 88.974)
Verbindungen der allgemeinen Formel I.4, in denen
R³ = Methyl
R⁴ = CH₃
R⁵ = 3,5-F₂-C₆H₃
bedeutet und der Substituent R⁶ für eine Verbindung einer Zeile der Tabelle A entspricht.

Tabelle 89: (Verbindungen 89.1 - 89.974)
Verbindungen der allgemeinen Formel I.1, in denen
R³ = Methyl
R⁴ = CH₃
R⁵ = 2,3-Cl₂-C₆H₃
bedeutet und der Substituent R⁶ für eine Verbindung einer Zeile der Tabelle A entspricht.

Tabelle 90: (Verbindungen 90.1 - 90.974)
Verbindungen der allgemeinen Formel I.2, in denen
R³ = Methyl
R⁴ = CH₃
R⁵ = 2,3-Cl₂-C₆H₃
bedeutet und der Substituent R⁶ für eine Verbindung einer Zeile der Tabelle A entspricht.

Tabelle 91: (Verbindungen 91.1 - 91.974)
Verbindungen der allgemeinen Formel I.3, in denen
R³ = Methyl
R⁴ = CH₃
R⁵ = 2,3-Cl₂-C₆H₃
bedeutet und der Substituent R⁶ für eine Verbindung einer Zeile der Tabelle A entspricht.

Tabelle 92: (Verbindungen 92.1 - 92.974)
Verbindungen der allgemeinen Formel I.4, in denen
R³ = Methyl
R⁴ = CH₃
R⁵ = 2,3-Cl₂-C₆H₃
bedeutet und der Substituent R⁶ für eine Verbindung einer Zeile der Tabelle A entspricht.

Tabelle 93: (Verbindungen 93.1 - 93.974)
Verbindungen der allgemeinen Formel I.1, in denen
R³ = Methyl
R⁴ = CH₃
R⁵ = 2,4-Cl₂-C₆H₃
bedeutet und der Substituent R⁶ für eine Verbindung einer Zeile der Tabelle A entspricht.

Tabelle 94: (Verbindungen 94.1 - 94.974)
Verbindungen der allgemeinen Formel I.2, in denen
R³ = Methyl
R⁴ = CH₃
R⁵ = 2,4-Cl₂-C₆H₃
bedeutet und der Substituent R⁶ für eine Verbindung einer Zeile der Tabelle A entspricht.

Tabelle 95: (Verbindungen 95.1 - 95.974)
Verbindungen der allgemeinen Formel I.3, in denen
R³ = Methyl
R⁴ = CH₃
R⁵ = 2,4-Cl₂-C₆H₃
bedeutet und der Substituent R⁶ für eine Verbindung einer Zeile der Tabelle A entspricht.

Tabelle 96: (Verbindungen 96.1 - 96.974)
Verbindungen der allgemeinen Formel I.4, in denen
R³ = Methyl
R⁴ = CH₃
R⁵ = 2,4-Cl₂-C₆H₃
bedeutet und der Substituent R⁶ für eine Verbindung einer Zeile der Tabelle A entspricht.

Tabelle 97: (Verbindungen 97.1 - 97.974)
Verbindungen der allgemeinen Formel I.1, in denen
R³ = Methyl
R⁴ = CH₃
R⁵ = 2,5-Cl₂-C₆H₃
bedeutet und der Substituent R⁶ für eine Verbindung einer Zeile der Tabelle A entspricht.

Tabelle 98: (Verbindungen 98.1 - 98.974)
Verbindungen der allgemeinen Formel I.2, in denen
R³ = Methyl
R⁴ = CH₃
R⁵ = 2,5-Cl₂-C₆H₃
bedeutet und der Substituent R⁶ für eine Verbindung einer Zeile der Tabelle A entspricht.

Tabelle 99: (Verbindungen 99.1 - 99.974)
Verbindungen der allgemeinen Formel I.3, in denen
R³ = Methyl
R⁴ = CH₃
R⁵ = 2,5-Cl₂-C₆H₃
bedeutet und der Substituent R⁶ für eine Verbindung einer Zeile der Tabelle A entspricht.

Tabelle 100: (Verbindungen 100.1 - 100.974)
Verbindungen der allgemeinen Formel I.4, in denen
R³ = Methyl
R⁴ = CH₃
R⁵ = 2,5-Cl₂-C₆H₃
bedeutet und der Substituent R⁶ für eine Verbindung einer Zeile der Tabelle A entspricht.

Tabelle 101: (Verbindungen 101.1 - 101.974)
Verbindungen der allgemeinen Formel I.1, in denen
R³ = Methyl
R⁴ = CH₃
R⁵ = 3,4-Cl₂-C₆H₃
bedeutet und der Substituent R⁶ für eine Verbindung einer Zeile der Tabelle A entspricht.

Tabelle 102: (Verbindungen 102.1 - 102.974)
Verbindungen der allgemeinen Formel I.2, in denen
R³ = Methyl
R⁴ = CH₃
R⁵ = 3,4-Cl₂-C₆H₃
bedeutet und der Substituent R⁶ für eine Verbindung einer Zeile der Tabelle A entspricht.

Tabelle 103: (Verbindungen 103.1 - 103.974)
Verbindungen der allgemeinen Formel I.3, in denen
R³ = Methyl
R⁴ = CH₃
R⁵ = 3,4-Cl₂-C₆H₃
bedeutet und der Substituent R⁶ für eine Verbindung einer Zeile der Tabelle A entspricht.

Tabelle 104: (Verbindungen 104.1 - 104.974)
Verbindungen der allgemeinen Formel I.4, in denen
R³ = Methyl
R⁴ = CH₃
R⁵ = 3,4-Cl₂-C₆H₃
bedeutet und der Substituent R⁶ für eine Verbindung einer Zeile der Tabelle A entspricht.

Tabelle 105: (Verbindungen 1.105 - 105.974)
Verbindungen der allgemeinen Formel I.1, in denen
R³ = Methyl
R⁴ = CH₃
R⁵ = 3,5-Cl₂-C₆H₃
bedeutet und der Substituent R⁶ für eine Verbindung einer Zeile der Tabelle A entspricht.

Tabelle 106: (Verbindungen 106.1 - 106.974)
Verbindungen der allgemeinen Formel I.2, in denen
R³ = Methyl
R⁴ = CH₃
R⁵ = 3,5-Cl₂-C₆H₃
bedeutet und der Substituent R⁶ für eine Verbindung einer Zeile der Tabelle A entspricht.

Tabelle 107: (Verbindungen 107.1 - 107.974)
Verbindungen der allgemeinen Formel I.3, in denen
R³ = Methyl
R⁴ = CH₃
R⁵ = 3,5-Cl₂-C₆H₃
bedeutet und der Substituent R⁶ für eine Verbindung einer Zeile der Tabelle A entspricht.

Tabelle 108: (Verbindungen 108.1 - 108.974)
Verbindungen der allgemeinen Formel I.4, in denen
R³ = Methyl
R⁴ = CH₃
R⁵ = 3,5-Cl₂-C₆H₃
bedeutet und der Substituent R⁶ für eine Verbindung einer Zeile der Tabelle A entspricht.

Tabelle 109: (Verbindungen 109.1 - 109.974)
Verbindungen der allgemeinen Formel I.1, in denen
R³ = Methyl
R⁴ = CH₃
R⁵ = 2-F; 3-Cl-C₆H₃
bedeutet und der Substituent R⁶ für eine Verbindung einer Zeile der Tabelle A entspricht.

Tabelle 110: (Verbindungen 110.1 - 110.974)
Verbindungen der allgemeinen Formel I.2, in denen
R³ = Methyl
R⁴ = CH₃
R⁵ = 2-F; 3-Cl-C₆H₃
bedeutet und der Substituent R⁶ für eine Verbindung einer Zeile der Tabelle A entspricht.

Tabelle 111: (Verbindungen 111.1 - 111.974)
Verbindungen der allgemeinen Formel I.3, in denen
R³ = Methyl
R⁴ = CH₃
R⁵ = 2-F; 3-Cl-C₆H₃
bedeutet und der Substituent R⁶ für eine Verbindung einer Zeile der Tabelle A entspricht.

Tabelle 112: (Verbindungen 112.1 - 112.974)
Verbindungen der allgemeinen Formel I.4, in denen
R³ = Methyl
R⁴ = CH₃
R⁵ = 2-F; 3-Cl-C₆H₃
bedeutet und der Substituent R⁶ für eine Verbindung einer Zeile der Tabelle A entspricht.

Tabelle 113: (Verbindungen 113.1 - 113.974)
Verbindungen der allgemeinen Formel I.1, in denen
R³ = Methyl
R⁴ = CH₃
R⁵ = 2-F; 4-Cl-C₆H₃
bedeutet und der Substituent R⁶ für eine Verbindung einer Zeile der Tabelle A entspricht.

Tabelle 114: (Verbindungen 114.1 - 114.974)
Verbindungen der allgemeinen Formel I.2, in denen
R³ = Methyl
R⁴ = CH₃
R⁵ = 2-F; 4-Cl-C₆H₃
bedeutet und der Substituent R⁶ für eine Verbindung einer Zeile der Tabelle A entspricht.

Tabelle 115: (Verbindungen 115.1 - 115.974)
Verbindungen der allgemeinen Formel I.3, in denen
R³ = Methyl
R⁴ = CH₃
R⁵ = 2-F; 4-Cl-C₆H₃
bedeutet und der Substituent R⁶ für eine Verbindung einer Zeile der Tabelle A entspricht.

Tabelle 116: (Verbindungen 116.1 - 116.974)
Verbindungen der allgemeinen Formel I.4, in denen
R³ = Methyl
R⁴ = CH₃
R⁵ = 2-F; 4-Cl-C₆H₃
bedeutet und der Substituent R⁶ für eine Verbindung einer Zeile der Tabelle A entspricht.

Tabelle 117: (Verbindungen 117.1 - 117.974)
Verbindungen der allgemeinen Formel I.1, in denen
R³ = Methyl
R⁴ = CH₃
R⁵ = 3-F; 4-Cl-C₆H₃
bedeutet und der Substituent R⁶ für eine Verbindung einer Zeile der Tabelle A entspricht.

Tabelle 118: (Verbindungen 118.1 - 118.974)
Verbindungen der allgemeinen Formel I.2, in denen
R³ = Methyl
R⁴ = CH₃
R⁵ = 3-F; 4-Cl-C₆H₃
bedeutet und der Substituent R⁶ für eine Verbindung einer Zeile der Tabelle A entspricht.

Tabelle 119: (Verbindungen 119.1 - 119.974)
Verbindungen der allgemeinen Formel I.3, in denen
R³ = Methyl
R⁴ = CH₃
R⁵ = 3-F; 4-Cl-C₆H₃
bedeutet und der Substituent R⁶ für eine Verbindung einer Zeile der Tabelle A entspricht.

Tabelle 120: (Verbindungen 120.1 - 120.974)
Verbindungen der allgemeinen Formel I.4, in denen
R³ = Methyl
R⁴ = CH₃
R⁵ = 3-F; 4-Cl-C₆H₃
bedeutet und der Substituent R⁶ für eine Verbindung einer Zeile der Tabelle A entspricht.

Tabelle 121: (Verbindungen 121.1 - 121.974)
Verbindungen der allgemeinen Formel I.1, in denen
R³ = Methyl
R⁴ = CH₃
R⁵ = 4-F; 3,5-Cl₂-C₆H₂
bedeutet und der Substituent R⁶ für eine Verbindung einer Zeile der Tabelle A entspricht.

Tabelle 122: (Verbindungen 122.1 - 122.974)
Verbindungen der allgemeinen Formel I.2, in denen
R³ = Methyl
R⁴ = CH₃
R⁵ = 4-F; 3,5-Cl₂-C₆H₂
bedeutet und der Substituent R⁶ für eine Verbindung einer Zeile der Tabelle A entspricht.

Tabelle 123: (Verbindungen 123.1 - 123.974)
Verbindungen der allgemeinen Formel I.3, in denen
R³ = Methyl
R⁴ = CH₃
R⁵ = 4-F; 3,5-Cl₂-C₆H₂
bedeutet und der Substituent R⁶ für eine Verbindung einer Zeile der Tabelle A entspricht.

Tabelle 124: (Verbindungen 124.1 - 124.974)
Verbindungen der allgemeinen Formel I.4, in denen
R³ = Methyl
R⁴ = CH₃
R⁵ = 4-F; 3,5-Cl₂-C₆H₂
bedeutet und der Substituent R⁶ für eine Verbindung einer Zeile der Tabelle A entspricht.

Tabelle 125: (Verbindungen 125.1 - 125.974)
Verbindungen der allgemeinen Formel I.1, in denen
R³ = Methyl
R⁴ = CH₃
R⁵ = 2,3,4-Cl₃-C₆H₂
bedeutet und der Substituent R⁶ für eine Verbindung einer Zeile der Tabelle A entspricht.

Tabelle 126: (Verbindungen 126.1 - 126.974)
Verbindungen der allgemeinen Formel I.2, in denen
R³ = Methyl
R⁴ = CH₃
R⁵ = 2,3,4-Cl₃-C₆H₂
bedeutet und der Substituent R⁶ für eine Verbindung einer Zeile der Tabelle A entspricht.

Tabelle 127: (Verbindungen 127.1 - 127.974)
Verbindungen der allgemeinen Formel I.3, in denen
R³ = Methyl
R⁴ = CH₃
R⁵ = 2,3,4-Cl₃-C₆H₂
bedeutet und der Substituent R⁶ für eine Verbindung einer Zeile der Tabelle A entspricht.

Tabelle 128: (Verbindungen 128.1 - 128.974)
Verbindungen der allgemeinen Formel I.4, in denen
R³ = Methyl
R⁴ = CH₃
R⁵ = 2,3,4-Cl₃-C₆H₂
bedeutet und der Substituent R⁶ für eine Verbindung einer Zeile der Tabelle A entspricht.

Tabelle 129: (Verbindungen 129.1 - 129.974)
Verbindungen der allgemeinen Formel I.1, in denen
R³ = Methyl
R⁴ = CH₃
R⁵ = 2,3,5-Cl₃-C₆H₂
bedeutet und der Substituent R⁶ für eine Verbindung einer Zeile der Tabelle A entspricht.

Tabelle 130: (Verbindungen 130.1 - 130.974)
Verbindungen der allgemeinen Formel I.2, in denen
R³ = Methyl
R⁴ = CH₃
R⁵ = 2,3,5-Cl₃-C₆H₂
bedeutet und der Substituent R⁶ für eine Verbindung einer Zeile der Tabelle A entspricht.

Tabelle 131: (Verbindungen 131.1 - 131.974)
Verbindungen der allgemeinen Formel I.3, in denen
R³ = Methyl
R⁴ = CH₃
R⁵ = 2,3,5-Cl₃-C₆H₂
bedeutet und der Substituent R⁶ für eine Verbindung einer Zeile der Tabelle A entspricht.

Tabelle 132: (Verbindungen 132.1 - 132.974)
Verbindungen der allgemeinen Formel I.4, in denen
R³ = Methyl
R⁴ = CH₃
R⁵ = 2,3,5-Cl₃-C₆H₂
bedeutet und der Substituent R⁶ für eine Verbindung einer Zeile der Tabelle A entspricht.

Tabelle 133: (Verbindungen 133.1 - 133.974)
Verbindungen der allgemeinen Formel I.1, in denen
R³ = Methyl
R⁴ = CH₃
R⁵ = 2,4,5-Cl₃-C₆H₂
bedeutet und der Substituent R⁶ für eine Verbindung einer Zeile der Tabelle A entspricht.

Tabelle 134: (Verbindungen 134.1 - 134.974)
Verbindungen der allgemeinen Formel I.2, in denen
R³ = Methyl
R⁴ = CH₃
R⁵ = 2,4,5-Cl₃-C₆H₂
bedeutet und der Substituent R⁶ für eine Verbindung einer Zeile der Tabelle A entspricht.

Tabelle 135: (Verbindungen 135.1 - 135.974)
Verbindungen der allgemeinen Formel I.3, in denen
R³ = Methyl
R⁴ = CH₃
R⁵ = 2,4,5-Cl₃-C₆H₂
bedeutet und der Substituent R⁶ für eine Verbindung einer Zeile der Tabelle A entspricht.

Tabelle 136: (Verbindungen 136.1 - 136.974)
Verbindungen der allgemeinen Formel I.4, in denen
R³ = Methyl
R⁴ = CH₃
R⁵ = 2,4,5-Cl₃-C₆H₂
bedeutet und der Substituent R⁶ für eine Verbindung einer Zeile der Tabelle A entspricht.

Tabelle 137: (Verbindungen 137.1 - 137.974)
Verbindungen der allgemeinen Formel I.1, in denen
R³ = Methyl
R⁴ = CH₃
R⁵ = 2-CN-C₆H₄
bedeutet und der Substituent R⁶ für eine Verbindung einer Zeile der Tabelle A entspricht.

Tabelle 138: (Verbindungen 138.1 - 138.974)
Verbindungen der allgemeinen Formel I.2, in denen
R³ = Methyl
R⁴ = CH₃
R⁵ = 2-CN-C₆H₄
bedeutet und der Substituent R⁶ für eine Verbindung einer Zeile der Tabelle A entspricht.

Tabelle 139: (Verbindungen 139.1 - 139.974)
Verbindungen der allgemeinen Formel I.3, in denen
R³ = Methyl
R⁴ = CH₃
R⁵ = 2-CN-C₆H₄
bedeutet und der Substituent R⁶ für eine Verbindung einer Zeile der Tabelle A entspricht.

Tabelle 140: (Verbindungen 140.1 - 140.974)
Verbindungen der allgemeinen Formel I.4, in denen
R³ = Methyl
R⁴ = CH₃
R⁵ = 2-CN-C₆H₄
bedeutet und der Substituent R⁶ für eine Verbindung einer Zeile der Tabelle A entspricht.

Tabelle 141: (Verbindungen 141.1 - 141.974)
Verbindungen der allgemeinen Formel I.1, in denen
R³ = Methyl
R⁴ = CH₃
R⁵ = 3-CN-C₆H₄
bedeutet und der Substituent R⁶ für eine Verbindung einer Zeile der Tabelle A entspricht.

Tabelle 142: (Verbindungen 142.1 - 142.974)
Verbindungen der allgemeinen Formel I.2, in denen
R³ = Methyl
R⁴ = CH₃
R⁵ = 3-CN-C₆H₄
bedeutet und der Substituent R⁶ für eine Verbindung einer Zeile der Tabelle A entspricht.

Tabelle 143: (Verbindungen 143.1 - 143.974)
Verbindungen der allgemeinen Formel I.3, in denen
R³ = Methyl
R⁴ = CH₃
R⁵ = 3-CN-C₆H₄
bedeutet und der Substituent R⁶ für eine Verbindung einer Zeile der Tabelle A entspricht.

Tabelle 144: (Verbindungen 144.1 - 144.974)
Verbindungen der allgemeinen Formel I.4, in denen
R³ = Methyl
R⁴ = CH₃
R⁵ = 3-CN-C₆H₄
bedeutet und der Substituent R⁶ für eine Verbindung einer Zeile der Tabelle A entspricht.

Tabelle 145: (Verbindungen 145.1 - 145.974)
Verbindungen der allgemeinen Formel I.1, in denen
R³ = Methyl
R⁴ = CH₃
R⁵ = 4-CN-C₆H₄
bedeutet und der Substituent R⁶ für eine Verbindung einer Zeile der Tabelle A entspricht.

Tabelle 146: (Verbindungen 146.1 - 146.974)
Verbindungen der allgemeinen Formel I.2, in denen
R³ = Methyl
R⁴ = CH₃
R⁵ = 4-CN-C₆H₄
bedeutet und der Substituent R⁶ für eine Verbindung einer Zeile der Tabelle A entspricht.

Tabelle 147: (Verbindungen 147.1 - 147.974)
Verbindungen der allgemeinen Formel I.3, in denen
R³ = Methyl
R⁴ = CH₃
R⁵ = 4-CN-C₆H₄
bedeutet und der Substituent R⁶ für eine Verbindung einer Zeile der Tabelle A entspricht.

Tabelle 148: (Verbindungen 148.1 - 148.974)
Verbindungen der allgemeinen Formel I.4, in denen
R³ = Methyl
R⁴ = CH₃
R⁵ = 4-CN-C₆H₄
bedeutet und der Substituent R⁶ für eine Verbindung einer Zeile der Tabelle A entspricht.

Tabelle 149: (Verbindungen 149.1 - 149.974)
Verbindungen der allgemeinen Formel I.1, in denen
R³ = Methyl
R⁴ = CH₃
R⁵ = 2-NO₂-C₆H₄
bedeutet und der Substituent R⁶ für eine Verbindung einer Zeile der Tabelle A entspricht.

Tabelle 150: (Verbindungen 150.1 - 150.974)
Verbindungen der allgemeinen Formel I.2, in denen
R³ = Methyl
R⁴ = CH₃
R⁵ = 2-NO₂-C₆H₄
bedeutet und der Substituent R⁶ für eine Verbindung einer Zeile der Tabelle A entspricht.

Tabelle 151: (Verbindungen 151.1 - 151.974)
Verbindungen der allgemeinen Formel I.3, in denen
R³ = Methyl
R⁴ = CH₃
R⁵ = 2-NO₂-C₆H₄
bedeutet und der Substituent R⁶ für eine Verbindung einer Zeile der Tabelle A entspricht.

Tabelle 152: (Verbindungen 152.1 - 152.974)
Verbindungen der allgemeinen Formel I.4, in denen
R³ = Methyl
R⁴ = CH₃
R⁵ = 2-NO₂-C₆H₄
bedeutet und der Substituent R⁶ für eine Verbindung einer Zeile der Tabelle A entspricht.

Tabelle 153: (Verbindungen 153.1 - 153.974)
Verbindungen der allgemeinen Formel I.1, in denen
R³ = Methyl
R⁴ = CH₃
R⁵ = 3-NO₂-C₆H₄
bedeutet und der Substituent R⁶ für eine Verbindung einer Zeile der Tabelle A entspricht.

Tabelle 154: (Verbindungen 154.1 - 154.974)
Verbindungen der allgemeinen Formel I.2, in denen
R³ = Methyl
R⁴ = CH₃
R⁵ = 3-NO₂-C₆H₄
bedeutet und der Substituent R⁶ für eine Verbindung einer Zeile der Tabelle A entspricht.

Tabelle 155: (Verbindungen 155.1 - 155.974)
Verbindungen der allgemeinen Formel I.3, in denen
R³ = Methyl
R⁴ = CH₃
R⁵ = 3-NO₂-C₆H₄
bedeutet und der Substituent R⁶ für eine Verbindung einer Zeile der Tabelle A entspricht.

Tabelle 156: (Verbindungen 156.1 - 156.974)
Verbindungen der allgemeinen Formel I.4, in denen
R³ = Methyl
R⁴ = CH₃
R⁵ = 3-NO₂-C₆H₄
bedeutet und der Substituent R⁶ für eine Verbindung einer Zeile der Tabelle A entspricht.

Tabelle 157: (Verbindungen 157.1 - 157.974)
Verbindungen der allgemeinen Formel I.1, in denen
R³ = Methyl
R⁴ = CH₃
R⁵ = 4-NO₂-C₆H₄
bedeutet und der Substituent R⁶ für eine Verbindung einer Zeile der Tabelle A entspricht.

Tabelle 158: (Verbindungen 158.1 - 158.974)
Verbindungen der allgemeinen Formel I.2, in denen
R³ = Methyl
R⁴ = CH₃
R5 = 4-NO₂-C₆H₄
bedeutet und der Substituent R⁶ für eine Verbindung einer Zeile der Tabelle A entspricht.

Tabelle 159: (Verbindungen 159.1 - 159.974)
Verbindungen der allgemeinen Formel I.3, in denen
R³ = Methyl
R⁴ = CH₃
R⁵ = 4-NO₂-C₆H₄
bedeutet und der Substituent R⁶ für eine Verbindung einer Zeile der Tabelle A entspricht.

Tabelle 160: (Verbindungen 160.1 - 160.974)
Verbindungen der allgemeinen Formel I.4, in denen
R³ = Methyl
R⁴ = CH₃
R⁵ = 4-NO₂-C₆H₄
bedeutet und der Substituent R⁶ für eine Verbindung einer Zeile der Tabelle A entspricht.

Tabelle 161: (Verbindungen 161.1 - 161.974)
Verbindungen der allgemeinen Formel I.1, in denen
R³ = Methyl
R⁴ = CH₃
R⁵ = 2-CH₃-C₆H₄
bedeutet und der Substituent R⁶ für eine Verbindung einer Zeile der Tabelle A entspricht.

Tabelle 162: (Verbindungen 162.1 - 162.974)
Verbindungen der allgemeinen Formel I.2, in denen
R³ = Methyl
R⁴ = CH₃
R⁵ = 2-CH₃-C₆H₄
bedeutet und der Substituent R⁶ für eine Verbindung einer Zeile der Tabelle A entspricht.

Tabelle 163: (Verbindungen 163.1 - 163.974)
Verbindungen der allgemeinen Formel I.3, in denen
R³ = Methyl
R⁴ = CH₃
R⁵ = 2-CH₃-C₆H₄
bedeutet und der Substituent R⁶ für eine Verbindung einer Zeile der Tabelle A entspricht.

Tabelle 164: (Verbindungen 164.1 - 164.974)
Verbindungen der allgemeinen Formel I.4, in denen
R³ = Methyl
R⁴ = CH₃
R⁵ = 2-CH₃-C₆H₄
bedeutet und der Substituent R⁶ für eine Verbindung einer Zeile der Tabelle A entspricht.

Tabelle 165: (Verbindungen 165.1 - 165.974)
Verbindungen der allgemeinen Formel I.1, in denen
R³ = Methyl
R⁴ = CH₃
R⁵ = 3-CH₃-C₆H₄
bedeutet und der Substituent R⁶ für eine Verbindung einer Zeile der Tabelle A entspricht.

Tabelle 166: (Verbindungen 166.1 - 166.974)
Verbindungen der allgemeinen Formel I.2, in denen
R³ = Methyl
R⁴ = CH₃
R⁵ = 3-CH₃-C₆H₄
bedeutet und der Substituent R⁶ für eine Verbindung einer Zeile der Tabelle A entspricht.

Tabelle 167: (Verbindungen 167.1 - 167.974)
Verbindungen der allgemeinen Formel I.3, in denen
R³ = Methyl
R⁴ = CH₃
R⁵ = 3-CH₃-C₆H₄
bedeutet und der Substituent R⁶ für eine Verbindung einer Zeile der Tabelle A entspricht.

Tabelle 168: (Verbindungen 168.1 - 168.974)
Verbindungen der allgemeinen Formel I.4, in denen
R³ = Methyl
R⁴ = CH₃
R⁵ = 3-CH₃-C₆H₄
bedeutet und der Substituent R⁶ für eine Verbindung einer Zeile der Tabelle A entspricht.

Tabelle 169: (Verbindungen 169.1 - 169.974)
Verbindungen der allgemeinen Formel I.1, in denen
R³ = Methyl
R⁴ = CH₃
R⁵ = 4-CH₃-C₆H₄
bedeutet und der Substituent R⁶ für eine Verbindung einer Zeile der Tabelle A entspricht.

Tabelle 170: (Verbindungen 170.1 - 170.974)
Verbindungen der allgemeinen Formel I.2, in denen
R³ = Methyl
R⁴ = CH₃
R⁵ = 4-CH₃-C₆H₄
bedeutet und der Substituent R⁶ für eine Verbindung einer Zeile der Tabelle A entspricht.

Tabelle 171: (Verbindungen 171.1 - 171.974)
Verbindungen der allgemeinen Formel I.3, in denen
R³ = Methyl
R⁴ = CH₃
R⁵ = 4-CH₃-C₆H₄
bedeutet und der Substituent R⁶ für eine Verbindung einer Zeile der Tabelle A entspricht.

Tabelle 172: (Verbindungen 172.1 - 172.974)
Verbindungen der allgemeinen Formel I.4, in denen
R³ = Methyl
R⁴ = CH₃
R⁵ = 4-CH₃-C₆H₄
bedeutet und der Substituent R⁶ für eine Verbindung einer Zeile der Tabelle A entspricht.

Tabelle 173: (Verbindungen 173.1 - 173.974)
Verbindungen der allgemeinen Formel I.1, in denen
R³ = Methyl
R⁴ = CH₃
R⁵ = 2,4-(CH₃)₂-C₆H₃
bedeutet und der Substituent R⁶ für eine Verbindung einer Zeile der Tabelle A entspricht.

Tabelle 174: (Verbindungen 174.1 - 174.974)
Verbindungen der allgemeinen Formel I.2, in denen
R³ = Methyl
R⁴ = CH₃
R⁵ = 2,4-(CH₃)₂-C₆H₃
bedeutet und der Substituent R⁶ für eine Verbindung einer Zeile der Tabelle A entspricht.

Tabelle 175: (Verbindungen 175.1 - 175.974)
Verbindungen der allgemeinen Formel I.3, in denen
R³ = Methyl
R⁴ = CH₃
R⁵ = 2,4-(CH₃)₂-C₆H₃
bedeutet und der Substituent R⁶ für eine Verbindung einer Zeile der Tabelle A entspricht.

Tabelle 176: (Verbindungen 176.1 - 176.974)
Verbindungen der allgemeinen Formel I.4, in denen
R³ = Methyl
R⁴ = CH₃
R⁵ = 2,4-(CH₃)₂-C₆H₃
bedeutet und der Substituent R⁶ für eine Verbindung einer Zeile der Tabelle A entspricht.

Tabelle 177: (Verbindungen 177.1 - 177.974)
Verbindungen der allgemeinen Formel I.1, in denen
R³ = Methyl
R⁴ = CH₃
R⁵ = 2,5-(CH₃)₂-C₆H₃
bedeutet und der Substituent R⁶ für eine Verbindung einer Zeile der Tabelle A entspricht.

Tabelle 178: (Verbindungen 178.1 - 178.974)
Verbindungen der allgemeinen Formel I.2, in denen
R³ = Methyl
R⁴ = CH³
R⁵ = 2,5-(CH₃)₂-C₆H₃
bedeutet und der Substituent R⁶ für eine Verbindung einer Zeile der Tabelle A entspricht.

Tabelle 179: (Verbindungen 179.1 - 179.974)
Verbindungen der allgemeinen Formel I.3, in denen
R³ = Methyl
R⁴ = CH₃
R⁵ = 2,5-(CH₃)₂-C₆H₃
bedeutet und der Substituent R⁶ für eine Verbindung einer Zeile der Tabelle A entspricht.

Tabelle 180: (Verbindungen 180.1 - 180.974)
Verbindungen der allgemeinen Formel I.4, in denen
R³ = Methyl
R⁴ = CH₃
R⁵ = 2,5-(CH₃)₂-C₆H₃
bedeutet und der Substituent R⁶ für eine Verbindung einer Zeile der Tabelle A entspricht.

Tabelle 181: (Verbindungen 181.1 - 181.974)
Verbindungen der allgemeinen Formel I.1, in denen
R³ = Methyl
R⁴= CH₃
R⁵ = 3,4-(CH₃)₂-C₆H₃
bedeutet und der Substituent R⁶ für eine Verbindung einer Zeile der Tabelle A entspricht.

Tabelle 182: (Verbindungen 182.1 - 182.974)
Verbindungen der allgemeinen Formel I.2, in denen
R³ = Methyl
R⁴ = CH₃
R⁵ = 3,4-(CH₃)₂-C₆H₃
bedeutet und der Substituent R⁶ für eine Verbindung einer Zeile der Tabelle A entspricht.

Tabelle 183: (Verbindungen 183.1 - 183.974)
Verbindungen der allgemeinen Formel I.3, in denen
R³ = Methyl
R⁴ = CH₃
R⁵ = 3,4-(CH₃)₂-C₆H₃
bedeutet und der Substituent R⁶ für eine Verbindung einer Zeile der Tabelle A entspricht.

Tabelle 184: (Verbindungen 184.1 - 184.974)
Verbindungen der allgemeinen Formel I.4, in denen
R³ = Methyl
R⁴ = CH₃
R⁵ = 3,4-(CH₃)₂-C₆H₃
bedeutet und der Substituent R⁶ für eine Verbindung einer Zeile der Tabelle A entspricht.

Tabelle 185: (Verbindungen 185.1 - 185.974)
Verbindungen der allgemeinen Formel I.1, in denen
R³ = Methyl
R⁴ = CH₃
R⁵ = 3,5-(CH₃)₂-C₆H₃
bedeutet und der Substituent R⁶ für eine Verbindung einer Zeile der Tabelle A entspricht.

Tabelle 186: (Verbindungen 186.1 - 186.974)
Verbindungen der allgemeinen Formel I.2, in denen
R³ = Methyl
R⁴ = CH₃
R⁵ = 3,5-(CH₃)₂-C₆H₃
bedeutet und der Substituent R⁶ für eine Verbindung einer Zeile der Tabelle A entspricht.

Tabelle 187: (Verbindungen 187.1 - 187.974)
Verbindungen der allgemeinen Formel I.3, in denen
R³ = Methyl
R⁴ = CH₃
R⁵ = 3,5-(CH₃)₂-C₆H₃
bedeutet und der Substituent R⁶ für eine Verbindung einer Zeile der Tabelle A entspricht.

Tabelle 188: (Verbindungen 188.1 - 188.974)
Verbindungen der allgemeinen Formel I.4, in denen
R³ = Methyl
R⁴ = CH₃
R⁵ = 3,5-(CH₃)₂-C₆H₃
bedeutet und der Substituent R⁶ für eine Verbindung einer Zeile der Tabelle A entspricht.

Tabelle 189: (Verbindungen 189.1 - 189.974)
Verbindungen der allgemeinen Formel I.1, in denen
R³ = Methyl
R⁴ = CH₃
R⁵ = 3,4,5-(CH₃)₃-C₆H₂
bedeutet und der Substituent R⁶ für eine Verbindung einer Zeile der Tabelle A entspricht.

Tabelle 190: (Verbindungen 190.1 - 190.974)
Verbindungen der allgemeinen Formel I.2, in denen
R³ = Methyl
R⁴ = CH₃
R⁵ = 3,4,5-(CH₃)₃-C₆H₂
bedeutet und der Substituent R⁶ für eine Verbindung einer Zeile der Tabelle A entspricht.

Tabelle 191: (Verbindungen 191.1 - 191.974)
Verbindungen der allgemeinen Formel I.3, in denen
R³ = Methyl
R⁴ = CH₃
R⁵ = 3,4,5-(CH₃)₃-C₆H₂
bedeutet und der Substituent R⁶ für eine Verbindung einer Zeile der Tabelle A entspricht.

Tabelle 192: (Verbindungen 192.1 - 192.974)
Verbindungen der allgemeinen Formel I.4, in denen
R³ = Methyl
R⁴ = CH₃
R⁵ = 3,4,5-(CH₃)₃-C₆H₂
bedeutet und der Substituent R⁶ für eine Verbindung einer Zeile der Tabelle A entspricht.

Tabelle 193: (Verbindungen 193.1 - 193.974)
Verbindungen der allgemeinen Formel I.1, in denen
R³ = Methyl
R⁴ = CH₃
R⁵ = 4-C₆H₅-C₆H₄
bedeutet und der Substituent R⁶ für eine Verbindung einer Zeile der Tabelle A entspricht.

Tabelle 194: (Verbindungen 194.1 - 194.974)
Verbindungen der allgemeinen Formel I.2, in denen
R³ = Methyl
R⁴ = CH₃
R⁵ = 4-C₆H₅-C₆H₄
bedeutet und der Substituent R⁶ für eine Verbindung einer Zeile der Tabelle A entspricht.

Tabelle 195: (Verbindungen 195.1 - 195.974)
Verbindungen der allgemeinen Formel I.3, in denen
R³ = Methyl
R⁴ = CH₃
R⁵ = 4-C₆H₅-C₆H₄
bedeutet und der Substituent R⁶ für eine Verbindung einer Zeile der Tabelle A entspricht.

Tabelle 196: (Verbindungen 196.1 - 196.974)
Verbindungen der allgemeinen Formel I.4, in denen
R³ = Methyl
R⁴ = CH₃
R⁵ = 4-C₆H₅-C₆H₄
bedeutet und der Substituent R⁶ für eine Verbindung einer Zeile der Tabelle A entspricht.

Tabelle 197: (Verbindungen 197.1 - 197.974)
Verbindungen der allgemeinen Formel I.1, in denen
R³ = Methyl
R⁴ = CH₃
R⁵ = 3-Cl-4-CH₃-C₆H₃
bedeutet und der Substituent R⁶ für eine Verbindung einer Zeile der Tabelle A entspricht.

Tabelle 198: (Verbindungen 198.1 - 198.974)
Verbindungen der allgemeinen Formel I.2, in denen
R³ = Methyl
R⁴ = CH₃
R⁵ = 3-Cl-4-CH₃-C₆H₃
bedeutet und der Substituent R⁶ für eine Verbindung einer Zeile der Tabelle A entspricht.

Tabelle 199: (Verbindungen 199.1 - 199.974)
Verbindungen der allgemeinen Formel I.3, in denen
R³ = Methyl
R⁴ = CH₃
R⁵ = 3-Cl-4-CH₃-C₆H₃
bedeutet und der Substituent R⁶ für eine Verbindung einer Zeile der Tabelle A entspricht.

Tabelle 200: (Verbindungen 200.1 - 200.974)
Verbindungen der allgemeinen Formel I.4, in denen
R³ = Methyl
R⁴ = CH₃
R⁵ = 3-Cl-4-CH₃-C₆H₃
bedeutet und der Substituent R⁶ für eine Verbindung einer Zeile der Tabelle A entspricht.

Tabelle 201: (Verbindungen 201.1 - 201.974)
Verbindungen der allgemeinen Formel I.1, in denen
R³ = Methyl
R⁴ = CH₃
R⁵ = 2-OCH₃-C₆H₄
bedeutet und der Substituent R⁶ für eine Verbindung einer Zeile der Tabelle A entspricht.

Tabelle 202: (Verbindungen 202.1 - 202.974)
Verbindungen der allgemeinen Formel I.2, in denen
R³ = Methyl
R⁴ = CH₃
R⁵ = 2-OCH₃-C₆H₄
bedeutet und der Substituent R⁶ für eine Verbindung einer Zeile der Tabelle A entspricht.

Tabelle 203: (Verbindungen 203.1 - 203.974)
Verbindungen der allgemeinen Formel I.3, in denen
R³ = Methyl
R⁴ = CH₃
R⁵ = 2-OCH₃-C₆H₄
bedeutet und der Substituent R⁶ für eine Verbindung einer Zeile der Tabelle A entspricht.

Tabelle 204: (Verbindungen 204.1 - 204.974)
Verbindungen der allgemeinen Formel I.4, in denen
R³ = Methyl
R⁴ = CH₃
R⁵ = 2-OCH₃-C₆H₄
bedeutet und der Substituent R⁶ für eine Verbindung einer Zeile der Tabelle A entspricht.

Tabelle 205: (Verbindungen 205.1 - 205.974)
Verbindungen der allgemeinen Formel I.1, in denen
R³ = Methyl
R⁴ = CH₃
R⁵ = 3-OCH₃-C₆H₄
bedeutet und der Substituent R⁶ für eine Verbindung einer Zeile der Tabelle A entspricht.

Tabelle 206: (Verbindungen 206.1 - 206.974)
Verbindungen der allgemeinen Formel I.2, in denen
R³ = Methyl
R⁴ = CH₃
R⁵ = 3-OCH₃-C₆H₄
bedeutet und der Substituent R⁶ für eine Verbindung einer Zeile der Tabelle A entspricht.

Tabelle 207: (Verbindungen 207.1 - 207.974)
Verbindungen der allgemeinen Formel I.3, in denen
R³ = Methyl
R⁴ = CH₃
R⁵ = 3-OCH₃-C₆H₄
bedeutet und der Substituent R⁶ für eine Verbindung einer Zeile der Tabelle A entspricht.

Tabelle 208: (Verbindungen 208.1 - 208.974)
Verbindungen der allgemeinen Formel I.4, in denen
R³ = Methyl
R⁴ = CH₃
R⁵ = 3-OCH₃-C₆H₄
bedeutet und der Substituent R⁶ für eine Verbindung einer Zeile der Tabelle A entspricht.

Tabelle 209: (Verbindungen 209.1 - 209.974)
Verbindungen der allgemeinen Formel I.1, in denen
R³ = Methyl
R⁴ = CH₃
R⁵ = 4-OCH₃-C₆H₄
bedeutet und der Substituent R⁶ für eine Verbindung einer Zeile der Tabelle A entspricht.

Tabelle 210: (Verbindungen 210.1 - 210.974)
Verbindungen der allgemeinen Formel I.2, in denen
R³ = Methyl
R⁴ = CH₃
R⁵ = 4-OCH₃-C₆H₄
bedeutet und der Substituent R⁶ für eine Verbindung einer Zeile der Tabelle A entspricht.

Tabelle 211: (Verbindungen 211.1 - 211.974)
Verbindungen der allgemeinen Formel I.3, in denen
R³ = Methyl
R⁴ = CH₃
R⁵ = 4-OCH₃-C₆H₄
bedeutet und der Substituent R⁶ für eine Verbindung einer Zeile der Tabelle A entspricht.

Tabelle 212: (Verbindungen 212.1 - 212.974)
Verbindungen der allgemeinen Formel I.4, in denen
R³ = Methyl
R⁴ = CH₃
R⁵ = 4-OCH₃-C₆H₄
bedeutet und der Substituent R⁶ für eine Verbindung einer Zeile der Tabelle A entspricht.

Tabelle 213: (Verbindungen 213.1 - 213.974)
Verbindungen der allgemeinen Formel I.1, in denen
R³ = Methyl
R⁴ = CH₃
R⁵ = 2-OCF₃-C₆H₄
bedeutet und der Substituent R⁶ für eine Verbindung einer Zeile der Tabelle A entspricht.

Tabelle 214: (Verbindungen 214.1 - 214.974)
Verbindungen der allgemeinen Formel I.2, in denen
R³ = Methyl
R⁴ = CH₃
R⁵ = 2-OCF₃-C₆H₄
bedeutet und der Substituent R⁶ für eine Verbindung einer Zeile der Tabelle A entspricht.

Tabelle 215: (Verbindungen 215.1 - 215.974)
Verbindungen der allgemeinen Formel I.3, in denen
R³ = Methyl
R⁴ = CH₃
R⁵ = 2-OCF₃-C₆H₄
bedeutet und der Substituent R⁶ für eine Verbindung einer Zeile der Tabelle A entspricht.

Tabelle 216: (Verbindungen 216.1 - 216.974)
Verbindungen der allgemeinen Formel I.4, in denen
R³ = Methyl
R⁴ = CH₃
R⁵ = 2-OCF₃-C₆H₄
bedeutet und der Substituent R⁶ für eine Verbindung einer Zeile der Tabelle A entspricht.

Tabelle 217: (Verbindungen 217.1 - 217.974)
Verbindungen der allgemeinen Formel I.1, in denen
R³ = Methyl
R⁴ = CH₃
R⁵ = 3-OCF₃-C₆H₄
bedeutet und der Substituent R⁶ für eine Verbindung einer Zeile der Tabelle A entspricht.

Tabelle 218: (Verbindungen 218.1 - 218.974)
Verbindungen der allgemeinen Formel I.2, in denen
R³ = Methyl
R⁴ = CH₃
R⁵ = 3-OCF₃-C₆H₄
bedeutet und der Substituent R⁶ für eine Verbindung einer Zeile der Tabelle A entspricht.

Tabelle 219: (Verbindungen 219.1 - 219.974)
Verbindungen der allgemeinen Formel I.3, in denen
R³ = Methyl
R⁴ = CH₃
R⁵ = 3-OCF₃-C₆H₄
bedeutet und der Substituent R⁶ für eine Verbindung einer Zeile der Tabelle A entspricht.

Tabelle 220: (Verbindungen 220.1 - 220.974)
Verbindungen der allgemeinen Formel I.4, in denen
R³ = Methyl
R⁴ = CH₃
R⁵ = 3-OCF₃-C₆H₄
bedeutet und der Substituent R⁶ für eine Verbindung einer Zeile der Tabelle A entspricht.

Tabelle 221: (Verbindungen 221.1 - 221.974)
Verbindungen der allgemeinen Formel I.1, in denen
R³ = Methyl
R⁴ = CH₃
R⁵ = 4-OCF₃-C₆H₄
bedeutet und der Substituent R⁶ für eine Verbindung einer Zeile der Tabelle A entspricht.

Tabelle 222: (Verbindungen 222.1 - 222.974)
Verbindungen der allgemeinen Formel I.2, in denen
R³ = Methyl
R⁴ = CH₃
R⁵ = 4-OCF₃-C₆H₄
bedeutet und der Substituent R⁶ für eine Verbindung einer Zeile der Tabelle A entspricht.

Tabelle 223: (Verbindungen 223.1 - 223.974)
Verbindungen der allgemeinen Formel I.3, in denen
R³ = Methyl
R⁴ = CH₃
R⁵ = 4-OCF₃-C₆H₄
bedeutet und der Substituent R⁶ für eine Verbindung einer Zeile der Tabelle A entspricht.

Tabelle 224: (Verbindungen 224.1 - 224.974)
Verbindungen der allgemeinen Formel I.4, in denen
R³ = Methyl
R⁴ = CH₃
R⁵ = 4-OCF₃-C₆H₄
bedeutet und der Substituent R⁶ für eine Verbindung einer Zeile der Tabelle A entspricht.

Tabelle 225: (Verbindungen 226.1 - 225.974)
Verbindungen der allgemeinen Formel I.1, in denen
R³ = Methyl
R⁴ = CH₃
R⁵ = 3-Cl; 4-OCH₃-C₆H₃
bedeutet und der Substituent R⁶ für eine Verbindung einer Zeile der Tabelle A entspricht.

Tabelle 226: (Verbindungen 226.1 - 226.974)
Verbindungen der allgemeinen Formel I.2, in denen
R³ = Methyl
R⁴ = CH₃
R⁵ = 3-Cl; 4-OCH₃-C₆H₃
bedeutet und der Substituent R⁶ für eine Verbindung einer Zeile der Tabelle A entspricht.

Tabelle 227: (Verbindungen 227.1 - 227.974)
Verbindungen der allgemeinen Formel I.3, in denen
R³ = Methyl
R⁴ = CH₃
R⁵ = 3-Cl; 4-OCH₃-C₆H₃
bedeutet und der Substituent R⁶ für eine Verbindung einer Zeile der Tabelle A entspricht.

Tabelle 228: (Verbindungen 228.1 - 228.974)
Verbindungen der allgemeinen Formel I.4, in denen
R³ = Methyl
R⁴ = CH₃
R⁵ = 3-Cl; 4-OCH₃-C₆H₃
bedeutet und der Substituent R⁶ für eine Verbindung einer Zeile der Tabelle A entspricht.

Tabelle 229: (Verbindungen 229.1 - 229.974)
Verbindungen der allgemeinen Formel I.1, in denen
R³ = Methyl
R⁴ = CH₃ 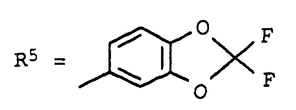 bedeutet und der Substituent R⁶ für eine Verbindung einer Zeile der Tabelle A entspricht.

Tabelle 230: (Verbindungen 230.1 - 230.974)
Verbindungen der allgemeinen Formel I.2, in denen
R³ = Methyl
R⁴ = CH₃ 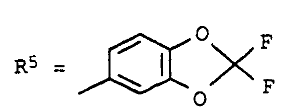 bedeutet und der Substituent R⁶ für eine Verbindung einer Zeile der Tabelle A entspricht.

Tabelle 231: (Verbindungen 231.1 - 231.974)
Verbindungen der allgemeinen Formel I.3, in denen
R³ = Methyl
R⁴ = CH₃ 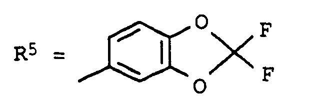 bedeutet und der Substituent R⁶ für eine Verbindung einer Zeile der Tabelle A entspricht.

Tabelle 232: (Verbindungen 232.1 - 232.974)
Verbindungen der allgemeinen Formel I.4, in denen
R³ = Methyl
R⁴ = CH₃ 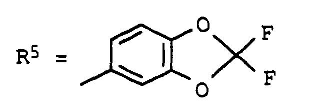 bedeutet und der Substituent R⁶ für eine Verbindung einer Zeile der Tabelle A entspricht.

Tabelle 233: (Verbindungen 233.1 - 233.974)
Verbindungen der allgemeinen Formel I.1, in denen
R³ = Methyl
R⁴ = CH₃
R⁵ = Pyridin-2-yl
bedeutet und der Substituent R⁶ für eine Verbindung einer Zeile der Tabelle A entspricht.

Tabelle 234: (Verbindungen 234.1 - 234.974)
Verbindungen der allgemeinen Formel I.2, in denen
R³ = Methyl
R⁴ = CH₃
R⁵ = Pyridin-2-yl
bedeutet und der Substituent R⁶ für eine Verbindung einer Zeile der Tabelle A entspricht.

Tabelle 235: (Verbindungen 235.1 - 235.974)
Verbindungen der allgemeinen Formel I.3, in denen
R³ = Methyl
R⁴ = CH₃
R⁵ = Pyridin-2-yl
bedeutet und der Substituent R⁶ für eine Verbindung einer Zeile der Tabelle A entspricht.

Tabelle 236: (Verbindungen 236.1 - 236.974)
Verbindungen der allgemeinen Formel I.4, in denen
R³ = Methyl
R⁴ = CH₃
R⁵ = Pyridin-2-yl
bedeutet und der Substituent R⁶ für eine Verbindung einer Zeile der Tabelle A entspricht.

Tabelle 237: (Verbindungen 237.1 - 237.974)
Verbindungen der allgemeinen Formel I.1, in denen
R³ = Methyl
R⁴ = CH₃
R⁵ = Pyridin-3-yl
bedeutet und der Substituent R⁶ für eine Verbindung einer Zeile der Tabelle A entspricht.

Tabelle 238: (Verbindungen 238.1 - 238.974)
Verbindungen der allgemeinen Formel I.2, in denen
R³ = Methyl
R⁴ = CH₃
R⁵ = Pyridin-3-yl
bedeutet und der Substituent R⁶ für eine Verbindung einer Zeile der Tabelle A entspricht.

Tabelle 239: (Verbindungen 239.1 - 239.974)
Verbindungen der allgemeinen Formel I.3, in denen
R³ = Methyl
R⁴ = CH₃
R⁵ = Pyridin-3-yl
bedeutet und der Substituent R⁶ für eine Verbindung einer Zeile der Tabelle A entspricht.

Tabelle 240: (Verbindungen 240.1 - 240.974)
Verbindungen der allgemeinen Formel I.4, in denen
R³ = Methyl
R⁴ = CH₃
R⁵ = Pyridin-3-yl
bedeutet und der Substituent R⁶ für eine Verbindung einer Zeile der Tabelle A entspricht.

Tabelle 241: (Verbindungen 241.1 - 241.974)
Verbindungen der allgemeinen Formel I.1, in denen
R³ = Methyl
R⁴ = CH₃
R⁵ = Pyridin-4-yl
bedeutet und der Substituent R⁶ für eine Verbindung einer Zeile der Tabelle A entspricht.

Tabelle 242: (Verbindungen 242.1 - 242.974)
Verbindungen der allgemeinen Formel I.2, in denen
R³ = Methyl
R⁴ = CH₃
R⁵ = Pyridin-4-yl
bedeutet und der Substituent R⁶ für eine Verbindung einer Zeile der Tabelle A entspricht.

Tabelle 243: (Verbindungen 243.1 - 243.974)
Verbindungen der allgemeinen Formel I.3, in denen
R³ = Methyl
R⁴ = CH₃
R⁵ = Pyridin-4-yl
bedeutet und der Substituent R⁶ für eine Verbindung einer Zeile der Tabelle A entspricht.

Tabelle 244: (Verbindungen 244.1 - 244.974)
Verbindungen der allgemeinen Formel I.4, in denen
R³ = Methyl
R⁴ = CH₃
R⁵ = Pyridin-4-yl
bedeutet und der Substituent R⁶ für eine Verbindung einer Zeile der Tabelle A entspricht.

Tabelle 245: (Verbindungen 245.1 - 245.974)
Verbindungen der allgemeinen Formel I.1, in denen
R³ = Methyl
R⁴ = CH₃
R⁵ = 3-CH₃-Pyridin-2-yl
bedeutet und der Substituent R⁶ für eine Verbindung einer Zeile der Tabelle A entspricht.

Tabelle 246: (Verbindungen 246.1 - 246.974)
Verbindungen der allgemeinen Formel I.2, in denen
R³ = Methyl
R⁴ = CH₃
R⁵ = 3-CH₃-Pyridin-2-yl
bedeutet und der Substituent R⁶ für eine Verbindung einer Zeile der Tabelle A entspricht.

Tabelle 247: (Verbindungen 247.1 - 247.974)
Verbindungen der allgemeinen Formel I.3, in denen
R³ = Methyl
R⁴ = CH₃
R⁵ = 3-CH₃-Pyridin-2-yl
bedeutet und der Substituent R⁶ für eine Verbindung einer Zeile der Tabelle A entspricht.

Tabelle 248: (Verbindungen 248.1 - 248.974)
Verbindungen der allgemeinen Formel I.4, in denen
R³ = Methyl
R⁴ = CH₃
R⁵ = 3-CH₃-Pyridin-2-yl
bedeutet und der Substituent R⁶ für eine Verbindung einer Zeile der Tabelle A entspricht.

Tabelle 249: (Verbindungen 249.1 - 249.974)
Verbindungen der allgemeinen Formel I.1, in denen
R³ = Methyl
R⁴ = CH₃
R⁵ = 4-CH₃-Pyridin-2-yl
bedeutet und der Substituent R⁶ für eine Verbindung einer Zeile der Tabelle A entspricht.

Tabelle 250: (Verbindungen 250.1 - 250.974)
Verbindungen der allgemeinen Formel I.2, in denen
R³ = Methyl
R⁴ = CH₃
R⁵ = 4-CH₃-Pyridin-2-yl
bedeutet und der Substituent R⁶ für eine Verbindung einer Zeile der Tabelle A entspricht.

Tabelle 251: (Verbindungen 251.1 - 251.974)
Verbindungen der allgemeinen Formel I.3, in denen
R³ = Methyl
R⁴ = CH₃
R⁵ = 4-CH₃-Pyridin-2-yl
bedeutet und der Substituent R⁶ für eine Verbindung einer Zeile der Tabelle A entspricht.

Tabelle 252: (Verbindungen 252.1 - 252.974)
Verbindungen der allgemeinen Formel I.4, in denen
R³ = Methyl
R⁴ = CH₃
R⁵ = 4-CH₃-Pyridin-2-yl
bedeutet und der Substituent R⁶ für eine Verbindung einer Zeile der Tabelle A entspricht.

Tabelle 253: (Verbindungen 253.1 - 253.974)
Verbindungen der allgemeinen Formel I.1, in denen
R³ = Methyl
R⁴ = CH₃
R⁵ = 3-OCH₃-Pyridin-2-yl
bedeutet und der Substituent R⁶ für eine Verbindung einer Zeile der Tabelle A entspricht.

Tabelle 254: (Verbindungen 254.1 - 254.974)
Verbindungen der allgemeinen Formel I.2, in denen
R³ = Methyl
R⁴ = CH₃
R⁵ = 3-OCH₃-Pyridin-2-yl
bedeutet und der Substituent R⁶ für eine Verbindung einer Zeile der Tabelle A entspricht.

Tabelle 255: (Verbindungen 255.1 - 255.974)
Verbindungen der allgemeinen Formel I.3, in denen
R³ = Methyl
R⁴ = CH₃
R⁵ = 3-OCH₃-Pyridin-2-yl
bedeutet und der Substituent R⁶ für eine Verbindung einer Zeile der Tabelle A entspricht.

Tabelle 256: (Verbindungen 256.1 - 256.974)
Verbindungen der allgemeinen Formel I.4, in denen
R³ = Methyl
R⁴ = CH₃
R⁵ = 3-OCH₃-Pyridin-2-yl
bedeutet und der Substituent R⁶ für eine Verbindung einer Zeile der Tabelle A entspricht.

Tabelle 257: (Verbindungen 257.1 - 257.974)
Verbindungen der allgemeinen Formel I.1, in denen
R³ = Methyl
R⁴ = CH₃
R⁵ = 4-Cl-Pyridin-2-yl
bedeutet und der Substituent R⁶ für eine Verbindung einer Zeile der Tabelle A entspricht.

Tabelle 258: (Verbindungen 258.1 - 258.974)
Verbindungen der allgemeinen Formel I.2, in denen
R³ = Methyl
R⁴ = CH₃
R⁵ = 4-Cl-Pyridin-2-yl
bedeutet und der Substituent R⁶ für eine Verbindung einer Zeile der Tabelle A entspricht.

Tabelle 259: (Verbindungen 259.1 - 259.974)
Verbindungen der allgemeinen Formel I.3, in denen
R³ = Methyl
R⁴ = CH₃
R⁵ = 4-Cl-Pyridin-2-yl
bedeutet und der Substituent R⁶ für eine Verbindung einer Zeile der Tabelle A entspricht.

Tabelle 260: (Verbindungen 260.1 - 260.974)
Verbindungen der allgemeinen Formel I.4, in denen
R³ = Methyl
R⁴ = CH₃
R⁵ = 4-Cl-Pyridin-2-yl
bedeutet und der Substituent R⁶ für eine Verbindung einer Zeile der Tabelle A entspricht.

Tabelle 261: (Verbindungen 261.1 - 261.974)
Verbindungen der allgemeinen Formel I.1, in denen
R³ = Methyl
R⁴ = CH₃
R⁵ = 5-Cl-Pyridin-2-yl
bedeutet und der Substituent R⁶ für eine Verbindung einer Zeile der Tabelle A entspricht.

Tabelle 262: (Verbindungen 262.1 - 262.974)
Verbindungen der allgemeinen Formel I.2, in denen
R³ = Methyl
R⁴ = CH₃
R⁵ = 5-Cl-Pyridin-2-yl
bedeutet und der Substituent R⁶ für eine Verbindung einer Zeile der Tabelle A entspricht.

Tabelle 263: (Verbindungen 263.1 - 263.974)
Verbindungen der allgemeinen Formel I.3, in denen
R³ = Methyl
R⁴ = CH₃
R⁵ = 5-Cl-Pyridin-2-yl
bedeutet und der Substituent R⁶ für eine Verbindung einer Zeile der Tabelle A entspricht.

Tabelle 264: (Verbindungen 264.1 - 264.974)
Verbindungen der allgemeinen Formel I.4, in denen
R³ = Methyl
R⁴ = CH₃
R⁵ = 5-Cl-Pyridin-2-yl
bedeutet und der Substituent R⁶ für eine Verbindung einer Zeile der Tabelle A entspricht.

Tabelle 265: (Verbindungen 265.1 - 265.974)
Verbindungen der allgemeinen Formel I.1, in denen
R³ = Methyl
R⁴ = CH₃
R⁵ = Pyrimidin-2-yl
bedeutet und der Substituent R⁶ für eine Verbindung einer Zeile der Tabelle A entspricht.

Tabelle 266: (Verbindungen 266.1 - 266.974)
Verbindungen der allgemeinen Formel I.2, in denen
R³ = Methyl
R⁴ = CH₃
R⁵ = Pyrimidin-2-yl
bedeutet und der Substituent R⁶ für eine Verbindung einer Zeile der Tabelle A entspricht.

Tabelle 267: (Verbindungen 267.1 - 267.974)
Verbindungen der allgemeinen Formel I.3, in denen
R³ = Methyl
R⁴ = CH₃
R⁵ = Pyrimidin-2-yl
bedeutet und der Substituent R⁶ für eine Verbindung einer Zeile der Tabelle A entspricht.

Tabelle 268: (Verbindungen 268.1 - 268.974)
Verbindungen der allgemeinen Formel I.4, in denen
R³ = Methyl
R⁴ = CH₃
R⁵ = Pyrimidin-2-yl
bedeutet und der Substituent R⁶ für eine Verbindung einer Zeile der Tabelle A entspricht.

Tabelle 269: (Verbindungen 269.1 - 269.974)
Verbindungen der allgemeinen Formel I.1, in denen
R³ = Methyl
R⁴ = CH₃
R⁵ = 5-Cl-Pyrimidin-2-yl
bedeutet und der Substituent R⁶ für eine Verbindung einer Zeile der Tabelle A entspricht.

Tabelle 270: (Verbindungen 270.1 - 270.974)
Verbindungen der allgemeinen Formel I.2, in denen
R³ = Methyl
R⁴ = CH₃
R⁵ = 5-Cl-Pyrimidin-2-yl
bedeutet und der Substituent R⁶ für eine Verbindung einer Zeile der Tabelle A entspricht.

Tabelle 271: (Verbindungen 271.1 - 271.974)
Verbindungen der allgemeinen Formel I.3, in denen
R³ = Methyl
R⁴ = CH₃
R⁵ = 5-Cl-Pyrimidin-2-yl
bedeutet und der Substituent R⁶ für eine Verbindung einer Zeile der Tabelle A entspricht.

Tabelle 272: (Verbindungen 272.1 - 272.974)
Verbindungen der allgemeinen Formel I.4, in denen
R³ = Methyl
R⁴ = CH₃
R⁵ = 5-Cl-Pyrimidin-2-yl
bedeutet und der Substituent R⁶ für eine Verbindung einer Zeile der Tabelle A entspricht.

Tabelle 273: (Verbindungen 273.1 - 273.974)
Verbindungen der allgemeinen Formel I.1, in denen
R³ = Methyl
R⁴ = CH₃
R⁵ = 4-CH₃-Pyrimidin-2-yl
bedeutet und der Substituent R⁶ für eine Verbindung einer Zeile der Tabelle A entspricht.

Tabelle 274: (Verbindungen 274.1 - 274.974)
Verbindungen der allgemeinen Formel I.2, in denen
R³ = Methyl
R⁴ = CH₃
R⁵ = 4-CH₃-Pyrimidin-2-yl
bedeutet und der Substituent R⁶ für eine Verbindung einer Zeile der Tabelle A entspricht.

Tabelle 275: (Verbindungen 275.1 - 275.974)
Verbindungen der allgemeinen Formel I.3, in denen
R³ = Methyl
R⁴ = CH₃
R⁵ = 4-CH₃-Pyrimidin-2-yl
bedeutet und der Substituent R⁶ für eine Verbindung einer Zeile der Tabelle A entspricht.

Tabelle 276: (Verbindungen 276.1 - 276.974)
Verbindungen der allgemeinen Formel I.4, in denen
R³ = Methyl
R⁴ = CH₃
R⁵ = 4-CH₃-Pyrimidin-2-yl
bedeutet und der Substituent R⁶ für eine Verbindung einer Zeile der Tabelle A entspricht.

Tabelle 277: (Verbindungen 277.1 - 277.974)
Verbindungen der allgemeinen Formel I.1, in denen
R³ = Methyl
R⁴ = CH₃
R⁵ = 4-Cl-Furan-2-yl
bedeutet und der Substituent R⁶ für eine Verbindung einer Zeile der Tabelle A entspricht.

Tabelle 278: (Verbindungen 278.1 - 278.974)
Verbindungen der allgemeinen Formel I.2, in denen
R³ = Methyl
R⁴ = CH₃
R⁵ = 4-Cl-Furan-2-yl
bedeutet und der Substituent R⁶ für eine Verbindung einer Zeile der Tabelle A entspricht.

Tabelle 279: (Verbindungen 279.1 - 279.974)
Verbindungen der allgemeinen Formel I.3, in denen
R³ = Methyl
R⁴ = CH₃
R⁵ = 4-Cl-Furan-2-yl
bedeutet und der Substituent R⁶ für eine Verbindung einer Zeile der Tabelle A entspricht.

Tabelle 280: (Verbindungen 280.1 - 280.974)
Verbindungen der allgemeinen Formel I.4, in denen
R³ = Methyl
R⁴ = CH₃
R⁵ = 4-Cl-Furan-2-yl
bedeutet und der Substituent R⁶ für eine Verbindung einer Zeile der Tabelle A entspricht.

Tabelle 281: (Verbindungen 281.1 - 281.974)
Verbindungen der allgemeinen Formel I.1, in denen
R³ = Methyl
R⁴ = CH₃
R⁵ = 4-CH₃-Furan-2-yl
bedeutet und der Substituent R⁶ für eine Verbindung einer Zeile der Tabelle A entspricht.

Tabelle 282: (Verbindungen 282.1 - 282.974)
Verbindungen der allgemeinen Formel I.2, in denen
R³ = Methyl
R⁴ = CH₃
R⁵ = 4-CH₃-Furan-2-yl
bedeutet und der Substituent R⁶ für eine Verbindung einer Zeile der Tabelle A entspricht.

Tabelle 283: (Verbindungen 283.1 - 283.974)
Verbindungen der allgemeinen Formel I.3, in denen
R³ = Methyl
R⁴ = CH₃
R⁵ = 4-CH₃-Furan-2-yl
bedeutet und der Substituent R⁶ für eine Verbindung einer Zeile der Tabelle A entspricht.

Tabelle 284: (Verbindungen 284.1 - 284.974)
Verbindungen der allgemeinen Formel I.4, in denen
R³ = Methyl
R⁴ = CH₃
R⁵ = 4-CH₃-Furan-2-yl
bedeutet und der Substituent R⁶ für eine Verbindung einer Zeile der Tabelle A entspricht.

Tabelle 285: (Verbindungen 285.1 - 285.974)
Verbindungen der allgemeinen Formel I.1, in denen
R³ = Methyl
R⁴ = CH₃
R⁵ = Thien-2-yl
bedeutet und der Substituent R⁶ für eine Verbindung einer Zeile der Tabelle A entspricht.

Tabelle 286: (Verbindungen 286.1 - 286.974)
Verbindungen der allgemeinen Formel I.2, in denen
R³ = Methyl
R⁴ = CH₃
R⁵ = Thien-2-yl
bedeutet und der Substituent R⁶ für eine Verbindung einer Zeile der Tabelle A entspricht.

Tabelle 287: (Verbindungen 287.1 - 287.974)
Verbindungen der allgemeinen Formel I.3, in denen
R³ = Methyl
R⁴ = CH₃
R⁵ = Thien-2-yl
bedeutet und der Substituent R⁶ für eine Verbindung einer Zeile der Tabelle A entspricht.

Tabelle 288: (Verbindungen 288.1 - 288.974)
Verbindungen der allgemeinen Formel I.4, in denen
R³ = Methyl
R⁴ = CH₃
R⁵ = Thien-2-yl
bedeutet und der Substituent R⁶ für eine Verbindung einer Zeile der Tabelle A entspricht.

Tabelle 289: (Verbindungen 289.1 - 289.974)
Verbindungen der allgemeinen Formel I.1, in denen
R³ = Methyl
R⁴ = CH₃
R⁵ = Thien-3-yl
bedeutet und der Substituent R⁶ für eine Verbindung einer Zeile der Tabelle A entspricht.

Tabelle 290: (Verbindungen 290.1 - 290.974)
Verbindungen der allgemeinen Formel I.2, in denen
R³ = Methyl
R⁴ = CH₃
R⁵ = Thien-3-yl
bedeutet und der Substituent R⁶ für eine Verbindung einer Zeile der Tabelle A entspricht.

Tabelle 291: (Verbindungen 291.1 - 291.974)
Verbindungen der allgemeinen Formel I.3, in denen
R³ = Methyl
R⁴ = CH₃
R⁵ = Thien-3-yl
bedeutet und der Substituent R⁶ für eine Verbindung einer Zeile der Tabelle A entspricht.

Tabelle 292: (Verbindungen 292.1 - 292.974)
Verbindungen der allgemeinen Formel I.4, in denen
R³ = Methyl
R⁴ = CH₃
R⁵ = Thien-3-yl
bedeutet und der Substituent R⁶ für eine Verbindung einer Zeile der Tabelle A entspricht.

Tabelle 293: (Verbindungen 293.1 - 293.974)
Verbindungen der allgemeinen Formel I.1, in denen
R³ = Methyl
R⁴ = CH₃
R⁵ = Oxazol-2-yl
bedeutet und der Substituent R⁶ für eine Verbindung einer Zeile der Tabelle A entspricht.

Tabelle 294: (Verbindungen 294.1 - 294.974)
Verbindungen der allgemeinen Formel I.2, in denen
R³ = Methyl
R⁴ = CH₃
R⁵ = Oxazol-2-yl
bedeutet und der Substituent R⁶ für eine Verbindung einer Zeile der Tabelle A entspricht.

Tabelle 295: (Verbindungen 295.1 - 295.974)
Verbindungen der allgemeinen Formel I.3, in denen
R³ = Methyl
R⁴ = CH₃
R⁵ = Oxazol-2-yl
bedeutet und der Substituent R⁶ für eine Verbindung einer Zeile der Tabelle A entspricht.

Tabelle 296: (Verbindungen 296.1 - 296.974)
Verbindungen der allgemeinen Formel I.4, in denen
R³ = Methyl
R⁴ = CH₃
R⁵ = Oxazol-2-yl
bedeutet und der Substituent R⁶ für eine Verbindung einer Zeile der Tabelle A entspricht.

Tabelle 297: (Verbindungen 297.1 - 297.974)
Verbindungen der allgemeinen Formel I.1, in denen
R³ = Methyl
R⁴ = CH₃
R⁵ = Isoxazol-3-yl
bedeutet und der Substituent R⁶ für eine Verbindung einer Zeile der Tabelle A entspricht.

Tabelle 298: (Verbindungen 298.1 - 298.974)
Verbindungen der allgemeinen Formel I.2, in denen
R³ = Methyl
R⁴ = CH₃
R⁵ = Isoxazol-3-yl
bedeutet und der Substituent R⁶ für eine Verbindung einer Zeile der Tabelle A entspricht.

Tabelle 299: (Verbindungen 299.1 - 299.974)
Verbindungen der allgemeinen Formel I.3, in denen
R³ = Methyl
R⁴ = CH₃
R⁵ = Isoxazol-3-yl
bedeutet und der Substituent R⁶ für eine Verbindung einer Zeile der Tabelle A entspricht.

Tabelle 300: (Verbindungen 300.1 - 300.974)
Verbindungen der allgemeinen Formel I.4, in denen
R³ = Methyl
R⁴ = CH₃
R⁵ = Isoxazol-3-yl
bedeutet und der Substituent R⁶ für eine Verbindung einer Zeile der Tabelle A entspricht.

Tabelle 301: (Verbindungen 301.1 - 301.974)
Verbindungen der allgemeinen Formel I.1, in denen
R³ = Methyl
R⁴ = CH₃
R⁵ = Isoxazol-4-yl
bedeutet und der Substituent R⁶ für eine Verbindung einer Zeile der Tabelle A entspricht.

Tabelle 302: (Verbindungen 302.1 - 302.974)
Verbindungen der allgemeinen Formel I.2, in denen
R³ = Methyl
R⁴ = CH₃
R⁵ = Isoxazol-4-yl
bedeutet und der Substituent R⁶ für eine Verbindung einer Zeile der Tabelle A entspricht.

Tabelle 303: (Verbindungen 303.1 - 303.974)
Verbindungen der allgemeinen Formel I.3, in denen
R³ = Methyl
R⁴ = CH₃
R⁵ = Isoxazol-4-yl
bedeutet und der Substituent R⁶ für eine Verbindung einer Zeile der Tabelle A entspricht.

Tabelle 304: (Verbindungen 304.1 -304.974)
Verbindungen der allgemeinen Formel I,4, in denen
R³ = Methyl
R⁴ = CH₃
R⁵ = Isoxazol-4-yl
bedeutet und der Substituent R⁶ für eine Verbindung einer Zeile der Tabelle A entspricht.

Tabelle 305: (Verbindungen 305.1 - 305.974)
Verbindungen der allgemeinen Formel I.1, in denen
R³ = Methyl
R⁴ = CH₃
R⁵ = Pyrazol-3-yl
bedeutet und der Substituent R⁶ für eine Verbindung einer Zeile der Tabelle A entspricht.

Tabelle 306: (Verbindungen 306.1 - 306.974)
Verbindungen der allgemeinen Formel I.2, in denen
R³ = Methyl
R⁴ = CH₃
R⁵ = Pyrazol-3-yl
bedeutet und der Substituent R⁶ für eine Verbindung einer Zeile der Tabelle A entspricht.

Tabelle 307: (Verbindungen 307.1 - 307.974)
Verbindungen der allgemeinen Formel I.3, in denen
R³ = Methyl
R⁴ = CH₃
R⁵ = Pyrazol-3-yl
bedeutet und der Substituent R⁶ für eine Verbindung einer Zeile der Tabelle A entspricht.

Tabelle 308: (Verbindungen 308.1 - 308.974)
Verbindungen der allgemeinen Formel I.4, in denen
R³ = Methyl
R⁴ = CH₃
R⁵ = Pyrazol-3-yl
bedeutet und der Substituent R⁶ für eine Verbindung einer Zeile der Tabelle A entspricht.

Tabelle 309: (Verbindungen 309.1 - 309.974)
Verbindungen der allgemeinen Formel I.1, in denen
R³ = Methyl
R⁴ = CH₃
R⁵ = 1,3,4-Oxadiazol-5-yl
bedeutet und der Substituent R⁶ für eine Verbindung einer Zeile der Tabelle A entspricht.

Tabelle 310: (Verbindungen 310.1 - 310.974)
Verbindungen der allgemeinen Formel I.2, in denen
R³ = Methyl
R⁴ = CH₃
R⁵ = 1,3,4-Oxadiazol-5-yl
bedeutet und der Substituent R⁶ für eine Verbindung einer Zeile der Tabelle A entspricht.

Tabelle 311: (Verbindungen 311.1 - 311.974)
Verbindungen der allgemeinen Formel I.3, in denen
R³ = Methyl
R⁴ = CH₃
R⁵ = 1,3,4-Oxadiazol-5-yl
bedeutet und der Substituent R⁶ für eine Verbindung einer Zeile der Tabelle A entspricht.

Tabelle 312: (Verbindungen 312.1 - 312.974)
Verbindungen der allgemeinen Formel I.4, in denen
R³ = Methyl
R⁴ = CH₃
R⁵ = 1,3,4-Oxadiazol-5-yl
bedeutet und der Substituent R⁶ für eine Verbindung einer Zeile der Tabelle A entspricht.

Tabelle 313: (Verbindungen 313.1 - 313.974)
Verbindungen der allgemeinen Formel I.1, in denen
R³ = Methyl
R⁴ = CH₃
R⁵ = 1,2,4-Triazol-3-yl
bedeutet und der Substituent R⁶ für eine Verbindung einer Zeile der Tabelle A entspricht.

Tabelle 314: (Verbindungen 314.1 - 314.974)
Verbindungen der allgemeinen Formel I.2, in denen
R³ = Methyl
R⁴ = CH₃
R⁵ = 1,2,4-Triazol-3-yl
bedeutet und der Substituent R⁶ für eine Verbindung einer Zeile der Tabelle A entspricht.

Tabelle 315: (Verbindungen 315.1 - 315.974)
Verbindungen der allgemeinen Formel I.3, in denen
R³ = Methyl
R⁴ = CH₃
R⁵ = 1,2,4-Triazol-3-yl
bedeutet und der Substituent R⁶ für eine Verbindung einer Zeile der Tabelle A entspricht.

Tabelle 316: (Verbindungen 316.1 - 316.974)
Verbindungen der allgemeinen Formel I.4, in denen
R³ = Methyl
R⁴ = CH₃
R⁵ = 1,2,4-Triazol-3-yl
bedeutet und der Substituent R⁶ für eine Verbindung einer Zeile der Tabelle A entspricht.

Tabelle 317: (Verbindungen 317.1 - 317.974)
Verbindungen der allgemeinen Formel I.1, in denen
R³ = Methyl
R⁴ = CH₃
R⁵ = Pyrrol-2-yl
bedeutet und der Substituent R⁶ für eine Verbindung einer Zeile der Tabelle A entspricht.

Tabelle 318: (Verbindungen 318.1 - 318.974)
Verbindungen der allgemeinen Formel I.2, in denen
R³ = Methyl
R⁴ = CH₃
R⁵ = Pyrrol-2-yl
bedeutet und der Substituent R⁶ für eine Verbindung einer Zeile der Tabelle A entspricht.

Tabelle 319: (Verbindungen 319.1 - 319.974)
Verbindungen der allgemeinen Formel I.3, in denen
R³ = Methyl
R⁴ = CH₃
R⁵ = Pyrrol-2-yl
bedeutet und der Substituent R⁶ für eine Verbindung einer Zeile der Tabelle A entspricht.

Tabelle 320: (Verbindungen 320.1 - 320.974)
Verbindungen der allgemeinen Formel I.4, in denen
R³ = Methyl
R⁴ = CH₃
R⁵ = Pyrrol-2-yl
bedeutet und der Substituent R⁶ für eine Verbindung einer Zeile der Tabelle A entspricht.

Tabelle 321: (Verbindungen 321.1 - 321.974)
Verbindungen der allgemeinen Formel I.1, in denen
R³ = Methyl
R⁴ = C₂H₅
R⁵ = H
bedeutet und der Substituent R⁶ für eine Verbindung einer Zeile der Tabelle A entspricht.

Tabelle 322: (Verbindungen 322.1 - 322.974)
Verbindungen der allgemeinen Formel I.2, in denen
R³ = Methyl
R⁴ = C₂H₅
R⁵ = H
bedeutet und der Substituent R⁶ für eine Verbindung einer Zeile der Tabelle A entspricht.

Tabelle 323: (Verbindungen 323.1 - 323.974)
Verbindungen der allgemeinen Formel I.3, in denen
R³ = Methyl
R⁴ = C₂H₅
R⁵ = H
bedeutet und der Substituent R⁶ für eine Verbindung einer Zeile der Tabelle A entspricht.

Tabelle 324: (Verbindungen 324.1 - 324.974)
Verbindungen der allgemeinen Formel I.4, in denen
R³ = Methyl
R⁴ = C₂H₅
R⁵ = H
bedeutet und der Substituent R⁶ für eine Verbindung einer Zeile der Tabelle A entspricht.

Tabelle 325: (Verbindungen 325.1 - 325.974)
Verbindungen der allgemeinen Formel I.1, in denen
R³ = Methyl
R⁴ = C₂H₅
R⁵ = CH₃
bedeutet und der Substituent R⁶ für eine Verbindung einer Zeile der Tabelle A entspricht.

Tabelle 326: (Verbindungen 326.1 - 326.974)
Verbindungen der allgemeinen Formel I.2, in denen
R³ = Methyl
R⁴ = C₂H₅
R⁵ = CH₃
bedeutet und der Substituent R⁶ für eine Verbindung einer Zeile der Tabelle A entspricht.

Tabelle 327: (Verbindungen 327.1 - 327.974)
Verbindungen der allgemeinen Formel I.3, in denen
R³ = Methyl
R⁴ = C₂H₅
R⁵ = CH₃
bedeutet und der Substituent R⁶ für eine Verbindung einer Zeile der Tabelle A entspricht.

Tabelle 328: (Verbindungen 328.1 - 328.974)
Verbindungen der allgemeinen Formel I.4, in denen
R³ = Methyl
R⁴ = C₂H₅
R⁵ = CH₃
bedeutet und der Substituent R⁶ für eine Verbindung einer Zeile der Tabelle A entspricht.

Tabelle 329: (Verbindungen 329.1 - 329.974)
Verbindungen der allgemeinen Formel I.1, in denen
R³ = Methyl
R⁴ = C₂H₅
R⁵ = C₂H₅
bedeutet und der Substituent R⁶ für eine Verbindung einer Zeile der Tabelle A entspricht.

Tabelle 330: (Verbindungen 330.1 - 330.974)
Verbindungen der allgemeinen Formel I.2, in denen
R³ = Methyl
R⁴ = C₂H₅
R⁵ = C₂H₅
bedeutet und der Substituent R⁶ für eine Verbindung einer Zeile der Tabelle A entspricht.

Tabelle 331: (Verbindungen 331.1 - 331.974)
Verbindungen der allgemeinen Formel I.3, in denen
R³ = Methyl
R⁴ = C₂H₅
R⁵ = C₂H₅
bedeutet und der Substituent R⁶ für eine Verbindung einer Zeile der Tabelle A entspricht.

Tabelle 332: (Verbindungen 332.1 - 332.974)
Verbindungen der allgemeinen Formel I.4, in denen
R³ = Methyl
R⁴ = C₂H₅
R⁵ = C₂H₅
bedeutet und der Substituent R⁶ für eine Verbindung einer Zeile der Tabelle A entspricht.

Tabelle 333: (Verbindungen 333.1 - 333.974)
Verbindungen der allgemeinen Formel I.1, in denen
R³ = Methyl
R⁴ = C₂H₅
R⁵ = n-C₃H₇
bedeutet und der Substituent R⁶ für eine Verbindung einer Zeile der Tabelle A entspricht.

Tabelle 334: (Verbindungen 334.1 - 334.974)
Verbindungen der allgemeinen Formel I.2, in denen
R³ = Methyl
R⁴ = C₂H₅
R⁵ = n-C₃H₇
bedeutet und der Substituent R⁶ für eine Verbindung einer Zeile der Tabelle A entspricht.

Tabelle 335: (Verbindungen 335.1 - 335.974)
Verbindungen der allgemeinen Formel I.3, in denen
R³ = Methyl
R⁴ = C₂H₅
R⁵ = n-C₃H₇
bedeutet und der Substituent R⁶ für eine Verbindung einer Zeile der Tabelle A entspricht.

Tabelle 336: (Verbindungen 336.1 - 336.974)
Verbindungen der allgemeinen Formel I.4, in denen
R³ = Methyl
R⁴ = C₂H₅
R⁵ = n-C₃H₇
bedeutet und der Substituent R⁶ für eine Verbindung einer Zeile der Tabelle A entspricht.

Tabelle 337: (Verbindungen 337.1 - 337.974)
Verbindungen der allgemeinen Formel I.1, in denen
R³ = Methyl
R⁴ = C₂H₅
R⁵ = i-C₃H₇
bedeutet und der Substituent R⁶ für eine Verbindung einer Zeile der Tabelle A entspricht.

Tabelle 338: (Verbindungen 338.1 - 338.974)
Verbindungen der allgemeinen Formel I.2, in denen
R³ = Methyl
R⁴ = C₂H₅
R⁵ = i-C₃H₇
bedeutet und der Substituent R⁶ für eine Verbindung einer Zeile der Tabelle A entspricht.

Tabelle 339: (Verbindungen 339.1 - 339.974)
Verbindungen der allgemeinen Formel I.3, in denen
R³ = Methyl
R⁴ = C₂H₅
R⁵ = i-C₃H₇
bedeutet und der Substituent R⁶ für eine Verbindung einer Zeile der Tabelle A entspricht.

Tabelle 340: (Verbindungen 340.1 - 340.974)
Verbindungen der allgemeinen Formel I.4, in denen
R³ = Methyl
R⁴ = C₂H₅
R⁵ = i-C₃H₇
bedeutet und der Substituent R⁶ für eine Verbindung einer Zeile der Tabelle A entspricht.

Tabelle 341: (Verbindungen 341.1 - 341.974)
Verbindungen der allgemeinen Formel I.1, in denen
R³ = Methyl
R⁴ = C₂H₅
R⁵ = Cyclopropyl
bedeutet und der Substituent R⁶ für eine Verbindung einer Zeile der Tabelle A entspricht.

Tabelle 342: (Verbindungen 342.1 - 342.974)
Verbindungen der allgemeinen Formel I.2, in denen
R³ = Methyl
R⁴ = C₂H₅
R⁵ = Cyclopropyl
bedeutet und der Substituent R⁶ für eine Verbindung einer Zeile der Tabelle A entspricht.

Tabelle 343: (Verbindungen 343.1 - 343.974)
Verbindungen der allgemeinen Formel I.3, in denen
R³ = Methyl
R⁴ = C₂H₅
R⁵ = Cyclopropyl
bedeutet und der Substituent R⁶ für eine Verbindung einer Zeile der Tabelle A entspricht.

Tabelle 344: (Verbindungen 344.1 - 344.974)
Verbindungen der allgemeinen Formel I.4, in denen
R³ = Methyl
R⁴ = C₂H₅
R⁵ = Cyclopropyl
bedeutet und der Substituent R⁶ für eine Verbindung einer Zeile der Tabelle A entspricht.

Tabelle 345: (Verbindungen 345.1 - 345.974)
Verbindungen der allgemeinen Formel I.1, in denen
R³ = Methyl
R⁴ = C₂H₅
R⁵ = CN
bedeutet und der Substituent R⁶ für eine Verbindung einer Zeile der Tabelle A entspricht.

Tabelle 346: (Verbindungen 346.1 - 346.974)
Verbindungen der allgemeinen Formel I.2, in denen
R³ = Methyl
R⁴ = C₂H₅
R⁵ = CN
bedeutet und der Substituent R⁶ für eine Verbindung einer Zeile der Tabelle A entspricht.

Tabelle 347: (Verbindungen 347.1 - 347.974)
Verbindungen der allgemeinen Formel I.3, in denen
R³ = Methyl
R⁴ = C₂H₅
R⁵ = CN
bedeutet und der Substituent R⁶ für eine Verbindung einer Zeile der Tabelle A entspricht.

Tabelle 348: (Verbindungen 348.1 - 348.974)
Verbindungen der allgemeinen Formel I.4, in denen
R³ = Methyl
R⁴ = C₂H₅
R⁵ = CN
bedeutet und der Substituent R⁶ für eine Verbindung einer Zeile der Tabelle A entspricht.

Tabelle 349: (Verbindungen 349.1 - 349.974)
Verbindungen der allgemeinen Formel I.1, in denen
R³ = Methyl
R⁴ = C₂H₅
R⁵ = C₆H₅
bedeutet und der Substituent R⁶ für eine Verbindung einer Zeile der Tabelle A entspricht.

Tabelle 350: (Verbindungen 350.1 - 350.974)
Verbindungen der allgemeinen Formel I.2, in denen
R³ = Methyl
R⁴ = C₂H₅
R⁵ = C₆H₅
bedeutet und der Substituent R⁶ für eine Verbindung einer Zeile der Tabelle A entspricht.

Tabelle 351: (Verbindungen 351.1 - 351.974)
Verbindungen der allgemeinen Formel I.3, in denen
R³ = Methyl
R⁴ = C₂H₅
R⁵ = C₆H₅
bedeutet und der Substituent R⁶ für eine Verbindung einer Zeile der Tabelle A entspricht.

Tabelle 352: (Verbindungen 352.1 - 352.974)
Verbindungen der allgemeinen Formel I.4, in denen
R³ = Methyl
R⁴ = C₂H₅
R⁵ = C₆H₅
bedeutet und der Substituent R⁶ für eine Verbindung einer Zeile der Tabelle A entspricht.

Tabelle 353: (Verbindungen 353.1 - 353.974)
Verbindungen der allgemeinen Formel I.1, in denen
R³ = Methyl
R⁴ = C₂H₅
R⁵ = 2-F-C₆H₄
bedeutet und der Substituent R⁶ für eine Verbindung einer Zeile der Tabelle A entspricht.

Tabelle 354: (Verbindungen 354.1 - 354.974)
Verbindungen der allgemeinen Formel I.2, in denen
R³ = Methyl
R⁴ = C₂H₅
R⁵ = 2-F-C₆H₄
bedeutet und der Substituent R⁶ für eine Verbindung einer Zeile der Tabelle A entspricht.

Tabelle 355: (Verbindungen 355.1 - 355.974)
Verbindungen der allgemeinen Formel I.3, in denen
R³ = Methyl
R⁴ = C₂H₅
R⁵ = 2-F-C₆H₄
bedeutet und der Substituent R⁶ für eine Verbindung einer Zeile der Tabelle A entspricht.

Tabelle 356: (Verbindungen 356.1 - 356.974)
Verbindungen der allgemeinen Formel I.4, in denen
R³ = Methyl
R⁴ = C₂H₅
R⁵ = 2-F-C₆H₄
bedeutet und der Substituent R⁶ für eine Verbindung einer Zeile der Tabelle A entspricht.

Tabelle 357: (Verbindungen 357.1 - 357.974)
Verbindungen der allgemeinen Formel I.1, in denen
R³ = Methyl
R⁴ = C₂H₅
R⁵ = 3-F-C₆H₄
bedeutet und der Substituent R⁶ für eine Verbindung einer Zeile der Tabelle A entspricht.

Tabelle 358: (Verbindungen 358.1 - 358.974)
Verbindungen der allgemeinen Formel I.2, in denen
R³ = Methyl
R⁴ = C₂H₅
R⁵ = 3-F-C₆H₄
bedeutet und der Substituent R⁶ für eine Verbindung einer Zeile der Tabelle A entspricht.

Tabelle 359: (Verbindungen 359.1 - 359.974)
Verbindungen der allgemeinen Formel I.3, in denen
R³ = Methyl
R⁴ = C₂H₅
R⁵ = 3-F-C₆H₄
bedeutet und der Substituent R⁶ für eine Verbindung einer Zeile der Tabelle A entspricht.

Tabelle 360: (Verbindungen 360.1 - 360.974)
Verbindungen der allgemeinen Formel I.4, in denen
R³ = Methyl
R⁴ = C₂H₅
R⁵ = 3-F-C₆H₄
bedeutet und der Substituent R⁶ für eine Verbindung einer Zeile der Tabelle A entspricht.

Tabelle 361: (Verbindungen 361.1 - 361.974)
Verbindungen der allgemeinen Formel I.1, in denen
R³ = Methyl
R⁴ = C₂H₅
R⁵ = 4-F-C₆H₄
bedeutet und der Substituent R⁶ für eine Verbindung einer Zeile der Tabelle A entspricht.

Tabelle 362: (Verbindungen 362.1 - 362.974)
Verbindungen der allgemeinen Formel I.2, in denen
R³ = Methyl
R⁴ = C₂H₅
R⁵ = 4-F-C₆H₄
bedeutet und der Substituent R⁶ für eine Verbindung einer Zeile der Tabelle A entspricht.

Tabelle 363: (Verbindungen 363.1 - 363.974)
Verbindungen der allgemeinen Formel I.3, in denen
R³ = Methyl
R⁴ = C₂H₅
R⁵ = 4-F-C₆H₄
bedeutet und der Substituent R⁶ für eine Verbindung einer Zeile der Tabelle A entspricht.

Tabelle 364: (Verbindungen 364.1 - 364.974)
Verbindungen der allgemeinen Formel I.4, in denen
R³ = Methyl
R⁴ = C₂H₅
R⁵ = 4-F-C₆H₄
bedeutet und der Substituent R⁶ für eine Verbindung einer Zeile der Tabelle A entspricht.

Tabelle 365: (Verbindungen 365.1 - 365.974)
Verbindungen der allgemeinen Formel I.1, in denen
R³ = Methyl
R⁴ = C₂H₅
R⁵ = 2-Cl-C₆H₄
bedeutet und der Substituent R⁶ für eine Verbindung einer Zeile der Tabelle A entspricht.

Tabelle 366: (Verbindungen 366.1 - 366.974)
Verbindungen der allgemeinen Formel I.2, in denen
R³ = Methyl
R⁴ = C₂H₅
R⁵ = 2-Cl-C₆H₄
bedeutet und der Substituent R⁶ für eine Verbindung einer Zeile der Tabelle A entspricht.

Tabelle 367: (Verbindungen 367.1 - 367.974)
Verbindungen der allgemeinen Formel I.3, in denen
R³ = Methyl
R⁴ = C₂H₅
R⁵ = 2-Cl-C₆H₄
bedeutet und der Substituent R⁶ für eine Verbindung einer Zeile der Tabelle A entspricht.

Tabelle 368: (Verbindungen 368.1 - 368.974)
Verbindungen der allgemeinen Formel I.4, in denen
R³ = Methyl
R⁴ = C₂H₅
R⁵ = 2-Cl-C₆H₄
bedeutet und der Substituent R⁶ für eine Verbindung einer Zeile der Tabelle A entspricht.

Tabelle 369: (Verbindungen 369.1 - 369.974)
Verbindungen der allgemeinen Formel I.1, in denen
R³ = Methyl
R⁴ = C₂H₅
R⁵ = 3-Cl-C₆H₄
bedeutet und der Substituent R⁶ für eine Verbindung einer Zeile der Tabelle A entspricht.

Tabelle 370: (Verbindungen 370.1 - 370.974)
Verbindungen der allgemeinen Formel I.2, in denen
R³ = Methyl
R⁴ = C₂H₅
R⁵ = 3-Cl-C₆H₄
bedeutet und der Substituent R⁶ für eine Verbindung einer Zeile der Tabelle A entspricht.

Tabelle 371: (Verbindungen 371.1 - 371.974)
Verbindungen der allgemeinen Formel I.3, in denen
R³ = Methyl
R⁴ = C₂H₅
R⁵ = 3-Cl-C₆H₄
bedeutet und der Substituent R⁶ für eine Verbindung einer Zeile der Tabelle A entspricht.

Tabelle 372: (Verbindungen 372.1 - 372.974)
Verbindungen der allgemeinen Formel I,4, in denen
R³ = Methyl
R⁴ = C₂H₅
R⁵ = 3-Cl-C₆H₄
bedeutet und der Substituent R⁶ für eine Verbindung einer Zeile der Tabelle A entspricht.

Tabelle 373: (Verbindungen 373.1 - 373.974)
Verbindungen der allgemeinen Formel I.1, in denen
R³ = Methyl
R⁴ = C₂H₅
R⁵ = 4-Cl-C₆H₄
bedeutet und der Substituent R⁶ für eine Verbindung einer Zeile der Tabelle A entspricht.

Tabelle 374: (Verbindungen 374.1 - 374.974)
Verbindungen der allgemeinen Formel I.2, in denen
R³ = Methyl
R⁴ = C₂H₅
R⁵ = 4-Cl-C₆H₄
bedeutet und der Substituent R⁶ für eine Verbindung einer Zeile der Tabelle A entspricht.

Tabelle 375: (Verbindungen 375.1 - 375.974)
Verbindungen der allgemeinen Formel I.3, in denen
R³ = Methyl
R⁴ = C₂H₅
R⁵ = 4-Cl-C₆H₄
bedeutet und der Substituent R⁶ für eine Verbindung einer Zeile der Tabelle A entspricht.

Tabelle 376: (Verbindungen 376.1 - 376.974)
Verbindungen der allgemeinen Formel I.4, in denen
R³ = Methyl
R⁴ = C₂H₅
R⁵ = 4-Cl-C₆H₄
bedeutet und der Substituent R⁶ für eine Verbindung einer Zeile der Tabelle A entspricht.

Tabelle 377: (Verbindungen 377.1 - 377.974)
Verbindungen der allgemeinen Formel I.1, in denen
R³ = Methyl
R⁴ = C₂H₅
R⁵ = 2-Br-C₆H₄
bedeutet und der Substituent R⁶ für eine Verbindung einer Zeile der Tabelle A entspricht.

Tabelle 378: (Verbindungen 378.1 - 378.974)
Verbindungen der allgemeinen Formel I.2, in denen
R³ = Methyl
R⁴ = C₂H₅
R⁵ = 2-Br-C₆H₄
bedeutet und der Substituent R⁶ für eine Verbindung einer Zeile der Tabelle A entspricht.

Tabelle 379: (Verbindungen 379.1 - 379.974)
Verbindungen der allgemeinen Formel I.3, in denen
R³ = Methyl
R⁴ = C₂H₅
R⁵ = 2-Br-C₆H₄
bedeutet und der Substituent R⁶ für eine Verbindung einer Zeile der Tabelle A entspricht.

Tabelle 380: (Verbindungen 380.1 - 380.974)
Verbindungen der allgemeinen Formel I.4, in denen
R³ = Methyl
R⁴ = C₂H₅
R⁵ = 2-Br-C₆H₄
bedeutet und der Substituent R⁶ für eine Verbindung einer Zeile der Tabelle A entspricht.

Tabelle 381: (Verbindungen 381.1 - 381.974)
Verbindungen der allgemeinen Formel I.1, in denen
R³ = Methyl
R⁴ = C₂H₅
R⁵ = 3-Br-C₆H₄
bedeutet und der Substituent R⁶ für eine Verbindung einer Zeile der Tabelle A entspricht.

Tabelle 382: (Verbindungen 382.1 - 382.974)
Verbindungen der allgemeinen Formel I.2, in denen
R³ = Methyl
R⁴ = C₂H₅
R⁵ = 3-Br-C₆H₄
bedeutet und der Substituent R⁶ für eine Verbindung einer Zeile der Tabelle A entspricht.

Tabelle 383: (Verbindungen 338.1 - 383.974)
Verbindungen der allgemeinen Formel I.3, in denen
R³ = Methyl
R⁴ = C₂H₅
R⁵ = 3-Br-C₆H₄
bedeutet und der Substituent R⁶ für eine Verbindung einer Zeile der Tabelle A entspricht.

Tabelle 384: (Verbindungen 384.1 - 384.974)
Verbindungen der allgemeinen Formel I.4, in denen
R³ = Methyl
R⁴ = C₂H₅
R⁵ = 3-Br-C₆H₄
bedeutet und der Substituent R⁶ für eine Verbindung einer Zeile der Tabelle A entspricht.

Tabelle 385: (Verbindungen 385.1 - 385.974)
Verbindungen der allgemeinen Formel I.1, in denen
R³ = Methyl
R⁴ = C₂H₅
R⁵ = 4-Br-C₆H₄
bedeutet und der Substituent R⁶ für eine Verbindung einer Zeile der Tabelle A entspricht.

Tabelle 386: (Verbindungen 386.1 - 386.974)
Verbindungen der allgemeinen Formel I.2, in denen
R³ = Methyl
R⁴ = C₂H₅
R⁵ = 4-Br-C₆H₄
bedeutet und der Substituent R⁶ für eine Verbindung einer Zeile der Tabelle A entspricht.

Tabelle 387: (Verbindungen 387.1 - 387.974)
Verbindungen der allgemeinen Formel I.3, in denen
R³ = Methyl
R⁴ = C₂H₅
R⁵ = 4-Br-C₆H₄
bedeutet und der Substituent R⁶ für eine Verbindung einer Zeile der Tabelle A entspricht.

Tabelle 388: (Verbindungen 388.1 - 388.974)
Verbindungen der allgemeinen Formel I.4, in denen
R³ = Methyl
R⁴ = C₂H₅
R⁵ = 4-Br-C₆H₄
bedeutet und der Substituent R⁶ für eine Verbindung einer Zeile der Tabelle A entspricht.

Tabelle 389: (Verbindungen 389.1 - 389.974)
Verbindungen der allgemeinen Formel I.1, in denen
R³ = Methyl
R⁴ = C₂H₅
R⁵ = 2,3-F₂-C₆H₃
bedeutet und der Substituent R⁶ für eine Verbindung einer Zeile der Tabelle A entspricht.

Tabelle 390: (Verbindungen 390.1 - 390.974)
Verbindungen der allgemeinen Formel I.2, in denen
R³ = Methyl
R⁴ = C₂H₅
R⁵ = 2,3-F₂-C₆H₃
bedeutet und der Substituent R⁶ für eine Verbindung einer Zeile der Tabelle A entspricht.

Tabelle 391: (Verbindungen 391.1 - 391.974)
Verbindungen der allgemeinen Formel I.3, in denen
R³ = Methyl
R⁴ = C₂H₅
R⁵ = 2,3-F₂-C₆H₃
bedeutet und der Substituent R⁶ für eine Verbindung einer Zeile der Tabelle A entspricht.

Tabelle 392: (Verbindungen 392.1 - 393.974)
Verbindungen der allgemeinen Formel I.4, in denen
R³ = Methyl
R⁴ = C₂H₅
R⁵ = 2,3-F₂-C₆H₃
bedeutet und der Substituent R⁶ für eine Verbindung einer Zeile der Tabelle A entspricht.

Tabelle 393: (Verbindungen 393.1 - 393.974)
Verbindungen der allgemeinen Formel I.1, in denen
R³ = Methyl
R⁴ = C₂H₅
R⁵ = 2,4-F₂-C₆H₃
bedeutet und der Substituent R⁶ für eine Verbindung einer Zeile der Tabelle A entspricht.

Tabelle 394: (Verbindungen 394.1 - 394.974)
Verbindungen der allgemeinen Formel I.2, in denen
R³ = Methyl
R⁴ = C₂H₅
R⁵ = 2,4-F₂-C₆H₃
bedeutet und der Substituent R⁶ für eine Verbindung einer Zeile der Tabelle A entspricht.

Tabelle 395: (Verbindungen 395.1 - 395.974)
Verbindungen der allgemeinen Formel I.3, in denen
R³ = Methyl
R⁴ = C₂H₅
R⁵ = 2,4-F₂-C₆H₃
bedeutet und der Substituent R⁶ für eine Verbindung einer Zeile der Tabelle A entspricht.

Tabelle 396: (Verbindungen 396.1 - 396.974)
Verbindungen der allgemeinen Formel I.4, in denen
R³ = Methyl
R⁴ = C₂H₅
R⁵ = 2,4-F₂-C₆H₃
bedeutet und der Substituent R⁶ für eine Verbindung einer Zeile der Tabelle A entspricht.

Tabelle 397: (Verbindungen 397.1 - 397.974)
Verbindungen der allgemeinen Formel I.1, in denen
R³ = Methyl
R⁴ = C₂H₅
R⁵ = 2,5-F₂-C₆H₃
bedeutet und der Substituent R⁶ für eine Verbindung einer Zeile der Tabelle A entspricht.

Tabelle 398: (Verbindungen 398.1 - 398.974)
Verbindungen der allgemeinen Formel I.2, in denen
R³ = Methyl
R⁴ = C₂H₅
R⁵ = 2,5-F₂-C₆H₃
bedeutet und der Substituent R⁶ für eine Verbindung einer Zeile der Tabelle A entspricht.

Tabelle 399: (Verbindungen 399.1 - 399.974)
Verbindungen der allgemeinen Formel I.3, in denen
R³ = Methyl
R⁴ = C₂H₅
R⁵ = 2,5-F₂-C₆H₃
bedeutet und der Substituent R⁶ für eine Verbindung einer Zeile der Tabelle A entspricht.

Tabelle 400: (Verbindungen 400.1 - 400.974)
Verbindungen der allgemeinen Formel I.4, in denen
R³ = Methyl
R⁴ = C₂H₅
R⁵ = 2,5-F₂-C₆H₃
bedeutet und der Substituent R⁶ für eine Verbindung einer Zeile der Tabelle A entspricht.

Tabelle 401: (Verbindungen 401.1 - 401.974)
Verbindungen der allgemeinen Formel I.1, in denen
R³ = Methyl
R⁴ = C₂H₅
R⁵ = 3,4-F₂-C₆H₃
bedeutet und der Substituent R⁶ für eine Verbindung einer Zeile der Tabelle A entspricht.

Tabelle 402: (Verbindungen 402.1 - 402.974)
Verbindungen der allgemeinen Formel I.2, in denen
R³ = Methyl
R⁴ = C₂H₅
R⁵ = 3,4-F₂-C₆H₃
bedeutet und der Substituent R⁶ für eine Verbindung einer Zeile der Tabelle A entspricht.

Tabelle 403: (Verbindungen 403.1 - 403.974)
Verbindungen der allgemeinen Formel I.3, in denen
R³ = Methyl
R⁴ = C₂H₅
R⁵ = 3,4-F₂-C₆H₃
bedeutet und der Substituent R⁶ für eine Verbindung einer Zeile der Tabelle A entspricht.

Tabelle 404: (Verbindungen 404.1 - 404.974)
Verbindungen der allgemeinen Formel I.4, in denen
R³ = Methyl
R⁴ = C₂H₅
R⁵ = 3,4-F₂-C₆H₃
bedeutet und der Substituent R⁶ für eine Verbindung einer Zeile der Tabelle A entspricht.

Tabelle 405: (Verbindungen 405.1 - 405.974)
Verbindungen der allgemeinen Formel I.1, in denen
R³ = Methyl
R⁴ = C₂H₅
R⁵ = 3,5-F₂-C₆H₃
bedeutet und der Substituent R⁶ für eine Verbindung einer Zeile der Tabelle A entspricht.

Tabelle 406: (Verbindungen 406.1 - 406.974)
Verbindungen der allgemeinen Formel I.2, in denen
R³ = Methyl
R⁴ = C₂H₅
R⁵ = 3,5-F₂-C₆H₃
bedeutet und der Substituent R⁶ für eine Verbindung einer Zeile der Tabelle A entspricht.

Tabelle 407: (Verbindungen 407.1 - 407.974)
Verbindungen der allgemeinen Formel I.3, in denen
R³ = Methyl
R⁴ = C₂H₅
R⁵ = 3,5-F₂-C₆H₃
bedeutet und der Substituent R⁶ für eine Verbindung einer Zeile der Tabelle A entspricht.

Tabelle 408: (Verbindungen 408.1 - 408.974)
Verbindungen der allgemeinen Formel I.4, in denen
R³ = Methyl
R⁴ = C₂H₅
R⁵ = 3,5-F₂-C₆H₃
bedeutet und der Substituent R⁶ für eine Verbindung einer Zeile der Tabelle A entspricht.

Tabelle 409: (Verbindungen 409.1 - 409.974)
Verbindungen der allgemeinen Formel I.1, in denen
R³ = Methyl
R⁴ = C₂H₅
R⁵ = 2,3-Cl₂-C₆H₃
bedeutet und der Substituent R⁶ für eine Verbindung einer Zeile der Tabelle A entspricht.

Tabelle 410: (Verbindungen 410.1 - 410.974)
Verbindungen der allgemeinen Formel I.2, in denen
R³ = Methyl
R⁴ = C₂H₅
R⁵ = 2,3-Cl₂-C₆H₃
bedeutet und der Substituent R⁶ für eine Verbindung einer Zeile der Tabelle A entspricht.

Tabelle 411: (Verbindungen 411.1 - 411.974)
Verbindungen der allgemeinen Formel I.3, in denen
R³ = Methyl
R⁴ = C₂H₅
R⁵ = 2,3-Cl₂-C₆H₃
bedeutet und der Substituent R⁶ für eine Verbindung einer Zeile der Tabelle A entspricht.

Tabelle 412: (Verbindungen 412.1 - 412.974)
Verbindungen der allgemeinen Formel I.4, in denen
R³ = Methyl
R⁴ = C₂H₅
R⁵ = 2,3-Cl₂-C₆H₃
bedeutet und der Substituent R⁶ für eine Verbindung einer Zeile der Tabelle A entspricht.

Tabelle 413: (Verbindungen 413.1 - 413.974)
Verbindungen der allgemeinen Formel I.1, in denen
R³ = Methyl
R⁴ = C₂H₅
R⁵ = 2,4-Cl₂-C₆H₃
bedeutet und der Substituent R⁶ für eine Verbindung einer Zeile der Tabelle A entspricht.

Tabelle 414: (Verbindungen 414.1 - 414.974)
Verbindungen der allgemeinen Formel I.2, in denen
R³ = Methyl
R⁴ = C₂H₅
R⁵ = 2,4-Cl₂-C₆H₃
bedeutet und der Substituent R⁶ für eine Verbindung einer Zeile der Tabelle A entspricht.

Tabelle 415: (Verbindungen 415.1 - 415.974)
Verbindungen der allgemeinen Formel I.3, in denen
R³ = Methyl
R⁴ = C₂H₅
R⁵ = 2,4-Cl₂-C₆H₃
bedeutet und der Substituent R⁶ für eine Verbindung einer Zeile der Tabelle A entspricht.

Tabelle 416: (Verbindungen 416.1 - 416.974)
Verbindungen der allgemeinen Formel I.4, in denen
R³ = Methyl
R⁴ = C₂H₅
R⁵ = 2,4-Cl₂-C₆H₃
bedeutet und der Substituent R⁶ für eine Verbindung einer Zeile der Tabelle A entspricht.

Tabelle 417: (Verbindungen 417.1 - 417.974)
Verbindungen der allgemeinen Formel I.1, in denen
R³ = Methyl
R⁴ = C₂H₅
R⁵ = 2,5-Cl₂-C₆H₃
bedeutet und der Substituent R⁶ für eine Verbindung einer Zeile der Tabelle A entspricht.

Tabelle 418: (Verbindungen 418.1 - 418.974)
Verbindungen der allgemeinen Formel I.2, in denen
R³ = Methyl
R⁴ = C₂H₅
R⁵ = 2,5-Cl₂-C₆H₃
bedeutet und der Substituent R⁶ für eine Verbindung einer Zeile der Tabelle A entspricht.

Tabelle 419: (Verbindungen 419.1 - 419.974)
Verbindungen der allgemeinen Formel I.3, in denen
R³ = Methyl
R⁴ = C₂H₅
R⁵ = 2,5-Cl₂-C₆H₃
bedeutet und der Substituent R⁶ für eine Verbindung einer Zeile der Tabelle A entspricht.

Tabelle 420: (Verbindungen 420.1 - 420.974)
Verbindungen der allgemeinen Formel I.4, in denen
R³ = Methyl
R⁴ = C₂H₅
R⁵ = 2,5-Cl₂-C₆H₃
bedeutet und der Substituent R⁶ für eine Verbindung einer Zeile der Tabelle A entspricht.

Tabelle 421: (Verbindungen 421.1 - 421.974)
Verbindungen der allgemeinen Formel I.1, in denen
R³ = Methyl
R⁴ = C₂H₅
R⁵ = 3,4-Cl₂-C₆H₃
bedeutet und der Substituent R⁶ für eine Verbindung einer Zeile der Tabelle A entspricht.

Tabelle 422: (Verbindungen 422.1 - 422.974)
Verbindungen der allgemeinen Formel I.2, in denen
R³ = Methyl
R⁴ = C₂H₅
R⁵ = 3,4-Cl₂-C₆H₃
bedeutet und der Substituent R⁶ für eine Verbindung einer Zeile der Tabelle A entspricht.

Tabelle 423: (Verbindungen 423.1 - 423.974)
Verbindungen der allgemeinen Formel I.3, in denen
R³ = Methyl
R⁴ = C₂H₅
R⁵ = 3,4-Cl₂-C₆H₃
bedeutet und der Substituent R⁶ für eine Verbindung einer Zeile der Tabelle A entspricht.

Tabelle 424: (Verbindungen 424.1 - 424.974)
Verbindungen der allgemeinen Formel I.4, in denen
R³ = Methyl
R⁴ = C₂H₅
R⁵ = 3,4-Cl₂-C₆H₃
bedeutet und der Substituent R⁶ für eine Verbindung einer Zeile der Tabelle A entspricht.

Tabelle 425: (Verbindungen 425.1 - 425.974)
Verbindungen der allgemeinen Formel I.1, in denen
R³ = Methyl
R⁴ = C₂H₅
R⁵ = 3,5-Cl₂-C₆H₃
bedeutet und der Substituent R⁶ für eine Verbindung einer Zeile der Tabelle A entspricht.

Tabelle 426: (Verbindungen 426.1 - 426.974)
Verbindungen der allgemeinen Formel I.2, in denen
R³ = Methyl
R⁴ = C₂H₅
R⁵ = 3,5-Cl₂-C₆H₃
bedeutet und der Substituent R⁶ für eine Verbindung einer Zeile der Tabelle A entspricht.

Tabelle 427: (Verbindungen 427.1 - 427.974)
Verbindungen der allgemeinen Formel I.3, in denen
R³ = Methyl
R⁴ = C₂H₅
R⁵ = 3,5-Cl₂-C₆H₃
bedeutet und der Substituent R⁶ für eine Verbindung einer Zeile der Tabelle A entspricht.

Tabelle 428: (Verbindungen 428.1 - 428.974)
Verbindungen der allgemeinen Formel I.4, in denen
R³ = Methyl
R⁴ = C₂H₅
R⁵ = 3,5-Cl₂-C₆H₃
bedeutet und der Substituent R⁶ für eine Verbindung einer Zeile der Tabelle A entspricht.

Tabelle 429: (Verbindungen 429.1 - 429.974)
Verbindungen der allgemeinen Formel I.1, in denen
R³ = Methyl
R⁴ = C₂H₅
R⁵ = 2-F; 3-Cl-C₆H₃
bedeutet und der Substituent R⁶ für eine Verbindung einer Zeile der Tabelle A entspricht.

Tabelle 430: (Verbindungen 430.1 - 430.974)
Verbindungen der allgemeinen Formel I.2, in denen
R³ = Methyl
R⁴ = C₂H₅
R⁵ = 2-F; 3-Cl-C₆H₃
bedeutet und der Substituent R⁶ für eine Verbindung einer Zeile der Tabelle A entspricht.

Tabelle 431: (Verbindungen 431.1 - 431.974)
Verbindungen der allgemeinen Formel I.3, in denen
R³ = Methyl
R⁴ = C₂H₅
R⁵ = 2-F; 3-Cl-C₆H₃
bedeutet und der Substituent R⁶ für eine Verbindung einer Zeile der Tabelle A entspricht.

Tabelle 432: (Verbindungen 432.1 - 432.974)
Verbindungen der allgemeinen Formel I.4, in denen
R³ = Methyl
R⁴ = C₂H₅
R⁵ = 2-F; 3-Cl-C₆H₃
bedeutet und der Substituent R⁶ für eine Verbindung einer Zeile der Tabelle A entspricht.

Tabelle 433: (Verbindungen 433.1 - 433.974)
Verbindungen der allgemeinen Formel I.1, in denen
R³ = Methyl
R⁴ = C₂H₅
R⁵ = 2-F; 4-Cl-C₆H₃
bedeutet und der Substituent R⁶ für eine Verbindung einer Zeile der Tabelle A entspricht.

Tabelle 434: (Verbindungen 434.1 - 434.974)
Verbindungen der allgemeinen Formel I.2, in denen
R³ = Methyl
R⁴ = C₂H₅
R⁵ = 2-F; 4-Cl-C₆H₃
bedeutet und der Substituent R⁶ für eine Verbindung einer Zeile der Tabelle A entspricht.

Tabelle 435: (Verbindungen 435.1 - 435.974)
Verbindungen der allgemeinen Formel I.3, in denen
R³ = Methyl
R⁴ = C₂H₅
R⁵ = 2-F; 4-Cl-C₆H₃
bedeutet und der Substituent R⁶ für eine Verbindung einer Zeile der Tabelle A entspricht.

Tabelle 436: (Verbindungen 436.1 - 436.974)
Verbindungen der allgemeinen Formel I.4, in denen
R³ = Methyl
R⁴ = C₂H₅
R⁵ = 2-F; 4-Cl-C₆H₃
bedeutet und der Substituent R⁶ für eine Verbindung einer Zeile der Tabelle A entspricht.

Tabelle 437: (Verbindungen 437.1 - 437.974)
Verbindungen der allgemeinen Formel I.1, in denen
R³ = Methyl
R⁴ = C₂H₅
R⁵ = 3-F; 4-Cl-C₆H₃
bedeutet und der Substituent R⁶ für eine Verbindung einer Zeile der Tabelle A entspricht.

Tabelle 438: (Verbindungen 438.1 - 438.974)
Verbindungen der allgemeinen Formel I.2, in denen
R³ = Methyl
R⁴ = C₂H₅
R⁵ = 3-F; 4-Cl-C₆H₃
bedeutet und der Substituent R⁶ für eine Verbindung einer Zeile der Tabelle A entspricht.

Tabelle 439: (Verbindungen 439.1 - 439.974)
Verbindungen der allgemeinen Formel I.3, in denen
R³ = Methyl
R⁴ = C₂H₅
R⁵ = 3-F; 4-Cl-C₆H₃
bedeutet und der Substituent R⁶ für eine Verbindung einer Zeile der Tabelle A entspricht.

Tabelle 440: (Verbindungen 440.1 - 440.974)
Verbindungen der allgemeinen Formel I.4, in denen
R³ = Methyl
R⁴ = C₂H₅
R⁵ = 3-F; 4-Cl-C₆H₃
bedeutet und der Substituent R⁶ für eine Verbindung einer Zeile der Tabelle A entspricht.

Tabelle 441: (Verbindungen 441.1 - 441.974)
Verbindungen der allgemeinen Formel I.1, in denen
R³ = Methyl
R⁴ = C₂H₅
R⁵ = 4-F; 3,5-Cl₂-C₆H₂
bedeutet und der Substituent R⁶ für eine Verbindung einer Zeile der Tabelle A entspricht.

Tabelle 442: (Verbindungen 442.1 - 442.974)
Verbindungen der allgemeinen Formel I.2, in denen
R³ = Methyl
R⁴ = C₂H₅
R⁵ = 4-F; 3,5-Cl₂-C₆H₂
bedeutet und der Substituent R⁶ für eine Verbindung einer Zeile der Tabelle A entspricht.

Tabelle 443: (Verbindungen 443.1 - 443.974)
Verbindungen der allgemeinen Formel I.3, in denen
R³ = Methyl
R⁴ = C₂H₅
R⁵ = 4-F; 3,5-Cl₂-C₆H₂
bedeutet und der Substituent R⁶ für eine Verbindung einer Zeile der Tabelle A entspricht.

Tabelle 444: (Verbindungen 444.1 - 444.974)
Verbindungen der allgemeinen Formel I.4, in denen
R³ = Methyl
R⁴ = C₂H₅
R⁵ = 4-F; 3,5-Cl₂-C₆H₂
bedeutet und der Substituent R⁶ für eine Verbindung einer Zeile der Tabelle A entspricht.

Tabelle 445: (Verbindungen 445.1 - 445.974)
Verbindungen der allgemeinen Formel I.1, in denen
R³ = Methyl
R⁴ = C₂H₅
R⁵ = 2,3,4-Cl₃-C₆H₂
bedeutet und der Substituent R⁶ für eine Verbindung einer Zeile der Tabelle A entspricht.

Tabelle 446: (Verbindungen 446.1 - 446.974)
Verbindungen der allgemeinen Formel I.2, in denen
R³ = Methyl
R⁴ = C₂H₅
R⁵ = 2,3,4-Cl₃-C₆H₂
bedeutet und der Substituent R⁶ für eine Verbindung einer Zeile der Tabelle A entspricht.

Tabelle 447: (Verbindungen 447.1 - 447.974)
Verbindungen der allgemeinen Formel I.3, in denen
R³ = Methyl
R⁴ = C₂H₅
R⁵ = 2,3,4-Cl₃-C₆H₂
bedeutet und der Substituent R⁶ für eine Verbindung einer Zeile der Tabelle A entspricht.

Tabelle 448: (Verbindungen 448.1 - 448.974)
Verbindungen der allgemeinen Formel I.4, in denen
R³ = Methyl
R⁴ = C₂H₅
R⁵ = 2,3,4-Cl₃-C₆H₂
bedeutet und der Substituent R⁶ für eine Verbindung einer Zeile der Tabelle A entspricht.

Tabelle 449: (Verbindungen 449.1 - 449.974)
Verbindungen der allgemeinen Formel I.1, in denen
R³ = Methyl
R⁴ = C₂H₅
R⁵ = 2,3,5-Cl₃-C₆H₂
bedeutet und der Substituent R⁶ für eine Verbindung einer Zeile der Tabelle A entspricht.

Tabelle 450: (Verbindungen 450.1 - 450.974)
Verbindungen der allgemeinen Formel I.2, in denen
R³ = Methyl
R⁴ = C₂H₅
R⁵ = 2,3,5-Cl₃-C₆H₂
bedeutet und der Substituent R⁶ für eine Verbindung einer Zeile der Tabelle A entspricht.

Tabelle 451: (Verbindungen 451.1 - 451.974)
Verbindungen der allgemeinen Formel I.3, in denen
R³ = Methyl
R⁴ = C₂H₅
R⁵ = 2,3,5-Cl₃-C₆H₂
bedeutet und der Substituent R⁶ für eine Verbindung einer Zeile der Tabelle A entspricht.

Tabelle 452: (Verbindungen 452.1 - 452.974)
Verbindungen der allgemeinen Formel I.4, in denen
R³ = Methyl
R⁴ = C₂H₅
R⁵ = 2,3,5-Cl₃-C₆H₂
bedeutet und der Substituent R⁶ für eine Verbindung einer Zeile der Tabelle A entspricht.

Tabelle 453: (Verbindungen 453.1 - 453.974)
Verbindungen der allgemeinen Formel I.1, in denen
R³ = Methyl
R⁴ = C₂H₅
R⁵ = 2,4,5-Cl₃-C₆H₂
bedeutet und der Substituent R⁶ für eine Verbindung einer Zeile der Tabelle A entspricht.

Tabelle 454: (Verbindungen 454.1 - 454.974)
Verbindungen der allgemeinen Formel I.2, in denen
R³ = Methyl
R⁴ = C₂H₅
R⁵ = 2,4,5-Cl₃-C₆H₂
bedeutet und der Substituent R⁶ für eine Verbindung einer Zeile der Tabelle A entspricht.

Tabelle 455: (Verbindungen 455.1 - 455.974)
Verbindungen der allgemeinen Formel I.3, in denen
R³ = Methyl
R⁴ = C₂H₅
R⁵ = 2,4,5-Cl₃-C₆H₂
bedeutet und der Substituent R⁶ für eine Verbindung einer Zeile der Tabelle A entspricht.

Tabelle 456: (Verbindungen 456.1 - 456.974)
Verbindungen der allgemeinen Formel I.4, in denen
R³ = Methyl
R⁴ = C₂H₅
R⁵ = 2,4,5-Cl₃-C₆H₂
bedeutet und der Substituent R⁶ für eine Verbindung einer Zeile der Tabelle A entspricht.

Tabelle 457: (Verbindungen 457.1 - 457.974)
Verbindungen der allgemeinen Formel I.1, in denen
R³ = Methyl
R⁴ = C₂H₅
R⁵ = 2-CN-C₆H₄
bedeutet und der Substituent R⁶ für eine Verbindung einer Zeile der Tabelle A entspricht.

Tabelle 458: (Verbindungen 458.1 - 458.974)
Verbindungen der allgemeinen Formel I.2, in denen
R³ = Methyl
R⁴ = C₂H₅
R⁵ = 2-CN-C₆H₄
bedeutet und der Substituent R⁶ für eine Verbindung einer Zeile der Tabelle A entspricht.

Tabelle 459: (Verbindungen 459.1 - 459.974)
Verbindungen der allgemeinen Formel I.3, in denen
R³ = Methyl
R⁴ = C₂H₅
R⁵ = 2-CN-C₆H₄
bedeutet und der Substituent R⁶ für eine Verbindung einer Zeile der Tabelle A entspricht.

Tabelle 460: (Verbindungen 460.1 - 460.974)
Verbindungen der allgemeinen Formel I.4, in denen
R³ = Methyl
R⁴ = C₂H₅
R⁵ = 2-CN-C₆H₄
bedeutet und der Substituent R⁶ für eine Verbindung einer Zeile der Tabelle A entspricht.

Tabelle 461: (Verbindungen 461.1 - 461.974)
Verbindungen der allgemeinen Formel I.1, in denen
R³ = Methyl
R⁴ = C₂H₅
R⁵ = 3-CN-C₆H₄
bedeutet und der Substituent R⁶ für eine Verbindung einer Zeile der Tabelle A entspricht.

Tabelle 462: (Verbindungen 462.1 - 462.974)
Verbindungen der allgemeinen Formel I.2, in denen
R³ = Methyl
R⁴ = C₂H₅
R⁵ = 3-CN-C₆H₄
bedeutet und der Substituent R⁶ für eine Verbindung einer Zeile der Tabelle A entspricht.

Tabelle 463: (Verbindungen 463.1 - 463.974)
Verbindungen der allgemeinen Formel I.3, in denen
R³ = Methyl
R⁴ = C₂H₅
R⁵ = 3-CN-C₆H₄
bedeutet und der Substituent R⁶ für eine Verbindung einer Zeile der Tabelle A entspricht.

Tabelle 464: (Verbindungen 464.1 - 464.974)
Verbindungen der allgemeinen Formel I.4, in denen
R³ = Methyl
R⁴ = C₂H₅
R⁵ = 3-CN-C₆H₄
bedeutet und der Substituent R⁶ für eine Verbindung einer Zeile der Tabelle A entspricht.

Tabelle 465: (Verbindungen 465.1 - 465.974)
Verbindungen der allgemeinen Formel I.1, in denen
R³ = Methyl
R⁴ = C₂H₅
R⁵ = 4-CN-C₆H₄
bedeutet und der Substituent R⁶ für eine Verbindung einer Zeile der Tabelle A entspricht.

Tabelle 466: (Verbindungen 466.1 - 466.974)
Verbindungen der allgemeinen Formel I.2, in denen
R³ = Methyl
R⁴ = C₂H₅
R⁵ = 4-CN-C₆H₄
bedeutet und der Substituent R⁶ für eine Verbindung einer Zeile der Tabelle A entspricht.

Tabelle 467: (Verbindungen 467.1 - 467.974)
Verbindungen der allgemeinen Formel I.3, in denen
R³ = Methyl
R⁴ = C₂H₅
R⁵ = 4-CN-C₆H₄
bedeutet und der Substituent R⁶ für eine Verbindung einer Zeile der Tabelle A entspricht.

Tabelle 468: (Verbindungen 468.1 - 468.974)
Verbindungen der allgemeinen Formel I.4, in denen
R³ = Methyl
R⁴ = C₂H₅
R⁵ = 4-CN-C₆H₄
bedeutet und der Substituent R⁶ für eine Verbindung einer Zeile der Tabelle A entspricht.

Tabelle 469: (Verbindungen 469.1 - 469.974)
Verbindungen der allgemeinen Formel I.1, in denen
R³ = Methyl
R⁴ = C₂H₅
R⁵ = 2-NO₂-C₆H₄
bedeutet und der Substituent R⁶ für eine Verbindung einer Zeile der Tabelle A entspricht.

Tabelle 470: (Verbindungen 470.1 - 470.974)
Verbindungen der allgemeinen Formel I.2, in denen
R³ = Methyl
R⁴ = C₂H₅
R⁵ = 2-NO₂-C₆H₄
bedeutet und der Substituent R⁶ für eine Verbindung einer Zeile der Tabelle A entspricht.

Tabelle 471: (Verbindungen 471.1 - 471.974)
Verbindungen der allgemeinen Formel I.3, in denen
R³ = Methyl
R⁴ = C₂H₅
R⁵ = 2-NO₂-C₆H₄
bedeutet und der Substituent R⁶ für eine Verbindung einer Zeile der Tabelle A entspricht.

Tabelle 472: (Verbindungen 472.1 - 472.974)
Verbindungen der allgemeinen Formel I.4, in denen
R³ = Methyl
R⁴ = C₂H₅
R⁵ = 2-NO₂-C₆H₄
bedeutet und der Substituent R⁶ für eine Verbindung einer Zeile der Tabelle A entspricht.

Tabelle 473: (Verbindungen 473.1 - 473.974)
Verbindungen der allgemeinen Formel I.1, in denen
R³ = Methyl
R⁴ = C₂H₅
R⁵ = 3-NO₂-C₆H₄
bedeutet und der Substituent R⁶ für eine Verbindung einer Zeile der Tabelle A entspricht.

Tabelle 474: (Verbindungen 474.1 - 474.974)
Verbindungen der allgemeinen Formel I.2, in denen
R³ = Methyl
R⁴ = C₂H₅
R⁵ = 3-NO₂-C₆H₄
bedeutet und der Substituent R⁶ für eine Verbindung einer Zeile der Tabelle A entspricht.

Tabelle 475: (Verbindungen 475.1 - 475.974)
Verbindungen der allgemeinen Formel I.3, in denen
R³ = Methyl
R⁴ = C₂H₅
R⁵ = 3-NO₂-C₆H₄
bedeutet und der Substituent R⁶ für eine Verbindung einer Zeile der Tabelle A entspricht.

Tabelle 476: (Verbindungen 476.1 - 476.974)
Verbindungen der allgemeinen Formel I.4, in denen
R³ = Methyl
R⁴ = C₂H₅
R⁵ = 3-NO₂-C₆H₄
bedeutet und der Substituent R⁶ für eine Verbindung einer Zeile der Tabelle A entspricht.

Tabelle 477: (Verbindungen 477.1 - 477.974)
Verbindungen der allgemeinen Formel I.1, in denen
R³ = Methyl
R⁴ = C₂H₅
R5 = 4-NO₂-C₆H₄
bedeutet und der Substituent R⁶ für eine Verbindung einer Zeile der Tabelle A entspricht.

Tabelle 478: (Verbindungen 478.1 - 478.974)
Verbindungen der allgemeinen Formel I.2, in denen
R³ = Methyl
R⁴ = C₂H₅
R⁵ = 4-NO₂-C₆H₄
bedeutet und der Substituent R⁶ für eine Verbindung einer Zeile der Tabelle A entspricht.

Tabelle 479: (Verbindungen 479.1 - 479.974)
Verbindungen der allgemeinen Formel I.3, in denen
R³ = Methyl
R⁴ = C₂H₅
R⁵ = 4-NO₂-C₆H₄
bedeutet und der Substituent R⁶ für eine Verbindung einer Zeile der Tabelle A entspricht.

Tabelle 480: (Verbindungen 480.1 - 480.974)
Verbindungen der allgemeinen Formel I.4, in denen
R³ = Methyl
R⁴ = C₂H₅
R⁵ = 4-NO₂-C₆H₄
bedeutet und der Substituent R⁶ für eine Verbindung einer Zeile der Tabelle A entspricht.

Tabelle 481: (Verbindungen 481.1 - 481.974)
Verbindungen der allgemeinen Formel I.1, in denen
R³ = Methyl
R⁴ = C₂H₅
R⁵ = 2-CH₃-C₆H₄
bedeutet und der Substituent R⁶ für eine Verbindung einer Zeile der Tabelle A entspricht.

Tabelle 482: (Verbindungen 482.1 - 482.974)
Verbindungen der allgemeinen Formel I.2, in denen
R³ = Methyl
R⁴ = C₂H₅
R⁵ = 2-CH₃-C₆H₄
bedeutet und der Substituent R⁶ für eine Verbindung einer Zeile der Tabelle A entspricht.

Tabelle 483: (Verbindungen 483.1 - 483.974)
Verbindungen der allgemeinen Formel I.3, in denen
R³ = Methyl
R⁴ = C₂H₅
R⁵ = 2-CH₃-C₆H₄
bedeutet und der Substituent R⁶ für eine Verbindung einer Zeile der Tabelle A entspricht.

Tabelle 484: (Verbindungen 484.1 - 484.974)
Verbindungen der allgemeinen Formel I.4, in denen
R³ = Methyl
R⁴ = C₂H₅
R⁵ = 2-CH₃-C₆H₄
bedeutet und der Substituent R⁶ für eine Verbindung einer Zeile der Tabelle A entspricht.

Tabelle 485: (Verbindungen 485.1 - 485.974)
Verbindungen der allgemeinen Formel I.1, in denen
R³ = Methyl
R⁴ = C₂H₅
R⁵ = 3-CH₃-C₆H₄
bedeutet und der Substituent R⁶ für eine Verbindung einer Zeile der Tabelle A entspricht.

Tabelle 486: (Verbindungen 486.1 - 486.974)
Verbindungen der allgemeinen Formel I.2, in denen
R³ = Methyl
R⁴ = C₂H₅
R⁵ = 3-CH₃-C₆H₄
bedeutet und der Substituent R⁶ für eine Verbindung einer Zeile der Tabelle A entspricht.

Tabelle 487: (Verbindungen 487.1 - 487.974)
Verbindungen der allgemeinen Formel I.3, in denen
R³ = Methyl
R⁴ = C₂H₅
R⁵ = 3-CH₃-C₆H₄
bedeutet und der Substituent R⁶ für eine Verbindung einer Zeile der Tabelle A entspricht.

Tabelle 488: (Verbindungen 488.1 - 488.974)
Verbindungen der allgemeinen Formel I.4, in denen
R³ = Methyl
R⁴ = C₂H₅
R⁵ = 3-CH₃-C₆H₄
bedeutet und der Substituent R⁶ für eine Verbindung einer Zeile der Tabelle A entspricht.

Tabelle 489: (Verbindungen 489.1 - 489.974)
Verbindungen der allgemeinen Formel I.1, in denen
R³ = Methyl
R⁴ = C₂H₅
R⁵ = 4-CH₃-C₆H₄
bedeutet und der Substituent R⁶ für eine Verbindung einer Zeile der Tabelle A entspricht.

Tabelle 490: (Verbindungen 490.1 - 490.974)
Verbindungen der allgemeinen Formel I.2, in denen
R³ = Methyl
R⁴ = C₂H₅
R⁵ = 4-CH₃-C₆H₄
bedeutet und der Substituent R⁶ für eine Verbindung einer Zeile der Tabelle A entspricht.

Tabelle 491: (Verbindungen 491.1 - 491.974)
Verbindungen der allgemeinen Formel I.3, in denen
R³ = Methyl
R⁴ = C₂H₅
R⁵ = 4-CH₃-C₆H₄
bedeutet und der Substituent R⁶ für eine Verbindung einer Zeile der Tabelle A entspricht.

Tabelle 492: (Verbindungen 492.1 - 492.974)
Verbindungen der allgemeinen Formel I.4, in denen
R³ = Methyl
R⁴ = C₂H₅
R⁵ = 4-CH₃-C₆H₄
bedeutet und der Substituent R⁶ für eine Verbindung einer Zeile der Tabelle A entspricht.

Tabelle 493: (Verbindungen 493.1 - 493.974)
Verbindungen der allgemeinen Formel I.1, in denen
R³ = Methyl
R⁴ = C₂H₅
R⁵ = 2,4-(CH₃)₂-C₆H₃
bedeutet und der Substituent R⁶ für eine Verbindung einer Zeile der Tabelle A entspricht.

Tabelle 494: (Verbindungen 494.1 - 494.974)
Verbindungen der allgemeinen Formel I.2, in denen
R³ = Methyl
R⁴ = C₂H₅
R⁵ = 2,4-(CH₃)₂-C₆H₃
bedeutet und der Substituent R⁶ für eine Verbindung einer Zeile der Tabelle A entspricht.

Tabelle 495: (Verbindungen 495.1 - 495.974)
Verbindungen der allgemeinen Formel I.3, in denen
R³ = Methyl
R⁴ = C₂H₅
R⁵ = 2,4-(CH₃)₂-C₆H₃
bedeutet und der Substituent R⁶ für eine Verbindung einer Zeile der Tabelle A entspricht.

Tabelle 496: (Verbindungen 496.1 - 496.974)
Verbindungen der allgemeinen Formel I.4, in denen
R³ = Methyl
R⁴ = C₂H₅
R⁵ = 2,4-(CH₃)₂-C₆H₃
bedeutet und der Substituent R⁶ für eine Verbindung einer Zeile der Tabelle A entspricht.

Tabelle 497: (Verbindungen 497.1 - 497.974)
Verbindungen der allgemeinen Formel I.1, in denen
R³ = Methyl
R⁴ = C₂H₅
R⁵ = 2,5-(CH₃)₂-C₆H₃
bedeutet und der Substituent R⁶ für eine Verbindung einer Zeile der Tabelle A entspricht.

Tabelle 498: (Verbindungen 498.1 - 498.974)
Verbindungen der allgemeinen Formel I.2, in denen
R³ = Methyl
R⁴ = C₂H₅
R⁵ = 2,5-(CH₃)₂-C₆H₃
bedeutet und der Substituent R⁶ für eine Verbindung einer Zeile der Tabelle A entspricht.

Tabelle 499: (Verbindungen 499.1 - 499.974)
Verbindungen der allgemeinen Formel I.3, in denen
R³ = Methyl
R⁴ = C₂H₅
R⁵ = 2,5-(CH₃)₂-C₆H₃
bedeutet und der Substituent R⁶ für eine Verbindung einer Zeile der Tabelle A entspricht.

Tabelle 500: (Verbindungen 500.1 - 500.974)
Verbindungen der allgemeinen Formel I.4, in denen
R³ = Methyl
R⁴ = C₂H₅
R⁵ = 2,5-(CH₃)₂-C₆H₃
bedeutet und der Substituent R⁶ für eine Verbindung einer Zeile der Tabelle A entspricht.

Tabelle 501: (Verbindungen 501.1 - 501.974)
Verbindungen der allgemeinen Formel I.1, in denen
R³ = Methyl
R⁴ = C₂H₅
R⁵ = 3,4-(CH₃)₂-C₆H₃
bedeutet und der Substituent R⁶ für eine Verbindung einer Zeile der Tabelle A entspricht.

Tabelle 502: (Verbindungen 502.1 - 502.974)
Verbindungen der allgemeinen Formel I.2, in denen
R³ = Methyl
R⁴ = C₂H₅
R⁵ = 3,4-(CH₃)₂-C₆H₃
bedeutet und der Substituent R⁶ für eine Verbindung einer Zeile der Tabelle A entspricht.

Tabelle 503: (Verbindungen 503.1 - 503.974)
Verbindungen der allgemeinen Formel I.3, in denen
R³ = Methyl
R⁴ = C₂H₅
R⁵ = 3,4-(CH₃)₂-C₆H₃
bedeutet und der Substituent R⁶ für eine Verbindung einer Zeile der Tabelle A entspricht.

Tabelle 504: (Verbindungen 504.1 - 504.974)
Verbindungen der allgemeinen Formel I.4, in denen
R³ = Methyl
R⁴ = C₂H₅
R⁵ = 3,4-(CH₃)₂-C₆H₃
bedeutet und der Substituent R⁶ für eine Verbindung einer Zeile der Tabelle A entspricht.

Tabelle 505: (Verbindungen 505.1 - 505.974)
Verbindungen der allgemeinen Formel I.1, in denen
R³ = Methyl
R⁴ = C₂H₅
R⁵ = 3,5-(CH₃)₂-C₆H₃
bedeutet und der Substituent R⁶ für eine Verbindung einer Zeile der Tabelle A entspricht.

Tabelle 506: (Verbindungen 506.1 - 506.974)
Verbindungen der allgemeinen Formel I.2, in denen
R³ = Methyl
R⁴ = C₂H₅
R⁵ = 3,5-(CH₃)₂-C₆H₃
bedeutet und der Substituent R⁶ für eine Verbindung einer Zeile der Tabelle A entspricht.

Tabelle 507: (Verbindungen 507.1 - 507.974)
Verbindungen der allgemeinen Formel I.3, in denen
R³ = Methyl
R⁴ = C₂H₅
R⁵ = 3,5-(CH₃)₂-C₆H₃
bedeutet und der Substituent R⁶ für eine Verbindung einer Zeile der Tabelle A entspricht.

Tabelle 508: (Verbindungen 508.1 - 508.974)
Verbindungen der allgemeinen Formel I.4, in denen
R³ = Methyl
R⁴ = C₂H₅
R⁵ = 3,5-(CH₃)₂-C₆H₃
bedeutet und der Substituent R⁶ für eine Verbindung einer Zeile der Tabelle A entspricht.

Tabelle 509: (Verbindungen 509.1 - 509.974)
Verbindungen der allgemeinen Formel I.1, in denen
R³ = Methyl
R⁴ = C₂H₅
R⁵ = 3,4,5-(CH₃)₃-C₆H₂
bedeutet und der Substituent R⁶ für eine Verbindung einer Zeile der Tabelle A entspricht.

Tabelle 510: (Verbindungen 510.1 - 510.974)
Verbindungen der allgemeinen Formel I.2, in denen
R³ = Methyl
R⁴ = C₂H₅
R⁵ = 3,4,5-(CH₃)₃-C₆H₂
bedeutet und der Substituent R⁶ für eine Verbindung einer Zeile der Tabelle A entspricht.

Tabelle 511: (Verbindungen 511.1 - 511.974)
Verbindungen der allgemeinen Formel I.3, in denen
R³ = Methyl
R⁴ = C₂H₅
R⁵ = 3,4,5-(CH₃)₃-C₆H₂
bedeutet und der Substituent R⁶ für eine Verbindung einer Zeile der Tabelle A entspricht.

Tabelle 512: (Verbindungen 512.1 - 512.974)
Verbindungen der allgemeinen Formel I.4, in denen
R³ = Methyl
R⁴ = C₂H₅
R⁵ = 3,4,5-(CH₃)₃-C₆H₂
bedeutet und der Substituent R⁶ für eine Verbindung einer Zeile der Tabelle A entspricht.

Tabelle 513: (Verbindungen 513.1 - 513.974)
Verbindungen der allgemeinen Formel I.1, in denen
R³ = Methyl
R⁴ = C₂H₅
R⁵ = 4-C₆H₅-C₆H₄
bedeutet und der Substituent R⁶ für eine Verbindung einer Zeile der Tabelle A entspricht.

Tabelle 514: (Verbindungen 514.1 - 514.974)
Verbindungen der allgemeinen Formel I.2, in denen
R³ = Methyl
R⁴ = C₂H₅
R⁵ = 4-C₆H₅-C₆H₄
bedeutet und der Substituent R⁶ für eine Verbindung einer Zeile der Tabelle A entspricht.

Tabelle 515: (Verbindungen 515.1 - 515.974)
Verbindungen der allgemeinen Formel I.3, in denen
R³ = Methyl
R⁴ = C₂H₅
R⁵ = 4-C₆H₅-C₆H₄
bedeutet und der Substituent R⁶ für eine Verbindung einer Zeile der Tabelle A entspricht.

Tabelle 516: (Verbindungen 516.1 - 516.974)
Verbindungen der allgemeinen Formel I.4, in denen
R³ = Methyl
R⁴ = C₂H₅
R⁵ = 4-C₆H₅-C₆H₄
bedeutet und der Substituent R⁶ für eine Verbindung einer Zeile der Tabelle A entspricht.

Tabelle 517: (Verbindungen 517.1 - 517.974)
Verbindungen der allgemeinen Formel I.1, in denen
R³ = Methyl
R⁴ = C₂H₅
R⁵ = 3-Cl-4-CH₃-C₆H₃
bedeutet und der Substituent R⁶ für eine Verbindung einer Zeile der Tabelle A entspricht.

Tabelle 518: (Verbindungen 518.1 - 518.974)
Verbindungen der allgemeinen Formel I.2, in denen
R³ = Methyl
R⁴ = C₂H₅
R⁵ = 3-Cl-4-CH₃-C₆H₃
bedeutet und der Substituent R⁶ für eine Verbindung einer Zeile der Tabelle A entspricht.

Tabelle 519: (Verbindungen 519.1 - 519.974)
Verbindungen der allgemeinen Formel I.3, in denen
R³ = Methyl
R⁴ = C₂H₅
R⁵ = 3-Cl-4-CH₃-C₆H₃
bedeutet und der Substituent R⁶ für eine Verbindung einer Zeile der Tabelle A entspricht.

Tabelle 520: (Verbindungen 520.1 - 520.974)
Verbindungen der allgemeinen Formel I.4, in denen
R³ = Methyl
R⁴ = C₂H₅
R⁵ = 3-Cl-4-CH₃-C₆H₃
bedeutet und der Substituent R⁶ für eine Verbindung einer Zeile der Tabelle A entspricht.

Tabelle 521: (Verbindungen 521.1 - 521.974)
Verbindungen der allgemeinen Formel I.1, in denen
R³ = Methyl
R⁴ = C₂H₅
R⁵ = 2-OCH₃-C₆H₄
bedeutet und der Substituent R⁶ für eine Verbindung einer Zeile der Tabelle A entspricht.

Tabelle 522: (Verbindungen 522.1 - 522.974)
Verbindungen der allgemeinen Formel I.2, in denen
R³ = Methyl
R⁴ = C₂H₅
R⁵ = 2-OCH₃-C₆H₄
bedeutet und der Substituent R⁶ für eine Verbindung einer Zeile der Tabelle A entspricht.

Tabelle 523: (Verbindungen 523.1 - 523.974)
Verbindungen der allgemeinen Formel I.3, in denen
R³ = Methyl
R⁴ = C₂H₅
R⁵ = 2-OCH₃-C₆H₄
bedeutet und der Substituent R⁶ für eine Verbindung einer Zeile der Tabelle A entspricht.

Tabelle 524: (Verbindungen 524.1 - 524.974)
Verbindungen der allgemeinen Formel I.4, in denen
R³ = Methyl
R⁴ = C₂H₅
R⁵ = 2-OCH₃-C₆H₄
bedeutet und der Substituent R⁶ für eine Verbindung einer Zeile der Tabelle A entspricht.

Tabelle 525: (Verbindungen 525.1 - 525.974)
Verbindungen der allgemeinen Formel I.1, in denen
R³ = Methyl
R⁴ = C₂H₅
R⁵ = 3-OCH₃-C₆H₄
bedeutet und der Substituent R⁶ für eine Verbindung einer Zeile der Tabelle A entspricht.

Tabelle 526: (Verbindungen 526.1 - 526.974)
Verbindungen der allgemeinen Formel I.2, in denen
R³ = Methyl
R⁴ = C₂H₅
R⁵ = 3-OCH₃-C₆H₄
bedeutet und der Substituent R⁶ für eine Verbindung einer Zeile der Tabelle A entspricht.

Tabelle 527: (Verbindungen 527.1 - 527.974)
Verbindungen der allgemeinen Formel I.3, in denen
R³ = Methyl
R⁴ = C₂H₅
R⁵ = 3-OCH₃-C₆H₄
bedeutet und der Substituent R⁶ für eine Verbindung einer Zeile. der Tabelle A entspricht.

Tabelle 528: (Verbindungen 528.1 - 528.974)
Verbindungen der allgemeinen Forme528 I.4, in denen
R³ = Methyl
R⁴ = C₂H₅
R⁵ = 3-OCH₃-C₆H₄
bedeutet und der Substituent R⁶ für eine Verbindung einer Zeile der Tabelle A entspricht.

Tabelle 529: (Verbindungen 529.1 - 529.974)
Verbindungen der allgemeinen Formel I.1, in denen
R³ = Methyl
R⁴ = C₂H₅
R⁵ = 4-OCH₃-C₆H₄
bedeutet und der Substituent R⁶ für eine Verbindung einer Zeile der Tabelle A entspricht.

Tabelle 530: (Verbindungen 530.1 - 530.974)
Verbindungen der allgemeinen Formel I.2, in denen
R³ = Methyl
R⁴ = C₂H₅
R⁵ = 4-OCH₃-C₆H₄
bedeutet und der Substituent R⁶ für eine Verbindung einer Zeile der Tabelle A entspricht.

Tabelle 531: (Verbindungen 531.1 - 531.974)
Verbindungen der allgemeinen Formel I.3, in denen
R³ = Methyl
R⁴ = C₂H₅
R⁵ = 4-OCH₃-C₆H₄
bedeutet und der Substituent R⁶ für eine Verbindung einer Zeile der Tabelle A entspricht.

Tabelle 532: (Verbindungen 532.1 - 532.974)
Verbindungen der allgemeinen Formel I.4, in denen
R³ = Methyl
R⁴ = C₂H₅
R⁵ = 4-OCH₃-C₆H₄
bedeutet und der Substituent R⁶ für eine Verbindung einer Zeile der Tabelle A entspricht.

Tabelle 533: (Verbindungen 533.1 - 533.974)
Verbindungen der allgemeinen Formel I.1, in denen
R³ = Methyl
R⁴ = C₂H₅
R⁵ = 2-OCF3-C6H4
bedeutet und der Substituent R⁶ für eine Verbindung einer Zeile der Tabelle A entspricht.

Tabelle 534: (Verbindungen 534.1 - 534.974)
Verbindungen der allgemeinen Formel I.2, in denen
R³ = Methyl
R⁴ = C₂H₅
R⁵ = 2-OCF₃-C₆H₄
bedeutet und der Substituent R⁶ für eine Verbindung einer Zeile der Tabelle A entspricht.

Tabelle 535: (Verbindungen 535.1 - 535.974)
Verbindungen der allgemeinen Formel I.3, in denen
R³ = Methyl
R⁴ = C₂H₅
R⁵ = 2-OCF₃-C₆H₄
bedeutet und der Substituent R⁶ für eine Verbindung einer Zeile der Tabelle A entspricht.

Tabelle 536: (Verbindungen 536.1 - 536.974)
Verbindungen der allgemeinen Formel I.4, in denen
R³ = Methyl
R⁴ = C₂H₅
R⁵ = 2-OCF₃-C₆H₄
bedeutet und der Substituent R⁶ für eine Verbindung einer Zeile der Tabelle A entspricht.

Tabelle 537: (Verbindungen 537.1 - 537.974)
Verbindungen der allgemeinen Formel I.1, in denen
R³ = Methyl
R⁴ = C₂H₅
R⁵ = 3-OCF₃-C₆H₄
bedeutet und der Substituent R⁶ für eine Verbindung einer Zeile der Tabelle A entspricht.

Tabelle 538: (Verbindungen 538.1 - 538.974)
Verbindungen der allgemeinen Formel I.2, in denen
R³ = Methyl
R⁴ = C₂H₅
R⁵ = 3-OCF₃-C₆H₄
bedeutet und der Substituent R⁶ für eine Verbindung einer Zeile der Tabelle A entspricht.

Tabelle 539: (Verbindungen 539.1 - 539.974)
Verbindungen der allgemeinen Formel I.3, in denen
R³ = Methyl
R⁴ = C₂H₅
R⁵ = 3-OCF₃-C₆H₄
bedeutet und der Substituent R⁶ für eine Verbindung einer Zeile der Tabelle A entspricht.

Tabelle 540: (Verbindungen 540.1 - 540.974)
Verbindungen der allgemeinen Formel I.4, in denen
R³ = Methyl
R⁴ = C₂H₅
R⁵ = 3-OCF₃-C₆H₄
bedeutet und der Substituent R⁶ für eine Verbindung einer Zeile der Tabelle A entspricht.

Tabelle 541: (Verbindungen 541.1 - 541.974)
Verbindungen der allgemeinen Formel I.1, in denen
R³ = Methyl
R⁴ = C₂H₅
R⁵ = 4-OCF₃-C₆H₄
bedeutet und der Substituent R⁶ für eine Verbindung einer Zeile der Tabelle A entspricht.

Tabelle 542: (Verbindungen 542.1 - 542.974)
Verbindungen der allgemeinen Formel I.2, in denen
R³ = Methyl
R⁴ = C₂H₅
R⁵ = 4-OCF₃-C₆H₄
bedeutet und der Substituent R⁶ für eine Verbindung einer Zeile der Tabelle A entspricht.

Tabelle 543: (Verbindungen 543.1 - 543.974)
Verbindungen der allgemeinen Formel I.3, in denen
R³ = Methyl
R⁴ = C₂H₅
R⁵ = 4-OCF₃-C₆H₄
bedeutet und der Substituent R⁶ für eine Verbindung einer Zeile der Tabelle A entspricht.

Tabelle 544: (Verbindungen 544.1 - 544.974)
Verbindungen der allgemeinen Formel I.4, in denen
R³ = Methyl
R⁴ = C₂H₅
R⁵ = 4-OCF₃-C₆H₄
bedeutet und der Substituent R⁶ für eine Verbindung einer Zeile der Tabelle A entspricht.

Tabelle 545: (Verbindungen 545.1 - 545.974)
Verbindungen der allgemeinen Formel I.1, in denen
R³ = Methyl
R⁴ = C₂H₅
R⁵ = 3-Cl; 4-OCH₃-C₆H₃
bedeutet und der Substituent R⁶ für eine Verbindung einer Zeile der Tabelle A entspricht.

Tabelle 546: (Verbindungen 546.1 - 546.974)
Verbindungen der allgemeinen Formel I.2, in denen
R³ = Methyl
R⁴ = C₂H₅
R⁵ = 3-Cl; 4-OCH₃-C₆H₃
bedeutet und der Substituent R⁶ für eine Verbindung einer Zeile der Tabelle A entspricht.

Tabelle 547: (Verbindungen 547.1 - 547.974)
Verbindungen der allgemeinen Formel I.3, in denen
R³ = Methyl
R⁴ = C₂H₅
R⁵ = 3-Cl; 4-OCH₃-C₆H₃
bedeutet und der Substituent R⁶ für eine Verbindung einer Zeile der Tabelle A entspricht.

Tabelle 548: (Verbindungen 548.1 - 548.974)
Verbindungen der allgemeinen Formel I.4, in denen
R³ = Methyl
R⁴ = C₂H₅
R⁵ = 3-Cl; 4-OCH₃-C₆H₃
bedeutet und der Substituent R⁶ für eine Verbindung einer Zeile der Tabelle A entspricht.

Tabelle 549: (Verbindungen 549.1 - 549.974)
Verbindungen der allgemeinen Formel I.1, in denen
R³ = Methyl
R⁴ = C₂H₅ 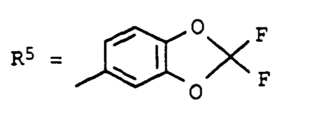 bedeutet und der Substituent R⁶ für eine Verbindung einer Zeile der Tabelle A entspricht.

Tabelle 550: (Verbindungen 550.1 - 550.974)
Verbindungen der allgemeinen Formel I.2, in denen
R³ = Methyl
R⁴ = C₂H₅ 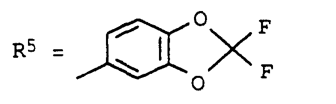 bedeutet und der Substituent R⁶ für eine Verbindung einer Zeile der Tabelle A entspricht.

Tabelle 551: (Verbindungen 551.1 - 551.974)
Verbindungen der allgemeinen Formel I.3, in denen
R³ = Methyl
R⁴ = C₂H₅ 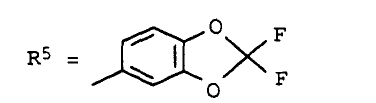 bedeutet und der Substituent R⁶ für eine Verbindung einer Zeile der Tabelle A entspricht.

Tabelle 552: (Verbindungen 552.1 - 552.974)
Verbindungen der allgemeinen Formel I.4, in denen
R³ = Methyl
R⁴ = C₂H₅ 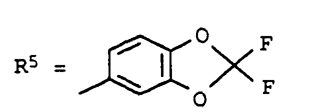 bedeutet und der Substituent R⁶ für eine Verbindung einer Zeile der Tabelle A entspricht.

Tabelle 553: (Verbindungen 553.1 - 553.974)
Verbindungen der allgemeinen Formel I.1, in denen
R³ = Methyl
R⁴ = C₂H₅
R⁵ = Pyridin-2-yl
bedeutet und der Substituent R⁶ für eine Verbindung einer Zeile der Tabelle A entspricht.

Tabelle 554: (Verbindungen 554.1 - 554.974)
Verbindungen der allgemeinen Formel I.2, in denen
R³ = Methyl
R⁴ = C₂H₅
R⁵ = Pyridin-2-yl
bedeutet und der Substituent R⁶ für eine Verbindung einer Zeile der Tabelle A entspricht.

Tabelle 555: (Verbindungen 555.1 - 555.974)
Verbindungen der allgemeinen Formel I.3, in denen
R³ = Methyl
R⁴ = C₂H₅
R⁵ = Pyridin-2-yl
bedeutet und der Substituent R⁶ für eine Verbindung einer Zeile der Tabelle A entspricht.

Tabelle 556: (Verbindungen 556.1 - 556.974)
Verbindungen der allgemeinen Formel I.4, in denen
R³ = Methyl
R⁴ = C₂H₅
R⁵ = Pyridin-2-yl
bedeutet und der Substituent R⁶ für eine Verbindung einer Zeile der Tabelle A entspricht.

Tabelle 557: (Verbindungen 557.1 - 557.974)
Verbindungen der allgemeinen Formel I.1, in denen
R³ = Methyl
R⁴ = C₂H₅
R⁵ = Pyridin-3-yl
bedeutet und der Substituent R⁶ für eine Verbindung einer Zeile der Tabelle A entspricht.

Tabelle 558: (Verbindungen 558.1 - 558.974)
Verbindungen der allgemeinen Formel I.2, in denen
R³ = Methyl
R⁴ = C₂H₅
R⁵ = Pyridin-3-yl
bedeutet und der Substituent R⁶ für eine Verbindung einer Zeile der Tabelle A entspricht.

Tabelle 559: (Verbindungen 559.1 - 559.974)
Verbindungen der allgemeinen Formel I.3, in denen
R³ = Methyl
R⁴ = C₂H₅
R⁵ = Pyridin-3-yl
bedeutet und der Substituent R⁶ für eine Verbindung einer Zeile der Tabelle A entspricht.

Tabelle 560: (Verbindungen 560.1 - 560.974)
Verbindungen der allgemeinen Formel I.4, in denen
R³ = Methyl
R⁴ = C₂H₅
R⁵ = Pyridin-3-yl
bedeutet und der Substituent R⁶ für eine Verbindung einer Zeile der Tabelle A entspricht.

Tabelle 561: (Verbindungen 561.1 - 561.974)
Verbindungen der allgemeinen Formel I.1, in denen
R³ = Methyl
R⁴ = C₂H₅
R⁵ = Pyridin-4-yl
bedeutet und der Substituent R⁶ für eine Verbindung einer Zeile der Tabelle A entspricht.

Tabelle 562: (Verbindungen 562.1 - 562.974)
Verbindungen der allgemeinen Formel I.2, in denen
R³ = Methyl
R⁴ = C₂H₅
R⁵ = Pyridin-4-yl
bedeutet und der Substituent R⁶ für eine Verbindung einer Zeile der Tabelle A entspricht.

Tabelle 563: (Verbindungen 563.1 - 563.974)
Verbindungen der allgemeinen Formel I.3, in denen
R³ = Methyl
R⁴ = C₂H₅
R⁵ = Pyridin-4-yl
bedeutet und der Substituent R⁶ für eine Verbindung einer Zeile der Tabelle A entspricht.

Tabelle 564: (Verbindungen 564.1 - 564.974)
Verbindungen der allgemeinen Formel I.4, in denen
R³ = Methyl
R⁴ = C₂H₅
R⁵ = Pyridin-4-yl
bedeutet und der Substituent R⁶ für eine Verbindung einer Zeile der Tabelle A entspricht.

Tabelle 565: (Verbindungen 565.1 - 565.974)
Verbindungen der allgemeinen Formel I.1, in denen
R³ = Methyl
R⁴ = C₂H₅
R⁵ = 3-CH₃-Pyridin-2-yl
bedeutet und der Substituent R⁶ für eine Verbindung einer Zeile der Tabelle A entspricht.

Tabelle 566: (Verbindungen 566.1 - 566.974)
Verbindungen der allgemeinen Formel I.2, in denen
R³ = Methyl
R⁴ = C₂H₅
R⁵ = 3-CH₃-Pyridin-2-yl
bedeutet und der Substituent R⁶ für eine Verbindung einer Zeile der Tabelle A entspricht.

Tabelle 567: (Verbindungen 567.1 - 567.974)
Verbindungen der allgemeinen Formel I.3, in denen
R³ = Methyl
R⁴ = C₂H₅
R⁵ = 3-CH₃-Pyridin-2-yl
bedeutet und der Substituent R⁶ für eine Verbindung einer Zeile der Tabelle A entspricht.

Tabelle 568: (Verbindungen 568.1 - 568.974)
Verbindungen der allgemeinen Formel I.4, in denen
R³ = Methyl
R⁴ = C₂H₅
R⁵ = 3-CH₃-Pyridin-2-yl
bedeutet und der Substituent R⁶ für eine Verbindung einer Zeile der Tabelle A entspricht.

Tabelle 569: (Verbindungen 569.1 - 569.974)
Verbindungen der allgemeinen Formel I.1, in denen
R³ = Methyl
R⁴ = C₂H₅
R⁵ = 4-CH₃-Pyridin-2-yl
bedeutet und der Substituent R⁶ für eine Verbindung einer Zeile der Tabelle A entspricht.

Tabelle 570: (Verbindungen 570.1 - 570.974)
Verbindungen der allgemeinen Formel I.2, in denen
R³ = Methyl
R⁴ = C₂H₅
R⁵ = 4-CH₃-Pyridin-2-yl
bedeutet und der Substituent R⁶ für eine Verbindung einer Zeile der Tabelle A entspricht.

Tabelle 571: (Verbindungen 571.1 - 571.974)
Verbindungen der allgemeinen Formel I.3, in denen
R³ = Methyl
R⁴ = C₂H₅
R⁵ = 4-CH₃-Pyridin-2-yl
bedeutet und der Substituent R⁶ für eine Verbindung einer Zeile der Tabelle A entspricht.

Tabelle 572: (Verbindungen 572.1 - 572.974)
Verbindungen der allgemeinen Formel I.4, in denen
R³ = Methyl
R⁴ = C₂H₅
R⁵ = 4-CH₃-Pyridin-2-yl
bedeutet und der Substituent R⁶ für eine Verbindung einer Zeile der Tabelle A entspricht.

Tabelle 573: (Verbindungen 573.1 - 573.974)
Verbindungen der allgemeinen Formel I.1, in denen
R³ = Methyl
R⁴ = C₂H₅
R⁵ = 3-OCH₃-Pyridin-2-yl
bedeutet und der Substituent R⁶ für eine Verbindung einer Zeile der Tabelle A entspricht.

Tabelle 574: (Verbindungen 574.1 - 574.974)
Verbindungen der allgemeinen Formel I.2, in denen
R³ = Methyl
R⁴ = C₂H₅
R⁵ = 3-OCH₃-Pyridin-2-yl
bedeutet und der Substituent R⁶ für eine Verbindung einer Zeile der Tabelle A entspricht.

Tabelle 575: (Verbindungen 575.1 - 575.974)
Verbindungen der allgemeinen Formel I.3, in denen
R³ = Methyl
R⁴ = C₂H₅
R⁵ = 3-OCH₃-Pyridin-2-yl
bedeutet und der Substituent R⁶ für eine Verbindung einer Zeile der Tabelle A entspricht.

Tabelle 576: (Verbindungen 576.1 - 576.974)
Verbindungen der allgemeinen Formel I.4, in denen
R³ = Methyl
R⁴ = C₂H₅
R⁵ = 3-OCH₃-Pyridin-2-yl
bedeutet und der Substituent R⁶ für eine Verbindung einer Zeile der Tabelle A entspricht.

Tabelle 577: (Verbindungen 577.1 - 577.974)
Verbindungen der allgemeinen Formel I.1, in denen
R³ = Methyl
R⁴ = C₂H₅
R⁵ = 4-Cl-Pyridin-2-yl
bedeutet und der Substituent R⁶ für eine Verbindung einer Zeile der Tabelle A entspricht.

Tabelle 578: (Verbindungen 578.1 - 578.974)
Verbindungen der allgemeinen Formel I.2, in denen
R³ = Methyl
R⁴ = C₂H₅
R⁵ = 4-Cl-Pyridin-2-yl
bedeutet und der Substituent R⁶ für eine Verbindung einer Zeile der Tabelle A entspricht.

Tabelle 579: (Verbindungen 579.1 - 579.974)
Verbindungen der allgemeinen Formel I.3, in denen
R³ = Methyl
R⁴ = C₂H₅
R⁵ = 4-Cl-Pyridin-2-yl
bedeutet und der Substituent R⁶ für eine Verbindung einer Zeile der Tabelle A entspricht.

Tabelle 580: (Verbindungen 580.1 - 580.974)
Verbindungen der allgemeinen Formel I.4, in denen
R³ = Methyl
R⁴ = C₂H₅
R⁵ = 4-Cl-Pyridin-2-yl
bedeutet und der Substituent R⁶ für eine Verbindung einer Zeile der Tabelle A entspricht.

Tabelle 581: (Verbindungen 581.1 - 581.974)
Verbindungen der allgemeinen Formel I.1, in denen
R³ = Methyl
R⁴ = C₂H₅
R⁵ = 5-Cl-Pyridin-2-yl
bedeutet und der Substituent R⁶ für eine Verbindung einer Zeile der Tabelle A entspricht.

Tabelle 582: (Verbindungen 582.1 - 582.974)
Verbindungen der allgemeinen Formel I.2, in denen
R³ = Methyl
R⁴ = C₂H₅
R⁵ = 5-Cl-Pyridin-2-yl
bedeutet und der Substituent R⁶ für eine Verbindung einer Zeile der Tabelle A entspricht.

Tabelle 583: (Verbindungen 583.1 - 583.974)
Verbindungen der allgemeinen Formel I.3, in denen
R³ = Methyl
R⁴ = C₂H₅
R⁵ = 5-Cl-Pyridin-2-yl
bedeutet und der Substituent R⁶ für eine Verbindung einer Zeile der Tabelle A entspricht.

Tabelle 584: (Verbindungen 584.1 - 584.974)
Verbindungen der allgemeinen Formel I.4, in denen
R³ = Methyl
R⁴ = C₂H₅
R⁵ = 5-Cl-Pyridin-2-yl
bedeutet und der Substituent R⁶ für eine Verbindung einer Zeile der Tabelle A entspricht.

Tabelle 585: (Verbindungen 585.1 - 585.974)
Verbindungen der allgemeinen Formel I.1, in denen
R³ = Methyl
R⁴ = C₂H₅
R⁵ = Pyrimidin-2-yl
bedeutet und der Substituent R⁶ für eine Verbindung einer Zeile der Tabelle A entspricht.

Tabelle 586: (Verbindungen 586.1 - 586.974)
Verbindungen der allgemeinen Formel I.2, in denen
R³ = Methyl
R⁴ = C₂H₅
R⁵ = Pyrimidin-2-yl
bedeutet und der Substituent R⁶ für eine Verbindung einer Zeile der Tabelle A entspricht.

Tabelle 587: (Verbindungen 587.1 - 587.974)
Verbindungen der allgemeinen Formel I.3, in denen
R³ = Methyl
R⁴ = C₂H₅
R⁵ = Pyrimidin-2-yl
bedeutet und der Substituent R⁶ für eine Verbindung einer Zeile der Tabelle A entspricht.

Tabelle 588: (Verbindungen 588.1 - 588.974)
Verbindungen der allgemeinen Formel I.4, in denen
R³ = Methyl
R⁴ = C₂H₅
R⁵ = Pyrimidin-2-yl
bedeutet und der Substituent R⁶ für eine Verbindung einer Zeile der Tabelle A entspricht.

Tabelle 589: (Verbindungen 589.1 - 589.974)
Verbindungen der allgemeinen Formel I.1, in denen
R³ = Methyl
R⁴ = C₂H₅
R⁵ = 5-Cl-Pyrimidin-2-yl
bedeutet und der Substituent R⁶ für eine Verbindung einer Zeile der Tabelle A entspricht.

Tabelle 590: (Verbindungen 590.1 - 590.974)
Verbindungen der allgemeinen Formel I.2, in denen
R³ = Methyl
R⁴ = C₂H₅
R⁵ = 5-Cl-Pyrimidin-2-yl
bedeutet und der Substituent R⁶ für eine Verbindung einer Zeile der Tabelle A entspricht.

Tabelle 591: (Verbindungen 591.1 - 591.974)
Verbindungen der allgemeinen Formel I.3, in denen
R³ = Methyl
R⁴ = C₂H₅
R⁵ = 5-Cl-Pyrimidin-2-yl
bedeutet und der Substituent R⁶ für eine Verbindung einer Zeile der Tabelle A entspricht.

Tabelle 592: (Verbindungen 592.1 - 592.974)
Verbindungen der allgemeinen Formel I.4, in denen
R³ = Methyl
R⁴ = C₂H₅
R⁵ = 5-Cl-Pyrimidin-2-yl
bedeutet und der Substituent R⁶ für eine Verbindung einer Zeile der Tabelle A entspricht.

Tabelle 593: (Verbindungen 593.1 - 593.974)
Verbindungen der allgemeinen Formel I.1, in denen
R³ = Methyl
R⁴ = C₂H₅
R⁵ = 4-CH₃-Pyrimidin-2-yl
bedeutet und der Substituent R⁶ für eine Verbindung einer Zeile der Tabelle A entspricht.

Tabelle 594: (Verbindungen 594.1 - 594.974)
Verbindungen der allgemeinen Formel I.2, in denen
R³ = Methyl
R⁴ = C₂H₅
R⁵ = 4-CH₃-Pyrimidin-2-yl
bedeutet und der Substituent R⁶ für eine Verbindung einer Zeile der Tabelle A entspricht.

Tabelle 595: (Verbindungen 595.1 - 595.974)
Verbindungen der allgemeinen Formel I.3, in denen
R³ = Methyl
R⁴ = C₂H₅
R⁵ = 4-CH₃-Pyrimidin-2-yl
bedeutet und der Substituent R⁶ für eine Verbindung einer Zeile der Tabelle A entspricht.

Tabelle 596: (Verbindungen 596.1 - 596.974)
Verbindungen der allgemeinen Formel I.4, in denen
R³ = Methyl
R⁴ = C₂H₅
R⁵ = 4-CH₃-Pyrimidin-2-yl
bedeutet und der Substituent R⁶ für eine Verbindung einer Zeile der Tabelle A entspricht.

Tabelle 597: (Verbindungen 597.1 - 597.974)
Verbindungen der allgemeinen Formel I.1, in denen
R³ = Methyl
R⁴ = C₂H₅
R⁵ = 4-Cl-Furan-2-yl
bedeutet und der Substituent R⁶ für eine Verbindung einer Zeile der Tabelle A entspricht.

Tabelle 598: (Verbindungen 598.1 - 598.974)
Verbindungen der allgemeinen Formel I.2, in denen
R³ = Methyl
R⁴ = C₂H₅
R⁵ = 4-Cl-Furan-2-yl
bedeutet und der Substituent R⁶ für eine Verbindung einer Zeile der Tabelle A entspricht.

Tabelle 599: (Verbindungen 599.1 - 599.974)
Verbindungen der allgemeinen Formel I.3, in denen
R³ = Methyl
R⁴ = C₂H₅
R⁵ = 4-Cl-Furan-2-yl
bedeutet und der Substituent R⁶ für eine Verbindung einer Zeile der Tabelle A entspricht.

Tabelle 600: (Verbindungen 600.1 - 600.974)
Verbindungen der allgemeinen Formel I.4, in denen
R³ = Methyl
R⁴ = C₂H₅
R⁵ = 4-Cl-Furan-2-yl
bedeutet und der Substituent R⁶ für eine Verbindung einer Zeile der Tabelle A entspricht.

Tabelle 601: (Verbindungen 601.1 - 601.974)
Verbindungen der allgemeinen Formel I.1, in denen
R³ = Methyl
R⁴ = C₂H₅
R⁵ = 4-CH₃-Furan-2-yl
bedeutet und der Substituent R⁶ für eine Verbindung einer Zeile der Tabelle A entspricht.

Tabelle 602: (Verbindungen 602.1 - 602.974)
Verbindungen der allgemeinen Formel I.2, in denen
R³ = Methyl
R⁴ = C₂H₅
R⁵ = 4-CH₃-Furan-2-yl
bedeutet und der Substituent R⁶ für eine Verbindung einer Zeile der Tabelle A entspricht.

Tabelle 603: (Verbindungen 603.1 - 603.974)
Verbindungen der allgemeinen Formel I.3, in denen
R³ = Methyl
R⁴ = C₂H₅
R⁵ = 4-CH₃-Furan-2-yl
bedeutet und der Substituent R⁶ für eine Verbindung einer Zeile der Tabelle A entspricht.

Tabelle 604: (Verbindungen 604.1 - 604.974)
Verbindungen der allgemeinen Formel I.4, in denen
R³ = Methyl
R⁴ = C₂H₅
R⁵ = 4-CH₃-Furan-2-yl
bedeutet und der Substituent R⁶ für eine Verbindung einer Zeile der Tabelle A entspricht.

Tabelle 605: (Verbindungen 605.1 - 605.974)
Verbindungen der allgemeinen Formel I.1, in denen
R³ = Methyl
R⁴ = C₂H₅
R⁵ = Thien-2-yl
bedeutet und der Substituent R⁶ für eine Verbindung einer Zeile der Tabelle A entspricht.

Tabelle 606: (Verbindungen 606.1 - 606.974)
Verbindungen der allgemeinen Formel I.2, in denen
R³ = Methyl
R⁴ = C₂H₅
R⁵ = Thien-2-yl
bedeutet und der Substituent R⁶ für eine Verbindung einer Zeile der Tabelle A entspricht.

Tabelle 607: (Verbindungen 607.1 - 607.974)
Verbindungen der allgemeinen Formel I.3, in denen
R³ = Methyl
R⁴ = C₂H₅
R⁵ = Thien-2-yl
bedeutet und der Substituent R⁶ für eine Verbindung einer Zeile der Tabelle A entspricht.

Tabelle 608: (Verbindungen 608.1 - 608.974)
Verbindungen der allgemeinen Formel I.4, in denen
R³ = Methyl
R⁴ = C₂H₅
R⁵ = Thien-2-yl
bedeutet und der Substituent R⁶ für eine Verbindung einer Zeile der Tabelle A entspricht.

Tabelle 609: (Verbindungen 609.1 - 609.974)
Verbindungen der allgemeinen Formel I.1, in denen
R³ = Methyl
R⁴ = C₂H₅
R⁵ = Thien-3-yl
bedeutet und der Substituent R⁶ für eine Verbindung einer Zeile der Tabelle A entspricht.

Tabelle 610: (Verbindungen 610.1 - 610.974)
Verbindungen der allgemeinen Formel I.2, in denen
R³ = Methyl
R⁴ = C₂H₅
R⁵ = Thien-3-yl
bedeutet und der Substituent R⁶ für eine Verbindung einer Zeile der Tabelle A entspricht.

Tabelle 611: (Verbindungen 611.1 - 611.974)
Verbindungen der allgemeinen Formel I.3, in denen
R³ = Methyl
R⁴ = C₂H₅
R⁵ = Thien-3-yl
bedeutet und der Substituent R⁶ für eine Verbindung einer Zeile der Tabelle A entspricht.

Tabelle 612: (Verbindungen 612.1 - 612.974)
Verbindungen der allgemeinen Formel I.4, in denen
R³ = Methyl
R⁴ = C₂H₅
R⁵ = Thien-3-yl
bedeutet und der Substituent R⁶ für eine Verbindung einer Zeile der Tabelle A entspricht.

Tabelle 613: (Verbindungen 613.1 - 613.974)
Verbindungen der allgemeinen Formel I.1, in denen
R³ = Methyl
R⁴ = C₂H₅
R⁵ = Oxazol-2-yl
bedeutet und der Substituent R⁶ für eine Verbindung einer Zeile der Tabelle A entspricht.

Tabelle 614: (Verbindungen 614.1 - 614.974)
Verbindungen der allgemeinen Formel I.2, in denen
R³ = Methyl
R⁴ = C₂H₅
R⁵ = Oxazol-2-yl
bedeutet und der Substituent R⁶ für eine Verbindung einer Zeile der Tabelle A entspricht.

Tabelle 615: (Verbindungen 615.1 - 615.974)
Verbindungen der allgemeinen Formel I.3, in denen
R³ = Methyl
R⁴ = C₂H₅
R⁵ = Oxazol-2-yl
bedeutet und der Substituent R⁶ für eine Verbindung einer Zeile der Tabelle A entspricht.

Tabelle 616: (Verbindungen 616.1 - 616.974)
Verbindungen der allgemeinen Formel I.4, in denen
R³ = Methyl
R⁴ = C₂H₅
R⁵ = Oxazol-2-yl
bedeutet und der Substituent R⁶ für eine Verbindung einer Zeile der Tabelle A entspricht.

Tabelle 617: (Verbindungen 617.1 - 617.974)
Verbindungen der allgemeinen Formel I.1, in denen
R³ = Methyl
R⁴ = C₂H₅
R⁵ = Isoxazol-3-yl
bedeutet und der Substituent R⁶ für eine Verbindung einer Zeile der Tabelle A entspricht.

Tabelle 618: (Verbindungen 618.1 - 618.974)
Verbindungen der allgemeinen Formel I.2, in denen
R³ = Methyl
R⁴ = C₂H₅
R⁵ = Isoxazol-3-yl
bedeutet und der Substituent R⁶ für eine Verbindung einer Zeile der Tabelle A entspricht.

Tabelle 619: (Verbindungen 619.1 - 619.974)
Verbindungen der allgemeinen Formel I.3, in denen
R³ = Methyl
R⁴ = C₂H₅
R⁵ = Isoxazol-3-yl
bedeutet und der Substituent R⁶ für eine Verbindung einer Zeile der Tabelle A entspricht.

Tabelle 620: (Verbindungen 620.1 - 620.974)
Verbindungen der allgemeinen Formel I.4, in denen
R³ = Methyl
R⁴ = C₂H₅
R⁵ = Isoxazol-3-yl
bedeutet und der Substituent R⁶ für eine Verbindung einer Zeile der Tabelle A entspricht.

Tabelle 621: (Verbindungen 621.1 - 621.974)
Verbindungen der allgemeinen Formel I.1, in denen
R³ = Methyl
R⁴ = C₂H₅
R⁵ = Isoxazol-4-yl
bedeutet und der Substituent R⁶ für eine Verbindung einer Zeile der Tabelle A entspricht.

Tabelle 622: (Verbindungen 622.1 - 622.974)
Verbindungen der allgemeinen Formel I.2, in denen
R³ = Methyl
R⁴ = C₂H₅
R⁵ = Isoxazol-4-yl
bedeutet und der Substituent R⁶ für eine Verbindung einer Zeile der Tabelle A entspricht.

Tabelle 623: (Verbindungen 623.1 - 623.974)
Verbindungen der allgemeinen Formel I.3, in denen
R³ = Methyl
R⁴ = C₂H₅
R⁵ = Isoxazol-4-yl
bedeutet und der Substituent R⁶ für eine Verbindung einer Zeile der Tabelle A entspricht.

Tabelle 624: (Verbindungen 624.1 - 624.974)
Verbindungen der allgemeinen Formel I.4, in denen
R³ = Methyl
R⁴ = C₂H₅
R⁵ = Isoxazol-4-yl
bedeutet und der Substituent R⁶ für eine Verbindung einer Zeile der Tabelle A entspricht.

Tabelle 625: (Verbindungen 625.1 - 625.974)
Verbindungen der allgemeinen Formel I.1, in denen
R³ = Methyl
R⁴ = C₂H₅
R⁵ = Pyrazol-3-yl
bedeutet und der Substituent R⁶ für eine Verbindung einer Zeile der Tabelle A entspricht.

Tabelle 626: (Verbindungen 626.1 - 626.974)
Verbindungen der allgemeinen Formel I.2, in denen
R³ = Methyl
R⁴ = C₂H₅
R⁵ = Pyrazol-3-yl
bedeutet und der Substituent R⁶ für eine Verbindung einer Zeile der Tabelle A entspricht.

Tabelle 627: (Verbindungen 627.1 - 627.974)
Verbindungen der allgemeinen Formel I.3, in denen
R³ = Methyl
R⁴ = C₂H₅
R⁵ = Pyrazol-3-yl
bedeutet und der Substituent R⁶ für eine Verbindung einer Zeile der Tabelle A entspricht.

Tabelle 628: (Verbindungen 628.1 - 628.974)
Verbindungen der allgemeinen Formel I.4, in denen
R³ = Methyl
R⁴ = C₂H₅
R⁵ = Pyrazol-3-yl
bedeutet und der Substituent R⁶ für eine Verbindung einer Zeile der Tabelle A entspricht.

Tabelle 629: (Verbindungen 629.1 - 629.974)
Verbindungen der allgemeinen Formel I.1, in denen
R³ = Methyl
R⁴ = C₂H₅
R⁵ = 1,3,4-Oxadiazol-5-yl
bedeutet und der Substituent R⁶ für eine Verbindung einer Zeile der Tabelle A entspricht.

Tabelle 630: (Verbindungen 630.1 - 630.974)
Verbindungen der allgemeinen Formel I.2, in denen
R³ = Methyl
R⁴ = C₂H₅
R⁵ = 1,3,4-Oxadiazol-5-yl
bedeutet und der Substituent R⁶ für eine Verbindung einer Zeile der Tabelle A entspricht.

Tabelle 631: (Verbindungen 631.1 - 631.974)
Verbindungen der allgemeinen Formel I.3, in denen
R³ = Methyl
R⁴ = C₂H₅
R⁵ = 1,3,4-Oxadiazol-5-yl
bedeutet und der Substituent R⁶ für eine Verbindung einer Zeile der Tabelle A entspricht.

Tabelle 632: (Verbindungen 632.1 - 632.974)
Verbindungen der allgemeinen Formel I.4, in denen
R³ = Methyl
R⁴ = C₂H₅
R⁵ = 1,3,4-Oxadiazol-5-yl
bedeutet und der Substituent R⁶ für eine Verbindung einer Zeile der Tabelle A entspricht.

Tabelle 633: (Verbindungen 633.1 - 633.974)
Verbindungen der allgemeinen Formel I.1, in denen
R³ = Methyl
R⁴ = C₂H₅
R⁵ = 1,2,4-Triazol-3-yl
bedeutet und der Substituent R⁶ für eine Verbindung einer Zeile der Tabelle A entspricht.

Tabelle 634: (Verbindungen 634.1 - 634.974)
Verbindungen der allgemeinen Formel I.2, in denen
R³ = Methyl
R⁴ = C₂H₅
R⁵ = 1,2,4-Triazol-3-yl
bedeutet und der Substituent R⁶ für eine Verbindung einer Zeile der Tabelle A entspricht.

Tabelle 635: (Verbindungen 635.1 - 635.974)
Verbindungen der allgemeinen Formel I.3, in denen
R³ = Methyl
R⁴ = C₂H₅
R⁵ = 1,2,4-Triazol-3-yl
bedeutet und der Substituent R⁶ für eine Verbindung einer Zeile der Tabelle A entspricht.

Tabelle 636: (Verbindungen 636.1 - 636.974)
Verbindungen der allgemeinen Formel I.4, in denen
R³ = Methyl
R⁴ = C₂H₅
R⁵ = 1,2,4-Triazol-3-yl
bedeutet und der Substituent R⁶ für eine Verbindung einer Zeile der Tabelle A entspricht.

Tabelle 637: (Verbindungen 637.1 - 637.974)
Verbindungen der allgemeinen Formel I.1, in denen
R³ = Methyl
R⁴ = C₂H₅
R⁵ = Pyrrol-2-yl
bedeutet und der Substituent R⁶ für eine Verbindung einer Zeile der Tabelle A entspricht.

Tabelle 638: (Verbindungen 638.1 - 638.974)
Verbindungen der allgemeinen Formel I.2, in denen
R³ = Methyl
R⁴ = C₂H₅
R⁵ = Pyrrol-2-yl
bedeutet und der Substituent R⁶ für eine Verbindung einer Zeile der Tabelle A entspricht.

Tabelle 639: (Verbindungen 639.1 - 639.974)
Verbindungen der allgemeinen Formel I.3, in denen
R³ = Methyl
R⁴ = C₂H₅
R⁵ = Pyrrol-2-yl
bedeutet und der Substituent R⁶ für eine Verbindung einer Zeile der Tabelle A entspricht.

Tabelle 640: (Verbindungen 640.1 - 640.974)
Verbindungen der allgemeinen Formel I.4, in denen
R³ = Methyl
R⁴ = C₂H₅
R⁵ = Pyrrol-2-yl
bedeutet und der Substituent R⁶ für eine Verbindung einer Zeile der Tabelle A entspricht.

Tabelle 641: (Verbindungen 641.1 - 641.974)
Verbindungen der allgemeinen Formel I.1, in denen
R³ = Methyl
R⁴ = C₃H₃
R⁵ = H
bedeutet und der Substituent R⁶ für eine Verbindung einer Zeile der Tabelle A entspricht.

Tabelle 642: (Verbindungen 642.1 - 642.974)
Verbindungen der allgemeinen Formel I.2, in denen
R³ = Methyl
R⁴ = C₃H₃
R⁵ = H
bedeutet und der Substituent R⁶ für eine Verbindung einer Zeile der Tabelle A entspricht.

Tabelle 643: (Verbindungen 643.1 - 643.974)
Verbindungen der allgemeinen Formel I.3, in denen
R³ = Methyl
R⁴ = C₃H₃
R⁵ = H
bedeutet und der Substituent R⁶ für eine Verbindung einer Zeile der Tabelle A entspricht.

Tabelle 644: (Verbindungen 644.1 - 644.974)
Verbindungen der allgemeinen Formel I.4, in denen
R³ = Methyl
R⁴ = C₃H₃
R⁵ = H
bedeutet und der Substituent R⁶ für eine Verbindung einer Zeile der Tabelle A entspricht.

Tabelle 645: (Verbindungen 645.1 - 645.974)
Verbindungen der allgemeinen Formel I.1, in denen
R³ = Methyl
R⁴ = C₃H₃
R⁵ = CH₃
bedeutet und der Substituent R⁶ für eine Verbindung einer Zeile der Tabelle A entspricht.

Tabelle 646: (Verbindungen 646.1 - 646.974)
Verbindungen der allgemeinen Formel I.2, in denen
R³ = Methyl
R⁴ = C₃H₃
R⁵ = CH₃
bedeutet und der Substituent R⁶ für eine Verbindung einer Zeile der Tabelle A entspricht.

Tabelle 647: (Verbindungen 647.1 - 647.974)
Verbindungen der allgemeinen Formel I.3, in denen
R³ = Methyl
R⁴ = C₃H₃
R⁵ = CH₃
bedeutet und der Substituent R⁶ für eine Verbindung einer Zeile der Tabelle A entspricht.

Tabelle 648: (Verbindungen 648.1 - 648.974)
Verbindungen der allgemeinen Formel I.4, in denen
R³ = Methyl
R⁴ = C₃H₃
R⁵ = CH₃
bedeutet und der Substituent R⁶ für eine Verbindung einer Zeile der Tabelle A entspricht.

Tabelle 649: (Verbindungen 649.1 - 649.974)
Verbindungen der allgemeinen Formel I.1, in denen
R³ = Methyl
R⁴ = C₃H₃
R⁵ = C₂H₅
bedeutet und der Substituent R⁶ für eine Verbindung einer Zeile der Tabelle A entspricht.

Tabelle 650: (Verbindungen 650.1 - 650.974)
Verbindungen der allgemeinen Formel I.2, in denen
R³ = Methyl
R⁴ = C₃H₃
R⁵ = C₂H₅
bedeutet und der Substituent R⁶ für eine Verbindung einer Zeile der Tabelle A entspricht.

Tabelle 651: (Verbindungen 651.1 - 651.974)
Verbindungen der allgemeinen Formel I.3, in denen
R³ = Methyl
R⁴ = C₃H₃
R⁵ = C₂H₅
bedeutet und der Substituent R⁶ für eine Verbindung einer Zeile der Tabelle A entspricht.

Tabelle 652: (Verbindungen 652.1 - 652.974)
Verbindungen der allgemeinen Formel I.4, in denen
R³ = Methyl
R⁴ = C₃H₃
R⁵ = C₂H₅
bedeutet und der Substituent R⁶ für eine Verbindung einer Zeile der Tabelle A entspricht.

Tabelle 653: (Verbindungen 653.1 - 653.974)
Verbindungen der allgemeinen Formel I.1, in denen
R³ = Methyl
R⁴ = C₃H₃
R⁵ = n-C₃H₇
bedeutet und der Substituent R⁶ für eine Verbindung einer Zeile der Tabelle A entspricht.

Tabelle 654: (Verbindungen 654.1 - 654.974)
Verbindungen der allgemeinen Formel I.2, in denen
R³ = Methyl
R⁴ = C₃H₃
R⁵ = n-C₃H₇
bedeutet und der Substituent R⁶ für eine Verbindung einer Zeile der Tabelle A entspricht.

Tabelle 655: (Verbindungen 655.1 - 655.974)
Verbindungen der allgemeinen Formel I.3, in denen
R³ = Methyl
R⁴ = C₃H₃
R⁵ = n-C₃H₇
bedeutet und der Substituent R⁶ für eine Verbindung einer Zeile der Tabelle A entspricht.

Tabelle 656: (Verbindungen 656.1 - 656.974)
Verbindungen der allgemeinen Formel I.4, in denen
R³ = Methyl
R⁴ = C₃H₃
R⁵ = n-C₃H₇
bedeutet und der Substituent R⁶ für eine Verbindung einer Zeile der Tabelle A entspricht.

Tabelle 657: (Verbindungen 657.1 - 657.974)
Verbindungen der allgemeinen Formel I.1, in denen
R³ = Methyl
R⁴ = C₃H₃
R⁵ = i-C₃H₇
bedeutet und der Substituent R⁶ für eine Verbindung einer Zeile der Tabelle A entspricht.

Tabelle 658: (Verbindungen 658.1 - 658.974)
Verbindungen der allgemeinen Formel I.2, in denen
R³ = Methyl
R⁴ = C₃H₃
R⁵ = i-C₃H₇
bedeutet und der Substituent R⁶ für eine Verbindung einer Zeile der Tabelle A entspricht.

Tabelle 659: (Verbindungen 659.1 - 659.974)
Verbindungen der allgemeinen Formel I.3, in denen
R³ = Methyl
R⁴ = C₃H₃
R⁵ = i-C₃H₇
bedeutet und der Substituent R⁶ für eine Verbindung einer Zeile der Tabelle A entspricht.

Tabelle 660: (Verbindungen 660.1 - 660.974)
Verbindungen der allgemeinen Formel I.4, in denen
R³ = Methyl
R⁴ = C₃H₃
R⁵ = i-C₃H₇
bedeutet und der Substituent R⁶ für eine Verbindung einer Zeile der Tabelle A entspricht.

Tabelle 661: (Verbindungen 661.1 - 661.974)
Verbindungen der allgemeinen Formel I.1, in denen
R³ = Methyl
R⁴ = C₃H₃
R⁵ = Cyclopropyl
bedeutet und der Substituent R⁶ für eine Verbindung einer Zeile der Tabelle A entspricht.

Tabelle 662: (Verbindungen 662.1 - 662.974)
Verbindungen der allgemeinen Formel I.2, in denen
R³ = Methyl
R⁴ = C₃H₃
R⁵ = Cyclopropyl
bedeutet und der Substituent R⁶ für eine Verbindung einer Zeile der Tabelle A entspricht.

Tabelle 663: (Verbindungen 663.1 - 663.974)
Verbindungen der allgemeinen Formel I.3, in denen
R³ = Methyl
R⁴ = C₃H₃
R⁵ = Cyclopropyl
bedeutet und der Substituent R⁶ für eine Verbindung einer Zeile der Tabelle A entspricht.

Tabelle 664: (Verbindungen 664.1 - 664.974)
Verbindungen der allgemeinen Formel I.4, in denen
R³ = Methyl
R⁴ = C₃H₃
R⁵ = Cyclopropyl
bedeutet und der Substituent R⁶ für eine Verbindung einer Zeile der Tabelle A entspricht.

Tabelle 665: (Verbindungen 665.1 - 665.974)
Verbindungen der allgemeinen Formel I.1, in denen
R³ = Methyl
R⁴ = C₃H₃
R⁵ = CN
bedeutet und der Substituent R⁶ für eine Verbindung einer Zeile der Tabelle A entspricht.

Tabelle 666: (Verbindungen 666.1 - 666.974)
Verbindungen der allgemeinen Formel I.2, in denen
R³ = Methyl
R⁴ = C₃H₃
R⁵ = CN
bedeutet und der Substituent R⁶ für eine Verbindung einer Zeile der Tabelle A entspricht.

Tabelle 667: (Verbindungen 667.1 - 667.974)
Verbindungen der allgemeinen Formel I.3, in denen
R³ = Methyl
R⁴ = C₃H₃
R⁵ = CN
bedeutet und der Substituent R⁶ für eine Verbindung einer Zeile der Tabelle A entspricht.

Tabelle 668: (Verbindungen 668.1 - 668.974)
Verbindungen der allgemeinen Formel I.4, in denen
R³ = Methyl
R⁴ = C₃H₃
R⁵ = CN
bedeutet und der Substituent R⁶ für eine Verbindung einer Zeile der Tabelle A entspricht.

Tabelle 669: (Verbindungen 669.1 - 349.974)
Verbindungen der allgemeinen Formel I.1, in denen
R³ = Methyl
R⁴ = C₃H₃
R⁵ = C₆H₅
bedeutet und der Substituent R⁶ für eine Verbindung einer Zeile der Tabelle A entspricht.

Tabelle 670: (Verbindungen 670.1 - 670.974)
Verbindungen der allgemeinen Formel I.2, in denen
R³ = Methyl
R⁴ = C₃H₃
R⁵ = C₆H₅
bedeutet und der Substituent R⁶ für eine Verbindung einer Zeile der Tabelle A entspricht.

Tabelle 671: (Verbindungen 671.1 - 671.974)
Verbindungen der allgemeinen Formel I.3, in denen
R³ = Methyl
R⁴ = C₃H₃
R⁵ = C₆H₅
bedeutet und der Substituent R⁶ für eine Verbindung einer Zeile der Tabelle A entspricht.

Tabelle 672: (Verbindungen 672.1 - 672.974)
Verbindungen der allgemeinen Formel I.4, in denen
R³ = Methyl
R⁴ = C₃H₃
R⁵ = C₆H₅
bedeutet und der Substituent R⁶ für eine Verbindung einer Zeile der Tabelle A entspricht.

Tabelle 673: (Verbindungen 673.1 - 673.974)
Verbindungen der allgemeinen Formel I.1, in denen
R³ = Methyl
R⁴ = C₃H₃
R⁵ = 2-F-C₆H₄
bedeutet und der Substituent R⁶ für eine Verbindung einer Zeile der Tabelle A entspricht.

Tabelle 674: (Verbindungen 674.1 - 674.974)
Verbindungen der allgemeinen Formel I.2, in denen
R³ = Methyl
R⁴ = C₃H₃
R⁵ = 2-F-C₆H₄
bedeutet und der Substituent R⁶ für eine Verbindung einer Zeile der Tabelle A entspricht.

Tabelle 675: (Verbindungen 675.1 - 675.974)
Verbindungen der allgemeinen Formel I.3, in denen
R³ = Methyl
R⁴ = C₃H₃
R⁵ = 2-F-C₆H₄
bedeutet und der Substituent R⁶ für eine Verbindung einer Zeile der Tabelle A entspricht.

Tabelle 676: (Verbindungen 676.1 - 676.974)
Verbindungen der allgemeinen Formel I.4, in denen
R³ = Methyl
R⁴ = C₃H₃
R⁵ = 2-F-C₆H₄
bedeutet und der Substituent R⁶ für eine Verbindung einer Zeile der Tabelle A entspricht.

Tabelle 677: (Verbindungen 677.1 - 677.974)
Verbindungen der allgemeinen Formel I.1, in denen
R³ = Methyl
R⁴ = C₃H₃
R⁵ = 3-F-C₆H₄
bedeutet und der Substituent R⁶ für eine Verbindung einer Zeile der Tabelle A entspricht.

Tabelle 678: (Verbindungen 678.1 - 678.974)
Verbindungen der allgemeinen Formel I.2, in denen
R³ = Methyl
R⁴ = C₃H₃
R⁵ = 3-F-C₆H₄
bedeutet und der Substituent R⁶ für eine Verbindung einer Zeile der Tabelle A entspricht.

Tabelle 679: (Verbindungen 679.1 - 679.974)
Verbindungen der allgemeinen Formel I.3, in denen
R³ = Methyl
R⁴ = C₃H₃
R⁵ = 3-F-C₆H₄
bedeutet und der Substituent R⁶ für eine Verbindung einer Zeile der Tabelle A entspricht.

Tabelle 680: (Verbindungen 680.1 - 680.974)
Verbindungen der allgemeinen Formel I.4, in denen
R³ = Methyl
R⁴ = C₃H₃
R⁵ = 3-F-C₆H₄
bedeutet und der Substituent R⁶ für eine Verbindung einer Zeile der Tabelle A entspricht.

Tabelle 681: (Verbindungen 681.1 - 681.974)
Verbindungen der allgemeinen Formel I.1, in denen
R³ = Methyl
R⁴ = C₃H₃
R⁵ = 4-F-C₆H₄
bedeutet und der Substituent R⁶ für eine Verbindung einer Zeile der Tabelle A entspricht.

Tabelle 682: (Verbindungen 682.1 - 682.974)
Verbindungen der allgemeinen Formel I.2, in denen
R³ = Methyl
R⁴ = C₃H₃
R⁵ = 4-F-C₆H₄
bedeutet und der Substituent R⁶ für eine Verbindung einer Zeile der Tabelle A entspricht.

Tabelle 683: (Verbindungen 683.1 - 683.974)
Verbindungen der allgemeinen Formel I.3, in denen
R³ = Methyl
R⁴ = C₃H₃
R⁵ = 4-F-C₆H₄
bedeutet und der Substituent R⁶ für eine Verbindung einer Zeile der Tabelle A entspricht.

Tabelle 684: (Verbindungen 684.1 - 684.974)
Verbindungen der allgemeinen Formel I.4, in denen
R³ = Methyl
R⁴ = C₃H₃
R⁵ = 4-F-C₆H₄
bedeutet und der Substituent R⁶ für eine Verbindung einer Zeile der Tabelle A entspricht.

Tabelle 685: (Verbindungen 685.1 - 685.974)
Verbindungen der allgemeinen Formel I.1, in denen
R³ = Methyl
R⁴ = C₃H₃
R⁵ = 2-Cl-C₆H₄
bedeutet und der Substituent R⁶ für eine Verbindung einer Zeile der Tabelle A entspricht.

Tabelle 686: (Verbindungen 686.1 - 686.974)
Verbindungen der allgemeinen Formel I.2, in denen
R³ = Methyl
R⁴ = C₃H₃
R⁵ = 2-Cl-C₆H₄
bedeutet und der Substituent R⁶ für eine Verbindung einer Zeile der Tabelle A entspricht.

Tabelle 687: (Verbindungen 687.1 - 687.974)
Verbindungen der allgemeinen Formel I.3, in denen
R³ = Methyl
R⁴ = C₃H₃
R⁵ = 2-Cl-C₆H₄
bedeutet und der Substituent R⁶ für eine Verbindung einer Zeile der Tabelle A entspricht.

Tabelle 688: (Verbindungen 688.1 - 688.974)
Verbindungen der allgemeinen Formel I.4, in denen
R³ = Methyl
R⁴ = C₃H₃
R⁵ = 2-Cl-C₆H₄
bedeutet und der Substituent R⁶ für eine Verbindung einer Zeile der Tabelle A entspricht.

Tabelle 689: (Verbindungen 689.1 - 689.974)
Verbindungen der allgemeinen Formel I.1, in denen
R³ = Methyl
R⁴ = C₃H₃
R⁵ = 3-Cl-C₆H₄
bedeutet und der Substituent R⁶ für eine Verbindung einer Zeile der Tabelle A entspricht.

Tabelle 690: (Verbindungen 690.1 - 690.974)
Verbindungen der allgemeinen Formel I.2, in denen
R³ = Methyl
R⁴ = C₃H₃
R⁵ = 3-Cl-C₆H₄
bedeutet und der Substituent R⁶ für eine Verbindung einer Zeile der Tabelle A entspricht.

Tabelle 691: (Verbindungen 691.1 - 691.974)
Verbindungen der allgemeinen Formel I.3, in denen
R³ = Methyl
R⁴ = C₃H₃
R⁵ = 3-Cl-C₆H₄
bedeutet und der Substituent R⁶ für eine Verbindung einer Zeile der Tabelle A entspricht.

Tabelle 692: (Verbindungen 692.1 - 692.974)
Verbindungen der allgemeinen Formel I.4, in denen
R³ = Methyl
R⁴ = C₃H₃
R⁵ = 3-Cl-C₆H₄
bedeutet und der Substituent R⁶ für eine Verbindung einer Zeile der Tabelle A entspricht.

Tabelle 693: (Verbindungen 693.1 - 693.974)
Verbindungen der allgemeinen Formel I.1, in denen
R³ = Methyl
R⁴ = C₃H₃
R⁵ = 4-Cl-C₆H₄
bedeutet und der Substituent R⁶ für eine Verbindung einer Zeile der Tabelle A entspricht.

Tabelle 694: (Verbindungen 694.1 - 694.974)
Verbindungen der allgemeinen Formel I.2, in denen
R³ = Methyl
R⁴ = C₃H₃
R⁵ = 4-Cl-C₆H₄
bedeutet und der Substituent R⁶ für eine Verbindung einer Zeile der Tabelle A entspricht.

Tabelle 695: (Verbindungen 695.1 - 695.974)
Verbindungen der allgemeinen Formel I.3, in denen
R³ = Methyl
R⁴ = C₃H₃
R⁵ = 4-Cl-C₆H₄
bedeutet und der Substituent R⁶ für eine Verbindung einer Zeile der Tabelle A entspricht.

Tabelle 696: (Verbindungen 696.1 - 696.974)
Verbindungen der allgemeinen Formel I.4, in denen
R³ = Methyl
R⁴ = C₃H₃
R⁵ = 4-Cl-C₆H₄
bedeutet und der Substituent R⁶ für eine Verbindung einer Zeile der Tabelle A entspricht.

Tabelle 697: (Verbindungen 697.1 - 697.974)
Verbindungen der allgemeinen Formel I.1, in denen
R³ = Methyl
R⁴ = C₃H₃
R⁵ = 2-Br-C₆H₄
bedeutet und der Substituent R⁶ für eine Verbindung einer Zeile der Tabelle A entspricht.

Tabelle 698: (Verbindungen 698.1 - 698.974)
Verbindungen der allgemeinen Formel I.2, in denen
R³ = Methyl
R⁴ = C₃H₃
R⁵ = 2-Br-C₆H₄
bedeutet und der Substituent R⁶ für eine Verbindung einer Zeile der Tabelle A entspricht.

Tabelle 699: (Verbindungen 699.1 - 699.974)
Verbindungen der allgemeinen Formel I.3, in denen
R³ = Methyl
R⁴ = C₃H₃
R⁵ = 2-Br-C₆H₄
bedeutet und der Substituent R⁶ für eine Verbindung einer Zeile der Tabelle A entspricht.

Tabelle 700: (Verbindungen 700.1 - 700.974)
Verbindungen der allgemeinen Formel I.4, in denen
R³ = Methyl
R⁴ = C₃H₃
R⁵ = 2-Br-C₆H₄
bedeutet und der Substituent R⁶ für eine Verbindung einer Zeile der Tabelle A entspricht.

Tabelle 701: (Verbindungen 701.1 - 701.974)
Verbindungen der allgemeinen Formel I.1, in denen
R³ = Methyl
R⁴ = C₃H₃
R⁵ = 3-Br-C₆H₄
bedeutet und der Substituent R⁶ für eine Verbindung einer Zeile der Tabelle A entspricht.

Tabelle 702: (Verbindungen 702.1 - 702.974)
Verbindungen der allgemeinen Formel I.2, in denen
R³ = Methyl
R⁴ = C₃H₃
R⁵ = 3-Br-C₆H₄
bedeutet und der Substituent R⁶ für eine Verbindung einer Zeile der Tabelle A entspricht.

Tabelle 703: (Verbindungen 708.1 - 703.974)
Verbindungen der allgemeinen Formel I.3, in denen
R³ = Methyl
R⁴ = C₃H₃
R⁵ = 3-Br-C₆H₄
bedeutet und der Substituent R⁶ für eine Verbindung einer Zeile der Tabelle A entspricht.

Tabelle 704: (Verbindungen 704.1 - 704.974)
Verbindungen der allgemeinen Formel I.4, in denen
R³ = Methyl
R⁴ = C₃H₃
R⁵ = 3-Br-C₆H₄
bedeutet und der Substituent R⁶ für eine Verbindung einer Zeile der Tabelle A entspricht.

Tabelle 705: (Verbindungen 705.1 - 705.974)
Verbindungen der allgemeinen Formel I.1, in denen
R³ = Methyl
R⁴ = C₃H₃
R⁵ = 4-Br-C₆H₄
bedeutet und der Substituent R⁶ für eine Verbindung einer Zeile der Tabelle A entspricht.

Tabelle 706: (Verbindungen 706.1 - 706.974)
Verbindungen der allgemeinen Formel I.2, in denen
R³ = Methyl
R⁴ = C₃H₃
R⁵ = 4-Br-C₆H₄
bedeutet und der Substituent R⁶ für eine Verbindung einer Zeile der Tabelle A entspricht.

Tabelle 707: (Verbindungen 707.1 - 707.974)
Verbindungen der allgemeinen Formel I.3, in denen
R³ = Methyl
R⁴ = C₃H₃
R⁵ = 4-Br-C₆H₄
bedeutet und der Substituent R⁶ für eine Verbindung einer Zeile der Tabelle A entspricht.

Tabelle 708: (Verbindungen 708.1 - 708.974)
Verbindungen der allgemeinen Formel I.4, in denen
R³ = Methyl
R⁴ = C₃H₃
R⁵ = 4-Br-C₆H₄
bedeutet und der Substituent R⁶ für eine Verbindung einer Zeile der Tabelle A entspricht.

Tabelle 709: (Verbindungen 709.1 - 709.974)
Verbindungen der allgemeinen Formel I.1, in denen
R³ = Methyl
R⁴ = C₃H₃
R⁵ = 2,3-F₂-C₆H₃
bedeutet und der Substituent R⁶ für eine Verbindung einer Zeile der Tabelle A entspricht.

Tabelle 710: (Verbindungen 710.1 - 710.974)
Verbindungen der allgemeinen Formel I.2, in denen
R³ = Methyl
R⁴ = C₃H₃
R⁵ = 2,3-F₂-C₆H₃
bedeutet und der Substituent R⁶ für eine Verbindung einer Zeile der Tabelle A entspricht.

Tabelle 711: (Verbindungen 711.1 - 711.974)
Verbindungen der allgemeinen Formel I.3, in denen
R³ = Methyl
R⁴ = C₃H₃
R⁵ = 2,3-F₂-C₆H₃
bedeutet und der Substituent R⁶ für eine Verbindung einer Zeile der Tabelle A entspricht.

Tabelle 712: (Verbindungen 712.1 - 712.974)
Verbindungen der allgemeinen Formel I.4, in denen
R³ = Methyl
R⁴ = C₃H₃
R⁵ = 2,3-F₂-C₆H₃
bedeutet und der Substituent R⁶ für eine Verbindung einer Zeile der Tabelle A entspricht.

Tabelle 713: (Verbindungen 713.1 - 713.974)
Verbindungen der allgemeinen Formel I.1, in denen
R³ = Methyl
R⁴ = C₃H₃
R⁵ = 2,4-F₂-C₆H₃
bedeutet und der Substituent R⁶ für eine Verbindung einer Zeile der Tabelle A entspricht.

Tabelle 714: (Verbindungen 714.1 - 714.974)
Verbindungen der allgemeinen Formel I.2, in denen
R³ = Methyl
R⁴ = C₃H₃
R⁵ = 2,4-F₂-C₆H₃
bedeutet und der Substituent R⁶ für eine Verbindung einer Zeile der Tabelle A entspricht.

Tabelle 715: (Verbindungen 715.1 - 755.974)
Verbindungen der allgemeinen Formel I.3, in denen
R³ = Methyl
R⁴ = C₃H₃
R⁵ = 2,4-F₂-C₆H₃
bedeutet und der Substituent R⁶ für eine Verbindung einer Zeile der Tabelle A entspricht.

Tabelle 716: (Verbindungen 716.1 - 716.974)
Verbindungen der allgemeinen Formel I.4, in denen
R³ = Methyl
R⁴ = C₃H₃
R⁵ = 2,4-F₂-C₆H₃
bedeutet und der Substituent R⁶ für eine Verbindung einer Zeile der Tabelle A entspricht.

Tabelle 717: (Verbindungen 717.1 - 717.974)
Verbindungen der allgemeinen Formel I.1, in denen
R³ = Methyl
R⁴ = C₃H₃
R⁵ = 2,5-F₂-C₆H₃
bedeutet und der Substituent R⁶ für eine Verbindung einer Zeile der Tabelle A entspricht.

Tabelle 718: (Verbindungen 718.1 - 718.974)
Verbindungen der allgemeinen Formel I.2, in denen
R³ = Methyl
R⁴ = C₃H₃
R⁵ = 2,5-F₂-C₆H₃
bedeutet und der Substituent R⁶ für eine Verbindung einer Zeile der Tabelle A entspricht.

Tabelle 719: (Verbindungen 719.1 - 719.974)
Verbindungen der allgemeinen Formel I.3, in denen
R³ = Methyl
R⁴ = C₃H₃
R⁵ = 2,5-F₂-C₆H₃
bedeutet und der Substituent R⁶ für eine Verbindung einer Zeile der Tabelle A entspricht.

Tabelle 720: (Verbindungen 720.1 - 720.974)
Verbindungen der allgemeinen Formel I.4, in denen
R³ = Methyl
R⁴ = C₃H₃
R⁵ = 2,5-F₂-C₆H₃
bedeutet und der Substituent R⁶ für eine Verbindung einer Zeile der Tabelle A entspricht.

Tabelle 721: (Verbindungen 721.1 - 721.974)
Verbindungen der allgemeinen Formel I.1, in denen
R³ = Methyl
R⁴ = C₃H₃
R⁵ = 3,4-F₂-C₆H₃
bedeutet und der Substituent R⁶ für eine Verbindung einer Zeile der Tabelle A entspricht.

Tabelle 722: (Verbindungen 722.1 - 722.974)
Verbindungen der allgemeinen Formel I.2, in denen
R³ = Methyl
R⁴ = C₃H₃
R⁵ = 3,4-F₂-C₆H₃
bedeutet und der Substituent R⁶ für eine Verbindung einer Zeile der Tabelle A entspricht.

Tabelle 723: (Verbindungen 723.1 - 723.974)
Verbindungen der allgemeinen Formel I.3, in denen
R³ = Methyl
R⁴ = C₃H₃
R⁵ = 3,4-F₂-C₆H₃
bedeutet und der Substituent R⁶ für eine Verbindung einer Zeile der Tabelle A entspricht.

Tabelle 724: (Verbindungen 724.1 - 724.974)
Verbindungen der allgemeinen Formel I.4, in denen
R³ = Methyl
R⁴ = C₃H₃
R⁵ = 3,4-F₂-C₆H₃
bedeutet und der Substituent R⁶ für eine Verbindung einer Zeile der Tabelle A entspricht.

Tabelle 725: (Verbindungen 725.1 - 725.974)
Verbindungen der allgemeinen Formel I.1, in denen
R³ = Methyl
R⁴ = C₃H₃
R⁵ = 3,5-F₂-C₆H₃
bedeutet und der Substituent R⁶ für eine Verbindung einer Zeile der Tabelle A entspricht.

Tabelle 726: (Verbindungen 726.1 - 726.974)
Verbindungen der allgemeinen Formel I.2, in denen
R³ = Methyl
R⁴ = C₃H₃
R⁵ = 3,5-F₂-C₆H₃
bedeutet und der Substituent R⁶ für eine Verbindung einer Zeile der Tabelle A entspricht.

Tabelle 727: (Verbindungen 727.1 - 727.974)
Verbindungen der allgemeinen Formel I.3, in denen
R³ = Methyl
R⁴ = C₃H₃
R⁵ = 3,5-F₂-C₆H₃
bedeutet und der Substituent R⁶ für eine Verbindung einer Zeile der Tabelle A entspricht.

Tabelle 728: (Verbindungen 728.1 - 728.974)
Verbindungen der allgemeinen Formel I.4, in denen
R³ = Methyl
R⁴ = C₃H₃
R⁵ = 3,5-F₂-C₆H₃
bedeutet und der Substituent R⁶ für eine Verbindung einer Zeile der Tabelle A entspricht.

Tabelle 729: (Verbindungen 729.1 - 729.974)
Verbindungen der allgemeinen Formel I.1, in denen
R³ = Methyl
R⁴ = C₃H₃
R⁵ = 2,3-Cl₂-C₆H₃
bedeutet und der Substituent R⁶ für eine Verbindung einer Zeile der Tabelle A entspricht.

Tabelle 730: (Verbindungen 730.1 - 730.974)
Verbindungen der allgemeinen Formel I.2, in denen
R³ = Methyl
R⁴ = C₃H₃
R⁵ = 2,3-Cl₂-C₆H₃
bedeutet und der Substituent R⁶ für eine Verbindung einer Zeile der Tabelle A entspricht.

Tabelle 731: (Verbindungen 731.1 - 731.974)
Verbindungen der allgemeinen Formel I.3, in denen
R³ = Methyl
R⁴ = C₃H₃
R⁵ = 2,3-Cl₂-C₆H₃
bedeutet und der Substituent R⁶ für eine Verbindung einer Zeile der Tabelle A entspricht.

Tabelle 732: (Verbindungen 732.1 - 732.974)
Verbindungen der allgemeinen Formel I.4, in denen
R³ = Methyl
R⁴ = C₃H₃
R⁵ = 2,3-Cl₂-C₆H₃
bedeutet und der Substituent R⁶ für eine Verbindung einer Zeile der Tabelle A entspricht.

Tabelle 733: (Verbindungen 733.1 - 733.974)
Verbindungen der allgemeinen Formel I.1, in denen
R³ = Methyl
R⁴ = C₃H₃
R⁵ = 2,4-Cl₂-C₆H₃
bedeutet und der Substituent R⁶ für eine Verbindung einer Zeile der Tabelle A entspricht.

Tabelle 734: (Verbindungen 734.1 - 734.974)
Verbindungen der allgemeinen Formel I.2, in denen
R³ = Methyl
R⁴ = C₃H₃
R⁵ = 2,4-Cl₂-C₆H₃
bedeutet und der Substituent R⁶ für eine Verbindung einer Zeile der Tabelle A entspricht.

Tabelle 735: (Verbindungen 735.1 - 735.974)
Verbindungen der allgemeinen Formel I.3, in denen
R³ = Methyl
R⁴ = C₃H₃
R⁵ = 2,4-Cl₂-C₆H₃
bedeutet und der Substituent R⁶ für eine Verbindung einer Zeile der Tabelle A entspricht.

Tabelle 736: (Verbindungen 736.1 - 736.974)
Verbindungen der allgemeinen Formel I.4, in denen
R³ = Methyl
R⁴ = C₃H₃
R⁵ = 2,4-Cl₂-C₆H₃
bedeutet und der Substituent R⁶ für eine Verbindung einer Zeile der Tabelle A entspricht.

Tabelle 737: (Verbindungen 737.1 - 737.974)
Verbindungen der allgemeinen Formel I.1, in denen
R³ = Methyl
R⁴ = C₃H₃
R⁵ = 2,5-Cl₂-C₆H₃
bedeutet und der Substituent R⁶ für eine Verbindung einer Zeile der Tabelle A entspricht.

Tabelle 738: (Verbindungen 738.1 - 738.974)
Verbindungen der allgemeinen Formel I.2, in denen
R³ = Methyl
R⁴ = C₃H₃
R⁵ = 2,5-Cl₂-C₆H₃
bedeutet und der Substituent R⁶ für eine Verbindung einer Zeile der Tabelle A entspricht.

Tabelle 739: (Verbindungen 739.1 - 739.974)
Verbindungen der allgemeinen Formel I.3, in denen
R³ = Methyl
R⁴ = C₃H₃
R⁵ = 2,5-Cl₂-C₆H₃
bedeutet und der Substituent R⁶ für eine Verbindung einer Zeile der Tabelle A entspricht.

Tabelle 740: (Verbindungen 740.1 - 740.974)
Verbindungen der allgemeinen Formel I.4, in denen
R³ = Methyl
R⁴ = C₃H₃
R⁵ = 2,5-Cl₂-C₆H₃
bedeutet und der Substituent R⁶ für eine Verbindung einer Zeile der Tabelle A entspricht.

Tabelle 741: (Verbindungen 741.1 - 741.974)
Verbindungen der allgemeinen Formel I.1, in denen
R³ = Methyl
R⁴ = C₃H₃
R⁵ = 3,4-Cl₂-C₆H₃
bedeutet und der Substituent R⁶ für eine Verbindung einer Zeile der Tabelle A entspricht.

Tabelle 742: (Verbindungen 742.1 - 742.974)
Verbindungen der allgemeinen Formel I.2, in denen
R³ = Methyl
R⁴ = C₃H₃
R⁵ = 3,4-Cl₂-C₆H₃
bedeutet und der Substituent R⁶ für eine Verbindung einer Zeile der Tabelle A entspricht.

Tabelle 743: (Verbindungen 743.1 - 743.974)
Verbindungen der allgemeinen Formel I.3, in denen
R³ = Methyl
R⁴ = C₃H₃
R⁵ = 3,4-Cl₂-C₆H₃
bedeutet und der Substituent R⁶ für eine Verbindung einer Zeile der Tabelle A entspricht.

Tabelle 744: (Verbindungen 744.1 - 744.974)
Verbindungen der allgemeinen Formel I.4, in denen
R³ = Methyl
R⁴ = C₃H₃
R⁵ = 3,4-Cl₂-C₆H₃
bedeutet und der Substituent R⁶ für eine Verbindung einer Zeile der Tabelle A entspricht.

Tabelle 745: (Verbindungen 745.1 - 745.974)
Verbindungen der allgemeinen Formel I.1, in denen
R³ = Methyl
R⁴ = C₃H₃
R⁵ = 3,5-Cl₂-C₆H₃
bedeutet und der Substituent R⁶ für eine Verbindung einer Zeile der Tabelle A entspricht.

Tabelle 746: (Verbindungen 746.1 - 746.974)
Verbindungen der allgemeinen Formel I.2, in denen
R³ = Methyl
R⁴ = C₃H₃
R⁵ = 3,5-Cl₂-C₆H₃
bedeutet und der Substituent R⁶ für eine Verbindung einer Zeile der Tabelle A entspricht.

Tabelle 747: (Verbindungen 747.1 - 747.974)
Verbindungen der allgemeinen Formel I.3, in denen
R³ = Methyl
R⁴ = C₃H₃
R⁵ = 3,5-Cl₂-C₆H₃
bedeutet und der Substituent R⁶ für eine Verbindung einer Zeile der Tabelle A entspricht.

Tabelle 748: (Verbindungen 748.1 - 748.974)
Verbindungen der allgemeinen Formel I.4, in denen
R³ = Methyl
R⁴ = C₃H₃
R⁵ = 3,5-Cl₂-C₆H₃
bedeutet und der Substituent R⁶ für eine Verbindung einer Zeile der Tabelle A entspricht.

Tabelle 749: (Verbindungen 749.1 - 749.974)
Verbindungen der allgemeinen Formel I.1, in denen
R³ = Methyl
R⁴ = C₃H₃
R⁵ = 2-F; 3-Cl-C₆H₃
bedeutet und der Substituent R⁶ für eine Verbindung einer Zeile der Tabelle A entspricht.

Tabelle 750: (Verbindungen 750.1 - 750.974)
Verbindungen der allgemeinen Formel I.2, in denen
R³ = Methyl
R⁴ = C₃H₃
R⁵ = 2-F; 3-Cl-C₆H₃
bedeutet und der Substituent R⁶ für eine Verbindung einer Zeile der Tabelle A entspricht.

Tabelle 751: (Verbindungen 751.1 - 751.974)
Verbindungen der allgemeinen Formel I.3, in denen
R³ = Methyl
R⁴ = C₃H₃
R⁵ = 2-F; 3-Cl-C₆H₃
bedeutet und der Substituent R⁶ für eine Verbindung einer Zeile der Tabelle A entspricht.

Tabelle 752: (Verbindungen 752.1 - 752.974)
Verbindungen der allgemeinen Formel I.4, in denen
R³ = Methyl
R⁴ = C₃H₃
R⁵ = 2-F; 3-Cl-C₆H₃
bedeutet und der Substituent R⁶ für eine Verbindung einer Zeile der Tabelle A entspricht.

Tabelle 753: (Verbindungen 753.1 - 753.974)
Verbindungen der allgemeinen Formel I.1, in denen
R³ = Methyl
R⁴ = C₃H₃
R⁵ = 2-F; 4-Cl-C₆H₃
bedeutet und der Substituent R⁶ für eine Verbindung einer Zeile der Tabelle A entspricht.

Tabelle 754: (Verbindungen 754.1 - 754.974)
Verbindungen der allgemeinen Formel I.2, in denen
R³ = Methyl
R⁴ = C₃H₃
R⁵ = 2-F; 4-Cl-C₆H₃
bedeutet und der Substituent R⁶ für eine Verbindung einer Zeile der Tabelle A entspricht.

Tabelle 755: (Verbindungen 755.1 - 755.974)
Verbindungen der allgemeinen Formel I.3, in denen
R³ = Methyl
R⁴ = C₃H₃
R⁵ = 2-F; 4-Cl-C₆H₃
bedeutet und der Substituent R⁶ für eine Verbindung einer Zeile der Tabelle A entspricht.

Tabelle 756: (Verbindungen 756.1 - 756.974)
Verbindungen der allgemeinen Formel I.4, in denen
R³ = Methyl
R⁴ = C₃H₃
R⁵ = 2-F; 4-Cl-C₆H₃
bedeutet und der Substituent R⁶ für eine Verbindung einer Zeile der Tabelle A entspricht.

Tabelle 757: (Verbindungen 757.1 - 757.974)
Verbindungen der allgemeinen Formel I.1, in denen
R³ = Methyl
R⁴ = C₃H₃
R⁵ = 3-F; 4-Cl-C₆H₃
bedeutet und der Substituent R⁶ für eine Verbindung einer Zeile der Tabelle A entspricht.

Tabelle 758: (Verbindungen 758.1 - 758.974)
Verbindungen der allgemeinen Formel I.2, in denen
R³ = Methyl
R⁴ = C₃H₃
R⁵ = 3-F; 4-Cl-C₆H₃
bedeutet und der Substituent R⁶ für eine Verbindung einer Zeile der Tabelle A entspricht.

Tabelle 759: (Verbindungen 759.1 - 759.974)
Verbindungen der allgemeinen Formel I.3, in denen
R³ = Methyl
R⁴ = C₃H₃
R⁵ = 3-F; 4-Cl-C₆H₃
bedeutet und der Substituent R⁶ für eine Verbindung einer Zeile der Tabelle A entspricht.

Tabelle 760: (Verbindungen 760.1 - 760.974)
Verbindungen der allgemeinen Formel I.4, in denen
R³ = Methyl
R⁴ = C₃H₃
R⁵ = 3-F; 4-Cl-C₆H₃
bedeutet und der Substituent R⁶ für eine Verbindung einer Zeile der Tabelle A entspricht.

Tabelle 761: (Verbindungen 761.1 - 761.974)
Verbindungen der allgemeinen Formel I.1, in denen
R³ = Methyl
R⁴ = C₃H₃
R⁵ = 4-F; 3,5-Cl₂-C₆H₂
bedeutet und der Substituent R⁶ für eine Verbindung einer Zeile der Tabelle A entspricht.

Tabelle 762: (Verbindungen 762.1 - 762.974)
Verbindungen der allgemeinen Formel I.2, in denen
R³ = Methyl
R⁴ = C₃H₃
R⁵ = 4-F; 3,5-Cl₂-C₆H₂
bedeutet und der Substituent R⁶ für eine Verbindung einer Zeile der Tabelle A entspricht.

Tabelle 763: (Verbindungen 763.1 - 763.974)
Verbindungen der allgemeinen Formel I.3, in denen
R³ = Methyl
R⁴ = C₃H₃
R⁵ = 4-F; 3,5-Cl₂-C₆H₂
bedeutet und der Substituent R⁶ für eine Verbindung einer Zeile der Tabelle A entspricht.

Tabelle 764: (Verbindungen 764.1 - 764.974)
Verbindungen der allgemeinen Formel I.4, in denen
R³ = Methyl
R⁴ = C₃H₃
R⁵ = 4-F; 3,5-Cl₂-C₆H₂
bedeutet und der Substituent R⁶ für eine Verbindung einer Zeile der Tabelle A entspricht.

Tabelle 765: (Verbindungen 765.1 - 765.974)
Verbindungen der allgemeinen Formel I.1, in denen
R³ = Methyl
R⁴ = C₃H₃
R⁵ = 2,3,4-Cl₃-C₆H₂
bedeutet und der Substituent R⁶ für eine Verbindung einer Zeile der Tabelle A entspricht.

Tabelle 766: (Verbindungen 766.1 - 766.974)
Verbindungen der allgemeinen Formel I.2, in denen
R³ = Methyl
R⁴ = C₃H₃
R⁵ = 2,3,4-Cl₃-C₆H₂
bedeutet und der Substituent R⁶ für eine Verbindung einer Zeile der Tabelle A entspricht.

Tabelle 767: (Verbindungen 767.1 - 767.974)
Verbindungen der allgemeinen Formel I.3, in denen
R³ = Methyl
R⁴ = C₃H₃
R⁵ = 2,3,4-Cl₃-C₆H₂
bedeutet und der Substituent R⁶ für eine Verbindung einer Zeile der Tabelle A entspricht.

Tabelle 768: (Verbindungen 768.1 - 768.974)
Verbindungen der allgemeinen Formel I.4, in denen
R³ = Methyl
R⁴ = C₃H₃
R⁵ = 2,3,4-Cl₃-C₆H₂
bedeutet und der Substituent R⁶ für eine Verbindung einer Zeile der Tabelle A entspricht.

Tabelle 769: (Verbindungen 769.1 - 769.974)
Verbindungen der allgemeinen Formel I.1, in denen
R³ = Methyl
R⁴ = C₃H₃
R⁵ = 2,3,5-Cl₃-C₆H₂
bedeutet und der Substituent R⁶ für eine Verbindung einer Zeile der Tabelle A entspricht.

Tabelle 770: (Verbindungen 770.1 - 770.974)
Verbindungen der allgemeinen Formel I.2, in denen
R³ = Methyl
R⁴ = C₃H₃
R⁵ = 2,3,5-Cl₃-C₆H₂
bedeutet und der Substituent R⁶ für eine Verbindung einer Zeile der Tabelle A entspricht.

Tabelle 771: (Verbindungen 771.1 - 771.974)
Verbindungen der allgemeinen Formel I.3, in denen
R³ = Methyl
R⁴ = C₃H₃
R⁵ = 2,3,5-Cl₃-C₆H₂
bedeutet und der Substituent R⁶ für eine Verbindung einer Zeile der Tabelle A entspricht.

Tabelle 772: (Verbindungen 772.1 - 772.974)
Verbindungen der allgemeinen Formel I.4, in denen
R³ = Methyl
R⁴ = C₃H₃
R⁵ = 2,3,5-Cl₃-C₆H₂
bedeutet und der Substituent R⁶ für eine Verbindung einer Zeile der Tabelle A entspricht.

Tabelle 773: (Verbindungen 773.1 - 773.974)
Verbindungen der allgemeinen Formel I.1, in denen
R³ = Methyl
R⁴ = C₃H₃
R⁵ = 2,4,5-Cl₃-C₆H₂
bedeutet und der Substituent R⁶ für eine Verbindung einer Zeile der Tabelle A entspricht.

Tabelle 774: (Verbindungen 774.1 - 774.974)
Verbindungen der allgemeinen Formel I.2, in denen
R³ = Methyl
R⁴ = C₃H₃
R⁵ = 2,4,5-Cl₃-C₆H₂
bedeutet und der Substituent R⁶ für eine Verbindung einer Zeile der Tabelle A entspricht.

Tabelle 775: (Verbindungen 775.1 - 775.974)
Verbindungen der allgemeinen Formel I.3, in denen
R³ = Methyl
R⁴ = C₃H₃
R⁵ = 2,4,5-Cl₃-C₆H₂
bedeutet und der Substituent R⁶ für eine Verbindung einer Zeile der Tabelle A entspricht.

Tabelle 776: (Verbindungen 776.1 - 776.974)
Verbindungen der allgemeinen Formel I.4, in denen
R³ = Methyl
R⁴ = C₃H₃
R⁵ = 2,4,5-Cl₃-C₆H₂
bedeutet und der Substituent R⁶ für eine Verbindung einer Zeile der Tabelle A entspricht.

Tabelle 777: (Verbindungen 777.1 - 777.974)
Verbindungen der allgemeinen Formel I.1, in denen
R³ = Methyl
R⁴ = C₃H₃
R⁵ = 2-CN-C₆H₄
bedeutet und der Substituent R⁶ für eine Verbindung einer Zeile der Tabelle A entspricht.

Tabelle 778: (Verbindungen 778.1 - 778.974)
Verbindungen der allgemeinen Formel I.2, in denen
R³ = Methyl
R⁴ = C₃H₃
R⁵ = 2-CN-C₆H₄
bedeutet und der Substituent R⁶ für eine Verbindung einer Zeile der Tabelle A entspricht.

Tabelle 779: (Verbindungen 779.1 - 779.974)
Verbindungen der allgemeinen Formel I.3, in denen
R³ = Methyl
R⁴ = C₃H₃
R⁵ = 2-CN-C₆H₄
bedeutet und der Substituent R⁶ für eine Verbindung einer Zeile der Tabelle A entspricht.

Tabelle 780: (Verbindungen 780.1 - 780.974)
Verbindungen der allgemeinen Formel I.4, in denen
R³ = Methyl
R⁴ = C₃H₃
R⁵ = 2-CN-C₆H₄
bedeutet und der Substituent R⁶ für eine Verbindung einer Zeile der Tabelle A entspricht.

Tabelle 781: (Verbindungen 781.1 - 781.974)
Verbindungen der allgemeinen Formel I.1, in denen
R³ = Methyl
R⁴ = C₃H₃
R⁵ = 3-CN-C₆H₄
bedeutet und der Substituent R⁶ für eine Verbindung einer Zeile der Tabelle A entspricht.

Tabelle 782: (Verbindungen 782.1 - 782.974)
Verbindungen der allgemeinen Formel I.2, in denen
R³ = Methyl
R⁴ = C₃H₃
R⁵ = 3-CN-C₆H₄
bedeutet und der Substituent R⁶ für eine Verbindung einer Zeile der Tabelle A entspricht.

Tabelle 783: (Verbindungen 783.1 - 783.974)
Verbindungen der allgemeinen Formel I.3, in denen
R³ = Methyl
R⁴ = C₃H₃
R⁵ = 3-CN-C₆H₄
bedeutet und der Substituent R⁶ für eine Verbindung einer Zeile der Tabelle A entspricht.

Tabelle 784: (Verbindungen 784.1 - 784.974)
Verbindungen der allgemeinen Formel I.4, in denen
R³ = Methyl
R⁴ = C₃H₃
R⁵ = 3-CN-C₆H₄
bedeutet und der Substituent R⁶ für eine Verbindung einer Zeile der Tabelle A entspricht.

Tabelle 785: (Verbindungen 785.1 - 785.974)
Verbindungen der allgemeinen Formel I.1, in denen
R³ = Methyl
R⁴ = C₃H₃
R⁵ = 4-CN-C₆H₄
bedeutet und der Substituent R⁶ für eine Verbindung einer Zeile der Tabelle A entspricht.

Tabelle 786: (Verbindungen 786.1 - 786.974)
Verbindungen der allgemeinen Formel I.2, in denen
R³ = Methyl
R⁴ = C₃H₃
R⁵ = 4-CN-C₆H₄
bedeutet und der Substituent R⁶ für eine Verbindung einer Zeile der Tabelle A entspricht.

Tabelle 787: (Verbindungen 7867.1 - 787.974)
Verbindungen der allgemeinen Formel I.3, in denen
R³ = Methyl
R⁴ = C₃H₃
R⁵ = 4-CN-C₆H₄
bedeutet und der Substituent R⁶ für eine Verbindung einer Zeile der Tabelle A entspricht.

Tabelle 788: (Verbindungen 788.1 - 788.974)
Verbindungen der allgemeinen Formel I.4, in denen
R³ = Methyl
R⁴ = C₃H₃
R⁵ = 4-CN-C₆H₄
bedeutet und der Substituent R⁶ für eine Verbindung einer Zeile der Tabelle A entspricht.

Tabelle 789: (Verbindungen 789.1 - 789.974)
Verbindungen der allgemeinen Formel I.1, in denen
R³ = Methyl
R⁴ = C₃H₃
R⁵ = 2-NO₂-C₆H₄
bedeutet und der Substituent R⁶ für eine Verbindung einer Zeile der Tabelle A entspricht.

Tabelle 790: (Verbindungen 790.1 - 790.974)
Verbindungen der allgemeinen Formel I.2, in denen
R³ = Methyl
R⁴ = C₃H₃
R⁵ = 2-NO₂-C₆H₄
bedeutet und der Substituent R⁶ für eine Verbindung einer Zeile der Tabelle A entspricht.

Tabelle 791: (Verbindungen 791.1 - 791.974)
Verbindungen der allgemeinen Formel I.3, in denen
R³ = Methyl
R⁴ = C₃H₃
R⁵ = 2-NO₂-C₆H₄
bedeutet und der Substituent R⁶ für eine Verbindung einer Zeile der Tabelle A entspricht.

Tabelle 792: (Verbindungen 792.1 - 792.974)
Verbindungen der allgemeinen Formel I.4, in denen
R³ = Methyl
R⁴ = C₃H₃
R⁵ = 2-NO₂-C₆H₄
bedeutet und der Substituent R⁶ für eine Verbindung einer Zeile der Tabelle A entspricht.

Tabelle 793: (Verbindungen 793.1 - 793.974)
Verbindungen der allgemeinen Formel I.1, in denen
R³ = Methyl
R⁴ = C₃H₃
R⁵ = 3-NO₂-C₆H₄
bedeutet und der Substituent R⁶ für eine Verbindung einer Zeile der Tabelle A entspricht.

Tabelle 794: (Verbindungen 794.1 - 794.974)
Verbindungen der allgemeinen Formel I.2, in denen
R³ = Methyl
R⁴ = C₃H₃
R⁵ = 3-NO₂-C₆H₄
bedeutet und der Substituent R⁶ für eine Verbindung einer Zeile der Tabelle A entspricht.

Tabelle 795: (Verbindungen 795.1 - 795.974)
Verbindungen der allgemeinen Formel I.3, in denen
R³ = Methyl
R⁴ = C₃H₃
R⁵ = 3-NO₂-C₆H₄
bedeutet und der Substituent R⁶ für eine Verbindung einer Zeile der Tabelle A entspricht.

Tabelle 796: (Verbindungen 796.1 - 796.974)
Verbindungen der allgemeinen Formel I.4, in denen
R³ = Methyl
R⁴ = C₃H₃
R⁵ = 3-NO₂-C₆H₄
bedeutet und der Substituent R⁶ für eine Verbindung einer Zeile der Tabelle A entspricht.

Tabelle 797: (Verbindungen 797.1 - 797.974)
Verbindungen der allgemeinen Formel I.1, in denen
R³ = Methyl
R⁴ = C₃H₃
R⁵ = 4-NO₂-C₆H₄
bedeutet und der Substituent R⁶ für eine Verbindung einer Zeile der Tabelle A entspricht.

Tabelle 798: (Verbindungen 798.1 - 798.974)
Verbindungen der allgemeinen Formel I.2, in denen
R³ = Methyl
R⁴ = C₃H₃
R⁵ = 4-NO₂-C₆H₄
bedeutet und der Substituent R⁶ für eine Verbindung einer Zeile der Tabelle A entspricht.

Tabelle 799: (Verbindungen 799.1 - 799.974)
Verbindungen der allgemeinen Formel I.3, in denen
R³ = Methyl
R⁴ = C₃H₃
R⁵ = 4-NO₂-C₆H₄
bedeutet und der Substituent R⁶ für eine Verbindung einer Zeile der Tabelle A entspricht.

Tabelle 800: (Verbindungen 800.1 - 800.974)
Verbindungen der allgemeinen Formel I.4, in denen
R³ = Methyl
R⁴ = C₃H₃
R⁵ = 4-NO₂-C₆H₄
bedeutet und der Substituent R⁶ für eine Verbindung einer Zeile der Tabelle A entspricht.

Tabelle 801: (Verbindungen 801.1 - 801.974)
Verbindungen der allgemeinen Formel I.1, in denen
R³ = Methyl
R⁴ = C₃H₃
R⁵ = 2-CH₃-C₆H₄
bedeutet und der Substituent R⁶ für eine Verbindung einer Zeile der Tabelle A entspricht.

Tabelle 802: (Verbindungen 802.1 - 802.974)
Verbindungen der allgemeinen Formel I.2, in denen
R³ = Methyl
R⁴ = C₃H₃
R⁵ = 2-CH₃-C₆H₄
bedeutet und der Substituent R⁶ für eine Verbindung einer Zeile der Tabelle A entspricht.

Tabelle 803: (Verbindungen 803.1 - 803.974)
Verbindungen der allgemeinen Formel I.3, in denen
R³ = Methyl
R⁴ = C₃H₃
R⁵ = 2-CH₃-C₆H₄
bedeutet und der Substituent R⁶ für eine Verbindung einer Zeile der Tabelle A entspricht.

Tabelle 804: (Verbindungen 804.1 - 804.974)
Verbindungen der allgemeinen Formel I.4, in denen
R³ = Methyl
R⁴ = C₃H₃
R⁵ = 2-CH₃-C₆H₄
bedeutet und der Substituent R⁶ für eine Verbindung einer Zeile der Tabelle A entspricht.

Tabelle 805: (Verbindungen 805.1 - 805.974)
Verbindungen der allgemeinen Formel I.1, in denen
R³ = Methyl
R⁴ = C₃H₃
R⁵ = 3-CH₃-C₆H₄
bedeutet und der Substituent R⁶ für eine Verbindung einer Zeile der Tabelle A entspricht.

Tabelle 806: (Verbindungen 806.1 - 806.974)
Verbindungen der allgemeinen Formel I.2, in denen
R³ = Methyl
R⁴ = C₃H₃
R⁵ = 3-CH₃-C₆H₄
bedeutet und der Substituent R⁶ für eine Verbindung einer Zeile der Tabelle A entspricht.

Tabelle 807: (Verbindungen 807.1 - 807.974)
Verbindungen der allgemeinen Formel I.3, in denen
R³ = Methyl
R⁴ = C₃H₃
R⁵ = 3-CH₃-C₆H₄
bedeutet und der Substituent R⁶ für eine Verbindung einer Zeile der Tabelle A entspricht.

Tabelle 808: (Verbindungen 808.1 - 808.974)
Verbindungen der allgemeinen Formel I.4, in denen
R³ = Methyl
R⁴ = C₃H₃
R⁵ = 3-CH₃-C₆H₄
bedeutet und der Substituent R⁶ für eine Verbindung einer Zeile der Tabelle A entspricht.

Tabelle 809: (Verbindungen 809.1 - 809.974)
Verbindungen der allgemeinen Formel I.1, in denen
R³ = Methyl
R⁴ = C₃H₃
R⁵ = 4-CH₃-C₆H₄
bedeutet und der Substituent R⁶ für eine Verbindung einer Zeile der Tabelle A entspricht.

Tabelle 810: (Verbindungen 810.1 - 810.974)
Verbindungen der allgemeinen Formel I.2, in denen
R³ = Methyl
R⁴ = C₃H₃
R⁵ = 4-CH₃-C₆H₄
bedeutet und der Substituent R⁶ für eine Verbindung einer Zeile der Tabelle A entspricht.

Tabelle 811: (Verbindungen 811.1 - 811.974)
Verbindungen der allgemeinen Formel I.3, in denen
R³ = Methyl
R⁴ = C₃H₃
R⁵ = 4-CH₃-C₆H₄
bedeutet und der Substituent R⁶ für eine Verbindung einer Zeile der Tabelle A entspricht.

Tabelle 812: (Verbindungen 812.1 - 812.974)
Verbindungen der allgemeinen Formel I.4, in denen
R³ = Methyl
R⁴ = C₃H₃
R⁵ = 4-CH₃-C₆H₄
bedeutet und der Substituent R⁶ für eine Verbindung einer Zeile der Tabelle A entspricht.

Tabelle 813: (Verbindungen 813.1 - 813.974)
Verbindungen der allgemeinen Formel I.1, in denen
R³ = Methyl
R⁴ = C₃H₃
R⁵ = 2,4-(CH₃)₂-C₆H₃
bedeutet und der Substituent R⁶ für eine Verbindung einer Zeile der Tabelle A entspricht.

Tabelle 814: (Verbindungen 814.1 - 814.974)
Verbindungen der allgemeinen Formel I.2, in denen
R³ = Methyl
R⁴ = C₃H₃
R⁵ = 2,4-(CH₃)₂-C₆H₃
bedeutet und der Substituent R⁶ für eine Verbindung einer Zeile der Tabelle A entspricht.

Tabelle 815: (Verbindungen 815.1 - 815.974)
Verbindungen der allgemeinen Formel I.3, in denen
R³ = Methyl
R⁴ = C₃H₃
R⁵ = 2,4-(CH₃)₂-C₆H₃
bedeutet und der Substituent R⁶ für eine Verbindung einer Zeile der Tabelle A entspricht.

Tabelle 816: (Verbindungen 816.1 - 816.974)
Verbindungen der allgemeinen Formel I.4, in denen
R³ = Methyl
R⁴ = C₃H₃
R⁵ = 2,4-(CH₃)₂-C₆H₃
bedeutet und der Substituent R⁶ für eine Verbindung einer Zeile der Tabelle A entspricht.

Tabelle 817: (Verbindungen 817.1 - 817.974)
Verbindungen der allgemeinen Formel I.1, in denen
R³ = Methyl
R⁴ = C₃H₃
R⁵ = 2,5-(CH₃)₂-C₆H₃
bedeutet und der Substituent R⁶ für eine Verbindung einer Zeile der Tabelle A entspricht.

Tabelle 818: (Verbindungen 818.1 - 818.974)
Verbindungen der allgemeinen Formel I.2, in denen
R³ = Methyl
R⁴ = C₃H₃
R⁵ = 2,5-(CH₃)₂-C₆H₃
bedeutet und der Substituent R⁶ für eine Verbindung einer Zeile der Tabelle A entspricht.

Tabelle 819: (Verbindungen 819.1 - 819.974)
Verbindungen der allgemeinen Formel I.3, in denen
R³ = Methyl
R⁴ = C₃H₃
R⁵ = 2,5-(CH₃)₂-C₆H₃
bedeutet und der Substituent R⁶ für eine Verbindung einer Zeile der Tabelle A entspricht.

Tabelle 820: (Verbindungen 820.1 - 820.974)
Verbindungen der allgemeinen Formel I.4, in denen
R³ = Methyl
R⁴ = C₃H₃
R⁵ = 2,5-(CH₃)₂-C₆H₃
bedeutet und der Substituent R⁶ für eine Verbindung einer Zeile der Tabelle A entspricht.

Tabelle 821: (Verbindungen 821.1 - 821.974)
Verbindungen der allgemeinen Formel I.1, in denen
R³ = Methyl
R⁴ = C₃H₃
R⁵ = 3,4-(CH₃)₂-C₆H₃
bedeutet und der Substituent R⁶ für eine Verbindung einer Zeile der Tabelle A entspricht.

Tabelle 822: (Verbindungen 822.1 - 822.974)
Verbindungen der allgemeinen Formel I.2, in denen
R³ = Methyl
R⁴ = C₃H₃
R⁵ = 3,4-(CH₃)₂-C₆H₃
bedeutet und der Substituent R⁶ für eine Verbindung einer Zeile der Tabelle A entspricht.

Tabelle 823: (Verbindungen 823.1 - 823.974)
Verbindungen der allgemeinen Formel I.3, in denen
R³ = Methyl
R⁴ = C₃H₃
R⁵ = 3,4-(CH₃)₂-C₆H₃
bedeutet und der Substituent R⁶ für eine Verbindung einer Zeile der Tabelle A entspricht.

Tabelle 824: (Verbindungen 824.1 - 824.974)
Verbindungen der allgemeinen Formel I.4, in denen
R³ = Methyl
R⁴ = C₃H₃
R⁵ = 3,4-(CH₃)₂-C₆H₃
bedeutet und der Substituent R⁶ für eine Verbindung einer Zeile der Tabelle A entspricht.

Tabelle 825: (Verbindungen 825.1 - 825.974)
Verbindungen der allgemeinen Formel I.1, in denen
R³ = Methyl
R⁴ = C₃H₃
R⁵ = 3,5-(CH₃)₂-C₆H₃
bedeutet und der Substituent R⁶ für eine Verbindung einer Zeile der Tabelle A entspricht.

Tabelle 826: (Verbindungen 826.1 - 826.974)
Verbindungen der allgemeinen Formel I.2, in denen
R³ = Methyl
R⁴ = C₃H₃
R⁵ = 3,5-(CH₃)₂-C₆H₃
bedeutet und der Substituent R⁶ für eine Verbindung einer Zeile der Tabelle A entspricht.

Tabelle 827: (Verbindungen 827.1 - 827.974)
Verbindungen der allgemeinen Formel I.3, in denen
R³ = Methyl
R⁴ = C₃H₃
R⁵ = 3,5-(CH₃)₂-C₆H₃
bedeutet und der Substituent R⁶ für eine Verbindung einer Zeile der Tabelle A entspricht.

Tabelle 828: (Verbindungen 828.1 - 828.974)
Verbindungen der allgemeinen Formel I.4, in denen
R³ = Methyl
R⁴ = C₃H₃
R⁵ = 3,5-(CH₃)₂-C₆H₃
bedeutet und der Substituent R⁶ für eine Verbindung einer Zeile der Tabelle A entspricht.

Tabelle 829: (Verbindungen 829.1 - 829.974)
Verbindungen der allgemeinen Formel I.1, in denen
R³ = Methyl
R⁴ = C₃H₃
R⁵ = 3,4,5-(CH₃)₃-C₆H₂
bedeutet und der Substituent R⁶ für eine Verbindung einer Zeile der Tabelle A entspricht.

Tabelle 830: (Verbindungen 830.1 - 830.974)
Verbindungen der allgemeinen Formel I.2, in denen
R³ = Methyl
R⁴ = C₃H₃
R⁵ = 3,4,5-(CH₃)₃-C₆H₂
bedeutet und der Substituent R⁶ für eine Verbindung einer Zeile der Tabelle A entspricht.

Tabelle 831: (Verbindungen 831.1 - 831.974)
Verbindungen der allgemeinen Formel I.3, in denen
R³ = Methyl
R⁴ = C₃H₃
R⁵ = 3,4,5-(CH₃)₃-C₆H₂
bedeutet und der Substituent R⁶ für eine Verbindung einer Zeile der Tabelle A entspricht.

Tabelle 832: (Verbindungen 832.1 - 832.974)
Verbindungen der allgemeinen Formel I.4, in denen
R³ = Methyl
R⁴ = C₃H₃
R⁵ = 3,4,5-(CH₃)₃-C₆H₂
bedeutet und der Substituent R⁶ für eine Verbindung einer Zeile der Tabelle A entspricht.

Tabelle 833: (Verbindungen 833.1 - 833.974)
Verbindungen der allgemeinen Formel I.1, in denen
R³ = Methyl
R⁴ = C₃H₃
R⁵ = 4-C₆H₅-C₆H₄
bedeutet und der Substituent R⁶ für eine Verbindung einer Zeile der Tabelle A entspricht.

Tabelle 834: (Verbindungen 834.1 - 834.974)
Verbindungen der allgemeinen Formel I.2, in denen
R³ = Methyl
R⁴ = C₃H₃
R⁵ = 4-C₆H₅-C₆H₄
bedeutet und der Substituent R⁶ für eine Verbindung einer Zeile der Tabelle A entspricht.

Tabelle 835: (Verbindungen 835.1 - 835.974)
Verbindungen der allgemeinen Formel I.3, in denen
R³ = Methyl
R⁴ = C₃H₃
R⁵ = 4-C₆H₅-C₆H₄
bedeutet und der Substituent R⁶ für eine Verbindung einer Zeile der Tabelle A entspricht.

Tabelle 836: (Verbindungen 836.1 - 836.974)
Verbindungen der allgemeinen Formel I.4, in denen
R³ = Methyl
R⁴ = C₃H₃
R⁵ = 4-C₆H₅-C₆H₄
bedeutet und der Substituent R⁶ für eine Verbindung einer Zeile der Tabelle A entspricht.

Tabelle 837: (Verbindungen 837.1 - 837.974)
Verbindungen der allgemeinen Formel I.1, in denen
R³ = Methyl
R⁴ = C₃H₃
R⁵ = 3-Cl-4-CH₃-C₆H₃
bedeutet und der Substituent R⁶ für eine Verbindung einer Zeile der Tabelle A entspricht.

Tabelle 838: (Verbindungen 838.1 - 838.974)
Verbindungen der allgemeinen Formel I.2, in denen
R³ = Methyl
R⁴ = C₃H₃
R⁵ = 3-Cl-4-CH₃-C₆H₃
bedeutet und der Substituent R⁶ für eine Verbindung einer Zeile der Tabelle A entspricht.

Tabelle 839: (Verbindungen 839.1 - 839.974)
Verbindungen der allgemeinen Formel I.3, in denen
R³ = Methyl
R⁴ = C₃H₃
R⁵ = 3-Cl-4-CH₃-C₆H₃
bedeutet und der Substituent R⁶ für eine Verbindung einer Zeile der Tabelle A entspricht.

Tabelle 840: (Verbindungen 840.1 - 840.974)
Verbindungen der allgemeinen Formel I.4, in denen
R³ = Methyl
R⁴ = C₃H₃
R⁵ = 3-Cl-4-CH₃-C₆H₃
bedeutet und der Substituent R⁶ für eine Verbindung einer Zeile der Tabelle A entspricht.

Tabelle 841: (Verbindungen 841.1 - 841.974)
Verbindungen der allgemeinen Formel I.1, in denen
R³ = Methyl
R⁴ = C₃H₃
R⁵ = 2-OCH₃-C₆H₄
bedeutet und der Substituent R⁶ für eine Verbindung einer Zeile der Tabelle A entspricht.

Tabelle 842: (Verbindungen 842.1 - 842.974)
Verbindungen der allgemeinen Formel I.2, in denen
R³ = Methyl
R⁴ = C₃H₃
R⁵ = 2-OCH₃-C₆H₄
bedeutet und der Substituent R⁶ für eine Verbindung einer Zeile der Tabelle A entspricht.

Tabelle 843: (Verbindungen 843.1 - 843.974)
Verbindungen der allgemeinen Formel I.3, in denen
R³ = Methyl
R⁴ = C₃H₃
R⁵ = 2-OCH₃-C₆H₄
bedeutet und der Substituent R⁶ für eine Verbindung einer Zeile der Tabelle A entspricht.

Tabelle 844: (Verbindungen 844.1 - 844.974)
Verbindungen der allgemeinen Formel I.4, in denen
R³ = Methyl
R⁴ = C₃H₃
R⁵ = 2-OCH₃-C₆H₄
bedeutet und der Substituent R⁶ für eine Verbindung einer Zeile der Tabelle A entspricht.

Tabelle 845: (Verbindungen 845.1 - 845.974)
Verbindungen der allgemeinen Formel I.1, in denen
R³ = Methyl
R⁴ = C₃H₃
R⁵ = 3-OCH₃-C₆H₄
bedeutet und der Substituent R⁶ für eine Verbindung einer Zeile der Tabelle A entspricht.

Tabelle 846: (Verbindungen 846.1 - 846.974)
Verbindungen der allgemeinen Formel I.2, in denen
R³ = Methyl
R⁴ = C₃H₃
R⁵ = 3-OCH₃-C₆H₄
bedeutet und der Substituent R⁶ für eine Verbindung einer Zeile der Tabelle A entspricht.

Tabelle 847: (Verbindungen 847.1 - 847.974)
Verbindungen der allgemeinen Formel I.3, in denen
R³ = Methyl
R⁴ = C₃H₃
R⁵ = 3-OCH₃-C₆H₄
bedeutet und der Substituent R⁶ für eine Verbindung einer Zeile der Tabelle A entspricht.

Tabelle 848: (Verbindungen 848.1 - 848.974)
Verbindungen der allgemeinen Forme528 I.4, in denen
R³ = Methyl
R⁴ = C₃H₃
R⁵ = 3-OCH₃-C₆H₄
bedeutet und der Substituent R⁶ für eine Verbindung einer Zeile der Tabelle A entspricht.

Tabelle 849: (Verbindungen 849.1 - 849.974)
Verbindungen der allgemeinen Formel I.1, in denen
R³ = Methyl
R⁴ = C₃H₃
R⁵ = 4-OCH₃-C₆H₄
bedeutet und der Substituent R⁶ für eine Verbindung einer Zeile der Tabelle A entspricht.

Tabelle 850: (Verbindungen 850.1 - 850.974)
Verbindungen der allgemeinen Formel I.2, in denen
R³ = Methyl
R⁴ = C₃H₃
R⁵ = 4-OCH₃-C₆H₄
bedeutet und der Substituent R⁶ für eine Verbindung einer Zeile der Tabelle A entspricht.

Tabelle 851: (Verbindungen 851.1 - 851.974)
Verbindungen der allgemeinen Formel I.3, in denen
R³ = Methyl
R⁴ = C₃H₃
R⁵ = 4-OCH₃-C₆H₄
bedeutet und der Substituent R⁶ für eine Verbindung einer Zeile der Tabelle A entspricht.

Tabelle 852: (Verbindungen 852.1 - 852.974)
Verbindungen der allgemeinen Formel I.4, in denen
R³ = Methyl
R⁴ = C₃H₃
R⁵ = 4-OCH₃-C₆H₄
bedeutet und der Substituent R⁶ für eine Verbindung einer Zeile der Tabelle A entspricht.

Tabelle 853: (Verbindungen 853.1 - 853.974)
Verbindungen der allgemeinen Formel I.1, in denen
R³ = Methyl
R⁴ = C₃H₃
R⁵ = 2-OCF₃-C₆H₄
bedeutet und der Substituent R⁶ für eine Verbindung einer Zeile der Tabelle A entspricht.

Tabelle 854: (Verbindungen 854.1 - 854.974)
Verbindungen der allgemeinen Formel I.2, in denen
R³ = Methyl
R⁴ = C₃H₃
R⁵ = 2-OCF₃-C₆H₄
bedeutet und der Substituent R⁶ für eine Verbindung einer Zeile der Tabelle A entspricht.

Tabelle 855: (Verbindungen 855.1 - 855.974)
Verbindungen der allgemeinen Formel I.3, in denen
R³ = Methyl
R⁴ = C₃H₃
R⁵ = 2-OCF₃-C₆H₄
bedeutet und der Substituent R⁶ für eine Verbindung einer Zeile der Tabelle A entspricht.

Tabelle 856: (Verbindungen 856.1 - 856.974)
Verbindungen der allgemeinen Formel I.4, in denen
R³ = Methyl
R⁴ = C₃H₃
R⁵ = 2-OCF₃-C₆H₄
bedeutet und der Substituent R⁶ für eine Verbindung einer Zeile der Tabelle A entspricht.

Tabelle 857: (Verbindungen 857.1 - 857.974)
Verbindungen der allgemeinen Formel I.1, in denen
R³ = Methyl
R⁴ = C₃H₃
R⁵ = 3-OCF₃-C₆H₄
bedeutet und der Substituent R⁶ für eine Verbindung einer Zeile der Tabelle A entspricht.

Tabelle 858: (Verbindungen 858.1 - 858.974)
Verbindungen der allgemeinen Formel I.2, in denen
R³ = Methyl
R⁴ = C₃H₃
R⁵ = 3-OCF₃-C₆H₄
bedeutet und der Substituent R⁶ für eine Verbindung einer Zeile der Tabelle A entspricht.

Tabelle 859: (Verbindungen 859.1 - 859.974)
Verbindungen der allgemeinen Formel I.3, in denen
R³ = Methyl
R⁴ = C₃H₃
R⁵ = 3-OCF₃-C₆H₄
bedeutet und der Substituent R⁶ für eine Verbindung einer Zeile der Tabelle A entspricht.

Tabelle 860: (Verbindungen 860.1 - 860.974)
Verbindungen der allgemeinen Formel I.4, in denen
R³ = Methyl
R⁴ = C₃H₃
R⁵ = 3-OCF₃-C₆H₄
bedeutet und der Substituent R⁶ für eine Verbindung einer Zeile der Tabelle A entspricht.

Tabelle 861: (Verbindungen 861.1 - 861.974)
Verbindungen der allgemeinen Formel I.1, in denen
R³ = Methyl
R⁴ = C₃H₃
R⁵ = 4-OCF₃-C₆H₄
bedeutet und der Substituent R⁶ für eine Verbindung einer Zeile der Tabelle A entspricht.

Tabelle 862: (Verbindungen 862.1 - 862.974)
Verbindungen der allgemeinen Formel I.2, in denen
R³ = Methyl
R⁴ = C₃H₃
R⁵ = 4-OCF₃-C₆H₄
bedeutet und der Substituent R⁶ für eine Verbindung einer Zeile der Tabelle A entspricht.

Tabelle 863: (Verbindungen 863.1 - 863.974)
Verbindungen der allgemeinen Formel I.3, in denen
R³ = Methyl
R⁴ = C₃H₃
R⁵ = 4-OCF₃-C₆H₄
bedeutet und der Substituent R⁶ für eine Verbindung einer Zeile der Tabelle A entspricht.

Tabelle 864: (Verbindungen 864.1 - 864.974)
Verbindungen der allgemeinen Formel I.4, in denen
R³ = Methyl
R⁴ = C₃H₃
R⁵ = 4-OCF₃-C₆H₄
bedeutet und der Substituent R⁶ für eine Verbindung einer Zeile der Tabelle A entspricht.

Tabelle 865: (Verbindungen 865.1 - 865.974)
Verbindungen der allgemeinen Formel I.1, in denen
R³ = Methyl
R⁴ = C₃H₃
R⁵ = 3-Cl; 4-OCH₃-C₆H₃
bedeutet und der Substituent R⁶ für eine Verbindung einer Zeile der Tabelle A entspricht.

Tabelle 866: (Verbindungen 866.1 - 866.974)
Verbindungen der allgemeinen Formel I.2, in denen
R³ = Methyl
R⁴ = C₃H₃
R⁵ = 3-Cl; 4-OCH₃-C₆H₃
bedeutet und der Substituent R⁶ für eine Verbindung einer Zeile der Tabelle A entspricht.

Tabelle 867: (Verbindungen 867.1 - 867.974)
Verbindungen der allgemeinen Formel I.3, in denen
R³ = Methyl
R⁴ = C₃H₃
R⁵ = 3-Cl; 4-OCH₃-C₆H₃
bedeutet und der Substituent R⁶ für eine Verbindung einer Zeile der Tabelle A entspricht.

Tabelle 868: (Verbindungen 868.1 - 868.974)
Verbindungen der allgemeinen Formel I.4, in denen
R³ = Methyl
R⁴ = C₃H₃
R⁵ = 3-Cl; 4-OCH₃-C₆H₃
bedeutet und der Substituent R⁶ für eine Verbindung einer Zeile der Tabelle A entspricht.

Tabelle 869: (Verbindungen 869.1 - 869.974)
Verbindungen der allgemeinen Formel I.1, in denen
R³ = Methyl
R⁴ = C₃H₃ 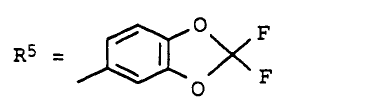 bedeutet und der Substituent R⁶ für eine Verbindung einer Zeile der Tabelle A entspricht.

Tabelle 870: (Verbindungen 870.1 - 870.974)
Verbindungen der allgemeinen Formel I.2, in denen
R³ = Methyl
R⁴ = C₃H₃ 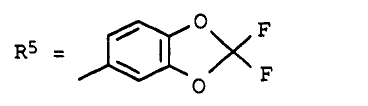 bedeutet und der Substituent R⁶ für eine Verbindung einer Zeile der Tabelle A entspricht.

Tabelle 871: (Verbindungen 871.1 - 871.974)
Verbindungen der allgemeinen Formel I.3, in denen
R³ = Methyl
R⁴ = C₃H₃ 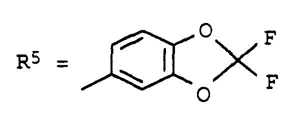 bedeutet und der Substituent R⁶ für eine Verbindung einer Zeile der Tabelle A entspricht.

Tabelle 872: (Verbindungen 872.1 - 872.974)
Verbindungen der allgemeinen Formel I.4, in denen
R³ = Methyl
R⁴ = C₃H₃ 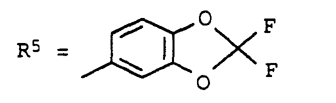 bedeutet und der Substituent R⁶ für eine Verbindung einer Zeile der Tabelle A entspricht.

Tabelle 873: (Verbindungen 873.1 - 873.974)
Verbindungen der allgemeinen Formel I.1, in denen
R³ = Methyl
R⁴ = C₃H₃
R⁵ = Pyridin-2-yl
bedeutet und der Substituent R⁶ für eine Verbindung einer Zeile der Tabelle A entspricht.

Tabelle 874: (Verbindungen 874.1 - 874.974)
Verbindungen der allgemeinen Formel I.2, in denen
R³ = Methyl
R⁴ = C₃H₃
R⁵ = Pyridin-2-yl
bedeutet und der Substituent R⁶ für eine Verbindung einer Zeile der Tabelle A entspricht.

Tabelle 875: (Verbindungen 875.1 - 875.974)
Verbindungen der allgemeinen Formel I.3, in denen
R³ = Methyl
R⁴ = C₃H₃
R⁵ = Pyridin-2-yl
bedeutet und der Substituent R⁶ für eine Verbindung einer Zeile der Tabelle A entspricht.

Tabelle 876: (Verbindungen 876.1 - 876.974)
Verbindungen der allgemeinen Formel I.4, in denen
R³ = Methyl
R⁴ = C₃H₃
R⁵ = Pyridin-2-yl
bedeutet und der Substituent R⁶ für eine Verbindung einer Zeile der Tabelle A entspricht.

Tabelle 877: (Verbindungen 877.1 - 877.974)
Verbindungen der allgemeinen Formel I.1, in denen
R³ = Methyl
R⁴ = C₃H₃
R⁵ = Pyridin-3-yl
bedeutet und der Substituent R⁶ für eine Verbindung einer Zeile der Tabelle A entspricht.

Tabelle 878: (Verbindungen 878.1 - 878.974)
Verbindungen der allgemeinen Formel I.2, in denen
R³ = Methyl
R⁴ = C₃H₃
R⁵ = Pyridin-3-yl
bedeutet und der Substituent R⁶ für eine Verbindung einer Zeile der Tabelle A entspricht.

Tabelle 879: (Verbindungen 879.1 - 879.974)
Verbindungen der allgemeinen Formel I.3, in denen
R³ = Methyl
R⁴ = C₃H₃
R⁵ = Pyridin-3-yl
bedeutet und der Substituent R⁶ für eine Verbindung einer Zeile der Tabelle A entspricht.

Tabelle 880: (Verbindungen 880.1 - 880.974)
Verbindungen der allgemeinen Formel I.4, in denen
R³ = Methyl
R⁴ = C₃H₃
R⁵ = Pyridin-3-yl
bedeutet und der Substituent R⁶ für eine Verbindung einer Zeile der Tabelle A entspricht.

Tabelle 881: (Verbindungen 881.1 - 881.974)
Verbindungen der allgemeinen Formel I.1, in denen
R³ = Methyl
R⁴ = C₃H₃
R⁵ = Pyridin-4-yl
bedeutet und der Substituent R⁶ für eine Verbindung einer Zeile der Tabelle A entspricht.

Tabelle 882: (Verbindungen 882.1 - 882.974)
Verbindungen der allgemeinen Formel I.2, in denen
R³ = Methyl
R⁴ = C₃H₃
R⁵ = Pyridin-4-yl
bedeutet und der Substituent R⁶ für eine Verbindung einer Zeile der Tabelle A entspricht.

Tabelle 883: (Verbindungen 883.1 - 883.974)
Verbindungen der allgemeinen Formel I.3, in denen
R³ = Methyl
R⁴ = C₃H₃
R⁵ = Pyridin-4-yl
bedeutet und der Substituent R⁶ für eine Verbindung einer Zeile der Tabelle A entspricht.

Tabelle 884: (Verbindungen 884.1 - 884.974)
Verbindungen der allgemeinen Formel I.4, in denen
R³ = Methyl
R⁴ = C₃H₃
R⁵ = Pyridin-4-yl
bedeutet und der Substituent R⁶ für eine Verbindung einer Zeile der Tabelle A entspricht.

Tabelle 885: (Verbindungen 885.1 - 885.974)
Verbindungen der allgemeinen Formel I.1, in denen
R³ = Methyl
R⁴ = C₃H₃
R⁵ = 3-CH₃-Pyridin-2-yl
bedeutet und der Substituent R⁶ für eine Verbindung einer Zeile der Tabelle A entspricht.

Tabelle 886: (Verbindungen 886.1 - 886.974)
Verbindungen der allgemeinen Formel I.2, in denen
R³ = Methyl
R⁴ = C₃H₃
R⁵ = 3-CH₃-Pyridin-2-yl
bedeutet und der Substituent R⁶ für eine Verbindung einer Zeile der Tabelle A entspricht.

Tabelle 887: (Verbindungen 887.1 - 887.974)
Verbindungen der allgemeinen Formel I.3, in denen
R³ = Methyl
R⁴ = C₃H₃
R⁵ = 3-CH₃-Pyridin-2-yl
bedeutet und der Substituent R⁶ für eine Verbindung einer Zeile der Tabelle A entspricht.

Tabelle 888: (Verbindungen 888.1 - 888.974)
Verbindungen der allgemeinen Formel I.4, in denen
R³ = Methyl
R⁴ = C₃H₃
R⁵ = 3-CH₃-Pyridin-2-yl
bedeutet und der Substituent R⁶ für eine Verbindung einer Zeile der Tabelle A entspricht.

Tabelle 889: (Verbindungen 889.1 - 889.974)
Verbindungen der allgemeinen Formel I.1, in denen
R³ = Methyl
R⁴ = C₃H₃
R⁵ = 4-CH₃-Pyridin-2-yl
bedeutet und der Substituent R⁶ für eine Verbindung einer Zeile der Tabelle A entspricht.

Tabelle 890: (Verbindungen 890.1 - 890.974)
Verbindungen der allgemeinen Formel I.2, in denen
R³ = Methyl
R⁴ = C₃H₃
R⁵ = 4-CH₃-Pyridin-2-yl
bedeutet und der Substituent R⁶ für eine Verbindung einer Zeile der Tabelle A entspricht.

Tabelle 891: (Verbindungen 891.1 - 891.974)
Verbindungen der allgemeinen Formel I.3, in denen
R³ = Methyl
R⁴ = C₃H₃
R⁵ = 4-CH₃-Pyridin-2-yl
bedeutet und der Substituent R⁶ für eine Verbindung einer Zeile der Tabelle A entspricht.

Tabelle 892: (Verbindungen 892.1 - 892.974)
Verbindungen der allgemeinen Formel I.4, in denen
R³ = Methyl
R⁴ = C₃H₃
R⁵ = 4-CH₃-Pyridin-2-yl
bedeutet und der Substituent R⁶ für eine Verbindung einer Zeile der Tabelle A entspricht.

Tabelle 893: (Verbindungen 893.1 - 893.974)
Verbindungen der allgemeinen Formel I.1, in denen
R³ = Methyl
R⁴ = C₃H₃
R⁵ = 3-OCH₃-Pyridin-2-yl
bedeutet und der Substituent R⁶ für eine Verbindung einer Zeile der Tabelle A entspricht.

Tabelle 894: (Verbindungen 894.1 - 894.974)
Verbindungen der allgemeinen Formel I.2, in denen
R³ = Methyl
R⁴ = C₃H₃
R⁵ = 3-OCH₃-Pyridin-2-yl
bedeutet und der Substituent R⁶ für eine Verbindung einer Zeile der Tabelle A entspricht.

Tabelle 895: (Verbindungen 895.1 - 895.974)
Verbindungen der allgemeinen Formel I.3, in denen
R³ = Methyl
R⁴ = C₃H₃
R⁵ = 3-OCH₃-Pyridin-2-yl
bedeutet und der Substituent R⁶ für eine Verbindung einer Zeile der Tabelle A entspricht.

Tabelle 896: (Verbindungen 896.1 - 896.974)
Verbindungen der allgemeinen Formel I.4, in denen
R³ = Methyl
R⁴ = C₃H₃
R⁵ = 3-OCH₃-Pyridin-2-yl
bedeutet und der Substituent R⁶ für eine Verbindung einer Zeile der Tabelle A entspricht.

Tabelle 897: (Verbindungen 897.1 - 897.974)
Verbindungen der allgemeinen Formel I.1, in denen
R³ = Methyl
R⁴ = C₃H₃
R⁵ = 4-Cl-Pyridin-2-yl
bedeutet und der Substituent R⁶ für eine Verbindung einer Zeile der Tabelle A entspricht.

Tabelle 898: (Verbindungen 898.1 - 898.974)
Verbindungen der allgemeinen Formel I.2, in denen
R³ = Methyl
R⁴ = C₃H₃
R⁵ = 4-Cl-Pyridin-2-yl
bedeutet und der Substituent R⁶ für eine Verbindung einer Zeile der Tabelle A entspricht.

Tabelle 899: (Verbindungen 899.1 - 899.974)
Verbindungen der allgemeinen Formel I.3, in denen
R³ = Methyl
R⁴ = C₃H₃
R⁵ = 4-Cl-Pyridin-2-yl
bedeutet und der Substituent R⁶ für eine Verbindung einer Zeile der Tabelle A entspricht.

Tabelle 900: (Verbindungen 900.1 - 900.974)
Verbindungen der allgemeinen Formel I.4, in denen
R³ = Methyl
R⁴ = C₃H₃
R⁵ = 4-Cl-Pyridin-2-yl
bedeutet und der Substituent R⁶ für eine Verbindung einer Zeile der Tabelle A entspricht.

Tabelle 901: (Verbindungen 901.1 - 901.974)
Verbindungen der allgemeinen Formel I.1, in denen
R³ = Methyl
R⁴ = C₃H₃
R⁵ = 5-Cl-Pyridin-2-yl
bedeutet und der Substituent R⁶ für eine Verbindung einer Zeile der Tabelle A entspricht.

Tabelle 902: (Verbindungen 902.1 - 902.974)
Verbindungen der allgemeinen Formel I.2, in denen
R³ = Methyl
R⁴ = C₃H₃
R⁵ = 5-Cl-Pyridin-2-yl
bedeutet und der Substituent R⁶ für eine Verbindung einer Zeile der Tabelle A entspricht.

Tabelle 903: (Verbindungen 903.1 - 903.974)
Verbindungen der allgemeinen Formel I.3, in denen
R³ = Methyl
R⁴ = C₃H₃
R⁵ = 5-Cl-Pyridin-2-yl
bedeutet und der Substituent R⁶ für eine Verbindung einer Zeile der Tabelle A entspricht.

Tabelle 904: (Verbindungen 904.1 - 904.974)
Verbindungen der allgemeinen Formel I.4, in denen
R³ = Methyl
R⁴ = C₃H₃
R⁵ = 5-Cl-Pyridin-2-yl
bedeutet und der Substituent R⁶ für eine Verbindung einer Zeile der Tabelle A entspricht.

Tabelle 905: (Verbindungen 905.1 - 905.974)
Verbindungen der allgemeinen Formel I.1, in denen
R³ = Methyl
R⁴ = C₃H₃
R⁵ = Pyrimidin-2-yl
bedeutet und der Substituent R⁶ für eine Verbindung einer Zeile der Tabelle A entspricht.

Tabelle 906: (Verbindungen 906.1 - 906.974)
Verbindungen der allgemeinen Formel I.2, in denen
R³ = Methyl
R⁴ = C₃H₃
R⁵ = Pyrimidin-2-yl
bedeutet und der Substituent R⁶ für eine Verbindung einer Zeile der Tabelle A entspricht.

Tabelle 907: (Verbindungen 907.1 - 907.974)
Verbindungen der allgemeinen Formel I.3, in denen
R³ = Methyl
R⁴ = C₃H₃
R⁵ = Pyrimidin-2-yl
bedeutet und der Substituent R⁶ für eine Verbindung einer Zeile der Tabelle A entspricht.

Tabelle 908: (Verbindungen 908.1 - 908.974)
Verbindungen der allgemeinen Formel I.4, in denen
R³ = Methyl
R⁴ = C₃H₃
R⁵ = Pyrimidin-2-yl
bedeutet und der Substituent R⁶ für eine Verbindung einer Zeile der Tabelle A entspricht.

Tabelle 909: (Verbindungen 909.1 - 909.974)
Verbindungen der allgemeinen Formel I.1, in denen
R³ = Methyl
R⁴ = C₃H₃
R⁵ = 5-Cl-Pyrimidin-2-yl
bedeutet und der Substituent R⁶ für eine Verbindung einer Zeile der Tabelle A entspricht.

Tabelle 910: (Verbindungen 910.1 - 910.974)
Verbindungen der allgemeinen Formel I.2, in denen
R³ = Methyl
R⁴ = C₃H₃
R⁵ = 5-Cl-Pyrimidin-2-yl
bedeutet und der Substituent R⁶ für eine Verbindung einer Zeile der Tabelle A entspricht.

Tabelle 911: (Verbindungen 911.1 - 911.974)
Verbindungen der allgemeinen Formel I.3, in denen
R³ = Methyl
R⁴ = C₃H₃
R⁵ = 5-Cl-Pyrimidin-2-yl
bedeutet und der Substituent R⁶ für eine Verbindung einer Zeile der Tabelle A entspricht.

Tabelle 912: (Verbindungen 912.1 - 912.974)
Verbindungen der allgemeinen Formel I.4, in denen
R³ = Methyl
R⁴ = C₃H₃
R⁵ = 5-Cl-Pyrimidin-2-yl
bedeutet und der Substituent R⁶ für eine Verbindung einer Zeile der Tabelle A entspricht.

Tabelle 913: (Verbindungen 913.1 - 913.974)
Verbindungen der allgemeinen Formel I.1, in denen
R³ = Methyl
R⁴ = C₃H₃
R⁵ = 4-CH₃-Pyrimidin-2-yl
bedeutet und der Substituent R⁶ für eine Verbindung einer Zeile der Tabelle A entspricht.

Tabelle 914: (Verbindungen 914.1 - 914.974)
Verbindungen der allgemeinen Formel I.2, in denen
R³ = Methyl
R⁴ = C₃H₃
R⁵ = 4-CH3-Pyrimidin-2-yl
bedeutet und der Substituent R⁶ für eine Verbindung einer Zeile der Tabelle A entspricht.

Tabelle 915: (Verbindungen 915.1 - 915.974)
Verbindungen der allgemeinen Formel I.3, in denen
R³ = Methyl
R⁴ = C₃H₃
R⁵ = 4-CH₃-Pyrimidin-2-yl
bedeutet und der Substituent R⁶ für eine Verbindung einer Zeile der Tabelle A entspricht.

Tabelle 916: (Verbindungen 916.1 - 916.974)
Verbindungen der allgemeinen Formel I.4, in denen
R³ = Methyl
R⁴ = C₃H₃
R⁵ = 4-CH₃-Pyrimidin-2-yl
bedeutet und der Substituent R⁶ für eine Verbindung einer Zeile der Tabelle A entspricht.

Tabelle 917: (Verbindungen 917.1 - 917.974)
Verbindungen der allgemeinen Formel I.1, in denen
R³ = Methyl
R⁴ = C₃H₃
R⁵ = 4-Cl-Furan-2-yl
bedeutet und der Substituent R⁶ für eine Verbindung einer Zeile der Tabelle A entspricht.

Tabelle 918: (Verbindungen 918.1 - 918.974)
Verbindungen der allgemeinen Formel I.2, in denen
R³ = Methyl
R⁴ = C₃H₃
R⁵ = 4-Cl-Furan-2-yl
bedeutet und der Substituent R⁶ für eine Verbindung einer Zeile der Tabelle A entspricht.

Tabelle 919: (Verbindungen 919.1 - 919.974)
Verbindungen der allgemeinen Formel I.3, in denen
R³ = Methyl
R⁴ = C₃H₃
R⁵ = 4-Cl-Furan-2-yl
bedeutet und der Substituent R⁶ für eine Verbindung einer Zeile der Tabelle A entspricht.

Tabelle 920: (Verbindungen 920.1 - 920.974)
Verbindungen der allgemeinen Formel I.4, in denen
R³ = Methyl
R⁴ = C₃H₃
R⁵= 4-Cl-Furan-2-yl
bedeutet und der Substituent R⁶ für eine Verbindung einer Zeile der Tabelle A entspricht.

Tabelle 921: (Verbindungen 921.1 - 921.974)
Verbindungen der allgemeinen Formel I.1, in denen
R³ = Methyl
R⁴ = C₃H₃
R⁵ = 4-CH₃-Furan-2-yl
bedeutet und der Substituent R⁶ für eine Verbindung einer Zeile der Tabelle A entspricht.

Tabelle 922: (Verbindungen 922.1 - 922.974)
Verbindungen der allgemeinen Formel I.2, in denen
R³ = Methyl
R⁴ = C₃H₃
R⁵ = 4-CH₃-Furan-2-yl
bedeutet und der Substituent R⁶ für eine Verbindung einer Zeile der Tabelle A entspricht.

Tabelle 923: (Verbindungen 923.1 - 923.974)
Verbindungen der allgemeinen Formel I.3, in denen
R³ = Methyl
R⁴ = C₃H₃
R⁵ = 4-CH₃-Furan-2-yl
bedeutet und der Substituent R⁶ für eine Verbindung einer Zeile der Tabelle A entspricht.

Tabelle 924: (Verbindungen 924.1 - 924.974)
Verbindungen der allgemeinen Formel I.4, in denen
R³ = Methyl
R⁴ = C₃H₃
R⁵ = 4-CH₃-Furan-2-yl
bedeutet und der Substituent R⁶ für eine Verbindung einer Zeile der Tabelle A entspricht.

Tabelle 925: (Verbindungen 925.1 - 925.974)
Verbindungen der allgemeinen Formel I.1, in denen
R³ = Methyl
R⁴ = C₃H₃
R⁵ = Thien-2-yl
bedeutet und der Substituent R⁶ für eine Verbindung einer Zeile der Tabelle A entspricht.

Tabelle 926: (Verbindungen 926.1 - 926.974)
Verbindungen der allgemeinen Formel I.2, in denen
R³ = Methyl
R⁴ = C₃H₃
R⁵ = Thien-2-yl
bedeutet und der Substituent R⁶ für eine Verbindung einer Zeile der Tabelle A entspricht.

Tabelle 927: (Verbindungen 927.1 - 927.974)
Verbindungen der allgemeinen Formel I.3, in denen
R³ = Methyl
R⁴ = C₃H₃
R⁵ = Thien-2-yl
bedeutet und der Substituent R⁶ für eine Verbindung einer Zeile der Tabelle A entspricht.

Tabelle 928: (Verbindungen 928.1 - 928.974)
Verbindungen der allgemeinen Formel I.4, in denen
R³ = Methyl
R⁴ = C₃H₃
R⁵ = Thien-2-yl
bedeutet und der Substituent R⁶ für eine Verbindung einer Zeile der Tabelle A entspricht.

Tabelle 929: (Verbindungen 929.1 - 929.974)
Verbindungen der allgemeinen Formel I.1, in denen
R³ = Methyl
R⁴ = C₃H₃
R⁵ = Thien-3-yl
bedeutet und der Substituent R⁶ für eine Verbindung einer Zeile der Tabelle A entspricht.

Tabelle 930: (Verbindungen 930.1 - 930.974)
Verbindungen der allgemeinen Formel I.2, in denen
R³ = Methyl
R⁴ = C₃H₃
R⁵ = Thien-3-yl
bedeutet und der Substituent R⁶ für eine Verbindung einer Zeile der Tabelle A entspricht.

Tabelle 931: (Verbindungen 931.1 - 931.974)
Verbindungen der allgemeinen Formel I.3, in denen
R³ = Methyl
R⁴ = C₃H₃
R⁵ = Thien-3-yl
bedeutet und der Substituent R⁶ für eine Verbindung einer Zeile der Tabelle A entspricht.

Tabelle 932: (Verbindungen 932.1 - 932.974)
Verbindungen der allgemeinen Formel I.4, in denen
R³ = Methyl
R⁴ = C₃H₃
R⁵ = Thien-3-yl
bedeutet und der Substituent R⁶ für eine Verbindung einer Zeile der Tabelle A entspricht.

Tabelle 933: (Verbindungen 933.1 - 933.974)
Verbindungen der allgemeinen Formel I.1, in denen
R³ = Methyl
R⁴ = C₃H₃
R⁵ = Oxazol-2-yl
bedeutet und der Substituent R⁶ für eine Verbindung einer Zeile der Tabelle A entspricht.

Tabelle 934: (Verbindungen 934.1 - 934.974)
Verbindungen der allgemeinen Formel I.2, in denen
R³ = Methyl
R⁴ = C₃H₃
R⁵ = Oxazol-2-yl
bedeutet und der Substituent R⁶ für eine Verbindung einer Zeile der Tabelle A entspricht.

Tabelle 935: (Verbindungen 935.1 - 935.974)
Verbindungen der allgemeinen Formel I.3, in denen
R³ = Methyl
R⁴ = C₃H₃
R⁵ = Oxazol-2-yl
bedeutet und der Substituent R⁶ für eine Verbindung einer Zeile der Tabelle A entspricht.

Tabelle 936: (Verbindungen 936.1 - 936.974)
Verbindungen der allgemeinen Formel I.4, in denen
R³ = Methyl
R⁴ = C₃H₃
R⁵ = Oxazol-2-yl
bedeutet und der Substituent R⁶ für eine Verbindung einer Zeile der Tabelle A entspricht.

Tabelle 937: (Verbindungen 937.1 - 937.974)
Verbindungen der allgemeinen Formel I.1, in denen
R³ = Methyl
R⁴ = C₃H₃
R⁵ = Isoxazol-3-yl
bedeutet und der Substituent R⁶ für eine Verbindung einer Zeile der Tabelle A entspricht.

Tabelle 938: (Verbindungen 938.1 - 938.974)
Verbindungen der allgemeinen Formel I.2, in denen
R³ = Methyl
R⁴ = C₃H₃
R⁵ = Isoxazol-3-yl
bedeutet und der Substituent R⁶ für eine Verbindung einer Zeile der Tabelle A entspricht.

Tabelle 939: (Verbindungen 939.1 - 939.974)
Verbindungen der allgemeinen Formel I.3, in denen
R³ = Methyl
R⁴ = C₃H₃
R⁵ = Isoxazol-3-yl
bedeutet und der Substituent R⁶ für eine Verbindung einer Zeile der Tabelle A entspricht.

Tabelle 940: (Verbindungen 940.1 - 940.974)
Verbindungen der allgemeinen Formel I.4, in denen
R³ = Methyl
R⁴ = C₃H₃
R⁵ = Isoxazol-3-yl
bedeutet und der Substituent R⁶ für eine Verbindung einer Zeile der Tabelle A entspricht.

Tabelle 941: (Verbindungen 941.1 - 941.974)
Verbindungen der allgemeinen Formel I.1, in denen
R³ = Methyl
R⁴ = C₃H₃
R⁵ = Isoxazol-4-yl
bedeutet und der Substituent R⁶ für eine Verbindung einer Zeile der Tabelle A entspricht.

Tabelle 942: (Verbindungen 942.1 - 942.974)
Verbindungen der allgemeinen Formel I.2, in denen
R³ = Methyl
R⁴ = C₃H₃
R⁵ = Isoxazol-4-yl
bedeutet und der Substituent R⁶ für eine Verbindung einer Zeile der Tabelle A entspricht.

Tabelle 943: (Verbindungen 943.1 - 943.974)
Verbindungen der allgemeinen Formel I.3, in denen
R³ = Methyl
R⁴ = C₃H₃
R⁵ = Isoxazol-4-yl
bedeutet und der Substituent R⁶ für eine Verbindung einer Zeile der Tabelle A entspricht.

Tabelle 944: (Verbindungen 944.1 - 944.974)
Verbindungen der allgemeinen Formel I.4, in denen
R³ = Methyl
R⁴ = C₃H₃
R⁵ = Isoxazol-4-yl
bedeutet und der Substituent R⁶ für eine Verbindung einer Zeile der Tabelle A entspricht.

Tabelle 945: (Verbindungen 945.1 - 945.974)
Verbindungen der allgemeinen Formel I.1, in denen
R³ = Methyl
R⁴ = C₃H₃
R⁵ = Pyrazol-3-yl
bedeutet und der Substituent R⁶ für eine Verbindung einer Zeile der Tabelle A entspricht.

Tabelle 946: (Verbindungen 946.1 - 946.974)
Verbindungen der allgemeinen Formel I.2, in denen
R³ = Methyl
R⁴ = C₃H₃
R⁵ = Pyrazol-3-yl
bedeutet und der Substituent R⁶ für eine Verbindung einer Zeile der Tabelle A entspricht.

Tabelle 947: (Verbindungen 947.1 - 947.974)
Verbindungen der allgemeinen Formel I.3, in denen
R³ = Methyl
R⁴ = C₃H₃
R⁵ = Pyrazol-3-yl
bedeutet und der Substituent R⁶ für eine Verbindung einer Zeile der Tabelle A entspricht.

Tabelle 948: (Verbindungen 948.1 - 948.974)
Verbindungen der allgemeinen Formel I.4, in denen
R³ = Methyl
R⁴ = C₃H₃
R⁵ = Pyrazol-3-yl
bedeutet und der Substituent R⁶ für eine Verbindung einer Zeile der Tabelle A entspricht.

Tabelle 949: (Verbindungen 949.1 - 949.974)
Verbindungen der allgemeinen Formel I.1, in denen
R³ = Methyl
R⁴ = C₃H₃
R⁵ = 1,3,4-Oxadiazol-5-yl
bedeutet und der Substituent R⁶ für eine Verbindung einer Zeile der Tabelle A entspricht.

Tabelle 950: (Verbindungen 950.1 - 950.974)
Verbindungen der allgemeinen Formel I.2, in denen
R³ = Methyl
R⁴ = C₃H₃
R⁵ = 1,3,4-Oxadiazol-5-yl
bedeutet und der Substituent R⁶ für eine Verbindung einer Zeile der Tabelle A entspricht.

Tabelle 951: (Verbindungen 951.1 - 951.974)
Verbindungen der allgemeinen Formel I.3, in denen
R³ = Methyl
R⁴ = C₃H₃
R⁵ = 1,3,4-Oxadiazol-5-yl
bedeutet und der Substituent R⁶ für eine Verbindung einer Zeile der Tabelle A entspricht.

Tabelle 952: (Verbindungen 952.1 - 952.974)
Verbindungen der allgemeinen Formel I.4, in denen
R³ = Methyl
R⁴ = C₃H₃
R⁵ = 1,3,4-Oxadiazol-5-yl
bedeutet und der Substituent R⁶ für eine Verbindung einer Zeile der Tabelle A entspricht.

Tabelle 953: (Verbindungen 953.1 - 953.974)
Verbindungen der allgemeinen Formel I.1, in denen
R³ = Methyl
R⁴ = C₃H₃
R⁵ = 1,2,4-Triazol-3-yl
bedeutet und der Substituent R⁶ für eine Verbindung einer Zeile der Tabelle A entspricht.

Tabelle 954: (Verbindungen 954.1 - 954.974)
Verbindungen der allgemeinen Formel I.2, in denen
R³ = Methyl
R⁴ = C₃H₃
R⁵ = 1,2,4-Triazol-3-yl
bedeutet und der Substituent R⁶ für eine Verbindung einer Zeile der Tabelle A entspricht.

Tabelle 955: (Verbindungen 955.1 - 955.974)
Verbindungen der allgemeinen Formel I.3, in denen
R³ = Methyl
R⁴ = C₃H₃
R⁵ = 1,2,4-Triazol-3-yl
bedeutet und der Substituent R⁶ für eine Verbindung einer Zeile der Tabelle A entspricht.

Tabelle 956: (Verbindungen 956.1 - 956.974)
Verbindungen der allgemeinen Formel I.4, in denen
R³ = Methyl
R⁴ = C₃H₃
R⁵ = 1,2,4-Triazol-3-yl
bedeutet und der Substituent R⁶ für eine Verbindung einer Zeile der Tabelle A entspricht.

Tabelle 957: (Verbindungen 957.1 - 957.974)
Verbindungen der allgemeinen Formel I.1, in denen
R³ = Methyl
R⁴ = C₃H₃
R⁵ = Pyrrol-2-yl
bedeutet und der Substituent R⁶ für eine Verbindung einer Zeile der Tabelle A entspricht.

Tabelle 958: (Verbindungen 558.1 - 658.974)
Verbindungen der allgemeinen Formel I.2, in denen
R³ = Methyl
R⁴ = C₃H₃
R⁵ = Pyrrol-2-yl
bedeutet und der Substituent R⁶ für eine Verbindung einer Zeile der Tabelle A entspricht.

Tabelle 959: (Verbindungen 959.1 - 959.974)
Verbindungen der allgemeinen Formel I.3, in denen
R³ = Methyl
R⁴ = C₃H₃
R⁵ = Pyrrol-2-yl
bedeutet und der Substituent R⁶ für eine Verbindung einer Zeile der Tabelle A entspricht.

Tabelle 960: (Verbindungen 960.1 - 960.974)
Verbindungen der allgemeinen Formel I.4, in denen
R³ = Methyl
R⁴ = C₃H₃
R⁵ = Pyrrol-2-yl
bedeutet und der Substituent R⁶ für eine Verbindung einer Zeile der Tabelle A entspricht.

Tabelle 961: (Verbindungen 961.1 - 961.974)
Verbindungen der allgemeinen Formel I.1, in denen
R³ = Trifluormethyl
R⁴ = CH₃
R⁵ = CH₃
bedeutet und der Substituent R⁶ für eine Verbindung einer Zeile der Tabelle A entspricht.

Tabelle 962: (Verbindungen 962.1 - 962.974)
Verbindungen der allgemeinen Formel I.2, in denen
R³ = Trifluormethyl
R⁴ = CH₃
R⁵ = CH₃
bedeutet und der Substituent R⁶ für eine Verbindung einer Zeile der Tabelle A entspricht.

Tabelle 963: (Verbindungen 963.1 - 963.974)
Verbindungen der allgemeinen Formel I.3, in denen
R³ = Trifluormethyl
R⁴ = CH₃
R⁵ = CH₃
bedeutet und der Substituent R⁶ für eine Verbindung einer Zeile der Tabelle A entspricht.

Tabelle 964: (Verbindungen 964.1 - 964.974)
Verbindungen der allgemeinen Formel I.4, in denen
R³ = Trifluormethyl
R⁴ = CH₃
R⁵ = CH₃
bedeutet und der Substituent R⁶ für eine Verbindung einer Zeile der Tabelle A entspricht.

Tabelle 965: (Verbindungen 965.1 - 965.974)
Verbindungen der allgemeinen Formel I.1, in denen
R³ = Trifluormethyl
R⁴ = C₂H₅
R⁵ = CH₃
bedeutet und der Substituent R⁶ für eine Verbindung einer Zeile der Tabelle A entspricht.

Tabelle 966: (Verbindungen 966.1 - 966.974)
Verbindungen der allgemeinen Formel I.2, in denen
R³ = Trifluormethyl
R⁴ = C₂H₅
R⁵ = CH₃
bedeutet und der Substituent R⁶ für eine Verbindung einer Zeile der Tabelle A entspricht.

Tabelle 967: (Verbindungen 967.1 - 967.974)
Verbindungen der allgemeinen Formel I.3, in denen
R³ = Trifluormethyl
R⁴ = C₂H₅
R⁵ = CH₃
bedeutet und der Substituent R⁶ für eine Verbindung einer Zeile der Tabelle A entspricht.

Tabelle 968: (Verbindungen 968.1 - 968.974)
Verbindungen der allgemeinen Formel I.4, in denen
R³ = Trifluormethyl
R⁴ = C₂H₅
R⁵ = CH₃
bedeutet und der Substituent R⁶ für eine Verbindung einer Zeile der Tabelle A entspricht.

Tabelle 969: (Verbindungen 969.1 - 969.974)
Verbindungen der allgemeinen Formel I.1, in denen
R³ = Trifluormethyl
R⁴ = C₃H₃
R⁵ = CH₃
bedeutet und der Substituent R⁶ für eine Verbindung einer Zeile der Tabelle A entspricht.

Tabelle 970: (Verbindungen 970.1 - 970.974)
Verbindungen der allgemeinen Formel I.2, in denen
R³ = Trifluormethyl
R⁴ = C₃H₃
R⁵ = CH₃
bedeutet und der Substituent R⁶ für eine Verbindung einer Zeile der Tabelle A entspricht.

Tabelle 971: (Verbindungen 971.1 - 971.974)
Verbindungen der allgemeinen Formel I.3, in denen
R³ = Trifluormethyl
R⁴ = C₃H₃
R⁵ = CH₃
bedeutet und der Substituent R⁶ für eine Verbindung einer Zeile der Tabelle A entspricht.

Tabelle 972: (Verbindungen 972.1 - 972.974)
Verbindungen der allgemeinen Formel I.4, in denen
R³ = Trifluormethyl
R⁴ = C₃H₃
R⁵ = CH₃
bedeutet und der Substituent R⁶ für eine Verbindung einer Zeile der Tabelle A entspricht.

Tabelle 973: (Verbindungen 973.1 - 731.974)
Verbindungen der allgemeinen Formel I.1, in denen
R³ = Cyclopropyl
R⁴ = CH₃
R⁵ = CH₃
bedeutet und der Substituent R⁶ für eine Verbindung einer Zeile der Tabelle A entspricht.

Tabelle 974: (Verbindungen 974.1 - 974.974)
Verbindungen der allgemeinen Formel I.2, in denen
R³ = Cyclopropyl
R⁴ = CH₃
R⁵ = CH₃
bedeutet und der Substituent R⁶ für eine Verbindung einer Zeile der Tabelle A entspricht.

Tabelle 975: (Verbindungen 975.1 - 975.974)
Verbindungen der allgemeinen Formel I.3, in denen
R³ = Cyclopropyl
R⁴ = CH₃
R⁵ = CH³
bedeutet und der Substituent R⁶ für eine Verbindung einer Zeile der Tabelle A entspricht.

Tabelle 976: (Verbindungen 976.1 - 976.974)
Verbindungen der allgemeinen Formel I.4, in denen
R³ = Cyclopropyl
R⁴ = CH₃
R⁵ = CH₃
bedeutet und der Substituent R⁶ für eine Verbindung einer Zeile der Tabelle A entspricht.

Tabelle 977: (Verbindungen 977.1 - 977.974)
Verbindungen der allgemeinen Formel I.1, in denen
R³ = Cyclopropyl
R⁴ = C₂H₅
R⁵ = CH₃
bedeutet und der Substituent R⁶ für eine Verbindung einer Zeile der Tabelle A entspricht.

Tabelle 978: (Verbindungen 978.1 - 978.974)
Verbindungen der allgemeinen Formel I.2, in denen
R³ = Cyclopropyl
R⁴ = C₂H₅
R⁵ = CH₃
bedeutet und der Substituent R⁶ für eine Verbindung einer Zeile der Tabelle A entspricht.

Tabelle 979: (Verbindungen 979.1 - 979.974)
Verbindungen der allgemeinen Formel I.3, in denen
R³ = Cyclopropyl
R⁴ = C₂H₅
R⁵ = CH₃
bedeutet und der Substituent R⁶ für eine Verbindung einer Zeile der Tabelle A entspricht.

Tabelle 980: (Verbindungen 980.1 - 980.974)
Verbindungen der allgemeinen Formel I.4, in denen
R³ = Cyclopropyl
R⁴ = C₂H₅
R⁵ = CH₃
bedeutet und der Substituent R⁶ für eine Verbindung einer Zeile der Tabelle A entspricht.

Tabelle 981: (Verbindungen 981.1 - 981.974)
Verbindungen der allgemeinen Formel I.1, in denen
R³ = Cyclopropyl
R⁴ = C₃H₃
R⁵ = CH₃
bedeutet und der Substituent R⁶ für eine Verbindung einer Zeile der Tabelle A entspricht.

Tabelle 982: (Verbindungen 982.1 - 982.974)
Verbindungen der allgemeinen Formel I.2, in denen
R³ = Cyclopropyl
R⁴ = C₃H₃
R⁵ = CH₃
bedeutet und der Substituent R⁶ für eine Verbindung einer Zeile der Tabelle A entspricht.

Tabelle 983: (Verbindungen 983.1 - 983.974)
Verbindungen der allgemeinen Formel I.3, in denen
R³ = Cyclopropyl
R⁴ = C₃H₃
R⁵ = CH₃
bedeutet und der Substituent R⁶ für eine Verbindung einer Zeile der Tabelle A entspricht.

Tabelle 984: (Verbindungen 984.1 - 984.974)
Verbindungen der allgemeinen Formel I.4, in denen
R³ = Cyclopropyl
R⁴ = C₃H₃
R⁵ = CH₃
bedeutet und der Substituent R⁶ für eine Verbindung einer Zeile der Tabelle A entspricht.

Tabelle 985: (Verbindungen 985.1 - 985.974)
Verbindungen der allgemeinen Formel I.1, in denen
R³ = Cyano
R⁴ = CH₃
R⁵ = CH₃
bedeutet und der Substituent R⁶ für eine Verbindung einer Zeile der Tabelle A entspricht.

Tabelle 986: (Verbindungen 986.1 - 986.974)
Verbindungen der allgemeinen Formel I.2, in denen
R³ = Cyano
R⁴ = CH₃
R⁵ = CH₃
bedeutet und der Substituent R⁶ für eine Verbindung einer Zeile der Tabelle A entspricht.

Tabelle 987: (Verbindungen 987.1 - 987.974)
Verbindungen der allgemeinen Formel I.3, in denen
R³ = Cyano
R⁴ = CH₃
R⁵ = CH₃
bedeutet und der Substituent R⁶ für eine Verbindung einer Zeile der Tabelle A entspricht.

Tabelle 988: (Verbindungen 988.1 - 988.974)
Verbindungen der allgemeinen Formel I.4, in denen
R³ = Cyano
R⁴ = CH₃
R⁵ = CH₃
bedeutet und der Substituent R⁶ für eine Verbindung einer Zeile der Tabelle A entspricht.

Tabelle 989: (Verbindungen 989.1 - 989.974)
Verbindungen der allgemeinen Formel I.1, in denen
R³ = Cyano
R⁴ = C₂H₅
R⁵ = CH₃
bedeutet und der Substituent R⁶ für eine Verbindung einer Zeile der Tabelle A entspricht.

Tabelle 990: (Verbindungen 990.1 - 990.974)
Verbindungen der allgemeinen Formel I.2, in denen
R³ = Cyano
R⁴ = C₂H₅
R⁵ = CH₃
bedeutet und der Substituent R⁶ für eine Verbindung einer Zeile der Tabelle A entspricht.

Tabelle 991: (Verbindungen 991.1 - 991.974)
Verbindungen der allgemeinen Formel I.3, in denen
R³ = Cyano
R⁴ = C₂H₅
R⁵ = CH₃
bedeutet und der Substituent R⁶ für eine Verbindung einer Zeile der Tabelle A entspricht.

Tabelle 992: (Verbindungen 992.1 - 992.974)
Verbindungen der allgemeinen Formel I.4, in denen
R³ = Cyano
R⁴ = C₂H₅
R⁵ = CH₃
bedeutet und der Substituent R⁶ für eine Verbindung einer Zeile der Tabelle A entspricht.

Tabelle 993: (Verbindungen 993.1 - 993.974)
Verbindungen der allgemeinen Formel I.1, in denen
R³ = Cyano
R⁴ = C₃H₃
R⁵ = CH₃
bedeutet und der Substituent R⁶ für eine Verbindung einer Zeile der Tabelle A entspricht.

Tabelle 994: (Verbindungen 994.1 - 994.974)
Verbindungen der allgemeinen Formel I.2, in denen
R³ = Cyano
R⁴ = C₃H₃
R⁵ = CH₃
bedeutet und der Substituent R⁶ für eine Verbindung einer Zeile der Tabelle A entspricht.

Tabelle 995: (Verbindungen 995.1 - 995.974)
Verbindungen der allgemeinen Formel I.3, in denen
R³ = Cyano
R⁴ = C₃H₃
R⁵ = CH₃
bedeutet und der Substituent R⁶ für eine Verbindung einer Zeile der Tabelle A entspricht.

Tabelle 996: (Verbindungen 996.1 - 996.974)
Verbindungen der allgemeinen Formel I.4, in denen
R³ = Cyano
R⁴ = C₃H₃
R⁵ = CH₃
bedeutet und der Substituent R⁶ für eine Verbindung einer Zeile der Tabelle A entspricht.

Die Verbindungen I eignen sich als Fungizide.

Die Verbindungen I zeichnen sich durch eine hervorragende Wirksamkeit gegen ein breites Spektrum von pflanzenpathogenen Pilzen, insbesondere aus der Klasse der Ascomyceten, Phycomyceten und Basidiomyceten, aus. Sie sind zum Teil systemisch wirksam und können im Pflanzenschutz als Blatt- und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Speziell eignen sie sich zur Bekämpfung folgender Pflanzenkrankheiten: Erysiphe graminis (echter Mehltau) in Getreide, Erysiphe cichoracearum und Sphaerotheca fuliginea an Kürbisgewächsen, Podosphaera leucotricha an Äpfeln, Uncinula necator an Reben, Puccinia-Arten an Getreide, Rhizoctonia-Arten an Baumwolle, Reis und Rasen, Ustilago-Arten an Getreide und Zuckerrohr, Venturia inaequalis (Schorf) an Äpfeln, Helminthosporium-Arten an Getreide, Septoria nodorum an Weizen, Botrytis cinerea (Grauschimmel) an Erdbeeren, Gemüse und Zierpflanzen, Reben, Cercospora arachidicola an Erdnüssen, Pseudocercosporella herpotrichoides an Weizen, Gerste, Pyricularia oryzae an Reis, Phytophthora infestans an Kartoffeln und Tomaten, Fusarium- und Verticillium-Arten an verschiedenen Pflanzen, Plasmopara viticola an Reben, Alternaria-Arten an Gemüse und Obst.

Die Verbindungen I eignen sich außerdem zur Bekämpfung von Schadpilzen im Materialschutz (z.B. Holz, Papier, Fasern bzw. Gewebe) und im Vorratsschutz.

Die Verbindungen I werden angewendet, indem man die Pilze oder die vor Pilzbefall zu schützenden Pflanzen, Saatgüter, Materialien oder den Erdboden mit einer fungizid wirksamen Menge der Wirkstoffe behandelt. Die Anwendung erfolgt vor oder nach der Infektion der Materialien, Pflanzen oder Samen durch die Pilze.

Sie können in die üblichen Formulierungen überführt werden, wie Lösungen, Emulsionen, Suspensionen, Stäube, Pulver, Pasten und Granulate. Die Anwendungsform richtet sich nach dem jeweiligen Verwendungszweck; sie soll in jedem Fall eine feine und gleichmäßige Verteilung der erfindungsgemäßen Verbindung gewährleisten. Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Lignin-Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen bei der Anwendung im Pflanzenschutz je nach Art des gewünschten Effektes zwischen 0,01 und 2,0 kg Wirkstoff pro ha.

Bei der Saatgutbehandlung werden im allgemeinen Wirkstoffmengen von 0,001 bis 0,1 g, vorzugsweise 0,01 bis 0,05 g je Kilogramm Saatgut benötigt.

Bei der Anwendung im Material- bzw. Vorratsschutz richtet sich die Aufwandmenge an Wirkstoff nach der Art des Einsatzgebietes und des gewünschten Effekts. Übliche Aufwandmengen sind im Materialschutz beispielsweise 0,001 g bis 2 kg, vorzugsweise 0,005 g bis 1 kg Wirkstoff pro Qubikmeter behandelten Materials.

Die erfindungsgemäßen Mittel können in der Anwendungsform als Fungizide auch zusammen mit anderen Wirkstoffen vorliegen, der z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

Die folgende Liste von Fungiziden, mit denen die erfindungsgemäßen Verbindungen gemeinsam angewendet werden können, soll die Kombinationsmöglichkeiten erläutern, nicht aber einschränken:
Schwefel, Dithiocarbamate und deren Derivate, wie Ferridimethyldithiocarbamat, Zinkdimethyldithiocarbamat, Zinkethylenbisdithiocarbamat, Manganethylenbisdithiocarbamat, Mangan-Zink-ethylendiamin-bis-dithiocarbamat, Tetramethylthiuramdisulfide, Ammoniak-Komplex von Zink-(N,N-ethylen-bis-dithiocarbamat), Ammoniak-Komplex von Zink-(N,N'-propylen-bis-dithiocarbamat), Zink-(N,N'-propylenbis-dithiocarbamat), N,N'-Polypropylen-bis-(thiocarbamoyl)disulfid;
Nitroderivate, wie Dinitro-(1-methylheptyl)-phenylcrotonat, 2-sec-Butyl-4,6-dinitrophenyl-3,3-dimethylacrylat, 2-sec-Butyl-4,6-dinitrophenyl-isopropylcarbonat, 5-Nitro-isophthalsäure-di-isopropylester;
heterocyclische Substanzen, wie 2-Heptadecyl-2-imidazolin-acetat, 2,4-Dichlor-6-(o-chloranilino)-s-triazin, O,O-Diethyl-phthalimidophosphonothioat, 5-Amino-1-[bis-(dimethylamino)-phosphinyl]-3-phenyl-1,2,4- triazol, 2,3-Dicyano-1,4-dithioanthrachinon, 2-Thio-1,3-dithiolo[4,5-b]chinoxalin, 1-(Butylcarbamoyl)-2-benzimidazol-carbaminsäuremethylester, 2-Methoxycarbonylamino-benzimidazol, 2-(Furyl-(2))-benzimidazol, 2-(Thiazolyl-(4))-benzimidazol, N-(1,1,2,2-Tetrachlorethylthio)-tetrahydrophthalimid, N-Trichlormethylthio-tetrahydrophthalimid, N-Trichlormethylthiophthalimid,
N-Dichlorfluormethylthio-N',N'-dimethyl-N-phenyl-schwefelsäurediamid, 5-Ethoxy-3-trichlormethyl-1,2,3-thiadiazol, 2-Rhodanmethylthiobenzthiazol, 1,4-Dichlor-2,5-dimethoxybenzol, 4-(2-Chlorphenylhydrazono)-3-methyl-5-isoxazolon, Pyridin-2-thio-1-oxid, 8-Hydroxychinolin bzw. dessen Kupfersalz, 2,3-Dihydro-5-carboxanilido-6-methyl-1,4-oxathiin, 2,3-Dihydro-5-carboxanilido-6-methyl-1,4-oxathiin-4,4-dioxid, 2-Methyl-5,6-dihydro-4H-pyran-3-carbonsäure-anilid, 2-Methyl-furan-3-carbonsäureanilid, 2,5-Dimethyl-furan-3-carbonsäureanilid, 2,4,5-Trimethyl-furan-3-carbonsäureanilid, 2,5-Dimethyl-furan-3-carbonsäurecyclohexylamid, N-Cyclohexyl-N-methoxy-2,5-dimethyl-furan-3-carbonsäureamid, 2-Methyl-benzoesäure-anilid, 2-Iod-benzoesäure-anilid, N-Formyl-N-morpholin-2,2,2-trichlorethylacetal, Piperazin-1,4-diylbis-(1-(2,2,2-trichlor-ethyl)-formamid, 1-(3,4-Dichloranilino)-1-formylamino-2,2,2-trichlorethan, 2,6-Dimethyl-N-tridecyl-morpholin bzw. dessen Salze, 2,6-Dimethyl-N-cyclododecyl-morpholin bzw. dessen Salze, N-[3-(p-tert.-Butylphenyl)-2-methylpropyl]-cis-2,6-dimethylmorpholin, N-[3-(p-tert.-Butylphenyl)-2-methylpropyl]-piperidin, 1-[2-(2,4-Dichlorphenyl)-4-ethyl-1,3-dioxolan-2-yl-ethyl]-1H-1,2,4-triazol, 1-[2-(2,4-Dichlorphenyl)-4-n-propyl-1,3-dioxolan-2-yl-ethyl]-1H-1,2,4-triazol, N-(n-Propyl)-N-(2,4,6-trichlorphenoxyethyl)-N'-imidazol-yl-harnstoff, 1-(4-Chlorphenoxy)-3,3-dimethyl-1-(1H-1,2,4-triazol-1-yl)-2-butanon, 1-(4-Chlorphenoxy)-3,3-dimethyl-1-(1H-1,2,4-triazol-1-yl)-2-butanol, α-(2-Chlorphenyl)-α-(4-chlorphenyl)-5-pyrimidinmethanol, 5-Butyl-2-dimethylamino-4-hydroxy-6-methyl-pyrimidin, Bis- (p-chlorphenyl)-3-pyridinmethanol, 1,2-Bis-(3-ethoxycarbonyl-2-thioureido)-benzol, 1,2-Bis-(3-methoxycarbonyl-2-thioureido)-benzol,sowie verschiedene Fungizide, wie Dodecylguanidinacetat, 3-[3-(3,5-Dimethyl-2-oxycyclohexyl)-2-hydroxyethyl]-glutarimid, Hexachlorbenzol, DL-Methyl-N-(2,6-dimethylphenyl)-N-furoyl(2)-alaninat, DL-N-(2,6-Dimethyl-phenyl)-N-(2'-methoxyacetyl)-alanin-methyl- ester, N-(2,6-Dimethylphenyl)-N-chloracetyl-D,L-2-aminobutyrolacton, DL-N-(2,6-Dimethylphenyl)-N-(phenylacetyl)-alaninmethylester, 5-Methyl-5-vinyl-3-(3,5-dichlorphenyl)-2,4-dioxo-1,3-oxazolidin, 3-[3,5-Dichlorphenyl(-5-methyl-5-methoxymethyl]-1,3-oxazolidin-2,4-dion, 3-(3,5-Dichlorphenyl)-1-isopropylcarbamoylhydantoin, N-(3,5-Dichlorphenyl)-1,2-dimethylcyclopropan-1,2-dicarbonsäureimid, 2-Cyano-[N-(ethylaminocarbonyl)-2-methoximino]-acetamid, 1-[2-(2,4-Dichlorphenyl)-pentyl]-lH-1,2,4-triazol, 2,4-Difluor-α-(1H-1,2,4-triazolyl-1-methyl)-benzhydrylalkohol, N-(3-Chlor-2,6-dinitro-4-trifluormethyl-phenyl)-5-trifluormethyl-3-chlor-2-aminopyridin, 1-((bis-(4-Fluorphenyl)-methylsilyl)-methyl)-1H-1,2,4-triazol.

Die Verbindungen der Formel I sind außerdem geeignet, Schädlinge aus der Klasse der Insekten, Spinnentiere und Nematoden wirksam zu bekämpfen. Sie können im Pflanzenschutz sowie auf dem Hygiene-, Vorratsschutz- und Veterinärsektor als Schädlingsbekämpfungsmittel eingesetzt werden.

Zu den schädlichen Insekten gehören aus der Ordnung der Schmetterlinge (Lepidoptera) beispielsweise Agrotis ypsilon, Agrotis segetum, Alabama argillacea, Anticarsia gemmatalis, Argyresthia conjugella, Autographa gamma, Bupalus piniarius, Cacoecia murinana, Capua reticulana, Cheimatobia brumata, Choristoneura fumiferana, Choristoneura occidentalis, Cirphis unipuncta, Cydia pomonella, Dendrolimus pini, Diaphania nitidalis, Diatraea grandiosella, Earias insulana, Elasmopalpus lignosellus, Eupoecilia ambiguella, Evetria bouliana, Feltia subterranea, Galleria mellonella, Grapholitha funebrana, Grapholitha molesta, Heliothis armigera, Heliothis virescens, Heliothis zea, Hellula undalis, Hibernia defoliaria, Hyphantria cunea, Hyponomeuta malinellus, Keiferia lycopersicella, Lambdina fiscellaria, Laphygma exigua, Leucoptera coffeella, Leucoptera scitella, Lithocolletis blancardella, Lobesia botrana, Loxostege sticticalis, Lymantria dispar, Lymantria monacha, Lyonetia clerkella, Malacosoma neustria, Mamestra brassicae, Orgyia pseudotsugata, Ostrinia nubilalis, Panolis flammea, Pectinophora gossypiella, Peridroma saucia, Phalera bucephala, Phthorimaea operculella, Phyllocnistis citrella, Pieris brassicae, Plathypena scabra, Plutella xylostella, Pseudoplusia includens, Rhyacionia frustrana, Scrobipalpula absoluta, Sitotroga cerealella, Sparganothis pilleriana, Spodoptera frugiperda, Spodoptera littoralis, Spodoptera litura, Thaumatopoea pityocampa, Tortrix viridana, Trichoplusia ni, Zeiraphera canadensis.

Aus der Ordnung der Käfer (Coleoptera) beispielsweise Agrilus sinuatus, Agriotes lineatus, Agriotes obscurus, Amphimallus solstitialis, Anisandrus dispar, Anthonomus grandis, Anthonomus pomorum, Atomaria linearis, Blastophagus piniperda, Blitophaga undata, Bruchus rufimanus, Bruchus pisorum, Bruchus lentis, Byctiscus betulae, Cassida nebulosa, Cerotoma trifurcata, Ceuthorrhynchus assimilis, Ceuthorrhynchus napi, Chaetocnema tibialis, Conoderus vespertinus, Crioceris asparagi, Diabrotica longicornis, Diabrotica 12-punctata, Diabrotica virgifera, Epilachna varivestis, Epitrix hirtipennis, Eutinobothrus brasiliensis, Hylobius abietis, Hypera brunneipennis, Hypera postica, Ips typographus, Lema bilineata, Lema melanopus, Leptinotarsa decemlineata, Limonius californicus, Lissorhoptrus oryzophilus, Melanotus communis, Meligethes aeneus, Melolontha hippocastani, Melolontha melolontha, Oulema oryzae, Ortiorrhynchus sulcatus, Otiorrhynchus ovatus, Phaedon cochleariae, Phyllotreta chrysocephala, Phyllophaga sp., Phyllopertha horticola, Phyllotreta nemorum, Phyllotreta striolata, Popillia japonica, Sitona lineatus, Sitophilus granaria.

Aus der Ordnung der Zweiflügler (Diptera) beispielsweise Aedes aegypti, Aedes vexans, Anastrepha ludens, Anopheles maculipennis, Ceratitis capitata, Chrysomya bezziana, Chrysomya hominivorax, Chrysomya macellaria, Contarinia sorghicola, Cordylobia anthropophaga, Culex pipiens, Dacus cucurbitae, Dacus oleae, Dasineura brassicae, Fannia canicularis, Gasterophilus intestinalis, Glossina morsitans, Haematobia irritans, Haplodiplosis equestris, Hylemyia platura, Hypoderma lineata, Liriomyza sativae, Liriomyza trifolii, Lucilia caprina, Lucilia cuprina, Lucilia sericata, Lycoria pectoralis, Mayetiola destructor, Musca domestica, Muscina stabulans, Oestrus ovis, Oscinella frit, Pegomya hysocyami, Phorbia antiqua, Phorbia brassicae, Phorbia coarctata, Rhagoletis cerasi, Rhagoletis pomonella, Tabanus bovinus, Tipula oleracea, Tipula paludosa.

Aus der Ordnung der Thripse (Thysanoptera) beispielsweise Frankliniella fusca, Frankliniella occidentalis, Frankliniella tritici, Scirtothrips citri, Thrips oryzae, Thrips palmi, Thrips tabaci.

Aus der Ordnung der Hautflügler (Hymenoptera) beispielsweise Athalia rosae, Atta cephalotes, Atta sexdens, Atta texana, Hoplocampa minuta, Hoplocampa testudinea, Monomorium pharaonis, Solenopsis geminata, Solenopsis invicta.

Aus der Ordnung der Wanzen (Heteroptera) beispielsweise Acrosternum hilare, Blissus leucopterus, Cyrtopeltis notatus, Dysdercus cingulatus, Dysdercus intermedius, Eurygaster integriceps, Euschistus impictiventris, Leptoglossus phyllopus, Lygus lineolaris, Lygus pratensis, Nezara viridula, Piesma quadrata, Solubea insularis, Thyanta perditor.

Aus der Ordnung der Pflanzensauger (Homoptera) beispielsweise Acyrthosiphon onobrychis, Adelges laricis, Aphidula nasturtii, Aphis fabae, Aphis pomi, Aphis sambuci, Brachycaudus cardui, Brevicoryne brassicae, Cerosipha gossypii, Dreyfusia nordmannianae, Dreyfusia piceae, Dysaphis radicola, Dysaulacorthum pseudosolani, Empoasca fabae, Macrosiphum avenae, Macrosiphum euphorbiae, Macrosiphon rosae, Megoura viciae, Metopolophium dirhodum, Myzodes persicae, Myzus cerasi, Nilaparvata lugens, Pemphigus bursarius, Perkinsiella saccharicida, Phorodon humuli, Psylla mali, Psylla piri, Rhopalomyzus ascalonicus, Rhopalosiphum maidis, Sappaphis mala, Sappaphis mali, Schizaphis graminum, Schizoneura lanuginosa, Trialeurodes vaporariorum, Viteus vitifolii.

Aus der Ordnung der Termiten (Isoptera) beispielsweise Calotermes flavicollis, Leucotermes flavipes, Reticulitermes lucifugus, Termes natalensis.

Aus der Ordnung der Geradflügler (Orthoptera) beispielsweise Acheta domestica, Blatta orientalis, Blattella germanica, Forficula auricularia, Gryllotalpa gryllotalpa, Locusta migratoria, Melanoplus bivittatus, Melanoplus femur-rubrum, Melanoplus mexicanus, Melanoplus sanguinipes, Melanoplus spretus, Nomadacris septemfasciata, Periplaneta americana, Schistocerca americana, Schistocerca peregrina, Stauronotus maroccanus, Tachycines asynamorus.

Aus der Klasse der Arachnoidea beispielsweise Spinnentiere (Acarina) wie Amblyomma americanum, Amblyomma variegatum, Argas persicus, Boophilus annulatus, Boophilus decoloratus, Boophilus microplus, Brevipalpus phoenicis, Bryobia praetiosa, Dermacentor silvarum, Eotetranychus carpini, Eriophyes sheldoni, Hyalomma truncatum, Ixodes ricinus, Ixodes rubicundus, Ornithodorus moubata, Otobius megnini, Paratetranychus pilosus, Dermanyssus gallinae, Phyllocoptruta oleivora, Polyphagotarsonemus latus, Psoroptes ovis, Rhipicephalus appendiculatus, Rhipicephalus evertsi, Sarcoptes scabiei, Tetranychus cinnabarinus, Tetranychus kanzawai, Tetranychus pacificus, Tetranychus telarius, Tetranychus urticae.

Aus der Klasse der Nematoden beispielsweise Wurzelgallennematoden, z.B. Meloidogyne hapla, Meloidogyne incognita, Meloidogyne javanica, Zysten bildende Nematoden, z.B. Globodera rostochiensis, Heterodera avenae, Heterodera glycines, Heterodera schachtii, Heterodera trifolii, Stock- und Blattälchen, z.B. Belonolaimus longicaudatus, Ditylenchus destructor, Ditylenchus dipsaci, Heliocotylenchus multicinctus, Longidorus elongatus, Radopholus similis, Rotylenchus robustus, Trichodorus primitivus, Tylenchorhynchus claytoni, Tylenchorhynchus dubius, Pratylenchus neglectus, Pratylenchus penetrans, Pratylenchus curvitatus, Pratylenchus goodeyi.

Die Wirkstoffe können als solche, in Form ihrer Formulierungen oder den daraus bereiteten Anwendungsformen, z.B. in Form von direkt versprühbaren Lösungen, Pulvern, Suspensionen oder Dispersionen, Emulsionen, Öldispersionen, Pasten, Stäubemitteln, Streumitteln, Granulaten durch Versprühen, Vernebeln, Verstäuben, Verstreuen oder Gießen angewendet werden. Die Anwendungsformen richten sich ganz nach den Verwendungszwecken; sie sollten in jedem Fall möglichst die feinste Verteilung der erfindungsgemäßen Wirkstoffe gewährleisten.

Die Wirkstoffkonzentrationen in den anwendungsfertigen Zubereitungen können in größeren Bereichen variiert werden.

Im allgemeinen liegen sie zwischen 0,0001 und 10 %, vorzugsweise zwischen 0,01 und 1 %.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Die Aufwandmenge an Wirkstoff zur Bekämpfung von Schädlingen beträgt unter Freilandbedingungen 0,1 bis 2,0, vorzugsweise 0,2 bis 1,0 kg/ha.

Zur Herstellung von direkt versprühbaren Lösungen, Emulsionen, Pasten oder Öldispersionen kommen Mineralölfraktionen von mittlerem bis hohem Siedepunkt, wie Kerosin oder Dieselöl, ferner Kohlenteeröle sowie Öle pflanzlichen oder tierischen Ursprungs, aliphatische, cyclische und aromatische Kohlenwasserstoffe, z.B.Benzol, Toluol, Xylol, Paraffin, Tetrahydronaphthalin, alkylierte Naphthaline oder deren Derivate, Methanol, Ethanol, Propanol, Butanol, Chloroform, Tetrachlorkohlenstoff, Cyclohexanol, Cyclohexanon, Chlorbenzol, Isophoron, stark polare Lösungsmittel, z.B. Dimethylformamid, Dimethylsulfoxid, N-Methylpyrrolidon, Wasser, in Betracht.

Wäßrige Anwendungsformen können aus Emulsionskonzentraten, Pasten oder netzbaren Pulvern (Spritzpulver, Öldispersionen) durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermitttel in Wasser homogenisiert werden. Es können aber auch aus wirksamer Substanz Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen Alkali-, Erdalkali-, Ammoniumsalze von Ligninsulfonsäure, Naphthalinsulfonsäure, Phenolsulfonsäure, Dibutylnaphthalinsulfonsäure, Alkylarylsulfonate, Alkylsulfate, Alkylsulfonate, Fettalkoholsulfate und Fettsäuren sowie deren Alkali- und Erdalkalisalze, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphtalinsulfonsäure mit Phenol und Formaldehyd, Polyoxyethylenoctylphenolether, ethoxyliertes Isooctylphenol, Octylphenol, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Ligninsulfitablaugen und Methylcellulose i Betracht.

Pulver-, Streu- und Stäubemittel können durch Mischen oder gemeinsames Vermahlen der wirksamen Substanzen mit einem festen Trägerstoff hergestellt werden.

Die Formulierungen enthalten im allgemeinen zwischen 0,01 und 95 Gew.%, vorzugsweise zwischen 0,1 und 90 Gew.% des Wirkstoffs. Die Wirkstoffe werden dabei in einer Reinheit von 90 % bis 100 %, vorzugsweise 95 % bis 100 % (nach NMR-Spektrum) eingesetzt. Beispiele für Formulierungen sind:
I. 5 Gew.-Teile einer erfindungsgemäßen Verbindung werden mit 95 Gew.-Teilen feinteiligem Kaolin innig vermischt. Man erhält auf diese Weise ein Stäubemittel, das 5 Gew.% des Wirkstoffs enthält.
II. 30 Gew.-Teile einer erfindungsgemäßen Verbindung werden mit einer Mischung aus 92 Gew.-Teilen pulverförmigem Kieselsäuregel und8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit (Wirkstoffgehalt 23 Gew.%).
III. 10 Gew.-Teile einer erfindungsgemäßen Verbindung werden in einer Mischung gelöst, die aus 90 Gew.-Teilen Xylol, 6Gew.-Teilen des Anlagerungsproduktes von 8 bis 10 Mol Ethylenoxid an 1Mol Ölsäure-N-monoethanolamid, 2 Gew.-Teilen Calciumsalz der Dodecylbenzolsulfonsäure und 2 Gew.-Teilen des Anlagerungsproduktes von 40 Mol Ethylenoxid an 1 Mol Ricinusöl besteht (Wirkstoffgehalt 9 Gew.%).
IV. 20 Gew.-Teile einer erfindungsgemäßen Verbindung werden in einer Mischung gelöst, die aus 60 Gew.-Teilen Cyclohexanon, 30Gew.-Teilen Isobutanol, 5 Gew.-Teilen des Anlagerungsproduktes von 7 Mol Ethylenoxid an 1 Mol Isooctylphenol und 5Gew.-Teilen des Anlagerungsproduktes von 40 Mol Ethylenoxid an 1 Mol Ricinusöl besteht (Wirkstoffgehalt 16Gew.%).
V. 80 Gew.-Teile einer erfindungsgemäßen Verbindung werden mit 3 Gew.-Teilen des Natriumsalzes der Diisobutylnaphthalin-alpha-sulfonsäure, 10 Gew.-Teilen des Natriumsalzes einer Ligninsulfonsäure aus einer Sulfit-Ablauge und 7 Gew.-Teilen pulverförmigem Kieselsäuregel gut vermischt und in einer Hammermühle vermahlen (Wirkstoffgehalt 80 Gew.%).
VI. Man vermischt 90 Gew.-Teile einer erfindungsgemäßen Verbindung mit 10 Gew.-Teilen N-Methyl-a-pyrrolidon und erhält eine Lösung, die zur Anwendung in Form kleinster Tropfen geeignet ist (Wirkstoffgehalt 90 Gew.%).
VII. 20 Gew.-Teile einer erfindungsgemäßen Verbindung werden in einer Mischung gelöst, die aus 40 Gew.-Teilen Cyclohexanon, 30Gew.-Teilen Isobutanol, 20 Gew.-Teilen des Anlagerungsproduktes von 7 Mol Ethylenoxid an 1 Mol Isooctylphenol und 10 Gew.-Teilen des Anlagerungsproduktes von 40 Mol Ethylenoxid an 1 Mol Ricinusöl besteht. Durch Eingießen und feines Verteilen der Lösung in 100 000 Gew.-Teilen Wasser erhält maneine wäßrige Dispersion, die 0,02 Gew.% des Wirkstoffs enthält.
VIII. 20 Gew.-Teile einer erfindungsgemäßen Verbindung werden mit 3 Gew.-Teilen des Natriumsalzes der Diisobutylnaphthalin-α-sulfonsäure, 17 Gew.-Teilen des Natriumsalzes einer Ligninsulfonsäure aus einer Sulfit-Ablauge und 60 Gew.-Teilen pulverförmigem Kieselsäuregel gut vermischt und in einer Hammermühle vermahlen. Durch feines Verteilen der Mischung in 20000 Gew.-Teilen Wasser erhält man eine Spritzbrühe, die0,1 Gew.% des Wirkstoffs enthält.

Granulate, z.B. Umhüllungs-, Imprägnierungs- und Homogengranulate, können durch Bindung der Wirkstoffe an feste Trägerstoffe hergestellt werden. Feste Trägerstoffe sind z.B. Mineralerden, wie Silicagel, Kieselsäuren, Kieselgele, Silikate, Talkum, Kaolin, Attaclay, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie z.B. Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver und andere feste Trägerstoffe.

Zu den Wirkstoffen können Öle verschiedenen Typs, Herbizide, Fungizide, andere Schädlingsbekämpfungsmittel, Bakterizide, gegebenenfalls auch erst unmittelbar vor der Anwendung (Tankmix), zugesetzt werden. Diese Mittel können zu den erfindungsgemäßen Mitteln im Gewichtsverhältnis 1:10 bis 10:1 zugemischt werden.

### Synthesebeispiele

Die in den nachstehenden Synthesebeispielen wiedergegebenen Vorschriften wurden unter entsprechender Abwandlung der Ausgangsverbindungen zur Gewinnung weiterer Verbindungen I benutzt. Die so erhaltenen Verbindungen sind in den anschließenden Tabellen mit physikalischen Angaben aufgeführt.

### Beispiel 1

### Darstellung von Pentan-2,3-dion-2-oxim

Zu 66,2 g (0,77 mol) 3-Pentanon in 300 ml Toluol werden bei -20°C 250 ml mit Chlorwasserstoff gesättigtes Diethylether zugetropft.

Zu dieser Mischung wird bei -20°C eine Lösung aus 88 g (0,85 mol) n-Butylnitrit in 250 ml Diethylether vorsichtig zugetropft und die gesamte Reaktionsmischung für zwei Stunden bei 0°C und weitere 12 Stunden bei Raumtemperatur nachgerührt.
Nach Zugabe von 250 ml Wasser wird die organische Phase abgetrennt und dreimal mit je 200 ml 1n Natriumhydroxidlösung extrahiert. Diese wäßrige Phase wird durch Zugabe von 1n Salzsäure auf pH = 4 eingestellt und dreimal mit je 200 ml Dichlormethan extrahiert. Nach Trocknung der organischen Phase über Natriumsulfat wird diese im Vakuum eingedampft. Man erhält 56 g eines Öls (63 %), welches schnell kristallisiert.
Schmelzpunkt: 58-60°C
¹H-NMR (CDCl₃): δ = 1.16 (+, 3H); 2.02 (s, 3H); 2.84 (q, 2H); 9.64 (s, 1H).

### Beispiel 2

### Darstellung von Pentan-2,3-dion-2-(O-methyloxim)

Zu einer Lösung von 40,2 g (0,55 mol) Pentan-2,3-dion-2-oxim und 52,0 g (0,38 mol) Kaliumcarbonat in 400 ml Aceton werden 49,7 g (0,35 mol) Methyliodid bei Raumtemperatur zugetropft und 12 Stunden bei dieser Temperatur nachgerührt.
Nach Abfiltrieren der anorganischen Salze wird die Acetonlösung im Vakuum eingedampft, der Rückstand zwischen Methyl-tert.-butylether und Wasser verteilt, die organische Phase mit Wasser gewaschen, über Natriumsulfat getrocknet und eingeengt. Man erhält 37,7 g (83 %) eines Öls.
¹H-NMR (CDCl₃): δ = 1.09 (t, 3H); 1.91 (s, 3H); 2,81 (q, 2H); 4.04 (s, 3H).

### Beispiel 3

### Darstellung von Pentan-2,3,4-trion-4-(O-methyloxim)-2-oxim

Zu einer Lösung aus 12,9 g (0,1 mol) Pentan-2,3-dion-2-(O-methyloxim) in 120 ml Methanol werden bei 10°C 10,3 g (0,1 mol) n-Butylnitrit und anschließend 54 g einer 30 %igen methanolischen Natriummethylatlösung vorsichtig zugetropft. Man läßt auf Raumtemperatur erwärmen und 12 Stunden bei dieser Temperatur rühren. Die Aufarbeitung erfolgt durch Zugabe verdünnter Salzsäure bis pH = 7 und nachfolgendes Einengen im Vakuum. Der Rückstand wird in 100 ml Wasser aufgenommen und zweimal mit je 100 ml Diethylether extrahiert. Die organische Phase wird über Natriumsulfat getrocknet und im Vakuum eingedampft. Man erhält 7,7 g (49 %) eines Öls.
¹H-NMR (CDCl₃): δ = 1.99, 2.11 (2s, 6H); 4.05 (s, 3H); 10,40 (s, 1H).

### Beispiel 4

### Darstellung von Pentan-2,3,4-trion-3,4-bis-(O-methyloxim)-2-oxim

Zu einer Lösung von 3,2 g (0,02 mol) Pentan-2,3,4-trion-4-(O-methyloxim)-2-oxim in 60 ml Methanol werden bei Raumtemperatur 4,7 ml (0,06 mol) Pyridin und 1,7 g (0,02 mol) O-Methylhydroxylaminhydrochlorid zugegeben. Nach zweistündigem Rühren bei Raumtemperatur wird die Mischung im Vakuum eingeengt, in 50 ml Diethylether aufgenommen, zweimal mit je 20 ml verd. Salzsäure und zweimal mit je 20 ml Wasser gewaschen, über Na₂SO₄ getrocknet und im Vakuum eingedampft. Nach Säulenchromatographie an Kieselgel (Cyclohexan / Essigester: 99:1 → 95:5) erhält man zwei Diastereomere als Öle. 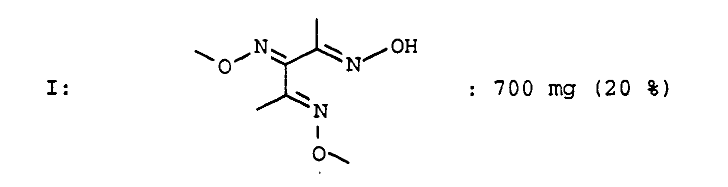 ¹H-NMR (CDCl₃): **δ** = 1.92, 2.12 (2s, 6H); 3.92, 3.99 (2s, 6H); 9.92 (s, 1H) . 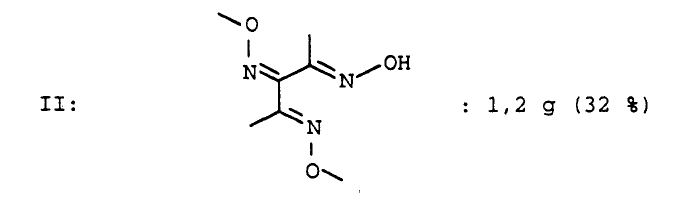 ¹H-NMR (CDCl₃): δ = 2.02, 2.11 (2s, 6H); 3.84, 4.07 (2s, 6H); 9.23 (s, 1H).

### Beispiel 5

### Darstellung von [2-(2,3-Bis-methoxyimino-1-methyl-butylidenaminooxymethyl)-phenyl]-methoxyimino-essigsäuremethylester

Zu einer Lösung von 0,9 g (4,8 mmol) Pentan-2,3,4-trion-3,4-bis-(O-methyloxim)-2-oxim und 0,4 g Kaliumhydroxid in 20 ml trockenem Dimethylformamid gibt man 1,4 g (4,8 mmol) 2-Methoxy-imino-2-(2)-brommethyl)phenylessigsäuremethylester und läßt 30 min bei Raumtemperatur rühren.
Die Aufarbeitung erfolgt durch Zugabe von 50 ml Eiswasser und nachfolgende dreimalige Extraktion mit je 20 ml Diethylether. Die vereinigten organischen Phasen werden über Natriumsulfat getrocknet und im Vakuum eingedampft. Der Rückstand wird aus Diisopropylether umkristallisiert. Man erhält 0,6 g (32 %) weißer Kristalle mit einem Schmelzpunkt von 105°C.
¹H-NMR (CDCl₃): δ = 1.88, 2.02 (2s, 6H); 3.83, 3.91, 3.93, 4.02 (4s, 12H); 5.05 (s, 2H); 7.15 (m, 1H); 7.39 (m, 3H).

### Beispiel 6

### Darstellung von [2-(2,3-Bis-methoxyimino-1-methyl-butylidenaminooxymethyl)-phenyl]-methoxyimino-essigsäuremethylamid

Zu einer Lösung von 0,6 g (1,5 mmol) [2-(2,3-Bis-methoxyimino-1-methyl-butylidenaminooxymethyl)-phenyl]-methoxyimino-essigsäuremethylester in 10 ml Tetrahydrofuran werden 2 ml 40 %ige wäßrige Methylaminlösung gegeben und für 12 h bei Raumtemperatur gerührt. Zur Aufarbeitung wird das Reaktionsgemisch im Vakuum eingedampft, in 20 ml Diethylether aufgenommen, mit 20 ml Wasser gewaschen, über Natriumsulfat getrocknet und im Vakuum eingedampft. Man erhält 0,6 g (98 %) eines Öls.
¹H-NMR (CDCl₃): **δ** = 1.90, 1.98 (2s, 6H); 2.88 (d, 3H); 3.93, 3.94 (3s, 9H); 5.05 (s, 2H); 6.72 (m, 1H); 7.20 (m, 1H); 7.38 (m, 3H).

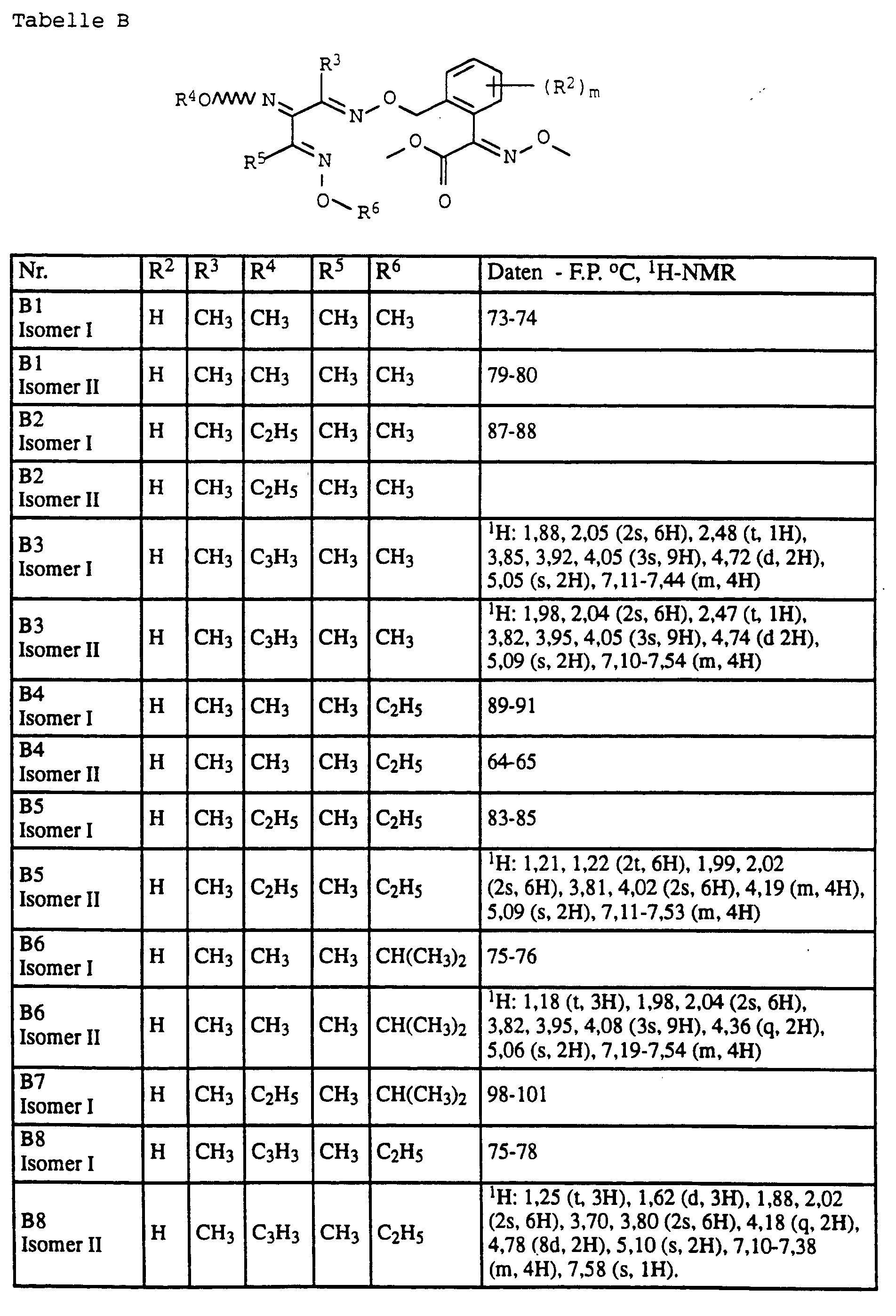

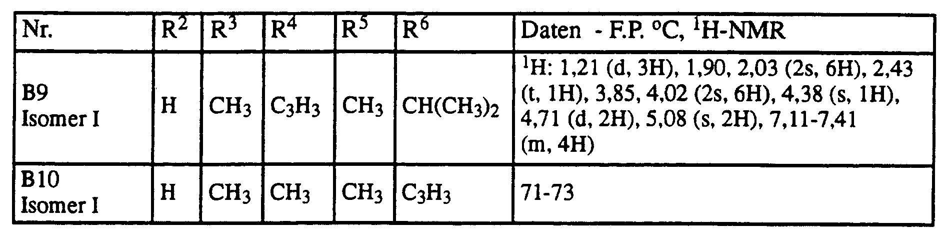

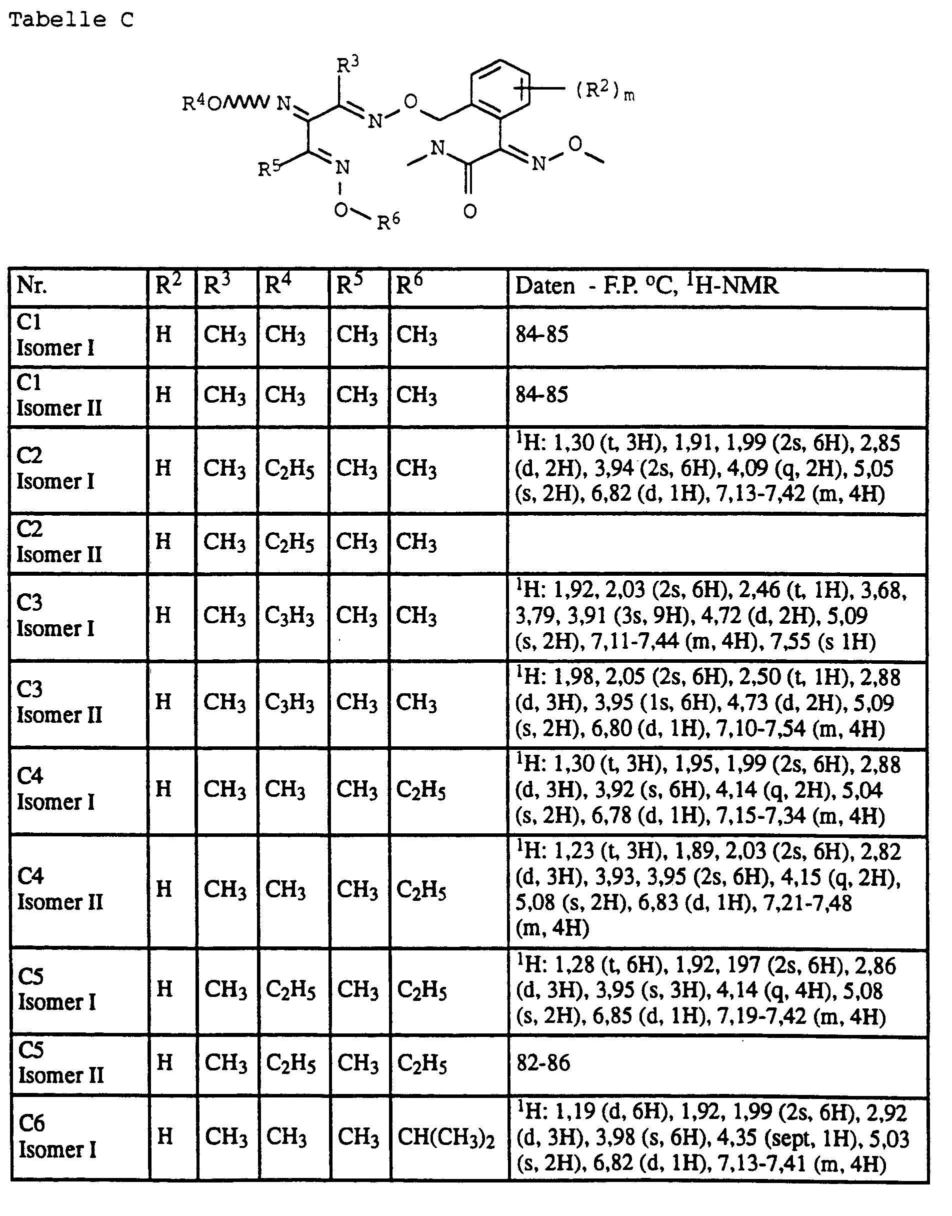

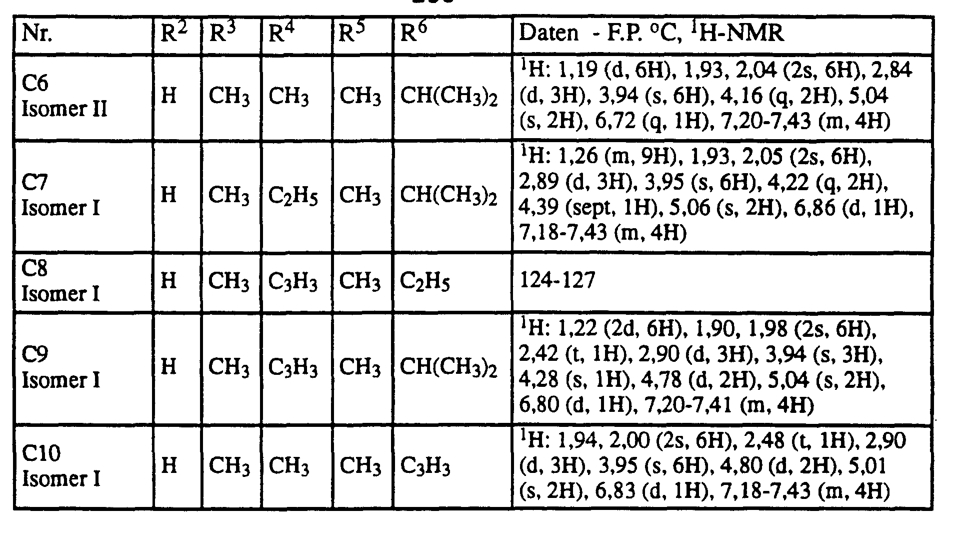

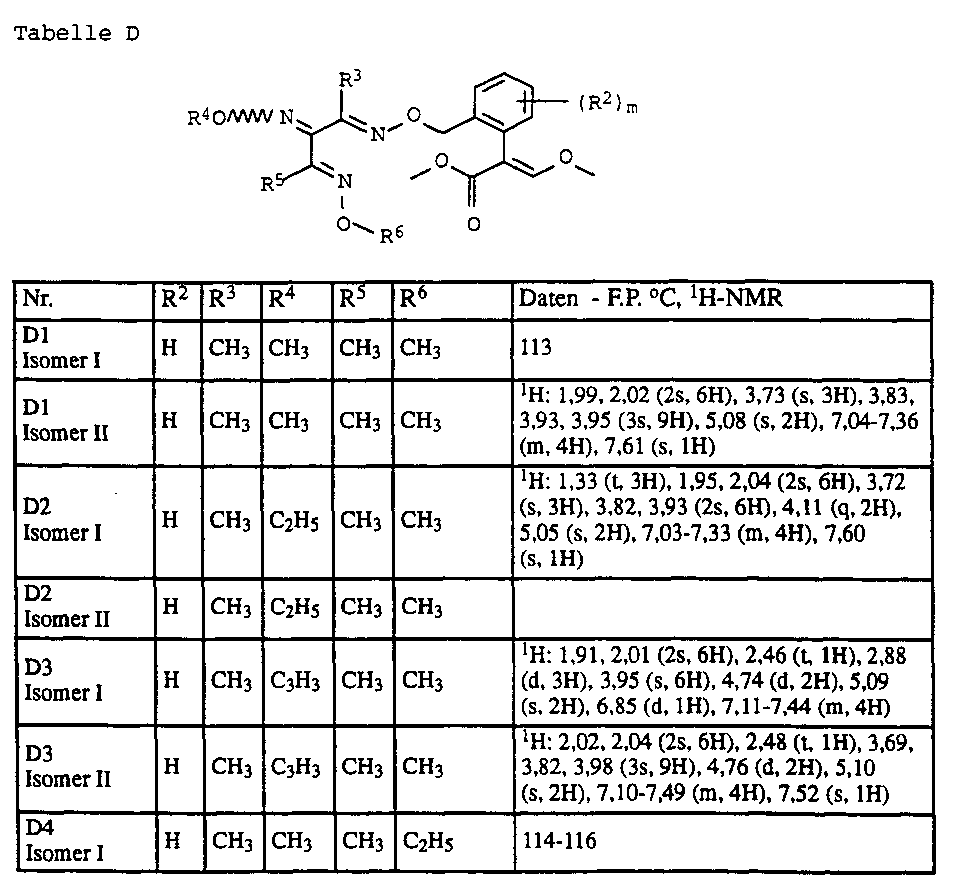

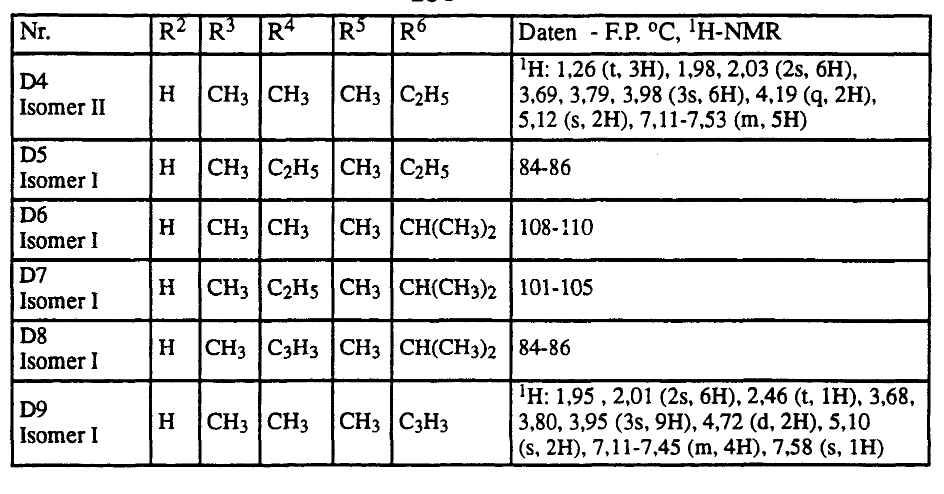

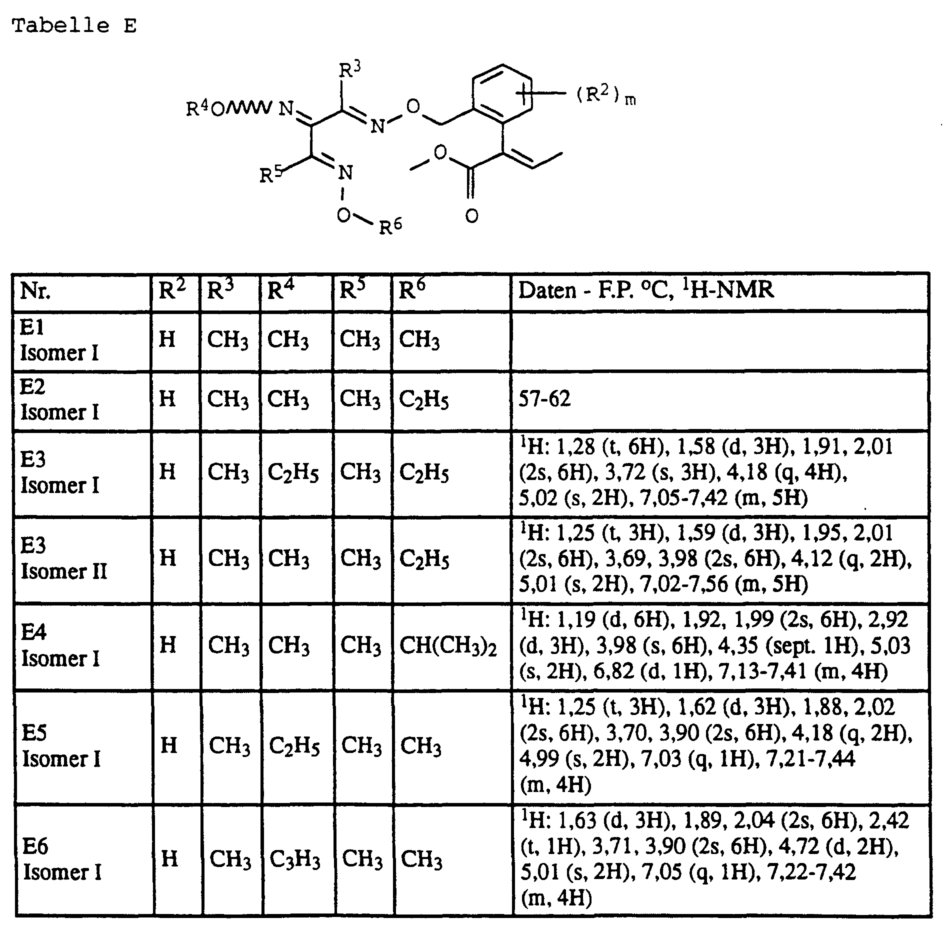

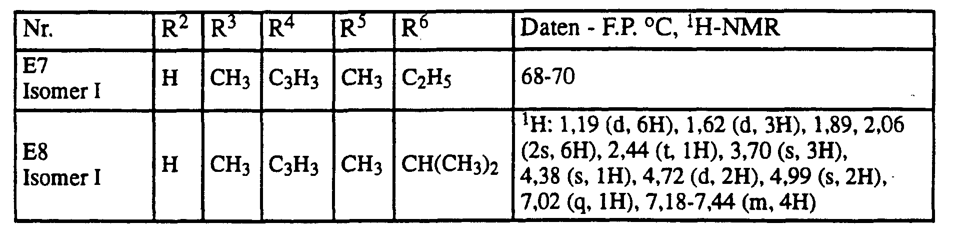

### Anwendungsbeispiel 1

### Wirksamkeit gegen Weizenmehltau

Blätter von in Töpfen gewachsenen Weizenkeimlingen der Sorte "Frühgold" wurden mit wäßriger Spritzbrühe, die 80 % Wirkstoff und 20 % Emulgiermittel in der Trockensubstanz enthielten, besprüht und 24 Stunden nach dem Antrocknen des Spritzbelages mit Oidien (Sporen) des Weizenmehltaus (Erysiphe graminis var. tritici) bestäubt. Die Versuchspflanzen wurden anschließend im Gewächshaus bei Temperaturen zwischen 20 und 22°C und 75 bis 80 % relativer Luftfeuchtigkeit aufgestellt. Nach 7 Tagen wurde das Ausmaß der Mehltauentwicklung in % befallener Blattfäche ermittelt.

| Wirkstoff-Nummer | %-Befall der Blätter nach Applikation von 4 ppm-haltiger wäßriger Wirkstoffaufbereitung |
|---|---|
| B1, Isomer I | 0 |
| B2, Isomer I | 15 |
| B4, Isomer I | 0 |
| B5, Isomer I | 15 |
| B6, Isomer I | 0 |
| B7, Isomer I | 15 |
| B8, Isomer I | 5 |
| B8, Isomer II | 15 |
| B9, Isomer I | 15 |
| C1, Isomer I | 5 |
| C2, Isomer I | 15 |
| C4, Isomer I | 5 |
| C5, Isomer I | 5 |
| C6, Isomer I | 15 |
| C7, Isomer I | 15 |
| D1, Isomer I | 15 |
| D2, Isomer I | 5 |
| D4, Isomer I | 15 |
| D5, Isomer I | 5 |
| D6, Isomer I | 15 |
| D8, Isomer I | 15 |
| E2, Isomer I | 0 |
| E3, Isomer I | 0 |
| E4, Isomer I | 5 |
| E5, Isomer I | 5 |
| E6, Isomer I | 15 |
| E7, Isomer I | 15 |
| E8, Isomer I | 5 |
| Unbehandelt | 75 |

### Anwendungsbeispiel 2

### Wirksamkeit gegen Plasmopara viticola

Blätter von Topfreben der Sorte "Müller-Thurgau" wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 48 Stunden in einer wasserdampfgesättigten Kammer bei 24°C und anschließend für 5 Tage in einem Gewächshaus bei Temperaturen zwischen 20 und 30°C aufgestellt. Nach dieser Zeit wurden die Pflanzen zur Beschleunigung des Sporangienträgerausbruchs abermals für 16 Stunden in der feuchten Kammer aufgestellt. Dann erfolgte die Beurteilung des Ausmaßes des Pilzausbruches in %-Befall auf den Blattunterseiten.

| Wirkstoff-Nummer | %-Befall der Blätter nach Applikation von 16 ppm-haltiger wäßriger Wirkstoffaufbereitung |
|---|---|
| B1, Isomer I | 5 |
| B2, Isomer I | 5 |
| B3, Isomer I | 0 |
| B4, Isomer I | 15 |
| B6, Isomer I | 10 |
| B7, Isomer I | 15 |
| B8, Isomer II | 5 |
| C1, Isomer I | 3 |
| C2, Isomer I | 10 |
| C3, Isomer I | 3 |
| C3, Isomer II | 10 |
| C4, Isomer I | 0 |
| C4, Isomer II | 15 |
| C5, Isomer I | 3 |
| C6, Isomer I | 3 |
| C6, Isomer II | 10 |
| C7, Isomer I | 3 |
| C9, Isomer I | 15 |
| C10, Isomer I | 0 |
| D1, Isomer I | 3 |
| D1, Isomer II | 3 |
| D2, Isomer I | 0 |
| D3, Isomer I | 0 |
| D4, Isomer I | 0 |
| D4, Isomer II | 5 |
| D5, Isomer I | 10 |
| D6, Isomer I | 0 |
| D7, Isomer I | 10 |
| D8, Isomer I | 15 |
| D9, Isomer I | 0 |
| Unbehandelt | 80 |

### Anwendungsbeispiel 3

### Wirksamkeit gegen Pyricularia oryzae (protektiv)

Blätter von in Töpfen gewachsenen Reiskeimlingen der Sorte "Tai-Nong 67" wurden mit wäßrigen Emulsionen, die 80 % Wirkstoff und 20 % Emulgiermittel in der Trockensubstanz enthielten, tropfnaß besprüht und 24 Stunden später mit einer wäßrigen Sporensuspension von Pyricularia oryzae inokuliert. Anschließend wurden die Versuchspflanzen in Klimakammern bei 22 bis 24°C und 95 bis 99 % relativer Luftfeuchtigkeit aufgestellt. Nach 6 Tagen wurde das Ausmaß des Krankheitsbefalls ermittelt.

| Wirkstoff-Nummer | %-Befall der Blätter nach Applikation von 4 ppm-haltiger wäßriger Wirkstoffaufbereitung |
|---|---|
| B1, Isomer I | 15 |
| B2, Isomer I | 15 |
| B3, Isomer I | 5 |
| C1, Isomer I | 0 |
| C2, Isomer I | 15 |
| C3, Isomer I | 0 |
| C5, Isomer I | 15 |
| C6, Isomer I | 15 |
| C7, Isomer I | 15 |
| C10, Isomer I | 15 |
| D1, Isomer I | 0 |
| D1, Isomer II | 15 |
| D2, Isomer I | 0 |
| D3, Isomer I | 0 |
| D4, Isomer I | 15 |
| D6, Isomer I | 15 |
| D7, Isomer I | 15 |
| D9, isomer I | 15 |
| E5, Isomer I | 15 |
| Unbehandelt | 85 |

## Patentansprüche

1. Phenylessigsäurederivate der Formel I 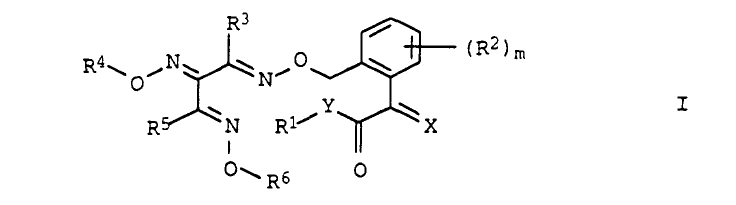 in der die Substituenten und der Index die folgende Bedeutung haben:
X NOCH₃, CHOCH₃, CHCH₃;
Y O, NR
R¹,R unabhängig voneinander Wasserstoff und C₁-C₄-Alkyl;
R² Cyano, Nitro, Trifluormethyl, Halogen, C₁-C₄-Alkyl und C₁-C₄-Alkoxy;
m 0, 1 oder 2, wobei die Reste R² verschieden sein können, wenn m für 2 steht;
R³ Wasserstoff, Cyano, C₁-C₄-Alkyl, C₁-C₄-Halogenalkyl, C₃-C₆-Cycloalkyl;
R⁴,R⁶ unabhängig voneinander Wasserstoff,
C₁-C₁₀-Alkyl, C₃-C₆-Cycloalkyl, C₂-C₁₀-Alkenyl, C₂-C₁₀-Alkinyl, C₁-C₁₀-Alkylcarbonyl, C₂-C₁₀-Alkenylcarbonyl, C₃-C₁₀-Alkinylcarbonyl oder C₁-C₁₀-Alkylsulfonyl, wobei diese Reste partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, C₃-C₆-Cycloalkyl, C₃-C₆-Cycloalkyloxy, Heterocyclyl, Heterocyclyloxy, Benzyl, Benzyloxy, Aryl, Aryloxy, Arylthio, Hetaryl, Hetaryloxy und Hetarylthio, wobei die cyclischen Gruppen ihrerseits partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkyloxycarbonyl, C₁-C₆-Alkylthio, C₁-C₆-Alkylamino, Di-C₁-C₆-Alkylamino, C₁-C₆-Alkylaminocarbonyl, Di-C₁-C₆-Alkylaminocarbonyl, C₁-C₆-Alkylaminothiocarbonyl, Di-C₁-C₆-Alkylaminothiocarbonyl, C₂-C₆-Alkenyl, C₂-C₆-Alkenyloxy, Benzyl, Benzyloxy, Aryl, Aryloxy, Arylthio, Hetaryl, Hetaryloxy, Hetarylthio oder C (=NOR⁷)-Aₙ-R⁸;
Aryl, Arylcarbonyl, Arylsulfonyl, Hetaryl, Hetarylcarbonyl oder Hetarylsulfonyl, wobei diese Reste partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylcarbonyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkyloxycarbonyl, C₁-C₆-Alkylthio, C₁-C₆-Alkylamino, Di-C₁-C₆-Alkylamino, C₁-C₆-Alkylaminocarbonyl, Di-C₁-C₆-Alkylaminocarbonyl, C₁-C₆-Alkylaminothiocarbonyl, Di-C₁-C₆-Alkylaminothiocarbonyl, C₂-C₆-Alkenyl, C₂-C₆-Alkenyloxy, Benzyl, Benzyloxy, Aryl, Aryloxy, Hetaryl, Hetaryloxy oder C(=NOR⁷)-Aₙ-R⁸;
R⁵ Wasserstoff,
C₁-C₆-Alkyl, C₂-C₆-Alkenyl, C₂-C₆-Alkinyl, wobei die Kohlenwasserstoffreste dieser Gruppen partiell oder vollständig halogeniert sein können oder einen bis drei der folgenden Reste tragen können: Cyano, Nitro, Hydroxy, Mercapto,Amino, Carboxyl, Aminocarbonyl, Aminothiocarbonyl, Halogen, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₁-C₆-Alkylsulfonyl, C₁-C₆-Alkylsulfoxyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₂-C₆-Alkenyloxy, C₃-C₆-Cycloalkyl, C₃-C₆-Cycloalkyloxy, Heterocyclyl, Heterocyclyloxy, Aryl, Aryloxy, Aryl-C₁-C₄-alkoxy, Arylthio, Aryl-C₁-C₄-alkylthio, Hetaryl, Hetaryloxy, Hetaryl-C₁-C₄-alkoxy, Hetarylthio, Hetaryl-C₁-C₄-alkylthio, wobei die cyclischen Reste ihrerseits partiell oder vollständig halogeniert sein können und/oder ein bis drei der folgenden Gruppen tragen können: Cyano, Nitro, Hydroxy, Mercapto, Amino, Carboxyl, Aminocarbonyl, Aminothiocarbonyl, C₁-C₆-Alkyl, C₁-C₆-Halogenalkyl, C₁-C₆-Alkylsulfonyl, C₁-C₆-Alkylsulfoxyl, C₃C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Arylthio, Hetaryl, Hetaryloxy, Hetarylthio und C(=NOR⁷)-Aₙ-R⁸;
C₃-C₆-Cycloalkyl, C₃-C₆-Cycloalkenyl, Heterocyclyl, Aryl, Hetaryl, wobei die cyclischen Reste partiell oder vollständig halogeniert sein können oder einen bis drei der folgenden Gruppen tragen können: Cyano, Nitro, Hydroxy, Mercapto, Amino, Carboxyl, Aminocarbonyl, Aminothiocarbonyl, Halogen, C₁-C₆-Alkyl, C₁-C₆-Halogenalkyl, C₁-C₆-Alkylsulfonyl, C₁-C₆-Alkylsulfoxyl, C₃-C₆-Cycloalkyl, C₁-C₆-Alkoxy, C₁-C₆-Halogenalkoxy, C₁-C₆-Alkoxycarbonyl, C₁-C₆-Alkylthio, C₁-C₆-Alkylamino, Di-C₁-C₆-alkylamino, C₁-C₆-Alkylaminocarbonyl, Di-C₁-C₆-alkylaminocarbonyl, C₁-C₆-Alkylaminothiocarbonyl, Di-C₁-C₆-alkylaminothiocarbonyl, C₂-C₆-Alkenyl, C₂-C₆-Alkenyloxy, Benzyl, Benzyloxy, Aryl, Aryloxy, Hetaryl und Hetaryloxy;
wobei
A für Sauerstoff, Schwefel oder Stickstoff steht und wobei der Stickstoff Wasserstoff oder C₁-C₆-Alkyl trägt;
n 0 oder 1 bedeutet;
R⁷ Wasserstoff oder C₁-C₆-Alkyl bedeutet und
R⁸ Wasserstoff oder C₁-C₆-Alkyl bedeutet,
sowie deren Salze.

2. Verbindungen der Formel I nach Anspruch 1, in der X für NOCH₃ steht.

3. Verbindungen der Formel I nach Anspruch 1 oder 2, in der X für CHOCH₃ steht.

4. Verbindungen der Formel I nach einem der Ansprüche 1 bis 3, in der X für CHCH₃ steht.

5. Verbindungen der Formel I nach einem der Ansprüche 1 bis 4, in denen m für 0 steht.

6. Verbindungen der Formel I nach einem der Ansprüche 1 bis 5, in denen R¹ für Methyl steht.

7. Verbindungen der Formel I nach einem der Ansprüche 1 bis 6, in denen R³ für Wasserstoff, Cyano, Cyclopropyl, Methyl, Ethyl oder 1-Methylethyl steht.

8. Verbindungen der Formel I nach einem der Ansprüche 1 bis 7, in denen R4 für Wasserstoff, C₁-C₆-Alkyl, C₂-C₆-Alkenyl, C₂-C₆-Alkinyl, Allyl, Arylalkyl, Hetarylalkyl, Aryloxyalkyl, Hetaryloxyalkyl, Aryl oder Hetaryl steht.

9. Verbindungen der Formel I nach einem der Ansprüche 1 bis 8, in der R⁵ für Wasserstoff, Cyclopropyl, Methyl, Ethyl, iso-Propyl, ggf. subst. Aryl oder Hetaryl steht.

10. Verbindungen der Formel I nach einem der Ansprüche 1 bis 9, in der R⁶ für C₁-C₆-Alkyl, C₂-C₆-Alkenyl, C₂-C₆-Alkinyl, Arylalkyl, Hetarylalkyl, Aryloxyalkyl oder Hetaryloxyalkyl steht.

11. Verfahren zur Herstellung der Verbindungen der Formel I, dadurch gekennzeichnet, daß man ein Benzylderivat der Formel II mit einem Hydroxyimin der Formel III, 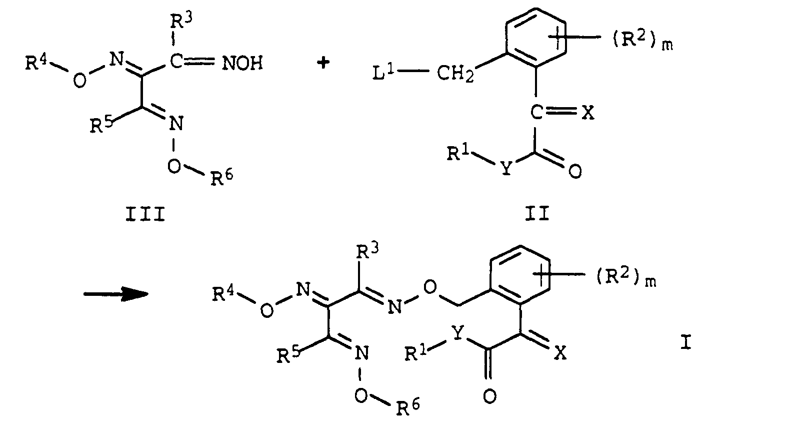 in der L¹ für eine nukleophil austauschbare Abgangsgruppe steht, umsetzt.

12. Mittel gegen tierische Schädlinge oder Schadpilze, enthaltend übliche Zusatzstoffe und eine wirksame Menge einer Verbindung der Formel I gemäß Anspruch 1.

13. Mittel nach Anspruch 12 zur Bekämpfung tierischer Schädlinge aus der Klasse der Insekten, Spinnentiere oder Nematoden.

14. Verfahren zur Bekämpfung von tierischen Schädlingen oder Schadpilzen, dadurch gekennzeichnet, daß man die Schädlinge oder Schadpilze, deren Lebensraum oder die von ihnen freizuhaltenden Pflanzen, Flächen, Materialien oder Räume mit einer wirksamen Menge mindestens einer Verbindung der Formel I gemäß Anspruch 1 behandelt.

15. Verwendung der Verbindungen I gemäß Anspruch 1 zur Herstellung von Mitteln gegen tierische Schädlinge oder Schadpilze.

16. Verwendung der Verbindungen I gemäß Anspruch 1 zur Bekämpfung von tierischen Schädlingen oder Schadpilzen.

17. Hydroxyimine der Formel III' 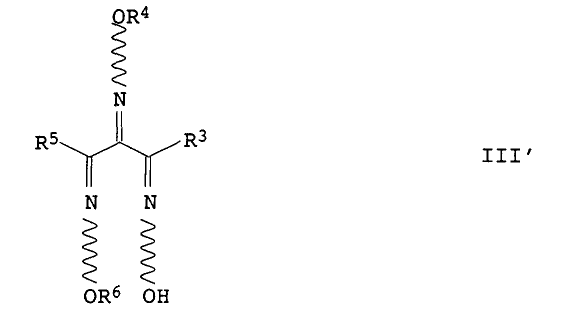 in der die Substituenten die in Anspruch 1 gegebene Bedeutung haben, wobei für R⁴ und R⁶ die Bedeutung Wasserstoff ausgenommen bleibt.

18. Hydroxyimine der Formel III gemäß Anspruch 11, in der die Substitutenten die in Anspruch 17 genannte Bedeutung haben.

19. Verfahren zur Herstellung der Verbindungen der Formel I, in denen Y für NH steht, dadurch gekennzeichnet, daß man ein Benzylderivat der Formel II, in der Y für Sauerstoff und L¹ für eine nukleophil austauschbare Abgangsgruppe steht, mit einem Hydroxyimin der Formel III und anschließend die erhaltenen Ester der Formel I mit Aminen der Formel R¹NH₂ umsetzt.

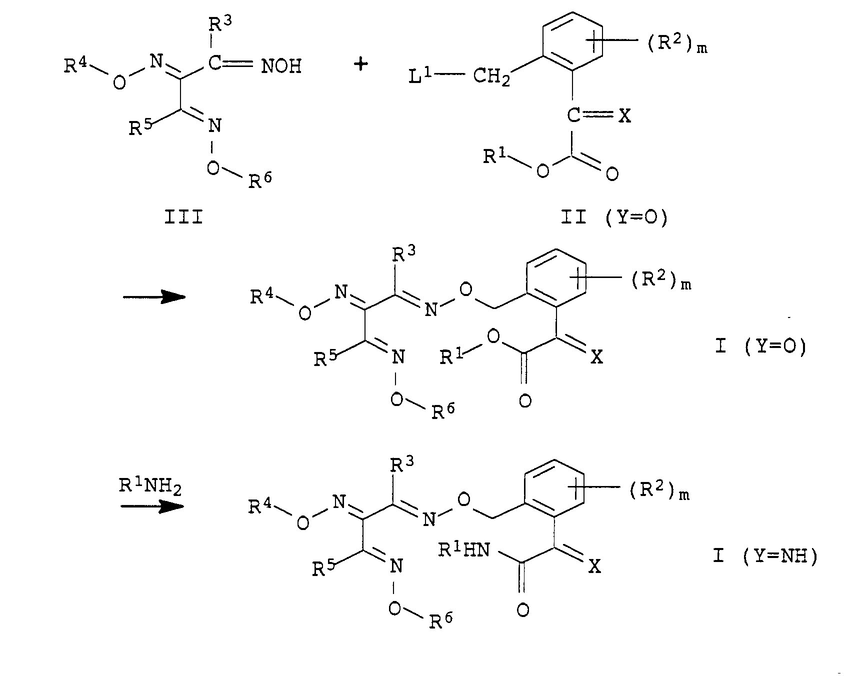

## Claims

1. A phenylacetic acid derivative of the formula I 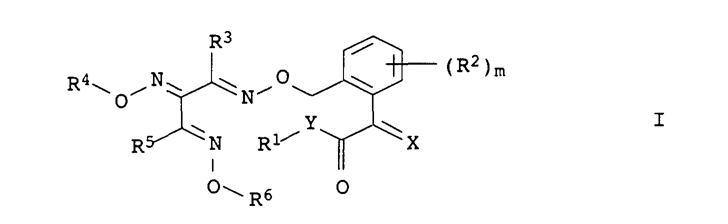 where the substituents and the index have the following meanings:
x is NOCH₃, CHOCH₃, CHCH₃;
Y is O, NR
R¹,R independently of one another are hydrogen and C₁-C₄-alkyl;
R² is cyano, nitro, trifluoromethyl, halogen, C₁-C₄-alkyl and C₁-C₄-alkoxy;
m is 0, 1 or 2, it being possible for the radicals R² to be different when m is 2;
R³ is hydrogen, cyano, C₁-C₄-alkyl, C₁-C₄-haloalkyl, C₃-C₆-cycloalkyl;
R⁴,R⁶ independently of one another are hydrogen,
C₁-C₁₀-alkyl, C₃-C₆-cycloalkyl, C₂-C₁₀-alkenyl, C₂-C₁₀-alkynyl, C₁-C₁₀-alkylcarbonyl, C₂-C₁₀-alkenylcarbonyl, C₃-C₁₀-alkynylcarbonyl or C₁-C₁₀-alkylsulfonyl, it being possible for these radicals to be partially or fully halogenated or to have attached to them one to three of the following groups: cyano, nitro, hydroxyl, mercapto, amino, carboxyl, aminocarbonyl, aminothiocarbonyl, halogen, C₁-C₆-alkyl, C₁-C₆-haloalkyl, C₁-C₆-alkylsulfonyl, C₁-C₆-alkylsulfoxyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, C₃-C₆-cycloalkyl, C₃-C₆-cycloalkyloxy, heterocyclyl, heterocyclyloxy, benzyl, benzyloxy, aryl, aryloxy, arylthio, hetaryl, hetaryloxy and hetarylthio, it being possible for the cyclic groups, in turn, to be partially or fully halogenated or to have attached to them one to three of the following groups: cyano, nitro, hydroxyl, mercapto, amino, carboxyl, aminocarbonyl, aminothiocarbonyl, halogen, C₁-C₆-alkyl, C₁-C₆-haloalkyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkyloxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, arylthio, hetaryl, hetaryloxy, hetarylthio or C(=NOR⁷)-Aₙ-R⁸;
aryl, arylcarbonyl, arylsulfonyl, hetaryl, hetarylcarbonyl or hetarylsulfonyl, it being possible for these radicals to be partially or fully halogenated or to have attached to them one to three of the following groups: cyano, nitro, hydroxyl, mercapto, amino, carboxyl, aminocarbonyl, aminothiocarbonyl, halogen, C₁-C₆-alkyl, C₁-C₆-haloalkyl, C₁-C₆-alkylcarbonyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkyloxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, hetaryl, hetaryloxy or C(=NOR⁷)-Aₙ-R⁸;
R⁵ is hydrogen,
C₁-C₆-alkyl, C₂-C₆-alkenyl, C₂-C₆-alkynyl, it being possible for the hydrocarbon radicals of these groups to be partially or fully halogenated or to have attached to them one to three of the following radicals: cyano, nitro, hydroxyl, mercapto, amino, carboxyl, aminocarbonyl, aminothiocarbonyl, halogen, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₁-C₆-alkylsulfonyl, C₁-C₆-alkylsulfoxyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₂-C₆-alkenyloxy, C₃-C₆-cycloalkyl, C₃-C₆-cycloalkyloxy, heterocyclyl, heterocyclyloxy, aryl, aryloxy, aryl-C₁-C₄-alkoxy, arylthio, aryl-C₁-C₄-alkylthio, hetaryl, hetaryloxy, hetaryl-C₁-C₄-alkoxy, hetarylthio, hetaryl-C₁-C₄-alkylthio, it being possible for the cyclic radicals, in turn, to be partially or fully halogenated and/or to have attached to them one to three of the following groups: cyano, nitro, hydroxyl, mercapto, amino, carboxyl, aminocarbonyl, aminothiocarbonyl, C₁-C₆-alkyl, C₁-C₆-haloalkyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, arylthio, hetaryl, hetaryloxy, hetarylthio and C(=NOR⁷)-Aₙ-R⁸;
C₃-C₆-cycloalkyl, C₃-C₆-cycloalkenyl, heterocyclyl, aryl, hetaryl, it being possible for the cyclic radicals to be partially or fully halogenated or to have attached to them one to three of the following groups: cyano, nitro, hydroxyl, mercapto, amino, carboxyl, aminocarbonyl, aminothiocarbonyl, halogen, C₁-C₆-alkyl, C₁-C₆-haloalkyl, C₁-C₆-alkylsulfonyl, C₁-C₆-alkylsulfoxyl, C₃-C₆-cycloalkyl, C₁-C₆-alkoxy, C₁-C₆-haloalkoxy, C₁-C₆-alkoxycarbonyl, C₁-C₆-alkylthio, C₁-C₆-alkylamino, di-C₁-C₆-alkylamino, C₁-C₆-alkylaminocarbonyl, di-C₁-C₆-alkylaminocarbonyl, C₁-C₆-alkylaminothiocarbonyl, di-C₁-C₆-alkylaminothiocarbonyl, C₂-C₆-alkenyl, C₂-C₆-alkenyloxy, benzyl, benzyloxy, aryl, aryloxy, hetaryl and hetaryloxy;
where
A is oxygen, sulfur or nitrogen and where the nitrogen has attached to it hydrogen or C₁-C₆-alkyl;
n is 0 or 1;
R⁷ is hydrogen or C₁-C₆-alkyl and
R⁸ is hydrogen or C₁-C₆-alkyl,
or a salt thereof.

2. A compound of the formula I as claimed in claim 1, where X is NOCH₃.

3. A compound of the formula I as claimed in claim 1 or 2, where X is CHOCH₃.

4. A compound of the formula I as claimed in any of claims 1 to 3 where X is CHCH₃.

5. A compound of the formula I as claimed in any of claims 1 to 4 where m is 0.

6. A compound of the formula I as claimed in any of claims 1 to 5 where R¹ is methyl.

7. A compound of the formula I as claimed in any of claims 1 to 6 where R³ is hydrogen, cyano, cyclopropyl, methyl, ethyl or 1-methylethyl.

8. A compound of the formula I as claimed in any of claims 1 to 7 where R⁴ is hydrogen, C₁-C₆-alkyl, C₂-C₆-alkenyl, C₂-C₆-alkynyl, allyl, arylalkyl, hetarylalkyl, aryloxyalkyl, hetaryloxyalkyl, aryl or hetaryl.

9. A compound of the formula I as claimed in any of claims 1 to 8 where R⁵ is hydrogen, cyclopropyl, methyl, ethyl, iso-propyl, unsubstituted or substituted aryl or hetaryl.

10. A compound of the formula I as claimed in any of claims 1 to 9 where R⁶ is C₁-C₆-alkyl, C₂-C₆-alkenyl, C₂-C₆-alkynyl, arylalkyl, hetarylalkyl, aryloxyalkyl or hetaryloxyalkyl.

11. A process for the preparation of the compounds of the formula I which comprises reacting a benzyl derivative of the formula II with a hydroxyimine of the formula III 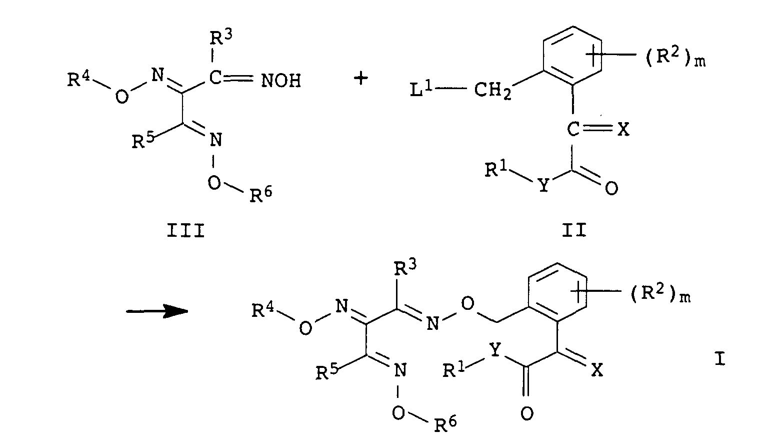 where L¹ is a nucleophilically exchangeable leaving group.

12. A composition against animal pests or harmful fungi comprising customary additives and an effective amount of a compound of the formula I as claimed in claim 1.

13. A composition as claimed in claim 12 for controlling animal pests from the classes of the insects, arachnids or nematodes.

14. A method of controlling animal pests or harmful fungi which comprises treating the pests or harmful fungi, their environment, or the plants, areas, materials or spaces to be kept free from them with an effective amount of at least one compound of the formula I as claimed in claim 1.

15. The use of the compounds I as claimed in claim 1 for the preparation of compositions against animal pests or harmful fungi.

16. The use of the compounds I as claimed in claim 1 for controlling animal pests or harmful fungi.

17. A hydroxyimine of the formula III' 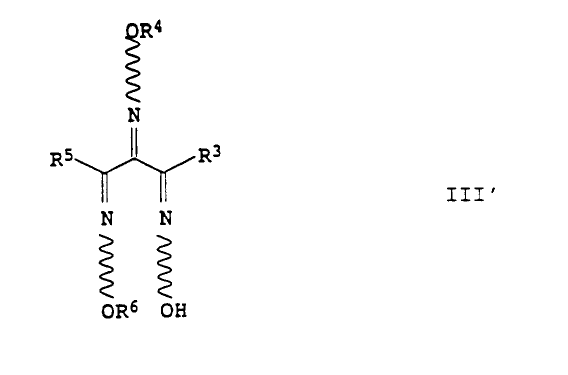 where the substituents have the meaning given in claim 1, the meaning hydrogen being excluded for R⁴ and R⁶.

18. A hydroxyimine of the formula III as claimed in claim 11, in which the substituents have the meaning given in claim 17.

19. A process for preparing the compounds of the formula I in which Y is NH, which comprises reacting a benzyl derivative of the formula II in which Y is oxygen and L¹ is a nucleophilically exchangeable leaving group with a hydroxyimine of the formula III and then reacting the resulting esters of the formula I with amines of the formula R¹NH₂.

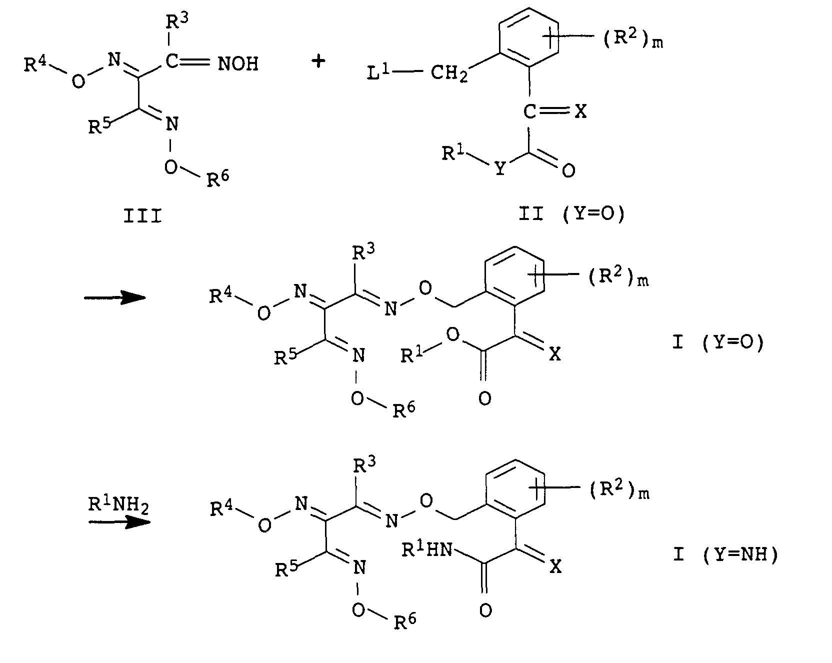

## Revendications

1. Dérivés de l'acide phénylacétique répondant à la formule I 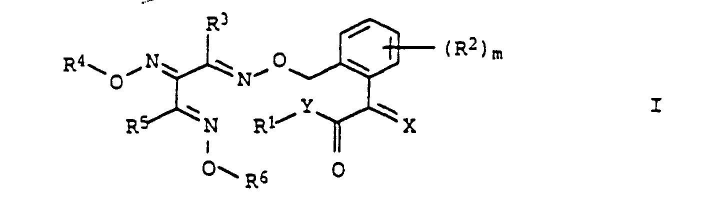 dans laquelle les symboles et l'indice ont les significations suivantes :
X : NOCH₃, CHOCH₃, CHCH₃ ;
Y: O, NR
R¹, R, indépendamment l'un de l'autre, l'hydrogène ou un groupe alkyle en C1-C4 ;
R² : un groupe cyano, nitro, trifluorométhyle, un halogène, un groupe alkyle en C1-C4 ou alcoxy en C1-C4 ;
m : 0, 1 ou 2, les substituants R² pouvant être différents lorsque m est égal à 2 ;
R³ : l'hydrogène, un groupe cyano, alkyle en C1-C4, halogénoalkyle en C1-C4, cycloalkyle en C3-C6 ;
R⁴, R⁶, indépendamment l'un de l'autre : l'hydrogène,
un groupe alkyle en C1-C10, cycloalkyle en C3-C6, alcényle en C2-C10, alcynyle en C2-C10, (alkyle en C1-C10)carbonyle, (alcényle en C2-C10)carbonyle, (alcynyle en C3-C10)carbonyle ou alkylsulfonyle en C1-C10, ces groupes pouvant être halogénés en totalité ou en partie ou pouvant porter un à trois des substituants suivants : cyano, nitro, hydroxy, mercapto, amino, carboxyle, aminocarbonyle, aminothiocarbonyle, halogéno, alkyle en C1-C6, halogénoalkyle en C1-C6, alkylsulfonyle en C1-C6, alkylsulfoxyle en C1-C6, alcoxy en C1-C6, halogénoalcoxy en C1-C6, (alcoxy en C1-C6)carbonyle, alkylthio en C1-C6, alkylamino en C1-C6, di-(alkyle en C1-C6)amino,
un groupe (alkyle en C1-C6)aminocarbonyle, di-(alkyle en C1-C6)aminocarbonyle, (alkyle en C1-C6)aminothiocarbonyle, di-(alkyle en C1-C6)aminothiocarbonyle, alcényle en C2-C6, alcényloxy en C2-C6, cycloalkyle en C3-C6, cycloalcoxy en C3-C6, hétérocyclyle, hétérocyclyloxy, benzyle, benzyloxy, aryle, aryloxy, arylthio, hétéroaryle, hétéroaryloxy et hétéroarylthio, les groupes cycliques pouvant eux-mêmes être halogénés en totalité ou en partie ou pouvant porter un à trois des substituants suivants : cyano, nitro, hydroxy, mercapto, amino, carboxyle, aminocarbonyle, aminothiocarbonyle, halogéno, alkyle en C1-C6, halogénoalkyle en C1-C6, alkylsulfonyle en C1-C6, alkylsulfoxyle en C1-C6, cycloalkyle en C3-C6, alcoxy en C1-C6, halogénoalcoxy en C1-C6, (alcoxy en C1-C6)carbonyle, alkylthio en C1-C6, alkylamino en C1-C6, di-(alkyle en C1-C6)amino, (alkyle en C1-C6)aminocarbonyle, di-(alkyle en C1-C6)aminocarbonyle, (alkyle en C1-C6)aminothiocarbonyle, di-(alkyle en C1-C6)aminothiocarbonyle, alcényle en C2-C6, alcényloxy en C2-C6, benzyle, benzyloxy, aryle, aryloxy, arylthio, hétéroaryle, hétéroaryloxy, hétéroarylthio ou C(=NOR⁷)-Aₙ-R⁸ ;
un groupe aryle, arylcarbonyle, arylsulfonyle, hétéroaryle, hétéroarylcarbonyle ou hétéroarylsulfonyle, ces groupes pouvant être halogénés en totalité ou en partie ou pouvant porter un à trois des substituants suivants : cyano, nitro, hydroxy, mercapto, amino, carboxyle, aminocarbonyle, aminothiocarbonyle, halogéno, alkyle en C1-C6, halogénoalkyle en C1-C6, (alkyle en C1-C6)carbonyle, alkylsulfonyle en C1-C6, alkylsulfoxyle en C1-C6, cycloalkyle en C3-C6, alcoxy en C1-C6, halogénoalcoxy en C1-C6, (alcoxy en C1-C6)carbonyle, alkylthio en C1-C6, alkylamino en C1-C6, di-(alkyle en C1-C6)amino, (alkyle en C1-C6)aminocarbonyle, di-(alkyle en C1-C6)aminocarbonyle, (alkyle en C1-C6)aminothiocarbonyle, di-(alkyle en C1-C6)aminothiocarbonyle, alcényle en C2-C6, alcényloxy en C2-C6, benzyle, benzyloxy, aryle, aryloxy, hétéroaryle, hétéroaryloxy ou C(=NOR⁷)-Aₙ-R⁸ ;
R⁵ : l'hydrogène,
un groupe alkyle en C1-C6, alcényle en C2-C6, alcynyle en C2-C6, les parties hydrocarbonées de ces groupes pouvant être halogénées en totalité ou en partie ou pouvant porter un à trois des substituants suivants : cyano, nitro, hydroxy, mercapto, amino, carboxyle, aminocarbonyle, aminothiocarbonyle, halogéno, (alkyle en C1-C6)aminocarbonyle, di-(alkyle en C1-C6)aminocarbonyle, (alkyle en C1-C6)aminothiocarbonyle, di-(alkyle en C1-C6)aminothiocarbonyle, alkylsulfonyle en C1-C6, alkylsulfoxyle en C1-C6, alcoxy en C1-C6, halogénoalcoxy en C1-C6, (alcoxy en C1-C6)carbonyle, alkylthio en C1-C6, alkylamino en C1-C6, di-(alkyle en C1-C6)amino, alcényloxy en C2-C6, cycloalkyle en C3-C6, cycloalcoxy en C3-C6, hétérocyclyle, hétérocyclyloxy, aryle, aryloxy, aryl-alcoxy en C1-C4, arylthio, arylalkylthio en C1-C4, hétéroaryle, hétéroaryloxy, hétéroaryl-alcoxy en C1-C4, hétéroarylthio, hétéroaryl-alkylthio en C1-C4, les radicaux cycliques pouvant eux-mêmes être halogénés en totalité ou en partie et/ou pouvant porter un à trois des substituants suivants : cyano, nitro, hydroxy, mercapto, amino, carboxyle, aminocarbonyle, aminothiocarbonyle, alkyle en C1-C6, halogénoalkyle en C1-C6, alkylsulfonyle en C1-C6, alkylsulfoxyle en C1-C6, cycloalkyle en C3-C6, alcoxy en C1-C6, halogénoalcoxy en C1-C6, (alcoxy en C1-C6)carbonyle, alkylthio en C1-C6, alkylamino en C1-C6, di-(alkyle en C1-C6)amino, (alkyle en C1-C6)aminocarbonyle, di-(alkyle en C1-C6)aminocarbonyle, (alkyle en C1-C6)aminothiocarbonyle, di-(alkyle en C1-C6)aminothiocarbonyle, alcényle en C2-C6, alcényloxy en C2-C6, benzyle, benzyloxy, aryle, aryloxy, arylthio, hétéroaryle, hétéroaryloxy, hétéroarylthio et C(=NOR⁷)-Aₙ-R⁸ ;
un groupe cycloalkyle en C3-C6, cycloalcényle en C3-C6, hétérocyclyle, aryle, hétéroaryle, les radicaux cycliques pouvant être halogénés en totalité ou en partie ou pouvant porter un à trois des substituants suivants : cyano, nitro, hydroxy, mercapto, amino, carboxyle, aminocarbonyle, aminothiocarbonyle, halogéno, alkyle en C1-C6, halogénoalkyle en C1-C6, alkylsulfonyle en C1-C6, alkylsulfoxyle en C1-C6, cycloalkyle en C3-C6, alcoxy en C1-C6, halogénoalcoxy en C1-C6, (alcoxy en C1-C6)carbonyle, alkylthio en C1-C6, alkylamino en C1-C6, di-(alkyle en C1-C6)amino, (alkyle en C1-C6)aminocarbonyle, di-(alkyle en C1-C6)aminocarbonyle, (alkyle en C1-C6)aminothiocarbonyle, di-(alkyle en C1-C6)aminothiocarbonyle, alcényle en C2-C6, alcényloxy en C2-C6, benzyle, benzyloxy, aryle, aryloxy, hétéroaryle et hétéroaryloxy ;
A représente l'oxygène, le soufre ou l'azote ou l'azote portant de l'hydrogène ou un groupe alkyle en C1-C6 ;
n est égal à 0 ou 1 ;
R⁷ représente l'hydrogène ou un groupe alkyle en C1-C6 et
R⁸ représente l'hydrogène ou un groupe alkyle en C1-C6,
et leurs sels.

2. Composés de formule I selon la revendication 1, dans laquelle X représente NOCH₃.

3. Composés de formule I selon la revendication 1 ou 2 dans laquelle X représente CHOCH₃.

4. Composés de formule I selon l'une des revendications 1 à 3, dans laquelle X représente CHCH₃.

5. Composés de formule I selon l'une des revendications 1 à 4, dans laquelle m est égal à 0.

6. Composés de formule I selon l'une des revendications 1 à 5, dans laquelle R¹ représente un groupe méthyle.

7. Composés de formule I selon l'une des revendications 1 à 6, dans laquelle R³ représente l'hydrogène, un groupe cyano, cyclopropyle, méthyle, éthyle ou 1-méthyléthyle.

8. Composés de formule I selon l'une des revendications 1 à 7, dans laquelle R⁴ représente l'hydrogène, un groupe alkyle en C1-C6, alcényle en C2-C6, alcynyle en C2-C6, allyle, arylalkyle, hétéroarylalkyle, aryloxyalkyle, hétéroaryloxyalkyle, aryle ou hétéroaryle.

9. Composés de formule I selon l'une des revendications 1 à 8, dans laquelle R⁵ représente l'hydrogène, un groupe cyclopropyle, méthyle, éthyle, isopropyle, aryle éventuellement substitué ou hétéroaryle.

10. Composés de formule I selon l'une des revendications 1 à 9, dans laquelle R⁶ représente un groupe alkyle en C1-C6, alcényle en C2-C6, alcynyle en C2-C6, arylalkyle, hétéroarylalkyle, aryloxyalkyle ou hétéroaryloxyalkyle.

11. Procédé pour la préparation des composés de formule I caractérisé par le fait que l'on fait réagir un dérivé benzylique de formule II avec une hydroxyimine de formule III 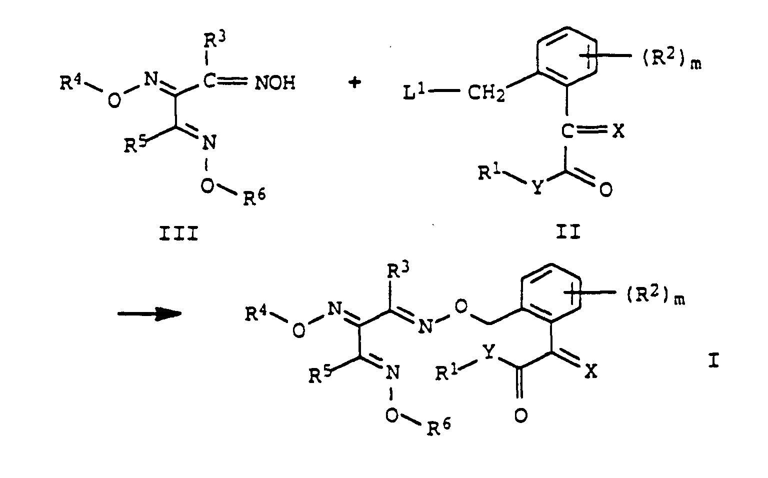 dans laquelle L¹ représente un groupe éliminable par échange nucléophile.

12. Produit pour combattre les parasites animaux ou les mycètes nuisibles, contenant des additifs usuels et une quantité efficace d'un composé de formule I selon la revendication 1.

13. Produit selon la revendication 12 pour la lutte contre les parasites animaux des classes des insectes, des arachnides ou des nématodes.

14. Procédé pour combattre les parasites animaux ou les mycètes nuisibles, caractérisé par le fait que l'on traite les parasites ou mycètes nuisibles, leur habitat ou les végétaux, aires, matériaux ou locaux qu'on veut protéger contre les parasites ou mycètes nuisibles par une quantité efficace d'au moins un composé de formule I selon la revendication 1.

15. Utilisation des composés I selon la revendication 1 pour la préparation de produits servant à combattre les parasites animaux ou mycètes nuisibles.

16. Utilisation des composés I selon la revendication 1, pour la lutte contre les parasites animaux ou les mycètes nuisibles.

17. Hydroxyimines de formule III' 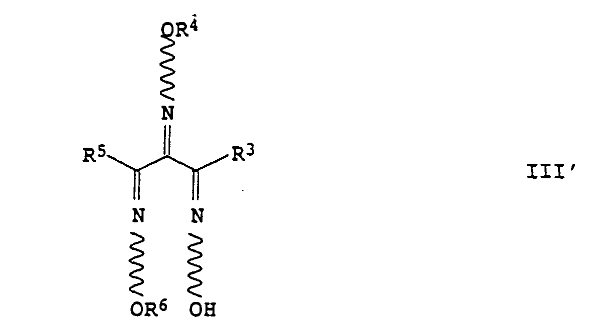 dans laquelle les symboles ont les significations indiquées dans la revendication 1, sous réserve que R⁴ et R⁶ ne peuvent représenter l'hydrogène.

18. Hydroxyimines de formule III selon la revendication 11, dans laquelle les symboles ont les significations indiquées dans la revendication 17.

19. Procédé de préparation des composés de formule I dans laquelle Y représente NH, caractérisé par le fait que l'on fait réagir un dérivé benzylique de formule II dans laquelle Y représente l'oxygène et L¹ un groupe éliminable par échange nucléophile, avec une hydroxyimine de formule III, ce qui donne un ester de formule I qu'on fait réagir avec des amines de formule R¹NH₂.
